(19) Europäisches Patentamt
European Patent Office
Office européen des brevets

(11) EP 3 906 065 B1

(12) **EUROPEAN PATENT SPECIFICATION**

(45) Date of publication and mention of the grant of the patent:
**30.04.2025 Bulletin 2025/18**

(21) Application number: **20700549.7**

(22) Date of filing: **03.01.2020**

(51) International Patent Classification (IPC):
***A61K 47/69*** *(2017.01)* ***A61K 47/60*** *(2017.01)*
***A61K 9/00*** *(2006.01)* ***A61K 31/4745*** *(2006.01)*
***A61P 35/00*** *(2006.01)*

(52) Cooperative Patent Classification (CPC):
**A61K 47/6903; A61K 31/4745; A61K 38/2013; A61K 45/06; A61K 47/60; A61K 47/62; A61P 35/00;** A61K 9/0019

(86) International application number:
**PCT/EP2020/050100**

(87) International publication number:
**WO 2020/141225 (09.07.2020 Gazette 2020/28)**

(54) **PRR AGONIST FOR USE IN THE TREATMENT OF CANCER**

PRR-AGONIST ZUR VERWENDUNG BEI DER BEHANDLUNG VON KREBS

AGONISTE DE PRR ET SON UTILISATION DANS LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.01.2019 EP 19150390**
**21.06.2019 EP 19181828**
**31.10.2019 EP 19206493**

(43) Date of publication of application:
**10.11.2021 Bulletin 2021/45**

(73) Proprietor: **Ascendis Pharma Oncology Division A/S**
**2900 Hellerup (DK)**

(72) Inventors:
- **ROSEN, David B**
  **Palo Alto, California 94304 (US)**
- **ZUNIGA, Luis**
  **Palo Alto, California 94304 (US)**
- **PUNNONEN, Juha**
  **Palo Alto, California 94304 (US)**
- **HOLTEN-ANDERSEN, Lars**
  **2900 Hellerup (DK)**
- **SPROGØE, Kennett**
  **2900 Hellerup (DK)**
- **YANG-MALTEN, Yang**
  **2900 Hellerup (DK)**
- **LESSMANN, Torben**
  **69120 Heidelberg (DE)**
- **BISEK, Nicola**
  **69120 Heidelberg (DE)**
- **WEISBROD, Samuel**
  **69120 Heidelberg (DE)**
- **STARK, Sebastian**
  **69120 Heidelberg (DE)**
- **VOIGT, Tobias**
  **69120 Heidelberg (DE)**

(74) Representative: **Büchel, Edwin**
**Patentanwälte**
**Isenbruck Bösl Hörschler PartG mbB**
**Eastsite One**
**Seckenheimer Landstraße 4**
**68163 Mannheim (DE)**

(56) References cited:
**WO-A1-2011/012715**
**WO-A1-2016/079332**
**WO-A1-2018/045058**
**WO-A2-2010/138193**
**US-A1- 2015 010 634**
**US-A1- 2016 082 123**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHUN GWON PARK ET AL: "Extended release of perioperative immunotherapy prevents tumor recurrence and eliminates metastases", SCIENCE TRANSLATIONAL MEDICINE, vol. 10, no. 433, 21 March 2018 (2018-03-21), US, pages eaar1916, XP055579366, ISSN: 1946-6234, DOI: 10.1126/scitranslmed.aar1916**

Remarks:

The complete document including Reference Table(s) and the Sequence Listing(s) can be downloaded from the EPO website

## Description

The present invention relates to a water-insoluble controlled-release pattern recognition receptor agonist ("PRRA") or its pharmaceutically acceptable salt or a pharmaceutical composition comprising such water-insoluble controlled-release PRRA or its pharmaceutical acceptable salt for use in the treatment of cancer, wherein the water-insoluble controlled-release PRRA is administered by intra-tumoral injection, wherein the water-insoluble controlled-release PRRA comprises a plurality of PRRA moieties covalently and reversibly conjugated to a carrier moiety, from which the PRRA moieties are released in aqueous buffer at pH 7.4 and 37°C with a half-life ranging from one day to three months, wherein the PRRA moieties are resiquimod moieties and wherein the carrier moiety is a hydrogel; and wherein the protein level of at least one cytokine selected from the group consisting of IL-6, CCL2 and IL-10 in plasma has a more than 10-fold lower maximum protein level within 24 hours compared to an equivalent molar dose of the corresponding free resquimod upon intra-tumoral injection; and to related aspects.

Toll-like receptors (TLRs) are a family of evolutionarily conserved pathogen recognition receptors that play a critical role in activating both innate and adaptive immunity. At least 13 different TLRs have been identified to date in mammals. TLR-1, -2, -4, -5 and -6 are located on the cell surfaces, while TLR-3, -7, -8 and -9 are located in the endosomal compartments with their ligand-binding domains facing the lumen of the vesicle.

TLRs bind pathogen and malignant cell-derived ligands called pathogen-associated molecular patterns (PAMPs) which, upon binding, trigger the NF-KB and interferon response factor (IRF) pathways resulting in the production of pro-inflammatory cytokines (e.g. IFN-$\alpha$, IFN-$\beta$, IL-1$\beta$, IL-6, TNF$\alpha$), chemokines (e.g. RANTES, MIP1$\alpha$, MIP1$\beta$), and expression of immune stimulatory molecules (e.g. CD80, CD86, CD40) by dendritic cells (DCs) and other antigen presenting cells such as macrophages. TLRs are crucial for stimulation of DC maturation, antigen uptake and presentation, immune cell recruitment, and the differentiation of $CD4^+$ T cells and control of regulatory T (Treg) cells. (Iwasaki & Medzhitov, Nat Immunol. 2004 Oct; 5(10): 987-995).

There are many known ligands for each TLR, especially as small synthetic molecules that can activate TLRs are actively being developed and widely pursued for therapeutic purposes. For example, imiquimod and resiquimod, which can activate TLR-7 and TLR-7/8, respectively, have been extensively evaluated in preclinical and clinical studies for their antiviral and anticancer effects.

Depending on the therapeutic purposes, TLR ligands have been administered via different routes, for example systemically, via oral or intravenous administration, or locally by topical cream application, by subcutaneous injection or by intratumoral injection. The efficacy, toxicity, bioavailability and other pharmacokinetic parameters vary greatly depending on the route of administration (Engel et al., Expert Rev Clin Pharmacol. 2011 Mar; 4(2): 275-289).

The lack of clinical anti-tumor efficacy and tumor-centric immunological effects following systemic administration of TLR agonists may be related to a failure of targeting the drug to the proposed site of action. As these drugs are meant to positively influence the immune response at the site of the tumor, systemic distribution may only serve to exacerbate global side effects due to systemic exposure of active drug while limiting bioavailability of the active compound in the tumor environment, thus precluding robust anti-tumor benefit (Engel et al., Expert Rev Clin Pharmacol. 2011 Mar; 4(2): 275-289).

Intratumoral injection of TLR agonists has been attempted using lipidation or different formulation methods, including suspending active drug in oily medium, mixing with biomaterials or conjugating to polymers to prolong exposure of tumor tissue to a given TLR drug. Diffusion of these soluble TLR agonists out from the tumor may lead to substantial systemic exposure. Furthermore, frequent intratumoral dosing of these compounds is required for prolonged continuous exposure of the tumor tissue to TLR drugs, making effective TLR agonist therapy impractical or unfeasible for patients.

Although there have been substantial efforts in developing new and improved TLR agonists that overcome one or more of the above-noted drawbacks, there remains a need to identify more effective TLR agonists. Furthermore, a need remains to modify TLR agonist treatment regimens such that they overcome the shortcomings of prior art compounds and their related treatment methodologies whilst also providing a favorable anti-tumoral response and reducing adverse events related to systemic exposure.

In summary, there is a need for a more efficacious treatment.

US 2016/0082123 A1 discloses a process for the preparation of a hydrogel-linked prodrug releasing a tag moiety-biologically active moiety conjugate, to a hydrogel-linked-prodrug releasing a tag moiety-biologically active moiety conjugate obtainable by such process, pharmaceutical compositions comprising said prodrug and their use as a medicament.

WO 2011/012715 A1 discloses biodegradable polyethylene glycol-based water-insoluble hydrogels comprising backbone moieties which are interconnected by hydrolytically degradable bonds exhibiting a particular degradation kinetic. It further describes conjugates of such hydrogels.

WO 2016/079332 A1 discloses a composition for use as controlled release of at least one active pharmaceutical ingredient that modulates an immunogenic response in a human or animal body, which comprises non-water-soluble carbohydrates and wherein the composition is a liquid before administration and increases in viscosity by more than 1000 centipoise after administration.

WO 2018/045058 A1 discloses drug delivery compositions and devices useful for the treatment and/or prevention of cancer and metastatic tumors, which may be implanted in the void volume of a resected tumor to prevent tumor regrowth and tumor metastasis. Park et al. (Sci. Transl. Med. 10, eaar1926 (2018)) discloses related data.

WO 2010/138193 A2 discloses compositions of synthetic nanocarriers that target sites of action in cells, such as antigen presenting cells and comprise immunomodulatory agents that dissociate from the synthetic nanocarriers in a pH sensitive manner.

US 2015/0010634 A1 discloses pharmaceutical compositions comprising hydrogel-linked prodrugs for use in the treatment, prevention and/or diagnosis of a condition of the eye and ophthalmic devices comprising said pharmaceutical composition.

It is an object of the present invention to at least partially overcome the above-described shortcomings.

This objective is achieved with a water-insoluble controlled-release pattern recognition receptor agonist ("PRRA") or its pharmaceutically acceptable salt or a pharmaceutical composition comprising such water-insoluble controlled-release PRRA or its pharmaceutically acceptable salt for use in the treatment of cancer as set out in claim 1.

It was surprisingly found that the water-insoluble controlled-release PRRA of the present invention can be used as stand-alone immunotherapeutic (i.e., as a mono-immunotherapeutic), or, in another aspect, can be used in combination with other therapeutic agents, that provide effective TLR agonist treatment regimens. Furthermore, using a water-insoluble controlled-release PRRA ensures high local PRRA concentrations for an extended period of time while keeping systemic PRRA concentrations low which minimizes side effects.

Within the present invention the terms are used having the meaning as follows.

As used herein the term "pattern recognition receptor agonist" ("PRRA") refers to a molecule that binds to and activates one or more immune cell-associated receptor that recognizes pathogen-associated molecular patterns (PAMPs) or damage-associated molecular patterns (DAMPs), leading to immune cell activation and/or pathogen- or damage-induced inflammatory responses. PRRs are typically expressed by cells of the innate immune system such as monocytes, macrophages, dendritic cells (DCs), neutrophils, and epithelial cells, as well as cells of the adaptive immune system.

As used herein the term "controlled-release pattern recognition receptor agonist" or "controlled-release PRRA" refers to any conjugate that comprises at least one pattern recognition receptor agonist and from which the at least one pattern recognition receptor agonist ("PRRA") is released with an *in vitro* release half-life under physiological conditions (aqueous buffer, pH 7.4, 37°C) ranging from one day to 3 months.

As used herein the term "maximum expression" refers to the highest plasma concentration of either IL-6, CCL2, or IL-10 measured within a 24-hour period following intra-tissue administration of either controlled-release PRRA or free PRRA to an animal (also defined above as "Plasma Cytokine Max".

As used herein the terms "cytotoxic agent" and "chemotherapeutic agent" are used synonymously and refer to compounds that are toxic to cells, which prevent cellular replication or growth, leading to cellular destruction/death. Examples of cytotoxic agents include chemotherapeutic agents and toxins, such as small molecule toxins or enzymatically active toxins of bacterial, fungal, plant or animal origin, including synthetic analogues and derivatives thereof.

As used herein the terms "immune checkpoint inhibitor" and "immune checkpoint antagonist" are used synonymously and refer to compounds that interfere with the function of, or inhibit binding of ligands that induce signaling through, cell-membrane expressed receptors that inhibit inflammatory immune cell function upon receptor activation. Such compounds may for example be biologics, such as antibodies, nanobodies, probodies, anticalins or cyclic peptides, or small molecule inhibitors.

As used herein the term "immune checkpoint agonist" refers to compounds that directly or indirectly activate cell-membrane expressed receptors that stimulate inflammatory immune cell function upon receptor activation.

As used herein the terms "multi-specific" and "multi-specific drugs" refer to compounds that simultaneously bind to two or more different antigens and can mediate antagonistic, agonistic, or specific antigen binding activity in a target-dependent manner.

As used herein the term "antibody-drug conjugate" (ADC) refers to compounds typically consisting of an antibody linked to a biologically active cytotoxic payload, radiotherapy, or other drug designed to deliver cytotoxic agents to the tumor environment. ADCs are particularly effective for reducing tumor burden without significant systemic toxicity and may act to improve the effectiveness of the immune response induced by checkpoint inhibitor antibodies.

As used herein the term "radionuclides" refers to radioactive isotopes that emit ionizing radiation leading to cellular destruction/death. Radionuclides conjugated to tumor targeting carriers are referred to as "targeted radionuclide therapeutics".

As used herein the term "DNA damage repair inhibitor" refers to a drug that targets DNA damage repair elements, such as for example CHK1, CHK2, ATM, ATR and PARP. Certain cancers are more susceptive to targeting these pathways due to existing mutations, such as BRCA1 mutated patients to PARP inhibitors due to the concept of synthetic lethality.

As used herein the term "tumor metabolism inhibitor" refers to a compound that interferes with the function of one or more enzymes expressed in the tumor environment that produce metabolic intermediates that may inhibit immune cell function.

As used herein the term "protein kinase inhibitor" refers to compounds that inhibit the activity of one or more protein kinases. Protein kinases are enzymes that phosphorylate proteins, which in turn can modulate protein function. It is understood that a protein kinase inhibitor may target more than one kinase and any classification for protein kinase inhibitors used herein refers to the main or most characterized target.

As used herein the term "chemokine receptor and chemoattractant receptor agonist" refers to compounds that activate chemokine or chemoattractant receptors, a subset of G-protein coupled receptors or G-protein coupled-like receptors that are expressed on a wide variety of cells and are primarily involved in controlling cell motility (chemotaxis or chemokinesis). These receptors may also participate in non-cell migratory processes, such as angiogenesis, cell maturation or inflammation.

As used herein the term "cytokine receptor agonist" refers to soluble proteins which control immune cell activation and proliferation. Cytokines include for example interferons, interleukins, lymphokines, and tumor necrosis factor.

As used herein the term "death receptor agonist" refers to a molecule which is capable of inducing pro-apoptotic signaling through one or more of the death receptors, such as DR4 (TRAIL-R1) or DR5 (TRAIL-R2). The death receptor agonist may be selected from the group consisting of antibodies, death ligands, cytokines, death receptor agonist expressing vectors, peptides, small molecule agonists, cells (such as for example stem cells) expressing the death receptor agonist, and drugs inducing the expression of death ligands.

As used herein the term "antigen-presenting cell" or "APC" refers to a cell, such as a macrophage, a B cell, or a dendritic cell, that presents processed antigenic peptides via MHC class II molecules to the T cell receptor on CD4 T cells. APCs can be identified by a person skilled in the art by using phenotypic techniques such as flow cytometry. Phenotypic markers used to identify APCs vary by species and by tissue but may include myeloid or dendritic cell surface markers (e.g. CD11b, CD11c, CD14, CD16, CD33, CD34, Ly6C, Ly6G, GR-1, F4/80) or B cell surface markers (e.g. CD19, CD20, B220).

As used herein the term "MHCII" refers to a class of major histocompatibility complex (MHC) molecules normally found only on antigen-presenting cells such as myeloid cells, dendritic cells, and B cells. MHCII presents processed antigenic peptides to the T cell receptor on CD4 T cells. MHCII expression can be measured by a person skilled in the art using protein expression profiling techniques such as flow cytometry. Changes in MHCII expression can be determined by analyzing changes in the median fluorescence intensity signal of MHCII, or the percentage of cells positive for MHCII, in a specific cell subset of interest.

As used herein the term "T cells" refers to a type of immune cell that plays a central role in the adaptive immune response. T cells are distinguished from other immune cells by the presence of either an $\alpha\beta$ or $\gamma\delta$ T cell receptor (TCR) on their cell surface. T cells also express CD3 - a protein complex critical for TCR signaling. $\alpha\beta$ T cells can be divided into either CD4, CD8, or CD4/CD8 double negative subsets. Due to the high surface density of CD4 and CD8 on $CD4^+$ and $CD8^+$ T cells, CD4 and CD8 alone can often be used to identify $CD4^+$ and $CD8^+$ T cells respectively. Following activation via TCR recognition of cognate antigen presented by MHC molecules, T cells can mature and divide to generate effector or memory T cells. Memory T cells are a subset of T cells that have previously encountered and responded to their cognate antigen. Such T cells can recognize pathogenic antigens, such as antigens derived from bacteria or viruses, as well as cancer-associated antigens. T cells can be identified by a person skilled in the art by using phenotypic techniques such as flow cytometry. Phenotypic markers used to identify T cells are generally conserved in mammals and include CD3, TCR$\alpha$, TCR$\beta$, TCR$\delta$, CD4, and CD8. Phenotypic markers used to identify memory T cells can vary by species and by tissue, but may include cell surface markers such as CD45RO, LY6C, CD44, and CD95.

As used herein the term "intra-tissue administration" refers to a type of administration, for example local injection, of a drug into a tissue of interest such as intratumoral, intra-muscular, subdermal or subcutaneous injections or injection into or adjacent to a normal or diseased tissue or organ.

As used herein, the term "intra-tumoral administration" refers to a mode of administration, in which the drug is administered directly into tumor tissue. The term "intra-tumoral administration" may in certain embodiments also refer to administration pre- or post-resection into or onto the tumor bed. When tumor boundary is not well defined, it is also understood that intra-tumoral administration includes administration to tissue adjacent to the tumor cells ("peri-tumoral administration"). Exemplary tumors for intra-tumoral administration are solid tumors and lymphomas, which are disclosed in more detail elsewhere herein. Administration may occur via injection, and includes intramuscular, and subcutaneous injection.

As used herein the term "baseline tissue" refers to a tissue sample taken from, or adjacent to, the area to be treated prior to treatment. For example, a biopsy of tissue to be treated can be taken immediately prior to treatment. It is understood that it may not always be possible to take a reference sample from the respective area prior to treatment, so the term "baseline tissue" may also refer to a non-treated control tissue that may be taken from a comparable location from the same animal or may be taken from a comparable location of a different animal of the same species. It is understood that the term "animal" also covers human and in certain embodiments means mouse, rat, non-human primate or human.

As used herein the term "anti-tumor activity" means the ability to inhibit a tumor from growing larger, i.e. tumor growth inhibition or tumor stasis, or the ability to cause a reduction in the size of a tumor, i.e. tumor regression. In certain embodiments the term also refers to the ability to reduce the speed of tumor growth by at least 20%, such as by at least

25%, by at least 30%, by at least 35%, by at least 40%, by at least 45%, or by at least 50%. Anti-tumor activity may be determined by comparing the mean relative tumor volumes between control and treatment conditions. Relative volumes of individual tumors (individual RTVs) for day "x" may be calculated by dividing the absolute individual tumor volume on day "x" ($T_x$) following treatment initiation by the absolute individual tumor volume of the same tumor on the day treatment started ($T_0$) multiplied by 100:

$$RTV_x\ [\%] = \frac{T_x}{T_0} \times 100$$

Anti-tumor activity may in certain embodiments be observed between 7 to 21 days following treatment initiation. In certain embodiments anti-tumor activity is observed 7 days following treatment initiation. In certain embodiments anti-tumor activity is observed 8 days following treatment initiation. In certain embodiments anti-tumor activity is observed 9 days following treatment initiation. In certain embodiments anti-tumor activity is observed 10 days following treatment initiation. In certain embodiments anti-tumor activity is observed 11 days following treatment initiation. In certain embodiments anti-tumor activity is observed 12 days following treatment initiation. In certain embodiments anti-tumor activity is observed 13 days following treatment initiation. In certain embodiments anti-tumor activity is observed 14 days following treatment initiation. In certain embodiments anti-tumor activity is observed 15 days following treatment initiation. In certain embodiments anti-tumor activity is observed 16 days following treatment initiation. In certain embodiments anti-tumor activity is observed 17 days following treatment initiation. In certain embodiments anti-tumor activity is observed 18 days following treatment initiation. In certain embodiments anti-tumor activity is observed 19 days following treatment initiation. In certain embodiments anti-tumor activity is observed 20 days following treatment initiation. In certain embodiments anti-tumor activity is observed 21 days following treatment initiation. It is understood that these time points indicate the earliest time point at which anti-tumor activity is observed.

Tumor size, reported in $mm^3$, can be measured physically by measuring the length (*L*) measured in mm and width (*W*) measured in mm of the tumor, which may include injected and non-injected tumors. Tumor volume can be determined by methods such as ultrasound imaging, magnetic resonance imaging, computed tomography scanning, or approximated by using the equation $V = \frac{1}{2} \times (L \times W^2)$, with *V* being the tumor volume. Tumor burden, i.e. the total number of cancer cells in an individuum, can also be measured in the case of an experimental tumor model that expresses a reporter, such as luciferase enzyme or a fluorescent protein or another measurable protein or enzyme, by measuring the reporter element, i.e. luminescence or fluorescence, or the expressed reporter protein or enzyme product as a measure of the total number of tumor cells present and total tumor size. The latter reporter models can be useful for tumors that are not readily measurable on the surface of the animals (i.e. orthotopic tumors). It is understood that in general the term "animal" also covers human and in certain embodiments means mouse, rat, non-human primate and human.

As used herein the term "local inflammation" refers to an inflammation that is restricted to an area near the site of administration of the controlled-release PRRA. The specific size of the area of inflammation will depend on the amount of agonist administered, the diffusion rate within the tissue, the time at which the signal is measured following injection, the rate of drug uptake by neighboring cells and the frequency of pattern recognition receptor responsive cells at and around the treated site, but would typically be detectable within a distance of 2 times the radius (r) from the injection site in any direction, wherein *r* is the distance in centimeters (cm) calculated from the volume (V) of water-insoluble controlled-release PRRA injected in cubic centimeters ($cm^3$) following the spheroid equation $V = \left(\frac{4}{3}\right) \times \pi r^3$. For example, if 0.5 $cm^3$ controlled-release PRRA is injected into a given tissue, a sample of tissue weighing at least 0.025g taken within 0.98 cm in any direction of the injection site displays a measurable inflammatory signal. Within a volume of 2 times *r* tissue samples are to be taken for determining the presence of a specific set of inflammation markers. However, this does not mean that said inflammation markers outside a volume of 2 times *r* may not be upregulated by at least a factor of 1.5. In general, inflammation intensity decreases with increasing distance from the administration site. However, the person skilled in the art understands that providing an outer boundary of such localized inflammation is not feasible, because the extend of inflammation depends on various factors, such as for example tumor type. In any way, the person skilled in the art will easily be able to distinguish between local and systemic inflammation.

As used herein, the term "water-insoluble" refers to a compound of which less than 1 g can be dissolved in one liter of water at 20°C to form a homogeneous solution. Accordingly, the term "water-soluble" refers to a compound of which 1 g or more can be dissolved in one liter of water at 20°C to form a homogeneous solution.

As used herein, the term "drug" refers to a substance used in the treatment, cure, prevention or diagnosis of a disease or used to otherwise enhance physical or mental well-being of a patient. If a drug is conjugated to another moiety, the moiety of the resulting product that originated from the drug is referred to as "drug moiety".

Any reference to a biologic drug herein, i.e. to a drug manufactured in, extracted from, or semisynthesized from biological sources such as a protein drug, also covers biosimilar versions of said drug.

As used herein the term "prodrug" refers to a drug moiety reversibly and covalently connected to a specialized protective group through a reversible prodrug linker moiety which is a linker moiety comprising a reversible linkage with the drug moiety and wherein the specialized protective group alters or eliminates undesirable properties in the parent molecule. This also includes the enhancement of desirable properties in the drug and the suppression of undesirable properties. The specialized non-toxic protective group may also be referred to as "carrier". A prodrug releases the reversibly and covalently bound drug moiety in the form of its corresponding drug. In other words, a prodrug is a conjugate comprising a drug moiety, which is covalently and reversibly conjugated to a carrier moiety via a reversible linker moiety, which covalent and reversible conjugation of the carrier to the reversible linker moiety is either directly or through a spacer. The reversible linker may also be referred to as "reversible prodrug linker". Such conjugate may release the formerly conjugated drug moiety in the form of a free drug, in which case the reversible linker or reversible prodrug linker is a traceless linker.

As used herein, the term "free form" of a drug means the drug in its unmodified, pharmacologically active form.

As used herein, the term "a $\pi$-electron-pair-donating heteroaromatic N-comprising moiety" refers to the moiety which after cleavage of the linkage between -D and -$L^1$- results in a drug D-H and wherein the drug moiety -D and analogously the corresponding D-H comprises at least one, such as one, two, three, four, five, six, seven, eight, nine or ten heteroaromatic nitrogen atoms that donate a $\pi$-electron pair to the aromatic $\pi$-system. Examples of chemical structures comprising such heteroaromatic nitrogens that donate a $\pi$-electron pair to the aromatic $\pi$-system include, but are not limited to, pyrrole, pyrazole, imidazole, isoindazole, indole, indazole, purine, tetrazole, triazole and carbazole. For example, in the imidazole ring below the heteroaromatic nitrogen which donates a $\pi$-electron pair to the aromatic $\pi$-system is marked with "#":

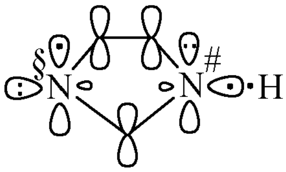

The $\pi$-electron-pair-donating heteroaromatic nitrogen atoms do not comprise heteroaromatic nitrogen atoms which only donate one electron (i.e. not a pair of $\pi$-electrons) to the aromatic $\pi$-system, such as for example the nitrogen that is marked with "§" in the abovementioned imidazole ring structure. The drug D-H may exist in one or more tautomeric forms, such as with one hydrogen atom moving between at least two heteroaromatic nitrogen atoms. In all such cases, the linker moiety is covalently and reversibly attached at a heteroaromatic nitrogen that donates a $\pi$-electron pair to the aromatic $\pi$-system.

As used herein the term "spacer" refers to a moiety that connects at least two other moieties with each other.

As used herein, the terms "reversible", "reversibly", "degradable" or "degradably" with regard to the attachment of a first moiety to a second moiety mean that the linkage that connects said first and second moiety is cleavable under physiological conditions, which physiological conditions are aqueous buffer at pH 7.4 and 37°C, with a half-life ranging from one day to three month, such as from two days to two months, such as from three days to one month. Such cleavage is in certain embodiments non-enzymatically. Accordingly, the term "stable" with regard to the attachment of a first moiety to a second moiety means that the linkage that connects said first and second moiety exhibits a half-life of more than three months under physiological conditions.

As used herein, the term "reagent" means a chemical compound, which comprises at least one functional group for reaction with the functional group of another chemical compound or drug. It is understood that a drug comprising a functional group is also a reagent.

As used herein, the term "moiety" means a part of a molecule, which lacks one or more atom(s) compared to the corresponding reagent. If, for example, a reagent of the formula "H-X-H" reacts with another reagent and becomes part of the reaction product, the corresponding moiety of the reaction product has the structure "H-X-" or "-X-" , whereas each "-" indicates attachment to another moiety. Accordingly, a drug moiety, such as an antibiotic moiety, is released from a reversible linkage as a drug, such as an antibiotic drug.

It is understood that if the chemical structure of a group of atoms is provided and if this group of atoms is attached to two moieties or is interrupting a moiety, said chemical structure can be attached to the two moieties in either orientation, unless explicitly stated otherwise. For example, a moiety "-C(O)N($R^1$)-" can be attached to two moieties or interrupting a moiety either as "-C(O)N($R^1$)-" or as "-N($R^1$)C(O)-". Similarly, a moiety

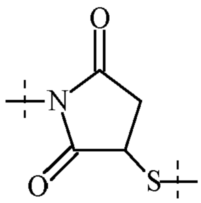

can be attached to two moieties or can interrupt a moiety either as

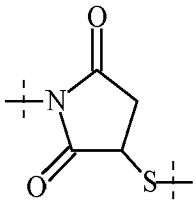

or as

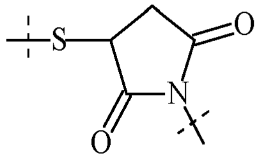

.

The term "substituted" as used herein means that one or more -H atom(s) of a molecule or moiety are replaced by a different atom or a group of atoms, which are referred to as "substituent".

As used herein, the term "substituent" in certain embodiments refers to a moiety selected from the group consisting of halogen, -CN, -COOR$^{x1}$, -OR$^{x1}$, -C(O)R$^{x1}$, -C(O)N(R$^{x1}$R$^{x1a}$), -S(O)$_2$N(R$^{x1}$R$^{x1a}$), -S(O)N(R$^{x1}$R$^{x1a}$), -S(O)$_2$R$^{x1}$, -S(O)R$^{x1}$, -N(R$^{x1}$)S(O)$_2$N(R$^{x1a}$R$^{x1b}$), -SR$^{x1}$, -N(R$^{x1}$R$^{x1a}$), -NO$_2$, -OC(O)R$^{x1}$, -N(R$^{x1}$)C(O)R$^{x1a}$, -N(R$^{x1}$)S(O)$_2$R$^{x1a}$, -N(R$^{x1}$)S(O)R$^{x1a}$, -N(R$^{x1}$)C(O)OR$^{x1a}$, -N(R$^{x1}$)C(O)N(R$^{x1a}$R$^{x1b}$), -OC(O)N(R$^{x1}$R$^{x1a}$), -T$^0$, C$_{1-50}$ alkyl, C$_{2-50}$ alkenyl, and C$_{2-50}$ alkynyl; wherein -T$^0$, C$_{1-50}$ alkyl, C$_{2-50}$ alkenyl, and C$_{2-50}$ alkynyl are optionally substituted with one or more -R$^{x2}$, which are the same or different and wherein C$_{1-50}$ alkyl, C$_{2-50}$ alkenyl, and C$_{2-50}$ alkynyl are optionally interrupted by one or more groups selected from the group consisting of -T$^0$-, -C(O)O-, -O-, -C(O)-, -C(O)N(R$^{x3}$)-, -S(O)$_2$N(R$^{x3}$)-, -S(O)N(R$^{x3}$)-, -S(O)$_2$-, -S(O)-, -N(R$^{x3}$)S(O)$_2$N(R$^{x3a}$)-, -S-, -N(R$^{x3}$)-, -OC(OR$^{x3}$)(R$^{x3a}$)-, -N(R$^{x3}$)C(O)N(R$^{x3a}$)-, and -OC(O)N(R$^{x3}$)-;

-R$^{x1}$, -R$^{x1a}$, -R$^{x1b}$ are independently of each other selected from the group consisting of -H, -T$^0$, C$_{1-50}$ alkyl, C$_{2-50}$ alkenyl, and C$_{2-50}$ alkynyl; wherein -T$^0$, C$_{1-50}$ alkyl, C$_{2-50}$ alkenyl, and C$_{2-50}$ alkynyl are optionally substituted with one or more -R$^{x2}$, which are the same or different and wherein C$_{1-50}$ alkyl, C$_{2-50}$ alkenyl, and C$_{2-50}$ alkynyl are optionally interrupted by one or more groups selected from the group consisting of -T$^0$-, -C(O)O-, -O-, -C(O)-, -C(O)N(R$^{x3}$)-, -S(O)$_2$N(R$^{x3}$)-, -S(O)N(R$^{x3}$)-; -S(O)$_2$-, -S(O)-, -N(R$^{x3}$)S(O)$_2$N(R$^{x3a}$)-, -S-, -N(R$^{x3}$)-, -OC(OR$^{x3}$)(R$^{x3a}$)-, -N(R$^{x3}$)C(O)N(R$^{x3a}$)-, and -OC(O)N(R$^{x3}$)-;

each T$^0$ is independently selected from the group consisting of phenyl, naphthyl, indenyl, indanyl, tetralinyl, C$_{3-10}$ cycloalkyl, 3- to 10-membered heterocyclyl, and 8- to 11-membered heterobicyclyl; wherein each T$^0$ is independently optionally substituted with one or more -R$^{x2}$, which are the same or different;

each -R$^{x2}$ is independently selected from the group consisting of halogen, -CN, oxo (=O), -COOR$^{x4}$, -OR$^{x4}$, -C(O)R$^{x4}$, -C(O)N(R$^{x4}$R$^{x4a}$), -S(O)$_2$N(R$^{x4}$R$^{x4a}$), -S(O)N(R$^{x4}$R$^{x4a}$), -S(O)$_2$R$^{x4}$, -S(O)R$^{x4}$, -N(R$^{x4}$)S(O)$_2$N(R$^{x4a}$R$^{x4b}$), -SR$^{x4}$, -N(R$^{x4}$R$^{x4a}$), -NO$_2$, -OC(O)R$^{x4}$, -N(R$^{x4}$)C(O)R$^{x4a}$, -N(R$^{x4}$)S(O)$_2$R$^{x4a}$, -N(R$^{x4}$)S(O)R$^{x4a}$, -N(R$^{x4}$)C(O)oR$^{x4a}$, -N(R$^{x4}$)C(O)N(R$^{x4a}$R$^{x4b}$), -OC(0)N(R$^{x4}$R$^{x4a}$), and C$_{1-6}$ alkyl; wherein C$_{1-6}$ alkyl is optionally substituted with one or more halogen, which are the same or different;

each -R$^{x3}$, -R$^{x3a}$, -R$^{x4}$, -R$^{x4a}$, -R$^{x4b}$ is independently selected from the group consisting of -H and C$_{1-6}$ alkyl; wherein C$_{1-6}$ alkyl is optionally substituted with one or more halogen, which are the same or different.

In certain embodiments a maximum of 6 -H atoms of an optionally substituted molecule are independently replaced by a substituent, e.g. 5 -H atoms are independently replaced by a substituent, 4 -H atoms are independently replaced by a substituent, 3 -H atoms are independently replaced by a substituent, 2 -H atoms are independently replaced by a substituent, or 1 -H atom is replaced by a substituent.

As used herein, the term "hydrogel" means a hydrophilic or amphiphilic polymeric network composed of homopolymers or copolymers, which is insoluble due to the presence of hydrophobic interactions, hydrogen bonds, ionic interactions and/or covalent chemical crosslinks. The crosslinks provide the network structure and physical integrity. In certain embodiments the hydrogel is insoluble due to the presence of covalent chemical crosslinks.

As used herein the term "crosslinker" refers to a moiety that is a connection between different elements of a hydrogel, such as between two or more backbone moieties or between two or more hyaluronic acid strands.

As used herein the term "about" in combination with a numerical value is used to indicate a range ranging from and including the numerical value plus and minus no more than 25% of said numerical value, such as no more than plus and minus 20% of said numerical value or such as no more than plus and minus 10% of said numerical value. For example, the phrase "about 200" is used to mean a range ranging from and including 200 +/- 25%, i.e. ranging from and including 150 to 250; such as 200 +/- 20%, i.e. ranging from and including 160 to 240; such as ranging from and including 200 +/-10%, i.e. ranging from and including 180 to 220. It is understood that a percentage given as "about 50%" does not mean "50% +/- 25%", i.e. ranging from and including 25 to 75%, but "about 50%" means ranging from and including 37.5 to 62.5%, i.e. plus and minus 25% of the numerical value which is 50.

As used herein, the term "polymer" means a molecule comprising repeating structural units, i.e. the monomers, connected by chemical bonds in a linear, circular, branched, crosslinked or dendrimeric way or a combination thereof, which may be of synthetic or biological origin or a combination of both. The monomers may be identical, in which case the polymer is a homopolymer, or may be different, in which case the polymer is a heteropolymer. A heteropolymer may also be referred to as a "copolymer" and includes, for example, alternating copolymers in which monomers of different types alternate, periodic copolymers, in which monomers of different types are arranged in a repeating sequence; statistical copolymers, in which monomers of different types are arranged randomly; block copolymers, in which blocks of different homopolymers consisting of only one type of monomers are linked by a covalent bond; and gradient copolymers, in which the composition of different monomers changes gradually along a polymer chain. It is understood that a polymer may also comprise one or more other moieties, such as, for example, one or more functional groups. The term "polymer" also relates to a peptide or protein, even though the side chains of individual amino acid residues may be different. It is understood that for covalently crosslinked polymers, such as hydrogels, no meaningful molecular weight ranges can be provided.

As used herein, the term "polymeric" refers to a reagent or a moiety comprising one or more polymers or polymer moieties. A polymeric reagent or moiety may optionally also comprise one or more other moieties, which in certain embodiments are selected from the group consisting of:

- $C_{1-50}$ alkyl, $C_{2-50}$ alkenyl, $C_{2-50}$ alkynyl, $C_{3-10}$ cycloalkyl, 3- to 10-membered heterocyclyl, 8- to 11-membered heterobicyclyl, phenyl, naphthyl, indenyl, indanyl, and tetralinyl;
- branching points, such as -CR<, >C< or -N<; and
- linkages selected from the group comprising

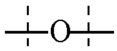, 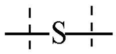, 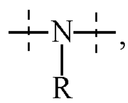, 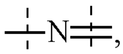, 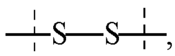, 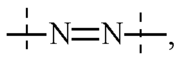,

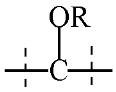, 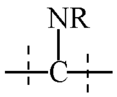, 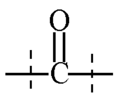, 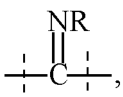, 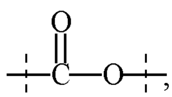, 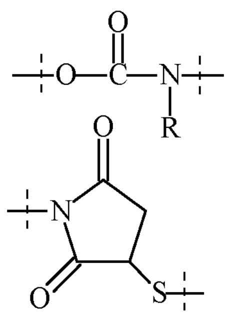,

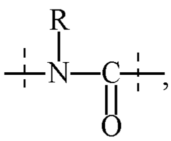, 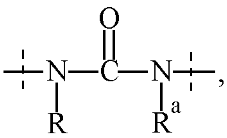, 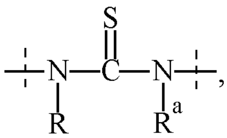, and ,

wherein

dashed lines indicate attachment to the remainder of the moiety or reagent, and
-R and -$R^a$ are independently of each other selected from the group consisting of -H, methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl; and
which moieties and linkages are optionally further substituted.

In certain embodiments a polymeric reagent or moiety may optionally also comprise one or more other moieties, which in certain embodiments are selected from the group consisting of:

- $C_{1-50}$ alkyl, $C_{2-50}$ alkenyl, $C_{2-50}$ alkynyl, $C_{3-10}$ cycloalkyl, 3- to 10-membered heterocyclyl, 8- to 11-membered heterobicyclyl, phenyl, naphthyl, indenyl, indanyl, and tetralinyl; and
- linkages selected from the group comprising

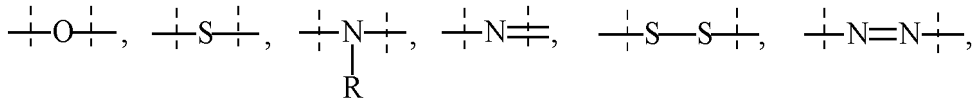

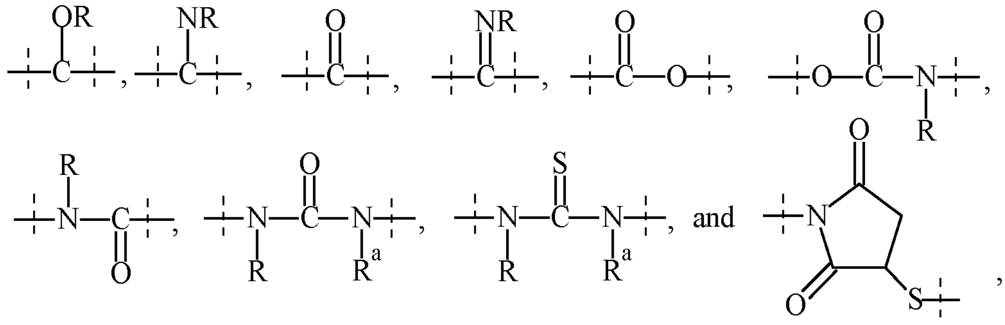

and

wherein

dashed lines indicate attachment to the remainder of the moiety or reagent, and
-R and -$R^a$ are independently of each other selected from the group consisting of -H, methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl; and
which moieties and linkages are optionally further substituted.

The person skilled in the art understands that the polymerization products obtained from a polymerization reaction do not all have the same molecular weight, but rather exhibit a molecular weight distribution. Consequently, the molecular weight ranges, molecular weights, ranges of numbers of monomers in a polymer and numbers of monomers in a polymer as used herein, refer to the number average molecular weight and number average of monomers, i.e. to the arithmetic mean of the molecular weight of the polymer or polymeric moiety and the arithmetic mean of the number of monomers of the polymer or polymeric moiety.

Accordingly, in a polymeric moiety comprising "x" monomer units any integer given for "x" therefore corresponds to the arithmetic mean number of monomers. Any range of integers given for "x" provides the range of integers in which the arithmetic mean numbers of monomers lies. An integer for "x" given as "about x" means that the arithmetic mean numbers of monomers lies in a range of integers of x +/- 25%, such as x +/- 20% or such as x +/- 10%.

As used herein, the term "number average molecular weight" means the ordinary arithmetic mean of the molecular weights of the individual polymers.

As used herein, the term "PEG-based" in relation to a moiety or reagent means that said moiety or reagent comprises PEG. Such PEG-based moiety or reagent comprises at least 10% (w/w) PEG, such as at least 20% (w/w) PEG, such as at least 30% (w/w) PEG, such as at least 40% (w/w) PEG, such as at least 50% (w/w), such as at least 60 (w/w) PEG, such as at least 70% (w/w) PEG, such as at least 80% (w/w) PEG, such as at least 90% (w/w) PEG, or such as at least 95% (w/w) PEG. The remaining weight percentage of the PEG-based moiety or reagent may be other moieties, such as those selected from the group consisting of:

- $C_{1-50}$ alkyl, $C_{2-50}$ alkenyl, $C_{2-50}$ alkynyl, $C_{3-10}$ cycloalkyl, 3- to 10-membered heterocyclyl, 8- to 11-membered heterobicyclyl, phenyl, naphthyl, indenyl, indanyl, and tetralinyl; and
- linkages selected from the group consisting of

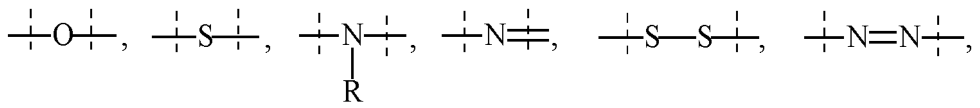

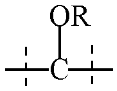, 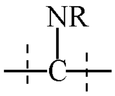, 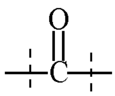, 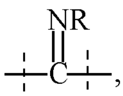, 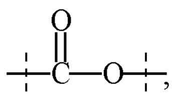, 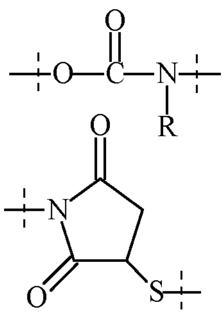,

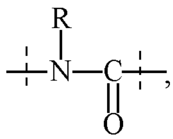, 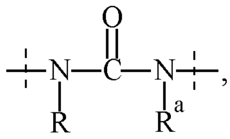, 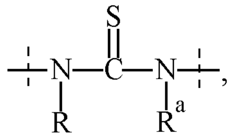, and ,

wherein

dashed lines indicate attachment to the remainder of the moiety or reagent, and
-R and -R$^a$ are independently of each other selected from the group consisting of -H, and $C_{1-6}$ alkyl; and which moieties and linkages are optionally further substituted.

The terms "poly(alkylene glycol)-based", "poly(propylene glycol)-based" and "hyaluronic acid-based" are used accordingly.

The term "interrupted" means that a moiety is inserted between two carbon atoms or - if the insertion is at one of the moiety's ends - between a carbon or heteroatom and a hydrogen atom.

As used herein, the term "$C_{1-4}$ alkyl" alone or in combination means a straight-chain or branched alkyl moiety having 1 to 4 carbon atoms. If present at the end of a molecule, examples of straight-chain or branched $C_{1-4}$ alkyl are methyl, ethyl, n-propyl, isopropyl, n-butyl, isobutyl, sec-butyl and tert-butyl. When two moieties of a molecule are linked by the $C_{1-4}$ alkyl, then examples for such $C_{1-4}$ alkyl groups are $-CH_2-$, $-CH_2-CH_2-$, $-CH(CH_3)-$, $-CH_2-CH_2-CH_2-$, $-CH(C_2H_5)-$, $-C(CH_3)_2-$. Each hydrogen of a $C_{1-4}$ alkyl carbon may optionally be replaced by a substituent as defined above. Optionally, a $C_{1-4}$ alkyl may be interrupted by one or more moieties as defined below.

As used herein, the term "$C_{1-6}$ alkyl" alone or in combination means a straight-chain or branched alkyl moiety having 1 to 6 carbon atoms. If present at the end of a molecule, examples of straight-chain and branched $C_{1-6}$ alkyl groups are methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl.

When two moieties of a molecule are linked by the $C_{1-6}$ alkyl group, then examples for such $C_{1-6}$ alkyl groups are $-CH_2-$, $-CH_2-CH_2-$, $-CH(CH_3)-$, $-CH_2-CH_2-CH_2-$, $-CH(C_2H_5)-$ and $-C(CH_3)_2-$. Each hydrogen atom of a $C_{1-6}$ carbon may optionally be replaced by a substituent as defined above. Optionally, a $C_{1-6}$ alkyl may be interrupted by one or more moieties as defined below.

Accordingly, "$C_{1-10}$ alkyl", "$C_{1-20}$ alkyl" or "$C_{1-50}$ alkyl" means an alkyl chain having 1 to 10, 1 to 20 or 1 to 50 carbon atoms, respectively, wherein each hydrogen atom of the $C_{1-10}$, $C_{1-20}$ or $C_{1-50}$ carbon may optionally be replaced by a substituent as defined above. Optionally, a $C_{1-10}$ or $C_{1-50}$ alkyl may be interrupted by one or more moieties as defined below.

As used herein, the term "$C_{2-6}$ alkenyl" alone or in combination means a straight-chain or branched hydrocarbon moiety comprising at least one carbon-carbon double bond having 2 to 6 carbon atoms. If present at the end of a molecule, examples are $-CH=CH_2$, $-CH=CH-CH_3$, $-CH_2-CH=CH_2$, $-CH=CHCH_2-CH_3$ and $-CH=CH-CH=CH_2$. When two moieties of a molecule are linked by the $C_{2-6}$ alkenyl group, then an example for such $C_{2-6}$ alkenyl is $-CH=CH-$. Each hydrogen atom of a $C_{2-6}$ alkenyl moiety may optionally be replaced by a substituent as defined above. Optionally, a $C_{2-6}$ alkenyl may be interrupted by one or more moieties as defined below.

Accordingly, the terms "$C_{2-10}$ alkenyl", "$C_{2-20}$ alkenyl" or "$C_{2-50}$ alkenyl" alone or in combination mean a straight-chain or branched hydrocarbon moiety comprising at least one carbon-carbon double bond having 2 to 10, 2 to 20 or 2 to 50 carbon atoms, respectively. Each hydrogen atom of a $C_{2-10}$ alkenyl, $C_{2-20}$ alkenyl or $C_{2-50}$ alkenyl group may optionally be replaced by a substituent as defined above. Optionally, a $C_{2-10}$ alkenyl, $C_{2-20}$ alkenyl or $C_{2-50}$ alkenyl may be interrupted by one or more moieties as defined below.

As used herein, the term "$C_{2-6}$ alkynyl" alone or in combination means a straight-chain or branched hydrocarbon moiety comprising at least one carbon-carbon triple bond having 2 to 6 carbon atoms. If present at the end of a molecule, examples are $-C\equiv CH$, $-CH_2-C\equiv CH$, $CH_2-CH_2-C\equiv CH$ and $CH_2-C\equiv C-CH_3$. When two moieties of a molecule are linked by the alkynyl group, then an example is $-C\equiv C-$. Each hydrogen atom of a $C_{2-6}$ alkynyl group may optionally be replaced by a substituent as defined above. Optionally, one or more double bond(s) may occur. Optionally, a $C_{2-6}$ alkynyl may be interrupted by one or more moieties as defined below.

Accordingly, as used herein, the term "$C_{2-10}$ alkynyl", "$C_{2-20}$ alkynyl" and "$C_{2-50}$ alkynyl" alone or in combination means a straight-chain or branched hydrocarbon moiety comprising at least one carbon-carbon triple bond having 2 to 10,

2 to 20 or 2 to 50 carbon atoms, respectively. Each hydrogen atom of a $C_{2-10}$ alkynyl, $C_{2-20}$ alkynyl or $C_{2-50}$ alkynyl group may optionally be replaced by a substituent as defined above. Optionally, one or more double bond(s) may occur. Optionally, a $C_{2-10}$ alkynyl, $C_{2-20}$ alkynyl or $C_{2-50}$ alkynyl may be interrupted by one or more moieties as defined below.

As mentioned above, a $C_{1-4}$ alkyl, $C_{1-6}$ alkyl, $C_{1-10}$ alkyl, $C_{1-20}$ alkyl, $C_{1-50}$ alkyl, $C_{2-6}$ alkenyl, $C_{2-10}$ alkenyl, $C_{2-20}$ alkenyl, $C_{2-50}$ alkenyl, $C_{2-6}$ alkynyl, $C_{2-10}$ alkynyl, $C_{2-20}$ alkenyl or $C_{2-50}$ alkynyl may optionally be interrupted by one or more moieties which may be selected from the group consisting of

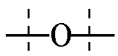, 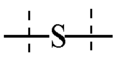, 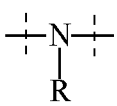, 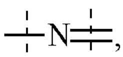, 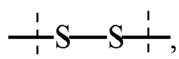, 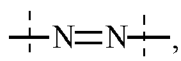,

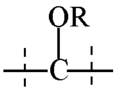, 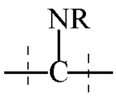, 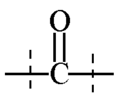, 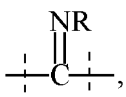, 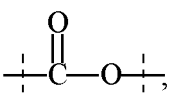, 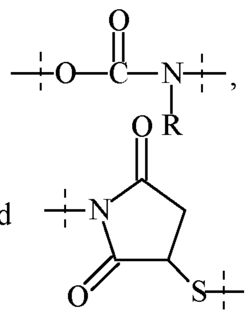,

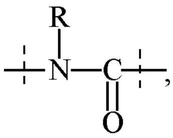, 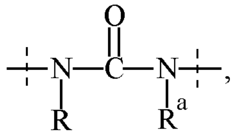, 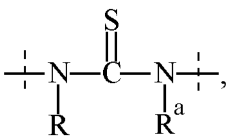, and ,

wherein

dashed lines indicate attachment to the remainder of the moiety or reagent; and
-R and -$R^a$ are independently of each other selected from the group consisting of -H and $C_{1-6}$ alkyl.

As used herein, the term "$C_{3-10}$ cycloalkyl" means a cyclic alkyl chain having 3 to 10 carbon atoms, which may be saturated or unsaturated, e.g. cyclopropyl, cyclobutyl, cyclopentyl, cyclohexyl, cyclohexenyl, cycloheptyl, cyclooctyl, cyclononyl or cyclodecyl. Each hydrogen atom of a $C_{3-10}$ cycloalkyl carbon may be replaced by a substituent as defined above. The term "$C_{3-10}$ cycloalkyl" also includes bridged bicycles like norbornane or norbornene.

The term "8- to 30-membered carbopolycyclyl" or "8- to 30-membered carbopolycycle" means a cyclic moiety of two or more rings with 8 to 30 ring atoms, where two neighboring rings share at least one ring atom and that may contain up to the maximum number of double bonds (aromatic or non-aromatic ring which is fully, partially or un-saturated). In one embodiment a 8- to 30-membered carbopolycyclyl means a cyclic moiety of two, three, four or five rings. In another embodiment a 8- to 30-membered carbopolycyclyl means a cyclic moiety of two, three or four rings.

As used herein, the term "3- to 10-membered heterocyclyl" or "3- to 10-membered heterocycle" means a ring with 3, 4, 5, 6, 7, 8, 9 or 10 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -$S(O)_2$-), oxygen and nitrogen (including =N(O)-) and wherein the ring is linked to the rest of the molecule via a carbon or nitrogen atom. Examples for 3- to 10-membered heterocycles include but are not limited to aziridine, oxirane, thiirane, azirine, oxirene, thiirene, azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine and homopiperazine. Each hydrogen atom of a 3- to 10-membered heterocyclyl or 3- to 10-membered heterocyclic group may be replaced by a substituent.

As used herein, the term "8- to 11-membered heterobicyclyl" or "8- to 11-membered heterobicycle" means a heterocyclic moiety of two rings with 8 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -$S(O)_2$-), oxygen and nitrogen (including =N(O)-) and wherein the ring is linked to the rest of the molecule via a carbon or nitrogen atom. Examples for an 8- to 11-membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline,

decahydroisoquinoline, tetrahydroisoquinoline, dihydroisoquinoline, benzazepine, purine and pteridine. The term 8- to 11-membered heterobicycle also includes spiro structures of two rings like 1,4-dioxa-8-azaspiro[4.5]decane or bridged heterocycles like 8-aza-bicyclo[3.2.1]octane. Each hydrogen atom of an 8- to 11-membered heterobicyclyl or 8- to 11-membered heterobicycle carbon may be replaced by a substituent.

Similary, the term "8- to 30-membered heteropolycyclyl" or "8- to 30-membered heteropolycycle" means a heterocyclic moiety of more than two rings with 8 to 30 ring atoms, such as of three, four or five rings, where two neighboring rings share at least one ring atom and that may contain up to the maximum number of double bonds (aromatic or non-aromatic ring which is fully, partially or unsaturated), wherein at least one ring atom up to 10 ring atoms are replaced by a heteroatom selected from the group of sulfur (including -S(O)-, $-S(O)_2-$), oxygen and nitrogen (including =N(O)-) and wherein the ring is linked to the rest of a molecule via a carbon or nitrogen atom.

It is understood that the phrase "the pair $R^x/R^y$ is joined together with the atom to which they are attached to form a $C_{3-10}$ cycloalkyl or a 3- to 10-membered heterocyclyl" in relation with a moiety of the structure

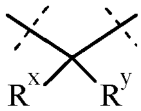

means that $R^x$ and $R^y$ form the following structure:

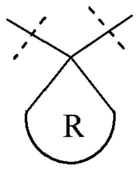

,

wherein R is $C_{3-10}$ cycloalkyl or 3- to 10-membered heterocyclyl.

It is also understood that the phrase "the pair $R^x/R^y$ is joint together with the atoms to which they are attached to form a ring A" in relation with a moiety of the structure

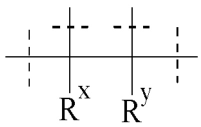

means that $R^x$ and $R^y$ form the following structure:

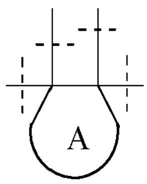

.

It is also understood that the phrase "$-R^1$ and an adjacent $-R^2$ form a carbon-carbon double bond provided that n is selected from the group consisting of 1, 2, 3 and 4" in relation with a moiety of the structure:

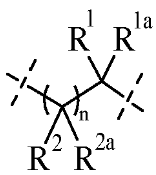

means that for example when n is 1, $-R^1$ and the adjacent $-R^2$ form the following structure:

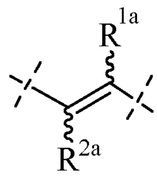

,

and if for example, n is 2, $R^1$ and the adjacent $-R^2$ form the following structure:

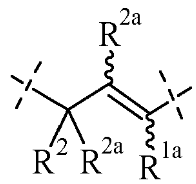

,

wherein the wavy bond means that $-R^{1a}$ and $-R^{2a}$ may be either on the same side of the double bond, i.e. in cis configuration, or on opposite sides of the double bond, i.e. in trans configuration and wherein the term "adjacent" means that $-R^1$ and $-R^2$ are attached to carbon atoms that are next to each other.

It is also understood that the phrase "two adjacent $-R^2$ form a carbon-carbon double bond provided that n is selected from the group consisting of 2, 3 and 4" in relation with a moiety of the structure:

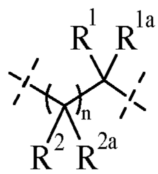

means that for example when n is 2, two adjacent $-R^2$ form the following structure:

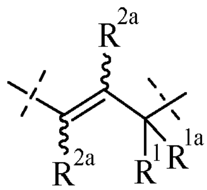

,

wherein the wavy bond means that each $-R^{2a}$ may be either on the same side of the double bond, i.e. in cis configuration, or on opposite sides of the double bond, i.e. in trans configuration and wherein the term "adjacent" means that two $-R^2$ are attached to carbon atoms that are next to each other.

It is understood that the "N" in the phrase "$\pi$-electron-pair-donating heteroaromatic N" refers to nitrogen.

It is understood that "$N^+$" in the phrases "an electron-donating heteroaromatic $N^+$-comprising moiety" and "attachment to the $N^+$ of $-D^+$" refers to a positively charged nitrogen atom.

As used herein, "halogen" means fluoro, chloro, bromo or iodo. In certain embodiments halogen is fluoro or chloro.

As used herein the term "alkali metal ion" refers to $Na^+$, $K^+$, $Li^+$, $Rb^+$ and $Cs^+$. In certain embodiments "alkali metal ion" refers to $Na^+$, $K^+$ and $Li^+$

As used herein the term "alkaline earth metal ion" refers to $Mg^{2+}$, $Ca^{2+}$, $Sr^{2+}$ and $Ba^{2+}$. In certain embodiments an alkaline earth metal ion is $Mg^{2+}$ or $Ca^{2+}$.

As used herein, the term "functional group" means a group of atoms which can react with other groups of atoms. Exemplary functional groups are carboxylic acid, primary amine, secondary amine, tertiary amine, maleimide, thiol, sulfonic acid, carbonate, carbamate, hydroxyl, aldehyde, ketone, hydrazine, isocyanate, isothiocyanate, phosphoric acid, phosphonic acid, haloacetyl, alkyl halide, acryloyl, aryl fluoride, hydroxylamine, disulfide, sulfonamides, sulfuric acid, vinyl sulfone, vinyl ketone, diazoalkane, oxirane, and aziridine.

In case the compounds of the present invention comprise one or more acidic or basic groups, the invention also comprises their corresponding pharmaceutically or toxicologically acceptable salts, in particular their pharmaceutically utilizable salts. Thus, the compounds of the present invention comprising acidic groups can be used according to the

invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine, amino acids, and quaternary ammonium salts, like tetrabutylammonium or cetyl trimethylammonium. Compounds of the present invention comprising one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, trifluoroacetic acid, and other acids known to the person skilled in the art. For the person skilled in the art further methods are known for converting the basic group into a cation like the alkylation of an amine group resulting in a positively-charge ammonium group and an appropriate counterion of the salt. If the compounds of the present invention simultaneously comprise acidic and basic groups, the invention also includes, in addition to the salt forms mentioned, inner salts or betaines (zwitterions). The respective salts can be obtained by customary methods, which are known to the person skilled in the art like, for example by contacting these compounds with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

The term "pharmaceutically acceptable" means a substance that does not cause harm when administered to a patient and in certain embodiments means approved by a regulatory agency, such as the EMA (Europe) and/or the FDA (US) and/or any other national regulatory agency for use in animals, such as for use in humans.

As used herein, the term "excipient" refers to a diluent, adjuvant, or vehicle with which the therapeutic, such as a drug or prodrug, is administered. Such pharmaceutical excipient may be sterile liquids, such as water and oils, including those of petroleum, animal, vegetable or synthetic origin, including but not limited to peanut oil, soybean oil, mineral oil or sesame oil. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, hyaluronic acid, propylene glycol, water and ethanol. The pharmaceutical composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(N-morpholino)ethanesulfonic acid), or may contain detergents, like Tween, poloxamers, poloxamines, CHAPS, Igepal, or amino acids like, for example, glycine, lysine, or histidine. These pharmaceutical compositions can take the form of solutions, suspensions, emulsions, tablets, pills, capsules, powders or sustained-release formulations. The pharmaceutical composition can be formulated as a suppository, with traditional binders and excipients such as triglycerides. Oral formulation can include standard excipients such as pharmaceutical grades of mannitol, lactose, starch, magnesium stearate, sodium saccharine, cellulose, magnesium carbonate, etc. Such compositions will contain a therapeutically effective amount of the drug or drug moiety, together with a suitable amount of excipient so as to provide the form for proper administration to the patient. The formulation should suit the mode of administration.

The term "peptide" as used herein refers to a chain of at least 2 and up to and including 50 amino acid monomer moieties, which may also be referred to as "amino acid residues", linked by peptide (amide) linkages. The amino acid monomers may be selected from the group consisting of proteinogenic amino acids and non-proteinogenic amino acids and may be D- or L-amino acids. The term "peptide" also includes peptidomimetics, such as peptoids, beta-peptides, cyclic peptides and depsipeptides and covers such peptidomimetic chains with up to and including 50 monomer moieties.

As used herein, the term "protein" refers to a chain of more than 50 amino acid monomer moieties, which may also be referred to as "amino acid residues", linked by peptide linkages, in which preferably no more than 12000 amino acid monomers are linked by peptide linkages, such as no more than 10000 amino acid monomer moieties, no more than 8000 amino acid monomer moieties, no more than 5000 amino acid monomer moieties or no more than 2000 amino acid monomer moieties.

In general, the terms "comprise" or "comprising" also encompasses "consist of" or "consisting of'.

For example, if the amount of water-insoluble controlled-release PRRA administered to an animal is 50 nmol of PRRA, as could be measured if all PRRA was released from the carrier, then an equivalent dose of free PRRA would also be 50 nmol. Fold differences in cytokine levels are calculated with the following equation:

$$\frac{\text{Plasma Cytokine Max Free PRRA}}{\text{Plasma Cytokine Max Controlled−Release PRRA}},$$

wherein

"Plasma Cytokine Max Free PRRA" is the highest plasma concentration of one of the measured cytokines within a 24-hour period following free PRRA intra-tumoral administration to a first group of animals and
"Plasma Cytokine Max Controlled-release PRRA" is the highest plasma concentration of the same cytokine measured within a 24-hour period following controlled-release PRRA intra-tumoral administration to a second group of animals.

In general the term "animal" also covers human and in certain embodiments means mouse, rat, non-human primate and human.
It is understood that the terms "first group of animals" and "second group of animals" may in certain embodiments relate to the same individuals, provided that a time period between the two administrations sufficient for complete clearance of the PRRA and conjugate is observed.
In one embodiment the at least one cytokine is IL-6. In another embodiment the at least one cytokine is CCL2. In another embodiment the at least one cytokine is IL-10. In another embodiment the at least one cytokine is IL-6 and CCL2, i.e. both IL-6 and CCL2 have a more than 10-fold lower maximum protein level. In another embodiment the at least one cytokine is CCL2 and IL-10. In another embodiment the at least one cytokine is IL-6 and IL-10. In another embodiment the at least one cytokine is IL-6, CCL2 and IL-10, i.e. all three cytokines have a more than 10-fold lower maximum protein level.
Protein levels can be measured by taking plasma samples prior to intra-tumoral administration and at various time points, such as at 3, 4, 5, 6, 7, or 8 time points, over a period of 24 hours after intra-tumoral administration and then determining the protein levels of the at least one cytokine. Suitable methods for quantifying protein levels are known to the person skilled in the art, such as for example by enzyme-linked immunosorbent assay (ELISA). Data points will be plotted and the maximum protein levels within the 24-hour period will be determined.
Maximum protein level of the at least one cytokine in plasma is more than 10-fold, such as more than 12-fold, more than 15-fold, more than 20-fold, more than 30-fold or more than 50-fold, lower following intra-tumoral administration of water-insoluble controlled-release PRRA compared to intra-tumoral administration of an equivalent molar dose of the corresponding free PRRA.
mRNA levels can be measured from PBMC samples at various time points, such as at 3, 4, 5, 6, 7, or 8 time points, over a period of 24 hours after intra-tumoral PRRA administration and can also include a sample taken prior to intra-tumoral administration by isolation of RNA and determination of the respective mRNA or cDNA levels. Suitable methods are known to the person skilled in the art, such as for example by isolation of PBMCs from blood using density gradient separation techniques, mRNA extraction from PBMCs using mRNA isolation kits, and measuring mRNA or corresponding cDNA levels using quantitative real-time PCR (qPCR). Data points will be plotted and the maximum mRNA expression levels within the 24-hour period will be determined.
The maximum plasma level of free PRRA can be measured by taking suitable plasma samples at various time points, such as 3, 4, 5, 6, 7, or 8 time points, over a period of 24 hours after intra-tumoral administration and can also include a sample taken prior to intra-tumoral administration, then determining the level of free PRRA. Suitable methods are known to the person skilled in art, such as for example by ultra high performance liquid chromatography coupled to tandem mass spectrophotometry. Data points will be plotted and the maximum PRRA level within the 24-hour period will be determined.
The maximum level of free PRRA following administration of controlled-release PRRA is more than 25-fold lower than the maximum plasma level within 24 hours after intra-tumoral administration of an equivalent molar dose of the corresponding free PRRA, such as more than 50-fold, more than 100-fold, or more than 200-fold lower.
In certain embodiments intra-tumoral administration of the water-insoluble controlled-release PRRA in a dose X induces a more than 1.5-fold, such as more than 1.5-fold, 1.7-fold, 2-fold, 2.2-fold, 2.5-fold, 3-fold, 3.5-fold, 4-fold or 5-fold, increase in the percent of antigen-presenting cells in tumor draining lymph nodes 7 days following said administration than intra-tumoral administration of a dose of 0.5 to 1.5 X of the corresponding free PRRA.
In certain embodiments intra-tumoral administration of the water-insoluble controlled-release PRRA in a dose X induces a more than 1.5-fold, such as more than 1.5-fold, 1.7-fold, 2-fold, 2.2-fold, 2.5-fold, 3-fold, 3.5-fold, 4-fold or 5-fold, increase in the expression of MHCII on antigen-presenting cell subsets in tumor-draining lymph nodes 7 days following said administration than intra-tumoral administration of a dose of 0.5 to 1.5 X of the corresponding free PRRA.
In certain embodiments intra-tumoral administration of the water-insoluble controlled-release PRRA results in local inflammation. In one embodiment local inflammation is an at least 1.5-fold, such as an at least 1.7-fold, at least 2-fold, at least 2.2-fold at least 2.5-fold, at least 3-fold, at least 3.5-fold, at least 4-fold, at least 4.5-fold, at least 5-fold, at least 5.5-fold, at least 6-fold, at least 7-fold, at least 8-fold, at least 9-fold or at least 10-fold increase, increase in the levels of at least four proteins selected from the group consisting of TNF$\alpha$, IL-1$\beta$, IL-10, IL-6, MCP-1, MIP-1$\alpha$, MIP-1$\beta$, MIP-2$\alpha$, MIP-3$\alpha$,

IP-10 and KC, in certain embodiments selected from the group consisting of TNFα, IL-1β, IL-6, MCP-1, MIP-1α, MIP-1β, MIP-2α, IP-10 and KC and in certain embodiments selected from the group consisting of are IL-1β, IL-6, MCP-1, MIP-1α, MIP-1β, MIP-2α, IP-10 and KC, compared to baseline tissue measured 3 days after intra-tumoral administration. This is not to be interpreted to mean that the local inflammation only lasts for 3 days. In fact, local inflammation may last significantly longer, such as for at least 4 days, at least 5 days, at least 6 days, at least 7 days, at least 8 days, at least 9 days, at least 10 days, at least 11 days, at least 12 days, at least 13 days, at least 14 days, at least 20 days, at least 30 days, or longer. Accordingly, measurement of the proteins selected from the group consisting of TNFα, IL-1β, IL-10, IL-6, MCP-1, MIP-1α, MIP-1β, MIP-2α, MIP-3α, IP-10 and KC, in certain embodiments selected from the group consisting of TNFα, IL-1β, IL-6, MCP-1, MIP-1α, MIP-1β, MIP-2α, IP-10 and KC and in certain embodiments selected from the group consisting of are IL-1β, IL-6, MCP-1, MIP-1α, MIP-1β, MIP-2α, IP-10 and KC, may also be performed at a later time point, such as at 4 days after intra-tumoral administration, at 5 days after intra- tumoral administration, at 6 days after intra-tumoral administration, at 7 days after intra- tumoral administration, at 8 days after intra- tumoral administration, at 9 days after intra-tumoral administration, at 10 days after intra- tumoral administration, at 11 days after intra-tumoral administration, at 12 days after intra- tumoral administration, at 13 days after intra-tumoral administration, at 14 days after intra-tumoral administration, at 20 days after intra-tumoral administration, at 30 days after intra- tumoral administration or even later than 30 days after intra- tumoral administration.

MCP-1 is also known as CCL2, MIP-1α is also known as CCL3, MIP-1β is also known as CCL4, MIP-2α is also known as MIP-2 and CXCL2, MIP-3α is also known as CCL20, IP-10 is also known as CXCL10 and KC is also known as GROα and CXCL1. CCL5 is also known as RANTES. CSF-2 is also known as GM-CSF. CCL8 is also known as MCP-2

It is understood that TNFα, IL-1β, IL-10, IL-6, MCP-1, MIP-1α, MIP-1β, MIP-2α, MIP-3α, IP-10 and KC are human proteins and that if the water-insoluble controlled-release PRRA is administered to a species other than human, the protein level of the corresponding homologous protein is measured.

Protein levels can be measured by methods known to the person skilled in the art. One method comprises the step of taking a sample of at least 0.025 g of tissue, such as at least 0.025 g, at least 0.05 g, at least 0.075 g, at least 0.1 g of tissue, from an area that is within 2 times the radius (r) from the injection site in any direction, wherein *r* is the distance in centimeters (cm) calculated from the volume (V) of water-insoluble controlled-release PRRA injected in cubic centimeters ($cm^3$) following the spheroid equation $V = \left(\frac{4}{3}\right)\pi r^3$. Protein may be isolated from such sample using standard methods known to the person skilled in the art, such as by tissue sample homogenization/disruption and cell lysis for protein analysis. The levels of at least four proteins selected from the group consisting of TNFα, IL-1β, IL-10, IL-6, MCP-1, MIP-1α, MIP-1β, MIP-2α, MIP-3α, IP-10 and KC, in certain embodiments selected from the group consisting of TNFα, IL-1β, IL-6, MCP-1, MIP-1α, MIP-1β, MIP-2α, IP-10 and KC and in certain embodiments selected from the group consisting of are IL-1β, IL-6, MCP-1, MIP-1α, MIP-1β, MIP-2α, IP-10 and KC, are then measured from such protein sample using standard methods known to the person skilled in the art, such as for example by enzyme-linked immunosorbent assay (ELISA).

In another embodiment local inflammation is an at least 1.5-fold, such as an at least 1.8-fold, at least 2-fold, at least 2.2-fold, at least 2.5-fold, at least 2.7-fold, at least 3-fold, at least 3.5-fold, at least 4-fold, at least 4.5-fold, at least 5-fold, at least 5.5-fold, at least 6-fold, at least 7-fold, at least 8-fold, at least 9-fold or at least 10-fold increase, increase in the expression levels of at least four mRNAs selected from the group consisting of *TNF, IL1A, IL1B, IL10, IL6, IL12B, CCL2, CCL8, CCL3, CCL4, CXCL2, CCL20, CSF2, pan-IFNA* subtype members, *IFNB1, IL18, CCL5, CXCL10* and *CXCL1,* in certain embodiments selected from the group consisting of *TNF, ILIB, IL10, IL6, CCL2, CCL3, CCL4, CXCL2, CSF2, IL18, CCL5, CXCL10* and *CXCL1* and in certain embodiments selected from the group consisting of *TNF, IL1B, IL6, CCL2, CCL3, CCL4, CXCL2, CCL5, CXCL10* and *CXCL1,* compared to baseline tissue measured 3 days after intra- tumoral administration. This is not to be interpreted to mean that the local inflammation only lasts for 3 days. In fact, local inflammation may last significantly longer, such as for at least 4 days, at least 5 days, at least 6 days, at least 7 days, at least 8 days, at least 9 days, at least 10 days, at least 11 days, at least 12 days, at least 13 days, at least 14 days, at least 20 days, at least 30 days or longer. Accordingly, measurement of the expression levels of at least four mRNAs selected from the group consisting of *TNF, IL1A, IL1B, IL10, IL6, IL12B, CCL2, CCL8, CCL3, CCL4, CXCL2, CCL20, CSF2, pan-IFNA* subtype members, *IFNB1, IL18, CCL5, CXCL10* and *CXCL1,* in certain embodiments selected from the group consisting of *TNF, IL1B, IL10, IL6, CCL2, CCL3, CCL4, CXCL2, CSF2, IL18, CCL5, CXCL10* and *CXCL1* and in certain embodiments selected from the group consisting of *TNF, IL1B, IL6, CCL2, CCL3, CCL4, CXCL2, CCL5, CXCL10* and *CXCL1,* may also be performed at a later time point, such as at 4 days after intra- tumoral administration, at 5 days after intra- tumoral administration, at 6 days after intra- tumoral administration, at 7 days after intra- tumoral administration, at 8 days after intra-tissue administration, at 9 days after intra- tumoral administration, at 10 days after intra- tumoral administration, at 11 days after intra- tumoral administration, at 12 days after intra- tumoral administration, at 13 days after intra- tumoral administration, at 14 days after intra- tumoral administration, at 20 days after intra- tumoral administration, at 30 days after intra- tumoral administration or even later than 30 days after intra- tumoral administration.

It is understood that *TNF, IL1A, IL1B, IL10, IL6, IL12B, CCL2, CCL8, CCL3, CCL4, CXCL2, CCL20, CSF2, pan-IFNA* subtype members, *IFNB1, IL18, CCL5, CXCL10* and *CXCL1* are human genes and that if the water-insoluble controlled-release pattern recognition receptor agonist is administered to a species other than human, mRNA expression of the corresponding homolog genes is measured. For mouse the respective homologs are *Tnf, Il1a, Il1b, Il10, Il6, Il12b, Ccl2, Ccl8, Ccl3, Ccl4, Cxcl2, Ccl20, Csf2, Ifna* (multiple subtype members), *Ifnb1, Il18, Ccl5, Cxcl10* and *Cxcl1.*

mRNA levels of a local inflammation can be measured by methods known to the person skilled in the art. One method comprises the step of taking a sample of at least 0.025 g of tissue, such as at least 0.025 g, at least 0.05 g, at least 0.075 g, at least 0.1 g of tissue, from an area that is within 2 times the radius (*r*) from the injection site in any direction, wherein *r* is the distance in centimeters (cm) calculated from the volume (V) of water-insoluble controlled-release PRRA injected in cubic centimeters ($cm^3$) following the spheroid equation $V = \left(\frac{4}{3}\right)\pi r^3$. Total RNA is isolated from such sample using standard methods known to the person skilled in the art, such as by tissue sample homogenization/disruption and cell lysis for RNA analysis. The expression levels of at least four mRNAs selected from the group consisting of *TNF, IL1A, ILIB, IL10, IL6, IL12B, CCL2, CCL8, CCL3, CCL4, CXCL2, CCL20, CSF2, pan-IFNA* subtype members, *IFNB1, IL18, CCL5, CXCL10* and *CXCL1,* in certain embodiments selected from the group consisting of *TNF, IL1B, IL10, IL6, CCL2, CCL3, CCL4, CXCL2, CSF2, IL18, CCL5, CXCL10* and *CXCL1* and in certain embodiments selected from the group consisting of *TNF, ILIB, IL6, CCL2, CCL3, CCL4, CXCL2, CCL5, CXCL10* and *CXCL1,* are then measured from such RNA sample using standard methods known to the person skilled in the art, such as for example by quantitative real-time PCR (qPCR).

The tumor into which the water-insoluble controlled-release PRRA is administered may be selected from the group selected from diseased tissues originating in the lymphoid tissue, such as lymph node, tonsil, spleen and bone marrow; gastrointestinal tract, such as salivary gland, oral mucosa, esophagus, stomach, duodenum, small intestine, colon and rectum; genitourinary tissues, such as fallopian tube, vagina, cervix, uterine, endometrium, ovaries, testes, prostate, epididymis and seminal vesicle; endocrine tissues, such as thyroid, parathyroid and adrenal glands; ocular tissues; oral tissues; auditory tissues; breast; skin; muscle, such as heart, skeletal and smooth muscle; lung; liver; heart; vascular tissue; central nervous tissue; peripheral nervous tissue; spinal tissue; brain; kidney; bladder; nasopharyngeal; bronchus; neck; pancreas; gall bladder; synovial; cartilage; connective tissue; fascia; pleural tissues; adipose tissues; and peritoneal tissues.

In certain embodiments the tumor into which the water-insoluble controlled-release PRRA is administered is diseased lymph node tissue. In certain embodiments the tumor into which the water-insoluble controlled-release PRRA is administered is diseased colon tissue. In certain embodiments the tumor into which the water-insoluble controlled-release PRRA is administered is diseased cervix tissue. In certain embodiments the tumor into which the water-insoluble controlled-release PRRA is administered is diseased uterine tissue. In certain embodiments the tumor into which the water-insoluble controlled-release PRRA is administered is diseased ovary tissue. In certain embodiments the tumor into which the water-insoluble controlled-release PRRA is administered is diseased prostate tissue. In certain embodiments the tumor into which the water-insoluble controlled-release PRRA is administered is diseased breast tissue. In certain embodiments the tumor into which the water-insoluble controlled-release PRRA is administered is diseased skin tissue. In certain embodiments the tumor into which the water-insoluble controlled-release PRRA is administered is diseased lung tissue. In certain embodiments the tumor into which the water-insoluble controlled-release PRRA is administered is diseased liver tissue. In certain embodiments the tumor into which the water-insoluble controlled-release PRRA is administered is diseased brain tissue. In certain embodiments the tumor into which the water-insoluble controlled-release PRRA is administered is diseased kidney tissue. In certain embodiments the tumor into which the water-insoluble controlled-release PRRA is administered is diseased bladder tissue. In certain embodiments the tumor into which the water-insoluble controlled-release PRRA is administered is diseased neck tissue. In certain embodiments the tumor into which the water-insoluble controlled-release PRRA is administered is diseased pancreas tissue.

In certain embodiments the treatment of the cancer may in addition to the administration of the water-insoluble controlled-release PRRA also include the administration of at least one cancer therapeutic, such as systemic immunotherapy. Such at least one additional cancer therapeutic may be selected from the group consisting of cytotoxic/-chemotherapeutic agents, immune checkpoint inhibitors or antagonists, immune checkpoint agonists, multi-specific drugs, antibody-drug conjugates (ADC), radionuclides or targeted radionuclide therapeutics, DNA damage repair inhibitors, tumor metabolism inhibitors, pattern recognition receptor agonists, protein kinase inhibitors, chemokine and chemoattractant receptor agonists, chemokine or chemokine receptor antagonists, cytokine receptor agonists, death receptor agonists, CD47 or SIRP$\alpha$ antagonists, oncolytic drugs, signal converter proteins, epigenetic modifiers, tumor peptides or tumor vaccines, heat shock protein (HSP) inhibitors, proteolytic enzymes, ubiquitin and proteasome inhibitors, adhesion molecule antagonists, and hormones including hormone peptides and synthetic hormones.

In certain embodiments the one or more additional drug is a cytotoxic/chemotherapeutic agent. In certain embodiments the one or more additional drug is an immune checkpoint inhibitor or antagonist. In certain embodiments the

one or more additional drug is a multi-specific drug. In certain embodiments the one or more additional drug is an antibody-drug conjugate (ADC). In certain embodiments the one or more additional drug is a radionuclide or a targeted radionuclide therapeutic. In certain embodiments the one or more additional drug is DNA damage repair inhibitor. In certain embodiments the one or more additional drug is a tumor metabolism inhibitor. In certain embodiments the one or more additional drug is a pattern recognition receptor agonist. In certain embodiments the one or more additional drug is a protein kinase inhibitor. In certain embodiments the one or more additional drug is a chemokine and chemoattractant receptor agonist. In certain embodiments the one or more additional drug is a chemokine or chemokine receptor antagonist. In certain embodiments the one or more additional drug is a cytokine receptor agonist. In certain embodiments the one or more additional drug is a death receptor agonist. In certain embodiments the one or more additional drug is a CD47 antagonist. In certain embodiments the one or more additional drug is a SIRPα antagonist. In certain embodiments the one or more additional drug is an oncolytic drug. In certain embodiments the one or more additional drug is a signal converter protein. In certain embodiments the one or more additional drug is an epigenetic modifier. In certain embodiments the one or more additional drug is a tumor peptide or tumor vaccine. In certain embodiments the one or more additional drug is a heat shock protein (HSP) inhibitor. In certain embodiments the one or more additional drug is a proteolytic enzyme. In certain embodiments the one or more additional drug is a ubiquitin and proteasome inhibitor. In certain embodiments the one or more additional drug is an adhesion molecule antagonist. In certain embodiments the one or more additional drug is a hormone including hormone peptides and synthetic hormones.

The cytotoxic or chemotherapeutic agent may be selected from the group consisting of alkylating agents, anti-metabolites, anti-microtubule agents, topoisomerase inhibitors, cytotoxic antibiotics, auristatins, enediynes, lexitropsins, duocarmycins, cyclopropylpyrroloindoles, puromycin, dolastatins, maytansine derivatives, alkylsufonates, triazenes and piperazine.

The alkylating agent may be selected from the group consisting of nitrogen mustards, such as mechlorethamine, cyclophosphamide, melphalan, chlorambucil, ifosfamide and busulfan; nitrosoureas, such as N-nitroso-N-methylurea, carmustine, lomustine, semustine, fotemustine and streptozotocin; tetrazines, such as dacarbazine, mitozolomide and temozolomide; ethylenimines, such as altretamine; aziridines, such as thiotepa, mitomycin and diaziquone; cisplatin and derivatives, such as cisplatin, carboplatin, oxaliplatin; and non-classical alkylating agents, such as procarbazine and hexamethylmelamine.

The anti-metabolite may be selected from the group consisting of anti-folates, such as methotrexate and pemetrexed; fluoropyrimidines, such as fluorouracil and capecitabine; deoxynucleoside analogues, such as cytarabine, gemcitabine, decitabine, azacytidine, fludarabine, nelarabine, cladribine, clofarabine and pentostatin; and thiopurines, such as thioguanine and mercaptopurine.

The anti-microtubule agent may be selected from the group consisting of *Vinca* alkaloids, such as vincristine, vinblastine, vinorelbine, vindesine and vinflunine; taxanes, such as paclitaxel and docetaxel; podophyllotoxins and derivatives, such as podophyllotoxin, etoposide and teniposide; stilbenoid phenol and derivatives, such as zybrestat (CA4P); and BNC105.

The topoisomerase inhibitor may be selected from the group consisting of topoisomerase I inhibitors, such as irinotecan, topotecan and camptothecin; and topoisomerase II inhibitors, such as etoposide, doxorubicin, mitoxantrone, teniposide, novobiocin, merbarone and aclarubicin.

The cytotoxic antibiotic may be selected from the group consisting of anthracyclines, such as doxorubicin, daunorubicin, epirubicin and idarubicin; pirarubicin, aclarubicin, bleomycin, mitomycin C, mitoxantrone, actinomycin, dactinomycin, adriamycin, mithramycin and tirapazamine.

The auristatin may be selected from the group consisting of monomethyl auristatin E (MMAE) and monomethyl auristatin F (MMAF).

The enediyne may be selected from the group consisting of neocarzinostatin, lidamycin (C-1027), calicheamicins, esperamicins, dynemicins and golfomycin A.

The maytansine derivative may be selected from the group consisting of ansamitocin, mertansine (emtansine, DM1) and ravtansine (soravtansine, DM14).

The immune checkpoint inhibitor or antagonist may be selected from the group consisting of inhibitors of CTLA-4 (cytotoxic T-lymphocyte-associated protein 4), such as ipilimumab, tremelimumab, MK-1308, FPT155, PRS010, BMS-986249, BPI-002, CBT509, JS007, ONC392, TE1254, IBI310, BR02001, CG0161, KN044, PBI5D3H5, BCD145, ADU1604, AGEN1884, AGEN1181, CS1002 and CP675206; inhibitors of PD-1 (programmed death 1), such as pembrolizumab, nivolumab, pidilizumab, AMP-224, BMS-936559, cemiplimab and PDR001; inhibitors of PD-L1 (programmed cell death protein 1), such as MDX-1105, MEDI4736, atezolizumab, avelumab, BMS-936559 and durvalumab; inhibitors of PD-L2 (programmed death-ligand 2); inhibitors of KIR (killer-cell immunoglobulin-like receptor), such as lirlumab (IPH2102) and IPH2101; inhibitors of B7-H3, such as MGA271; inhibitors of B7-H4, such as FPA150; inhibitors of BTLA (B- and T-lymphocyte attenuator); inhibitors of LAG3 (lymphocyte-activation gene 3), such as IMP321 (eftilagimod alpha), relatlimab, MK-4280, AVA017, BI754111, ENUM006, GSK2831781, INCAGN2385, LAG3Ig, LAG525, REGN3767, Sym016, Sym022, TSR033, TSR075 and XmAb22841; inhibitors of TIM-3 (T-cell immunoglobulin and

mucin-domain containing-3), such as LY3321367, MBG453, and TSR-022; inhibitors of VISTA (V-domain Ig suppressor of T cell activation), such as JNJ-61610588; inhibitors of ILT2/LELRB1 (Ig-like transcript 2/leukocyte Ig-like receptor 1); inhibitor of ILT3/LILRB4 (Ig-like transcript 3/leukocyte Ig-like receptor 4); inhibitors of ILT4/LILRB2 (Ig-like transcript 4/leukocyte Ig-like receptor 2), such as MK-4830; inhibitors of TIGIT (T cell immunoreceptor with Ig and ITIM domains), such as MK-7684, PTZ-201, RG6058 and COM902; inhibitors of NKG2A, such as IPH-2201; and inhibitors of PVRIG, such as COM701.

One example of a an inhibitor of CTLA-4 is an anti-CTLA4 conjugate or a pharmaceutically acceptable salt thereof, wherein said conjugate comprises a plurality of anti-CTLA4 moieties $-D_{CTLA4}$ covalently conjugated via at least one moiety $-L^1-L^2-$ to a polymeric moiety Z, wherein $-L^1-$ is covalently and reversibly conjugated to $-D_{CTLA4}$ and $-L^2-$ is covalently conjugated to Z and wherein $-L^1-$ is a linker moiety and $-L^2-$ is a chemical bond or a spacer moiety, wherein the moieties $-L^1-$, $-L^2-$ and Z are as described elsewhere herein for the conjugate of the present invention. In certain embodiments $-D_{CTLA4}$ is selected from the group consisting of wild-type $F_c$ anti-CTLA4 antibodies, Fc enhanced for effector function/FcγR binding anti-CTLA4 antibodies, anti-CTLA4 antibodies conditionally active in tumor microenvironment, anti-CTLA4 small molecules, CTLA4 antagonist fusion proteins, anti-CTLA4 anticalins, anti-CTLA4 nanobodies and anti-CTLA4 multispecific biologics based on antibodies, scFVs or other formats. In certain embodiments $-D_{CTLA4}$ is ipilimumab. In certain embodiments $-D_{CTLA4}$ is tremelimumab. In certain embodiments the anti-CTLA4 conjugate has the following structure:

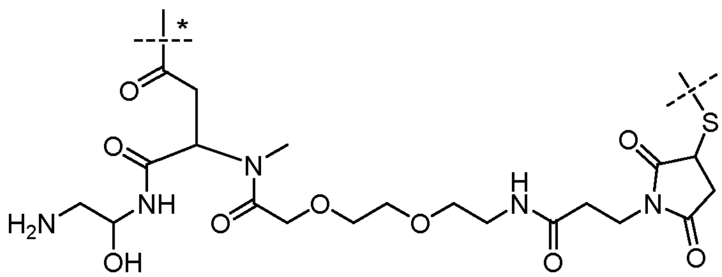

,

wherein

the dashed line marked with the asterisk indicates attachment to the nitrogen of an amine of $-D_{CTLA4}$, in particular to the nitrogen of an amine of ipilimumab; and

the unmarked dashed line indicates attachment to Z, such as a hydrogel, in particular to a crosslinked hyaluronic acid hydrogel.

It is understood that that a multitude of moieties $-D_{CTLA4}-L^1-L^2-$ are connected to Z, if Z is a hydrogel, such as a crosslinked hyaluronic acid hydrogel.

In certain embodiments the nitrogen of an amine functional group of $-D_{CTLA4}$ and in particular of ipilimumab is an amine of a lysine residue. In certain embodiments the nitrogen of an amine functional group of $-D_{CTLA4}$ and in particular of ipilimumab is the N-terminal amine.

In certain embodiments the one or more additional drug is an inhibitor of CTLA4 as described above.

The immune checkpoint agonist may be selected from the group consisting of agonists of CD27, such as recombinant CD70, such as HERA-CD27L, and varlilumab (CDX-1127); agonists of CD28, such as recombinant CD80, recombinant CD86, TGN1412 and FPT155; agonists of CD40, such as recombinant CD40L, CP-870,893, dacetuzumab (SGN-40), Chi Lob 7/4, ADC-1013 and CDX1140; agonists of 4-1BB (CD137), such as recombinant 4-1BBL, urelumab, utomilumab and ATOR-1017; agonists of OX40, such as recombinant OX40L, MEDI0562, GSK3174998, MOXR0916 and PF-04548600; agonists of GITR, such as recombinant GITRL, TRX518, MEDI1873, INCAGN01876, MK-1248, MK-4166, GWN323 and BMS-986156; and agonists of ICOS, such as recombinant ICOSL, JTX-2011 and GSK3359609.

The multi-specific drug may be selected from the group consisting of biologics and small molecule immune checkpoint inhibitors. Examples for biologics are multi-specific immune checkpoint inhibitors, such as CD137/HER2 lipocalin, PD1/LAG3, FS118, XmAb22841 and XmAb20717; and multi-specific immune checkpoint agonists. Such multi-specific immune checkpoint agonists may be selected from the group consisting of Ig superfamily agonists, such as ALPN-202; TNF superfamily agonists, such as ATOR-1015, ATOR-1144, ALG.APV-527, lipocalin/PRS-343, PRS344/ONC0055, FAP-CD40 DARPin, MP0310 DARPin, FAP-0X40 DARPin, EGFR-CD40 DARPin, EGFR41BB/CD137 DARPin, EGFR-0X40/DARFPin, HER2-CD40 DARPin, HER2-41BB/CD137 DARPin, HER2-0X40 DARPin, FIBRONECTIN ED-B-CD40 DARPin, FIBRONECTIN ED-B-41BB/CD137 and FIBRONECTIN ED-B-0X40 DARPin; CD3 multispecific agonists, such as blinatumomab, solitomab, MEDI-565, ertumaxomab, anti-HER2/CD3 1Fab-immunoblobulin G TDB, GBR 1302, MGD009, MGD007, EGFRBi, EGFR-CD Probody, RG7802, PF-06863135,

PF-06671008, MOR209/ES414, AMG212/BAY2010112 and CD3-5T4; and CD16 multispecific agonists, such as 1633 BiKE, 161533 TriKE, OXS-3550, OXS-C3550, AFM13 and AFM24.

An example for a small molecule immune checkpoint inhibitor is CA-327 (TIM3/PD-L1 antagonist).

The antibody-drug conjugate may be selected from the group consisting of ADCs targeting hematopoietic cancers, such as gemtuzumab ozogamicin, brentuximab vedotin, inotuzumab ozogamicin, SAR3419, BT062, SGN-CD19A, IMGN529, MDX-1203, polatuzumab vedotin (RG7596), pinatuzumab vedotin (RG7593), RG7598, milatuzumab-doxorubicin and OXS-1550; and ADCs targeting solid tumor antigens, such as trastuzumab emtansine, glembatumomab vedotin, SAR56658, AMG-172, AMG-595, BAY-94-9343, BIIB015, vorsetuzumab mafodotin (SGN-75), ABT-414, ASG-5ME, enfortumab vedotin (ASG-22ME), ASG-16M8F, IMGN853, indusatumab vedotin (MLN-0264), vadortuzumab vedotin (RG7450), sofituzumab vedotin (RG7458), lifastuzumab vedotin (RG7599), RG7600, DEDN6526A (RG7636), PSMA TTC, 1095 from Progenics Pharmaceuticals, lorvotuzumab mertansine, lorvotuzumab emtansine, IMMU-130, sacituzumab govitecan (IMMU-132), PF-06263507 and MEDI0641.

The radionuclides may be selected from the group consisting of $\beta$-emitters, such as $^{177}$Lutetium, $^{166}$Holmium, $^{186}$Rhenium, $^{188}$Rhenium, $^{67}$Copper, $^{149}$Promethium, $^{199}$Gold, $^{77}$Bromine, $^{153}$Samarium, $^{105}$Rhodium, $^{89}$Strontium, $^{90}$Yttrium, $^{131}$Iodine; $\alpha$-emitters, such as $^{213}$Bismuth, $^{223}$Radium, $^{225}$ Actinium, $^{211}$Astatine; and Auger electron-emitters, such as $^{77}$Bromine, $^{111}$Indium, $^{123}$Iodine and $^{125}$Iodine.

The targeted radionuclide therapeutics may be selected from the group consisting of zevalin ($^{90}$Y-ibritumomab tiuxetan), bexxar ($^{131}$I-tositumomab), oncolym ($^{131}$I-Lym 1), lymphocide ($^{90}$Y-epratuzumab), cotara ($^{131}$I-chTNT-1/B), labetuzumab ($^{90}$Y or $^{131}$I-CEA), theragyn ($^{90}$Y-pemtumomab), licartin ($^{131}$I-metuximab), radretumab ($^{131}$I-L19) PAM4 ($^{90}$Y-clivatuzumab tetraxetan), xofigo ($^{223}$Ra dichloride), lutathera ($^{177}$Lu-DOTA-Tyr$^{3}$-Octreotate) and $^{131}$I-MIBG.

The DNA damage repair inhibitor may be selected from the group consisting of poly (ADP-ribose) polymerase (PARP) inhibitors, such as olaparib, rucaparib, niraparib, veliparib, CEP 9722 and E7016; CHK1/CHK2 dual inhibitors, such as AZD7762, V158411, CBP501 and XL844; CHK1 selective inhibitors, such as PF477736, MK8776/SCH900776, CCT244747, CCT245737, LY2603618, LY2606368/prexasertib, AB-IsoG, ARRY575, AZD7762, CBP93872, ESP01, GDC0425, SAR020106, SRA737, V158411 and VER250840; CHK2 inhibitors, such as CCT241533 and PV1019; ATM inhibitors, such as AZD0156, AZD1390, KU55933, M3541 and SX-RDS1; ATR inhibitors, such as AZD6738, BAY1895344, M4344 and M6620 (VX-970); and DNA-PK inhibitors, such as M3814.

The tumor metabolism inhibitor may be selected from the group consisting of inhibitors of the adenosine pathway, inhibitors of the tryptophan metabolism and inhibitors of the arginine pathway.

Examples for an inhibitor of the adenosine pathway are inhibitors of A2AR (adenosine A2A receptor), such as ATL-444, istradefylline (KW-6002), MSX-3, preladenant (SCH-420,814), SCH-58261, SCH412,348, SCH-442,416, ST-1535, caffeine, VER-6623, VER-6947, VER-7835, vipadenant (BIIB-014), ZM-241,385, PBF-509 and V81444; inhibitors of CD73, such as IPH53 and SRF373; and inhibitors of CD39, such as IPH52.

Examples for an inhibitor of the tryptophane metabolism are inhibitors of IDO, such as indoximod (NLG8189), epacadostat, navoximod, BMS-986205 and MK-7162; inhibitors of TDO, such as 680C91; and IDO/TDO dual inhibitors.

Examples for inhibitors of the arginine pathway are inhibitors of arginase, such as INCB001158.

The pattern recognition agonist may be selected from the group consisting of Toll-like receptor agonists, NOD-like receptors, RIG-I-like receptors, cytosolic DNA sensors, STING, and aryl hydrocarbon receptors (AhR).

The Toll-like receptor agonists may be selected from the group consisting of agonists of TLR1/2, such as peptidoglycans, lipoproteins, Pam3CSK4, Amplivant, SLP-AMPLIVANT, HESPECTA, ISA101 and ISA201; agonists of TLR2, such as LAM-MS, LPS-PG, LTA-BS, LTA-SA, PGN-BS, PGN-EB, PGN-EK, PGN-SA, CL429, FSL-1, Pam2CSK4, Pam3CSK4, zymosan, CBLB612, SV-283, ISA204, SMP105, heat killed *Listeria monocytogenes;* agonists of TLR3, such as poly(A:U), poly(I:C) (poly-ICLC), rintatolimod, apoxxim, IPH3102, poly-ICR, PRV300, RGCL2, RGIC.1, Riboxxim (RGC100, RGIC100), Riboxxol (RGIC50) and Riboxxon; agonists of TLR4, such as lipopolysaccharides (LPS), neoceptin-3, glucopyranosyl lipid adjuvant (GLA), GLA-SE, G100, GLA-AF, clinical center reference endotoxin (CCRE), monophosphoryl lipid A, grass MATA MPL, PEPA10, ONT-10 (PET-Lipid A, oncothyreon), G-305, ALD046, CRX527, CRX675 (RC527, RC590), GSK1795091, OM197MPAC, OM294DP and SAR439794; agonists of TLR2/4, such as lipid A, OM174 and PGN007; agonists of TLR5, such as flagellin, entolimod, mobilan, protectan CBLB501; agonists of TLR6/2, such as diacylated lipoproteins, diacylated lipopeptides, FSL-1, MALP-2 and CBLB613; agonists of TLR7, such as CL264, CL307, imiquimod (R837), TMX-101, TMX-201, TMX-202, TMX-302, gardiquimod, S-27609, 851, UC-IV150, 852A (3M-001, PF-04878691), loxoribine, polyuridylic acid, GSK2245035, GS-9620, RO6864018 (ANA773, RG7795), RO7020531, isatoribine, AN0331, ANA245, ANA971, ANA975, DSP0509, DSP3025 (AZD8848), GS986, MBS2, MBS5, RG7863 (RO6870868), sotirimod, SZU101 and TQA3334; agonists of TLR8, such as ssPolyUridine, ssRNA40, TL8-506, XG-1-236, VTX-2337 (motolimod), VTX-1463, TMX-302, VTX-763, DN1508052 and GS9688; agonists of TLR7/8, such as CL075, CL097, poly(dT), resiquimod (R-848, VML600, S28463), MEDI9197 (3M-052), NKTR262, DV1001, IMO4200, IPH3201 and VTX1463; agonists of TLR9, such as CpG DNA, CpG ODN, lefitolimod (MGN1703), SD-101, QbG10, CYT003, CYT003-QbG10, DUK-CpG-001, CpG-7909 (PF-3512676), GNKG168, EMD 1201081, IMO-2125, IMO-2055, CpG10104, AZD1419, AST008, IMO2134, MGN1706, IRS 954, 1018 ISS, actilon

(CPG10101), ATP00001, AVE0675, AVE7279, CMP001, DIMS0001, DIMS9022, DIMS9054, DIMS9059, DV230, DV281, EnanDIM, heplisav (V270), kappaproct (DIMS0150), NJP834, NPI503, SAR21609 and tolamba; and agonists of TLR7/9, such as DV1179.

Examples for CpG ODN are ODN 1585, ODN 2216, ODN 2336, ODN 1668, ODN 1826, ODN 2006, ODN 2007, ODN BW006, ODN D-SL01, ODN 2395, ODN M362 and ODN D-SL03.

The NOD-like receptors may be selected from the group consisting of agonists of NOD1, such as C12-iE-DAP, C14-Tri-LAN-Gly, iE-DAP, iE-Lys, and Tri-DAP; and agonists of NOD2, such as L18-MDP, MDP, M-TriLYS, murabutide and N-glycolyl-MDP.

The RIG-I-like receptors may be selected from the group consisting of 3p-hpRNA, 5'ppp-dsRNA, 5'ppp RNA (M8), 5'OH RNA with kink (CBS-13-BPS), 5'PPP SLR, KIN100, KIN 101, KIN1000, KIN1400, KIN1408, KIN1409, KIN1148, KIN131A, poly(dA:dT), SB9200, RGT100 and hiltonol.

The cytosolic DNA sensors may be selected from the group consisting of cGAS agonists, dsDNA-EC, G3-YSD, HSV-60, ISD, ODN TTAGGG (A151), poly(dG:dC) and VACV-70.

The STING may be selected from the group consisting of MK-1454, ADU-S100 (MIW815), 2'3'-cGAMP, 3'3'-cGAMP, c-di-AMP, c-di-GMP, cAIMP (CL592), cAIMP difluor (CL614), cAIM$(PS)_2$ difluor (Rp/Sp) (CL656), 2'2'-cGAMP, 2'3'-cGAM(PS)2 (Rp/Sp), 3'3'-cGAM fluorinated, c-di-AMP fluorinated, 2'3'-c-di-AMP, 2'3'-c-di-AM(PS)2 (Rp,Rp), c-di-GMP fluorinated, 2'3'-c-di-GMP, c-di-IMP, c-di-UMP and DMXAA (vadimezan, ASA404).

The aryl hydrocarbon receptor (AhR) may be selected from the group consisting of FICZ, ITE and L-kynurenine.

The protein kinase inhibitor may be selected from the group consisting of receptor tyrosine kinase inhibitors, intracellular kinase inhibitors, cyclin dependent kinase inhibitors, phosphoinositide-3-kinase inhibitors, mitogen-activated protein kinase inhibitors, inhibitors of nuclear factor kappa-β kinase (IKK), and Wee-1 inhibitors.

Examples for receptor tyrosine kinase inhibitors are EGF receptor inhibitors, such as afatinib, cetuximab, erlotinib, gefitinib, pertuzumab and margetuximab; VEGF receptor inhibitors, such as axitinib, lenvatinib, pegaptanib and linifanib (ABT-869); C-KIT Receptor inhibitors, such as CDX0158 (KTN0158); ERBB2 (HER2) inhibiors, such as herceptin (trastuzumab); ERBB3 receptor inhibitors, such as CDX3379 (MEDI3379, KTN3379) and AZD8931 (sapitinib); FGF receptor inhibitors, such as erdafitinib; AXL receptor inhibitors, such as BGB324 (BGB 324, R 428, R428, bemcentinib) and SLC391; and MET receptor inhibitors, such as CGEN241.

Examples for intracellular kinase inhibitors are Bruton's tyrosine kinase (BTK) inhibitors, such as ibrutinib, acalabrutinib, GS-4059, spebrutinib, BGB-3111, HM71224, zanubrutinib, ARQ531, BI-BTK1 and vecabrutinib; spleen tyrosine kinase inhibitors, such as fostamatinib; Bcr-Abl tyrosine kinase inhibitors, such as imatinib and nilotinib; Janus kinase inhibitors, such as ruxolitinib, tofacitinib and fedratinib; and multi-specific tyrosine kinase inhibitors, such as bosutinib, crizotinib, cabozantinib, dasatinib, entrectinib, lapatinib, mubritinib, pazopanib, sorafenib, sunitinib, SU6656 and vandetanib.

One example of a tyrosine kinase inhibitor is a tyrosine kinase inhibitor ("TKI") conjugate or a pharmaceutically acceptable salt thereof, wherein said conjugate comprises a plurality of TKI moieties $-D_{TKI}$ covalently conjugated via at least one moiety $-L^1-L^2-$ to a polymeric moiety Z, wherein $-L^1-$ is covalently and reversibly conjugated to $-D_{TKI}$ and $-L^2-$ is covalently conjugated to Z and wherein $-L^1-$ is a linker moiety and $-L^2-$ is a chemical bond or a spacer moiety, wherein the moieties $-L^1-$, $-L^2-$ and Z are as described elsewhere herein for the conjugate of the present invention. In certain embodiments $-D_{TKI}$ is selected from the group consisting of receptor tyrosine kinase inhibitors, intracellular kinase inhibitors, cyclin dependent kinase inhibitors, phosphoinositide-3-kinase (PI3K) inhibitors, mitogen-activated protein kinase inhibitors, inhibitors of nuclear factor kappa-β kinase (IKK), and Wee-1 inhibitors. In certain embodiments $-D_{TKI}$ is axitinib. In certain embodiments $-D_{TKI}$ is lenvatinib. In certain embodiments $-D_{TKI}$ is pegaptanib. In certain embodiments $-D_{TKI}$ is linifanib.

In certain embodiments the TKI conjugate has the following structure

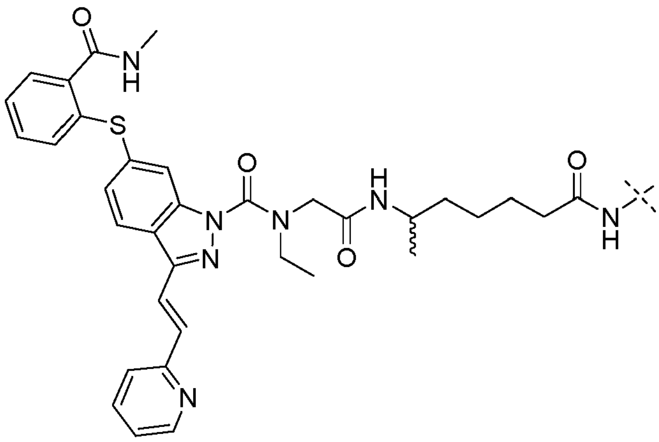

,

wherein
the dashed line indicates attachment to Z, such as a PEG-based hydrogel or a hyaluronic acid-based hydrogel.

In certain embodiments the TKI conjugate has the following structure

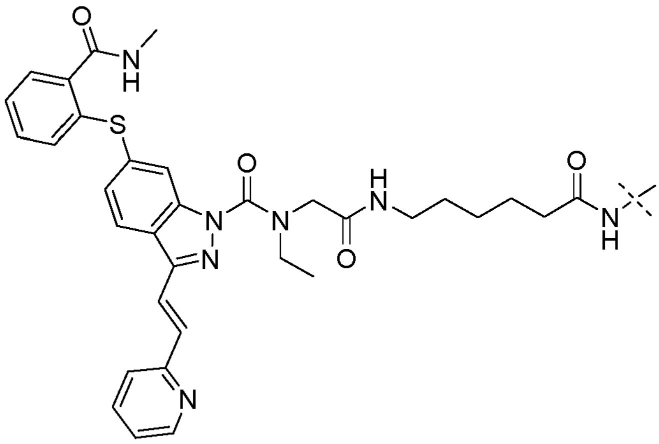

,

wherein
the dashed line indicates attachment to Z, such as a PEG-based hydrogel or a hyaluronic acid-based hydrogel.

In certain embodiments the TKI conjugate has the following structure

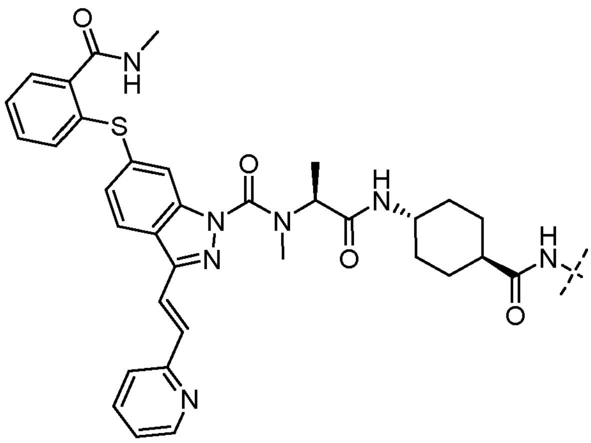

,

wherein
the dashed line indicates attachment to Z, such as a PEG-based hydrogel or a hyaluronic acid-based hydrogel.

In certain embodiments the TKI conjugate has the following structure

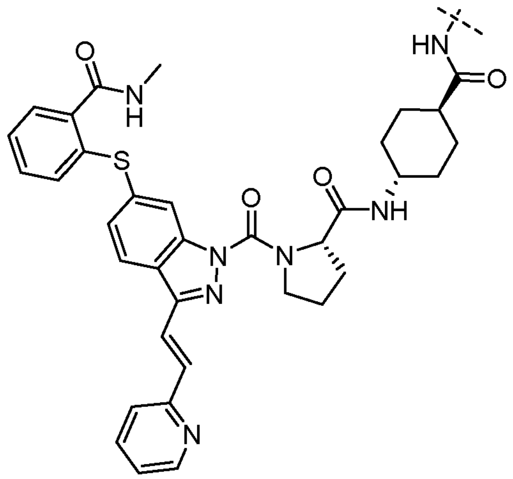

,

wherein

the dashed line indicates attachment to Z, such as a PEG-based hydrogel or a hyaluronic acid-based hydrogel.

Examples for cyclin dependent kinase inhibitors are ribociclib, palbociclib, abemaciclib, trilaciclib, purvalanol A, olomucine II and MK-7965.

Examples for phophoinositide-3-kinase inhibitors are IPI549, GDc-0326, pictilisib, serabelisib, IC-87114, AMG319, seletalisib, idealisib and CUDC907.

Examples for mitogen-activated protein kinase inhibitors are Ras/farnesyl transferase inhibitors, such as tipirafinib and LB42708; Raf inhibitors, such as regorafenib, encorafenib, vemurafenib, dabrafenib, sorafenib, PLX-4720, GDC-0879, AZ628, lifirafenib, PLX7904 and RO5126766; MEK inhibitors, such as cobimetinib, trametinib, binimetinib, selumetinib, pimasertib, refametinib and PD0325901; ERK inhibitors, such as MK-8353, GDC-0994, ulixertinib and SCH772984.

Examples for inhibitors of nuclear factor kappa-β kinase (IKK) are BPI-003 and AS602868.

An example of a Wee-1 inhibitor is adavosertib.

The chemokine receptor and chemoattractant receptor agonist may be selected from the group consisting of CXC chemokine receptors, CC chemokine receptors, C chemokine receptors, CX3C chemokine receptors and chemoattractant receptors.

The CXC chemokine receptor may be selected from the group consisting of CXCR1 agonists, such as recombinant CXCL8 and recombinant CXCL6; CXCR2 agonists, such as recombinant CXCL8, recombinant CXCL1, recombinant CXCL2, recombinant CXCL3, recombinant CXCL5, recombinant CXCL6, MGTA 145 and SB251353; CXCR3 agonists, such as recombinant CXCL9, recombinant CXCL10, recombinant CXCL11 and recombinant CXCL4; CXCR4 agonists, such as recombinant CXCL12, ATI2341, CTCE0214, CTCE0324 and NNZ4921; CXCR5 agonists, such as recombinant CXCL13; CXCR6 agonists, such as recombinant CXCL16; and CXCL7 agonists, such as recombinant CXCL11.

The CC chemokine receptor may be selected from the group consisting of CCR1 agonists, such as recombinant CCL3, ECI301, recombinant CCL4, recombinant CCL5, recombinant CCL6, recombinant CCL8, recombinant CCL9/10, recombinant CCL14, recombinant CCL15, recombinant CCL16, recombinant CCL23, PB103, PB105 and MPIF1; CCR2 agonists, such as recombinant CCL2, recombinant CCL8, recombinant CCL16, PB103 and PB105; CCR3 agonists, such as recombinant CCL11, recombinant CCL26, recombinant CCL7, recombinant CCL13, recombinant CCL15, recombinant CCL24, recombinant CCL5, recombinant CCL28 and recombinant CCL18; CCR4 agonists, such as recombinant CCL3, ECI301, recombinant CCL5, recombinant CCL17 and recombinant CCL22; CCR5 agonists, such as recombinant CCL3, ECI301, recombinant CCL5, recombinant CCL8, recombinant CCL11, recombinant CCL13, recombinant CCL14, recombinant CCL16, PB103 and PB105; CCR6 agonists, such as recombinant CCL20; CCR7 agonists, such as recombinant CCL19 and recombinant CCL21; CCR8 agonists, such as recombinant CCL1, recombinant CCL16, PB103 and PB105; CCR9 agonists, such as recombinant CCL25; CCR10 agonists, such as recombinant CCL27 and recombinant CCL28; and CCR11 agonists, such as recombinant CCL19, recombinant CCL21 and recombinant CCL25.

The C chemokine receptors may be a XCR1 agonist, such as recombinant XCL1 or recombinant XCL2.

The CX3C chemokine receptors may be a CX3CR1 agonist, such as recombinant CX3CL1.

The chemoattractant receptors may be selected from the group consisting of formyl peptide receptor agonists, such as N-formyl peptides, N-formylmethionine-leucyl-phenylalanine, enfuvirtide, T21/DP107, annexin A1, Ac2-26 and Ac9-25; C5a receptor agonists; and chemokine-like receptor 1 agonists, such as chemerin.

The chemokine antagonists may be selected from the group consisting of inhibitors of CXCL chemokines, such

as UNBS5162; inhibitors of CXCL8, such as BMS986253 and PA620; inhibitors of CXCL10, such as TM110, eldelumab and NI0801; inhibitors of CXCL12, such as NOX-A12 and JVS100; inhibitors of CXCL13, such as VX5; inhibitors of CCL2, such as PA508, ABN912, AF2838, BN83250, BN83470, C243, CGEN54, CNTO888, NOXE36, VT224 and SSR150106; inhibitors of CCL5, such as HGS1025 and NI0701; inhibitors of CCL2/CCL5, such as BKTP46; inhibitors of CCL5/FMLP receptor, such as RAP160; inhibitors of CCL11, such as bertilimumab and RAP701; inhibitors of CCL5/CXCL4, such as CT2008 and CT2009; inhibitors of CCL20, such as GSK3050002; and inhibitors of CX3CL1, such as quetmolimab.

The chemokine receptor antagonists may be selected from the group consisting of inhibitors of CXCR1, such as repertaxin, CCX832, FX68 and KB03; inhibitors of CXCR2, such as AZD5069, AZD5122, AZD8309, GSK1325756, GSK1325756H, PS291822, SB332235 and SB656933; inhibitors of CXCR1/CXCR2, such as DF1970, DF2156A, DF2162, DF2755A, reparixin, SX576, SX682, PACG31P, AZD4721 and PA401; inhibitors of CXCR3; inhibitors of CXCR4, such as BL8040; inhibitors of CXCR4/E-selectin, such as GMI1359; inhibitors of CXCR6, such as CCX5224; inhibitors of CCR1, such as AZD4818, BAY865047, BMS817399, CCX354, CCX634, CCX9588, CP481715, MLN3701, MLN3897, PS031291, PS375179 and PS386113; inhibitors of CCR2, such as AZD2423, BL2030, BMS741672, CCX140, CCX598, CCX872, CCX915, CNTX6970, INCB3284, INCB3344, INCB8696, JNJ17166864, JNJ27141491, MK0812, OPLCCL2LPM, PF4136309, serocion, STIB0201, STIB0211, STIB0221, STIB0232, STIB0234, TAK202, TPI526; inhibitors of CCR2/CCR5, such as PF04634817, RAP103 and TBR652; inhibitors of CCR2/CCR5/CCR8, such as RAP310; inhibitors of CCR3, such as ASM8, AXP1275, BMS639623, CM101, DPC168, GW766994, GW824575, MT0814, OPLCCL11LPM and QAP642; inhibitors of CCR4, such as AT008, AZD2098, CCX6239, FLX193, FLX475, GBV3019, GSK2239633, IC487892 and poteligeo; inhibitors of CCR5, such as 5P12-RANTES, AZD5672, AZD8566, CMPD167, ESN196, GSK706769, GW873140, HGS004, INCB15050, INCB9471, L872, microbicide, PF232798, PRO140, RAP101, SAR113244, SCH350634, SCH351125, SCH417690, selzentry, TAK779, TBR220, TD0232 and VX286; inhibitors of CCR5/CXCR4, such as AMD887, ND401 and SP01A; inhibitors of CCR6, such as CCX507, CCX9664 and STIB100X; inhibitors of CCR6, such as CCX025, CCX507, CCX807, eut22, MLN3126, POL7085, traficet-EN; inhibitors of CXCR3, such as AMG487, AT010, STIA120X; inhibitors of CXCR4, such as AD114, AD214, ALX0651, ALX40-4C, AMD070, AT007, AT009, BKT170, BMS936564, celixafor, CTCE9908, GBV4086, GSK812397, KRH2731, KRH3140, LY2510924, LY2624587, mozobil, OPLCXCL12LPM, PF06747143, POL6326, Q122, revixil, TG0054, USL311, X4P001 and X4P002; and inhibitors of CXCR7, such as CCX650 and CCX662.

The cytokine receptor agonist may be selected from the group consisting of mRNAs, DNAs or plasmids encoding the genes for IL-2, IL-15, IL-7, IL-10, IL-12, IL-21, IFN$\alpha$ 1-17, IFN$\beta$, IFN$\gamma$, IL-18, IL-27, TNF$\alpha$, GM-CSF, FLT3L and TRAIL and recombinant proteins, such as agonists of IL-2/IL-15 $\beta/\gamma$ receptors, agonists of IL-10 receptor, agonists of IL-12 receptor, agonists of IL-18 receptor, agonists of IL-21 receptor, agonists of IL-7 receptor, agonists of IFN$\alpha/\beta$ receptor, agonists of IFN $\gamma$ receptor, agonists of FLT3 receptor and agonists of TNF$\alpha$ receptor.

Examples for agonists of IL-2/IL-15 $\beta/\gamma$ receptor are recombinant IL-2, recombinant IL-15, ALKS4230, ALT803, APN301, MDNA109, NKTR214, RG7461, RG7813, AM0015, NIZ985, NKTR255, RTX-212, SO-C101, XmAb24306, L19-IL2, THOR-707 and PB101.

In certain embodiments an agonist of IL-2 is as described in WO2019/185705A1. In particular the agonist of IL-2 is in certain embodiments a conjugate comprising an IL-2 protein of SEQ ID NO:1

PTSSSTKKTQ LQLEHLLLDL QMILNGINNY KNPKLTCMLT FKFYMPKKAT
ELKHLQCLEE ELKPLEEVLN LAQSKNFHLR PRDLISNINV IVLELKGSET
TFMCEYADET ATIVEFLNRW ITFSQSIIST LT,

wherein the sulfur of the cysteine at position 37 of SEQ ID NO: 1 is conjugated to a moiety of formula (2)

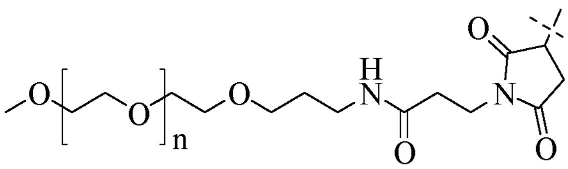 (2),

wherein the dashed line indicates attachment to said sulfur, and
n is about 113 or about 226;
and wherein the nitrogen of the amine of the side chain of any one of the lysine residues, i.e. one of the lysine residues

selected from the group consisting of the lysine residues at position 7, 8, 31, 34, 42, 47, 48, 53, 63, 75 and 96 of SEQ ID NO:1, is conjugated to a moiety of formula (3)

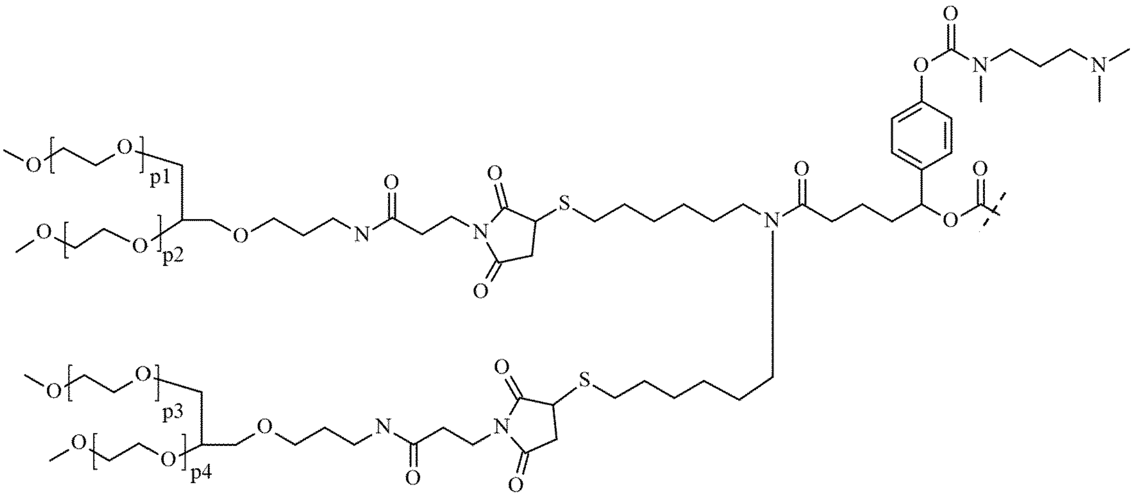

(3),

wherein the dashed line indicates attachment to said nitrogen of the side chain of said lysine residue; and p1, p2, p3 and p4 are independently an integer ranging from 200 to 250.

In certain embodiments the sequence of the IL-2 protein varies by at least one amino acid from the sequence of SEQ ID NO: 1, such as by one amino acid, by two amino acids, by three amino acids, by four amino acids or by five amino acids.

In certain embodiments n of formula (2) is 113. In certain embodiments n of formula (2) is 226.

In certain embodiments p1, p2, p3 and p4 are independently an integer ranging from 220 to 240. In certain embodiments p1, p2, p3 and p4 are the same integer.

The cytokine receptor agonist may be selected from the group consisting of mRNAs, DNAs or plasmids encoding the genes for IL-2, IL-15, IL-7, IL-10, IL-12, IL-21, IFNα 1-17, IFNβ, IFNγ, IL-18, IL-27, TNFα, GM-CSF and TRAIL and recombinant proteins, such as agonists of IL-2/IL-15 β/γ receptors, agonists of IL-10 receptor, agonists of IL-12 receptor, agonists of IL-18 receptor, agonists of IL-21 receptor, agonists of IL-7 receptor, and agonists of TNFα receptor.

Examples for agonists of IL-2/IL-15 β/γ receptor are recombinant IL-2, recombinant IL-15, ALKS4230, ALT803, APN301, MDNA109, NKTR214, RG7461, RG7813, AM0015, NIZ985, NKTR255, RTX-212, SO-C101, XmAb24306, L19-IL2 and PB101.

Examples for agonists of IL-10 receptor are AG011, dekavil, EG10, IL10Nanocap, Ilodecakin, AM0010, tenovil and VT310 VIRON.

Examples for agonists of IL-12 receptor are AM0012, AS1409, dodekin, HemaMax, LipoVIL12, MSB0010360N and NHS-IL12.

An example for an agonist of IL-18 receptor is SB485232.

An example for an agonist of IL-21 receptor is BMS982470 (denenicokin).

Examples for agonists of IL-7 receptor are CYT107, CYT99007 and GX-I7.

Examples for agonist of TNFα receptor are L19-TNFα, aurimune, beromun, BreMel/TNFα, fibromun, refnot and TNFPEG20.

The death receptor agonists may be selected from the group consisting of TRAILR1/DR4 agonists, such as AMG951 (dulanermin), APG350, APG880, HGSETR1 (mapatumumab) and SL231; and TRAILR2/DR5 agonists, such as AMG655, DS8273, HGSETR2 (lexatumumab), HGSTR2J, IDD004/GEN1029, INBRX109, LBY135, MEDI3039, PRO95780, RG7386 and TAS266.

The CD47 antagonists may be selected from the group consisting of ALX148, CC-90002, Hu5F9G4, SRF231, TI061, TTI-621, TTI-622, AO176, IBI188, IMC002 and LYN00301.

An example for a SIRPα antagonist is FSI89.

Examples for oncolytic drugs are CAVATAK, BCG, mobilan, TG4010, Pexa-Vec (JX-594), JX-900, JX-929 and JX-970.

Examples for signal converter proteins are Fn14-TRAIL (KAHR101), CTLA4-FasL (KAHR102), PD1-41BBL (DSP 105), PD1-CD70 (DSP 106) and SIRPα-41BBL (DSP 107).

The epigenetic modifiers may be selected from the group consisting of DNA methyltransferase inhibitors, lysine-specific demethylase 1 inhibitors, Zeste homolog 2 inhibitors, bromodomain and extra-terminal motif (BET) protein inhibitors such as GSK525762, and histone deacetylase (HDAC) inhibitors such as beleodaq, SNDX275 and CKD-M808.

Examples for tumor peptides/vaccines are NY-ESO, WT1, MART-1, IO102 and PF-06753512.

Examples for heat shock protein (HSP) inhibitors are inhibitors of HSP90, such as PF-04929113 (SNX-5422).

Examples of proteolytic enzymes are recombinant hyaluronidase, such as rHuPH20 and PEGPH20.

The ubiquitin and proteasome inhibitors may be selected from the group consisting of ubiquitin-specific protease (USP) inhibitors, such as P005091; 20S proteasome inhibitors, such as bortezimib, carfilzomib, ixazomib, oprozomib, delanzomib and celastrol; and immunoproteasome inhibitors, such as ONX-0914.

The adhesion molecule antagonists may be selected from the group consisting of β2-integrin antagonists, such as imprime PGG; and selectin antagonists.

The hormones may be selected from the group consisting of hormone receptor agonists and hormone receptor antagonists.

An example for a hormone receptor agonist are somatostatin receptor agonists, such as somatostatin, lanreotide, octreotide, FX125L, FX141L and FX87L.

Example for hormone receptor antagonists are anti-androgens, anti-estrogens and anti-progestogens. Examples for anti-androgens are steroidal antiandrogens, such as cyproterone acetate, megestrol acetate, chlormadinone acetate, spironolactone, oxendolone and osaterone acetate; nonsteroidal anti-androgens, such as flutamide, bicalutamide, nilutamide, topilutamide, enzalutamide and apalutamide; androgen synthesis inhibitors, such as ketoconazole, abiraterone acetate, seviteronel, aminoglutethimide, finasteride, dutasteride, epristeride and alfatradiol. Examples for anti-estrogens are selective estrogen receptor modulators (SERMs), such as tamoxifen, clomifene, Fareston and raloxifene; ER silent antagonists and selective estrogen receptor degrader (SERD), such as fulvestrant; aromatase inhibitors, such as anastrozole, letrozole, exemestane, vorozole, formestane and fadrozole; and anti-gonadotropins, such as testosterone, progestogens and GnRH analogues. Examples for anti-progestogens are mifepristone, lilopristone and onapristone.

In certain embodiments the water-insoluble controlled-released PRRA releases resiquimod and the one or more additional drug of the pharmaceutical composition is nivolumab. In certain embodiments the water-insoluble controlled-released PRRA releases resiquimod and the one or more additional drug is pembrolizumab. In certain embodiments the water-insoluble controlled-released PRRA releases resiquimod and the one or more additional drug is atezolizumab. In certain embodiments the water-insoluble controlled-released PRRA releases resiquimod and the one or more additional drug is avelumab. In certain embodiments the water-insoluble controlled-released PRRA releases resiquimod and the one or more additional drug is durvalumab. In certain embodiments the water-insoluble controlled-released PRRA releases resiquimod and the one or more additional drug is ipilimumab. In certain embodiments the water-insoluble controlled-released PRRA releases resiquimod and the one or more additional drug is tremelimumab. In certain embodiments the water-insoluble controlled-released PRRA releases resiquimod and the one or more additional drug is trastuzumab. In certain embodiments the water-insoluble controlled-released PRRA releases resiquimod and the one or more additional drug is cetuximab. In certain embodiments the water-insoluble controlled-released PRRA releases resiquimod and the one or more additional drug is margetuximab. In certain embodiments the water-insoluble controlled-released PRRA releases resiquimod and the one or more additional drug is one of the CD47 or SIRPα blockers described above.

In certain embodiments the treatment with the water-insoluble PRRA may be initiated prior to, concomitant with, or following surgical removal of a tumor or radiation therapy. In addition, such treatment may optionally be combined with at least one other cancer therapeutic, such as systemic immunotherapy. Examples for the at least one cancer therapeutic are as provided elsewhere herein. In certain embodiments the water-insoluble PRRA is administered intratumorally prior to, concomitant with, or following combination with at least one systemic immunotherapy, prior to radiation therapy or surgical removal of the injected tumor. In certain embodiments the water-insoluble PRRA is administered intratumorally prior to, concomitant with, or following combination with at least one systemic immunotherapy, following radiation therapy or surgical removal of a tumor. In certain embodiments the water-insoluble PRRA is administered into tumor draining lymph nodes prior to, concomitant with, or following surgical removal of a tumor or radiation therapy. In certain embodiments the water-insoluble PRRA is administered into tumor draining lymph nodes prior to, concomitant with, or following combination with at least one systemic immunotherapy, and prior to, concomitant with, or following surgical removal of a tumor or radiation therapy. In certain embodiments the water-insoluble PRRA is administered intratumorally into metastatic tumors that may arise prior to or following surgical removal or radiation therapy of primary tumor. In certain embodiments the water-insoluble PRRA is administered intratumorally into metastatic tumors that may arise prior to, concomitant with, or following combination with at least one systemic immunotherapy, and prior to, concomitant with, or following surgical removal or radiation therapy of primary tumor. In certain embodiments at least one systemic therapy is administered prior to surgical removal of a tumor or radiation therapy, followed by intratumoral administration of the water-insoluble PRRA. In certain embodiments intratumoral administration of the water-insoluble PRRA is administered first, followed by subsequent treatment in combination with at least one systemic therapy. In certain embodiments at least one systemic therapy is administered prior to surgical removal of a tumor, followed by administration of the water-insoluble PRRA to the tumor

bed following surgery or by intratumoral administration in tumor not removed by surgery.

In certain embodiments intra-tumoral administration is a single injection of the water-insoluble controlled-release PRRA into a tissue as described above. In certain embodiments intra-tumoral administration is via repeated intra-tumoral administration. In certain embodiments such repeated intra-tumoral administration is into the same tumor and may be at the same or a different administration site within said tumor. In certain embodiments the repeated intra-tumor administration may be into different tumors. In case of repeated intra-tumoral administration, the time interval between two intra-tumoral administrations may range from 1 minute to 28 weeks.

The tissue into which the water-insoluble controlled-release PRRA is administered is cancer tissue. Suitable cancers may be selected from the group consisting of solid tumors and lymphomas.

The cancer may be selected from the group consisting of lip and oral cavity cancer, oral cancer, liver cancer/hepatocellular cancer, primary liver cancer, lung cancer, lymphoma, malignant mesothelioma, malignant thymoma, skin cancer, intraocular melanoma, metastasic squamous neck cancer with occult primary, childhood multiple endocrine neoplasia syndrome, mycosis fungoides, nasal cavity and paranasal sinus cancer, nasopharyngeal cancer, neuroblastoma, oropharyngeal cancer, ovarian cancer, pancreatic cancer, parathyroid cancer, pheochromocytoma, pituitary tumor, adrenocortical carcinoma, AIDS-related malignancies, anal cancer, bile duct cancer, bladder cancer, brain and nervous system cancer, breast cancer, bronchial adenoma/carcinoid, gastrointestinal carcinoid tumor, carcinoma, colorectal cancer, endometrial cancer, esophageal cancer, extracranial germ cell tumor, extragonadal germ cell tumor, extrahepatic bile duct cancer, gallbladder cancer, gastric (stomach) cancer, gestational trophoblastic tumor, head and neck cancer, hypopharyngeal cancer, islet cell carcinoma (endocrine pancreas), kidney cancer/renal cell cancer, laryngeal cancer, pleuropulmonary blastoma, prostate cancer, transitional cell cancer of the renal pelvis and ureter, retinoblastoma, salivary gland cancer, sarcoma, Sezary syndrome, small intestine cancer, genitourinary cancer, malignant thymoma, thyroid cancer, Wilms' tumor and cholangiocarcinoma.

In certain embodiments the cancer is a liver cancer/hepatocellular cancer. In certain embodiments the cancer is a lung cancer. In certain embodiments the cancer is a lymphoma. In certain embodiments the cancer is a malignant thymoma. In certain embodiments the cancer is a skin cancer. In certain embodiments the cancer is a is a metastasic squamous neck cancer with occult primary. In certain embodiments the cancer is a neuroblastoma. In certain embodiments the cancer is an ovarian cancer. In certain embodiments the cancer is a pancreatic cancer. In certain embodiments the cancer is a bile duct cancer. In certain embodiments the cancer is a bladder cancer. In certain embodiments the cancer is a brain and nervous system cancer. In certain embodiments the cancer is a breast cancer. In certain embodiments the cancer is a gastrointestinal carcinoid tumor. In certain embodiments the cancer is a carcinoma. In certain embodiments the cancer is a colorectal cancer. In certain embodiments the cancer is an extrahepatic bile duct cancer. In certain embodiments the cancer is a gallbladder cancer. In certain embodiments the cancer is a gastric (stomach) cancer. In certain embodiments the cancer is a head and neck cancer. In certain embodiments the cancer is a kidney cancer/renal cell cancer. In certain embodiments the cancer is a prostate cancer. In certain embodiments the cancer is a sarcoma. In certain embodiments the cancer is a small intestine cancer. In certain embodiments the cancer is a genitourinary cancer.

Examples for lung cancer are non-small cell lung cancer and small cell lung cancer. In certain embodiments the cancer is a non-small cell lung cancer. In certain embodiment the cancer is a small cell lung cancer.

Example for lymphomas are AIDS-related lymphoma, primary central nervous system lymphoma, T-cell lymphoma, cutaneous T-cell lymphoma, Hodgkin's lymphoma, Hodgkin's lymphoma during pregnancy, non-Hodgkin's lymphoma, follicular lymphoma, marginal zone lymphoma, diffuse large B-cell lymphoma, non-Hodgkin's lymphoma during pregnancy and angioimmunoblastic lymphoma. In certain embodiments the cancer is a cutaneous T-cell lymphoma.

Examples for skin cancer are melanoma and Merkel cell carcinoma. In certain embodiments the cancer is a skin cancer. In certain embodiments the cancer is a Merkel cell carcinoma.

An ovarian cancer may for example be an epithelial cancer, a germ cell tumor or a low malignant potential tumor. In certain embodiments the cancer is an epithelial cancer. In certain embodiments the cancer is a germ cell tumor. In certain embodiments the cancer is a low malignant potential tumor.

A pancreatic cancer may for example be an exocrine tumor/adenocarcinoma, pancreatic endocrine tumor (PET) or neuroendocrine tumor (NET). In certain embodiments the cancer is an exocrine tumor/adenocarcinoma. In certain embodiments the tumor is a pancreatic endocrine tumor. In certain embodiments the cancer is a neuroendocrine tumor.

A brain and nervous system cancer may be for example be a medulloblastoma, such as a childhood medulloblastoma, astrocytoma, ependymoma, neuroectodermal tumors, schwannoma, meningioma, pituitary adenoma and glioma. In certain embodiment the cancer is a medullablastoma. In certain embodiments the cancer is a childhood medullablastoma. In certain embodiments the cancer is an astrocytoma. In certain embodiments the cancer is an ependymoma. In certain embodiments the cancer is a neuroectodermal tumor. In certain embodiments the tumor is a schwannoma. In certain embodiments the cancer is a meningioma. In certain embodiments the cancer is a pituitary adenoma. In certain embodiments the cancer is a glioma.

An astrocytoma may be selected from the group consisting of giant cell glioblastoma, glioblastoma, secondary

glioblastoma, primary adult glioblastoma, primary pediatric glioblastoma, oligodendroglial tumor, oligodendroglioma, anaplastic oligodendroglioma, oligoastrocytic tumor, oligoastrocytoma, anaplastic oligodendroglioma, oligoastrocytic tumor, oligoastrocytoma, anaplastic oligoastrocytoma, anaplastic astrocytoma, pilocytic astrocytoma, subependymal giant-cell astrocytoma, diffuse astrocytoma, pleomorphic xanthoastrocytoma and cerebellar astrocytoma.

Examples for an neuroectodermal tumor are a pineal primitive neuroectodermal tumor and a supratentorial primitive neuroectodermal tumor.

An ependymoma may be selected from the group consisting of subependymoma, ependymoma, myxopapillary ependymoma and anaplastic ependymoma.

A meningioma may be an atypical meningioma or an anaplastic meningioma.

A glioma may be selected from the group consisting of glioblastoma multiforme, paraganglioma, suprantentorial primordial neuroectodermal tumor (sPNET), brain stem glioma, childhood brain stem glioma, hypothalamic and visual pathway glioma, childhood hypothalamic and visual pathway glioma and malignant glioma.

Examples for breast cancer are breast cancer during pregnancy, triple negative breast cancer, ductal carcinoma in situ (DCIS), invasive ductal carcinoma (IDC), tubular carcinoma of the breast, medullary carcinoma of the breast, mucinous carcinoma of the breast, papillary carcinoma of the breast, cribriform carcinoma of the breast, invasive lobular carcinoma (ILC), inflammatory breast cancer, lobular carcinoma in situ (LCIS), male breast cancer, Paget's disease of the nipple, phyllodes tumors of the breast and metastasic breast cancer. In certain embodiments the cancer is a breast cancer during pregnancy. In certain embodiments the cancer is a triple negative breast cancer. In certain embodiments the cancer is a ductal carcinoma in situ. In certain embodiments the cancer is an invasive ductal carcinoma. In certain embodiments the cancer is a tubular carcinoma of the breast. In certain embodiments the cancer is a medullary carcinoma of the breast. In certain embodiments the cancer is a mucinous carcinoma of the breast. In certain embodiments the cancer is a papillary carcinoma of the breast. In certain embodiments the cancer is a cribriform carcinoma of the breast. In certain embodiments the cancer is an invasive lobular carcinoma. In certain embodiments the cancer is an inflammatory breast cancer. In certain embodiments the cancer is a lobular carcinoma in situ. In certain embodiments the cancer is a male breast cancer. In certain embodiments the cancer is a Paget's disease of the nipple. In certain embodiments the cancer is a phyllodes tumor of the breast. In certain embodiments the cancer is a metastatic breast cancer.

Examples for a carcinoma are neuroendocrine carcinoma, adrenocortical carcinoma and Islet cell carcinoma. In certain embodiments the cancer is a neuroendocrine carcinoma. In certain embodiments the cancer is an adrenocortical carcinoma. In certain embodiments the cancer is an Islet cell carcinoma.

Examples for a colorectal cancer are colon cancer and rectal cancer. In certain embodiments the cancer is a colon cancer. In certain embodiments the cancer is a rectal cancer.

A sarcoma may be selected from the group consisting of Kaposi's sarcoma, osteosarcoma/malignant fibrous histiocytoma of bone, soft tissue sarcoma, Ewing's family of tumors/sarcomas, rhabdomyosarcoma, clear cell sarcoma of tendon sheaths, central chondrosarcoma, central and periosteal chondroma, fibrosarcoma and uterine sarcoma. In certain embodiments the cancer may be a Kaposi's sarcoma. In certain embodiments the cancer may be an osteosarcoma/malignant fibrous histiocytoma of bone. In certain embodiments the cancer may be a soft tissue sarcoma. In certain embodiments the cancer may be an Ewing's family of tumors/sarcomas. In certain embodiments the cancer may be a rhabdomyosarcoma. In certain embodiments the cancer may be a clear cell sarcoma of tendon sheaths. In certain embodiments the cancer may be a central chondrosarcoma. In certain embodiments the cancer may be a central and periosteal chondroma. In certain embodiments the cancer may be a fibrosarcoma. In certain embodiments the cancer may be a uterine sarcoma.

Examples for a genitourinary cancer are testicular cancer, urethral cancer, vaginal cancer, cervical cancer, penile cancer and vulvar cancer. In certain embodiments the cancer may be a testicular cancer. In certain embodiments the cancer may be a urethral cancer. In certain embodiments the cancer may be a vaginal cancer. In certain embodiments the cancer may be a cervical cancer. In certain embodiments the cancer may be a penile cancer. In certain embodiments the cancer may be a vaginal cancer.

The water-insoluble controlled-release PRRA releases resiquimod.

In certain embodiments PRRA is released from the water-insoluble controlled-release PRRA with a release half-life under physiological conditions (aqueous buffer, pH 7.4, 37°C) of at least 3 days, such as at least 4 days, at least 5 days, at least 6 days, at least 7 days, at least 8 days, at least 9 days, at least 10 days, at least 12 days, at least 15 days, at least 17 days, at least 20 days or at least 25 days.

The carrier is a hydrogel. The one PRRA is covalently and reversibly conjugated to such hydrogel carrier. Such hydrogel may be degradable or may be non-degradable, i.e. stable. In certain embodiments such hydrogel is degradable. In certain embodiments such hydrogel is non-degradable.

In certain embodiments the hydrogel carrier is a PEG- or hyaluronic acid-based hydrogel. In certain embodiments hydrogel carrier is a PEG-based hydrogel. Such PEG-based hydrogel may be degradable or may be non-degradable, i.e. stable. In certain embodiments such PEG-based hydrogel is degradable. In certain embodiments such PEG-based hydrogel is non-degradable. In certain embodiments the hydrogel carrier is a hyaluronic acid-based hydrogel.

Such hyaluronic acid-based hydrogel may be degradable or may be non-degradable, i.e. stable. In certain embodiments such hyaluronic acid-based hydrogel is degradable. In certain embodiments such hyaluronic acid-based hydrogel is non-degradable.

In certain embodiments the water-insoluble controlled-release PRRA is a conjugate, wherein said conjugate is water-insoluble and comprises a carrier moiety Z to which one or more moieties $-L^2-L^1-D$ are conjugated, wherein

each $-L^2-$ is individually a chemical bond or a spacer moiety;
each $-L^1-$ is individually a linker moiety to which -D is reversibly and covalently conjugated; and
each -D is individually a pattern recognition receptor agonist.

In certain embodiments the water-insoluble controlled-release PRRA is a conjugate, wherein said conjugate is water-insoluble and comprises a carrier moiety Z to which one or more moieties $-L^2-L^1-D-L^1-L^2-$ are conjugated, wherein

each $-L^2-$ is individually a chemical bond or a spacer moiety and conjugated to Z;
each $-L^1-$ is individually a linker moiety to which -D is reversibly and covalently conjugated; and
each -D is individually a pattern recognition receptor agonist.

It is understood that in this embodiment a moiety $-L^2-L^1-D-L^1-L^2-$ is attached at both of its ends to Z.

The one or more moieties $-L^2-L^1-D$ are covalently conjugated to Z. In certain embodiments the one or more moieties $-L^2-L^1-D$ are stably conjugated to Z, i.e. the linkage between Z and $-L^2-$ is a stable linkage. As Z is a hydrogel it is understood that the number of moieties $-L^2-L^1-D$ conjugated to such hydrogel carrier is too large to specify.

-D is resiquimod.

In certain embodiments $-L^1-$ is conjugated to -D via a hydroxyl group of -D.

In certain embodiments $-L^1-$ is conjugated to -D via a primary amine group of -D.

In certain embodiments $-L^1-$ is conjugated to -D via an amidine group of -D.

$-L^1-$ is in certain embodiments conjugated to -D via its aromatic amine, i.e. the amine functional group marked with the asterisk

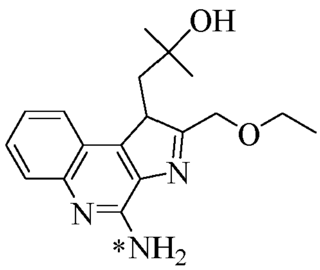

In certain embodiments cleavage of the linkage between -D and $-L^1-$ occurs with a release half-life under physiological conditions (aqueous buffer, pH 7.4, 37°C) of at least 3 days, such as at least 4 days, at least 5 days, at least 6 days, at least 7 days, at least 8 days, at least 9 days, at least 10 days, at least 12 days, at least 15 days, at least 17 days, at least 20 days or at least 25 days.

In certain embodiments $-L^1-$ is connected to -D through an ester linkage.

In certain embodiments $-L^1-$ is connected to -D through a carbonate linkage.

In certain embodiments $-L^1-$ is connected to -D through an acylamidine linkage.

In certain embodiments $-L^1-$ is connected to -D through a carbamate linkage.

In certain embodiments $-L^1-$ is connected to -D through an amide linkage.

It is understood that some of these linkages may not be reversible *per se,* but that in the present invention neighboring groups present in $-L^1-$ render these linkages reversible.

With -D being resiquimod, the linkage between -D and $-L^1-$ is in certain embodiments through an amide linkage, in which the aromatic amine functional group of -D forms an amide linkage with a carbonyl (-(C=O)-) of $-L^1-$

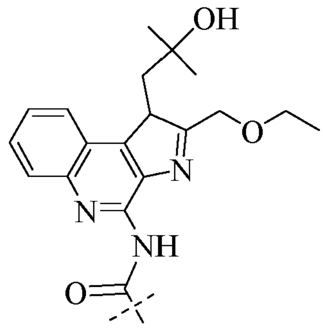

,

wherein the dashed line indicates attachment to the remainder of $-L^1-$.

The moiety $-L^1-$ is a linker moiety from which -D is released in its free form, i.e. generally in the form of D-H or D-OH. Such moieties are also known as "prodrug linkers" or "reversible prodrug linkers" and are known in the art, such as for example the reversible linker moieties disclosed in WO 2005/099768 A2, WO 2006/136586 A2, WO 2011/089216 A1, WO 2013/024053 A1, WO 2011/012722 A1, WO 2011/089214 A1, WO 2011/089215 A1, WO 2013/024052 A1 and WO 2013/160340 A1.

In one embodiment $-L^1-$ has a structure as disclosed in WO 2009/095479 A2. Accordingly, in certain embodiments the moiety $-L^1-$ is of formula (II):

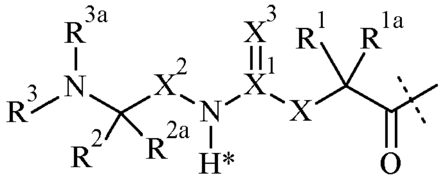 (II)

,

wherein the dashed line indicates attachment to a nitrogen of -D by forming an amide bond;
-X- is $-C(R^4R^{4a})-$; $-N(R^4)-$; -O-; $-C(R^4R^{4a})-C(R^5R^{5a})-$; $-C(R^5R^{5a})-C(R^4R^{4a})-$; $-C(R^4R^{4a})-N(R^6)-$; $-N(R^6)-C(R^4R^{4a})-$; $-C(R^4R^{4a})-O-$; $-O-C(R^4R^{4a})-$; or $-C(R^7R^{7a})-$;
$X^1$ is C; or S(O);
$-X^2-$ is $-C(R^8R^{8a})-$; or $-C(R^8R^{8a})-C(R^9R^{9a})-$;
$=X^3$ is =O; =S; or =N-CN;
$-R^1$, $-R^{1a}$, $-R^2$, $-R^{2a}$, $-R^4$, $-R^{4a}$, $-R^5$, $-R^{5a}$, $-R^6$, $-R^8$, $-R^{8a}$, $-R^9$, $-R^{9a}$ are independently selected from the group consisting of -H; and $C_{1-6}$ alkyl;
$-R^3$, $-R^{3a}$ are independently selected from the group consisting of -H; and $C_{1-6}$ alkyl, provided that in case one of $-R^3$, $-R^{3a}$ or both are other than -H they are connected to N to which they are attached through an $sp^3$-hybridized carbon atom;
$-R^7$ is $-N(R^{10}R^{10a})$; or $-NR^{10}-(C=O)-R^{11}$;
$-R^{7a}$, $-R^{10}$, $-R^{10a}$, $-R^{11}$ are independently of each other -H; or $C_{1-6}$ alkyl;
optionally, one or more of the pairs $-R^{1a}/-R^{4a}$, $-R^{1a}/-R^{5a}$, $-R^{1a}/-R^{7a}$, $-R^{4a}/-R^{5a}$, $-R^{8a}/-R^{9a}$ form a chemical bond;
optionally, one or more of the pairs $-R^1/-R^{1a}$, $-R^2/-R^{2a}$, $-R^4/-R^{4a}$, $-R^5/-R^{5a}$, $-R^8/-R^{8a}$, $-R^9/-R^{9a}$ are joined together with the atom to which they are attached to form a $C_{3-10}$ cycloalkyl; or 3- to 10-membered heterocyclyl;
optionally, one or more of the pairs $-R^1/-R^4$, $-R^1/-R^5$, $-R^1/-R^6$, $-R^1/-R^{7a}$, $-R^4/-R^5$, $-R^4/-R^6$, $-R^8/-R^9$, $-R^2/-R^3$ are joined together with the atoms to which they are attached to form a ring A;
optionally, $R^3/R^{3a}$ are joined together with the nitrogen atom to which they are attached to form a 3- to 10-membered heterocycle;
A is selected from the group consisting of phenyl; naphthyl; indenyl; indanyl; tetralinyl; $C_{3-10}$ cycloalkyl; 3- to 10-membered heterocyclyl; and 8- to 11-membered heterobicyclyl; and
wherein $-L^1-$ is substituted with at least one $-L^2-$ and wherein $-L^1-$ is optionally further substituted, provided that the hydrogen marked with the asterisk in formula (II) is not replaced by $-L^2-$ or a substituent.

Preferably $-L^1-$ of formula (II) is substituted with one moiety $-L^2-$.

In one embodiment $-L^1-$ of formula (II) is not further substituted.

It is understood that if $-R^3/-R^{3a}$ of formula (II) are joined together with the nitrogen atom to which they are attached to form a 3- to 10-membered heterocycle, only such 3- to 10-membered heterocycles may be formed in which the atoms

directly attached to the nitrogen are $sp^3$-hybridized carbon atoms. In other words, such 3- to 10-membered heterocycle formed by $-R^3/-R^{3a}$ together with the nitrogen atom to which they are attached has the following structure:

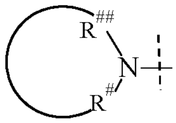
,

wherein

the dashed line indicates attachment to the rest of $-L^1-$;
the ring comprises 3 to 10 atoms comprising at least one nitrogen; and
$R^{\#}$ and $R^{\#\#}$ represent an $sp^3$-hydridized carbon atom.

It is also understood that the 3- to 10-membered heterocycle may be further substituted.
Exemplary embodiments of suitable 3- to 10-membered heterocycles formed by $-R^3/-R^{3a}$ of formula (II) together with the nitrogen atom to which they are attached are the following:

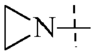
, 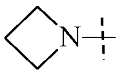
, 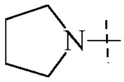
, 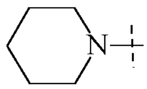
,

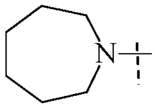
, 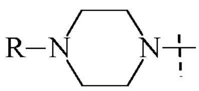
and 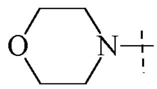
,

wherein

dashed lines indicate attachment to the rest of the molecule; and
-R is selected from the group consisting of -H and $C_{1-6}$ alkyl.

$-L^1-$ of formula (II) may optionally be further substituted. In general, any substituent may be used as far as the cleavage principle is not affected, i.e. the hydrogen marked with the asterisk in formula (II) is not replaced and the nitrogen of the moiety

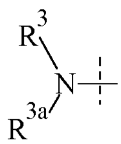

of formula (II) remains part of a primary, secondary or tertiary amine, i.e. $-R^3$ and $-R^{3a}$ are independently of each other -H or are connected to -N< through an $SP^3$-hybridized carbon atom.
In one embodiment $-R^1$ or $-R^{1a}$ of formula (II) is substituted with $-L^2-$. In another embodiment $-R^2$ or $-R^{2a}$ of formula (II) is substituted with $-L^2-$. In another embodiment $-R^3$ or $-R^{3a}$ of formula (II) is substituted with $-L^2-$. In another embodiment $-R^4$ of formula (II) is substituted with $-L^2-$. In another embodiment $-R^5$ or $-R^{5a}$ of formula (II) is substituted with $-L^2-$. In another embodiment $-R^6$ of formula (II) is substituted with $-L^2-$. In another embodiment $-R^7$ or $-R^{7a}$ of formula (II) is substituted with $-L^2-$. In another embodiment $-R^8$ or $-R^{8a}$ of formula (II) is substituted with $-L^2-$. In another embodiment $-R^9$ or $-R^{9a}$ of formula (II) is substituted with $-L^2-$. In another embodiment $-R^{10}$ or $-R^{10a}$ of formula (II) is substituted with $-L^2-$. In another embodiment $-R^{11}$ of formula (II) is substituted with $-L^2-$.
In certain embodiments $-L^1-$ has a structure as disclosed in WO2016/020373A1. Accordingly, in certain embodiments the moiety $-L^1-$ is of formula (III):

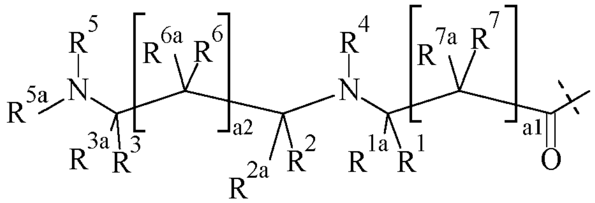

(III),

wherein

the dashed line indicates attachment to a primary or secondary amine or hydroxyl of -D by forming an amide or ester linkage, respectively;
$-R^1$, $-R^{1a}$, $-R^2$, $-R^{2a}$, $-R^3$ and $-R^{3a}$ are independently of each other selected from the group consisting of -H, $-C(R^8R^{8a}R^{8b})$, $-C(=O)R^8$, -C=N, $-C(=NR^8)R^{8a}$, $-CR^8(=CR^{8a}R^{8b})$, $-C\equiv CR^8$ and -T;
$-R^4$, $-R^5$ and $-R^{5a}$ are independently of each other selected from the group consisting of -H, $-C(R^9R^{9a}R^{9b})$ and -T;
a1 and a2 are independently of each other 0 or 1;
each $-R^6$, $-R^{6a}$, $-R^7$, $-R^{7a}$, $-R^8$, $-R^{8a}$, $-R^{8b}$, $-R^9$, $-R^{9a}$, $-R^{9b}$ are independently of each other selected from the group consisting of -H, halogen, -CN, $-COOR^{10}$, $-OR^{10}$, $-C(O)R^{10}$, $-C(O)N(R^{10}R^{10a})$, $-S(O)_2N(R^{10}R^{10a})$, $-S(O)N(R^{10}R^{10a})$, $-S(O)_2R^{10}$, $-S(O)R^{10}$, $-N(R^{10})S(O)_2N(R^{10a}R^{10b})$, $-SR^{10}$, $-N(R^{10}R^{10a})$, $-NO_2$, $-OC(O)R^{10}$, $-N(R^{10})C(O)R^{10a}$, $-N(R^{10})S(O)_2R^{10a}$, $-N(R^{10})S(O)R^{10a}$, $-N(R^{10})C(O)OR^{10a}$, $-N(R^{10})C(O)N(R^{10a}R^{10b})$, $-OC(O)N(R^{10}R^{10a})$, -T, $C_{1-20}$ alkyl, $C_{2-20}$ alkenyl, and $C_{2-20}$ alkynyl; wherein -T, $C_{1-20}$ alkyl, $C_{2-20}$ alkenyl, and $C_{2-20}$ alkynyl are optionally substituted with one or more $-R^{11}$, which are the same or different and wherein $C_{1-20}$ alkyl, $C_{2-20}$ alkenyl, and $C_{2-20}$ alkynyl are optionally interrupted by one or more groups selected from the group consisting of -T-, -C(O)O-, -O-, -C(O)-, $-C(O)N(R^{12})-$, $-S(O)_2N(R^{12})-$, $-S(O)N(R^{12})-$, $-S(O)_2-$, -S(O)-, $-N(R^{12})S(O)_2N(R^{12a})-$, -S-, $-N(R^{12})-$, $-OC(OR^{12})(R^{12a})-$, $-N(R^{12})C(O)N(R^{12a})-$, and $-OC(O)N(R^{12})-$;
each $-R^{10}$, $-R^{10a}$, $-R^{10b}$ is independently selected from the group consisting of -H, -T, $C_{1-20}$ alkyl, $C_{2-20}$ alkenyl, and $C_{2-20}$ alkynyl; wherein -T, $C_{1-20}$ alkyl, $C_{2-20}$ alkenyl, and $C_{2-20}$ alkynyl are optionally substituted with one or more $-R^{11}$, which are the same or different and wherein $C_{1-20}$ alkyl, $C_{2-20}$ alkenyl, and $C_{2-20}$ alkynyl are optionally interrupted by one or more groups selected from the group consisting of -T-, -C(O)O-, -O-, -C(O)-, $-C(O)N(R^{12})-$, $-S(O)_2N(R^{12})-$, $-S(O)N(R^{12})-$, $-S(O)_2-$, -S(O)-, $-N(R^{12})S(O)_2N(R^{12a})-$, -S-, $-N(R^{12})-$, $-OC(OR^{12})(R^{12a})-$, $-N(R^{12})C(O)N(R^{12a})-$, and $-OC(O)N(R^{12})-$;
each T is independently of each other selected from the group consisting of phenyl, naphthyl, indenyl, indanyl, tetralinyl, $C_{3-10}$ cycloalkyl, 3- to 10-membered heterocyclyl, and 8- to 11-membered heterobicyclyl; wherein each T is independently optionally substituted with one or more $-R^{11}$, which are the same or different;
each $-R^{11}$ is independently of each other selected from halogen, -CN, oxo (=O), $-COOR^{13}$, $-OR^{13}$, $-C(O)R^{13}$, $-C(O)N(R^{13}R^{13a})$, $-S(O)_2N(R^{13}R^{13a})$, $-S(O)N(R^{13}R^{13a})$, $-S(O)_2R^{13}$, $-S(O)R^{13}$, $-N(R^{13})S(O)_2N(R^{13a}R^{13b})$, $-SR^{13}$, $-N(R^{13}R^{13a})$, $-NO_2$, $-OC(O)R^{13}$, $-N(R^{13})C(O)R^{13a}$, $-N(R^{13})S(O)_2R^{13a}$, $-N(R^{13})S(O)R^{13a}$, $-N(R^{13})C(O)OR^{13a}$, $-N(R^{13})C(O)N(R^{13a}R^{13b})$, $-OC(O)N(R^{13}R^{13a})$, and $C_{1-6}$ alkyl; wherein $C_{1-6}$ alkyl is optionally substituted with one or more halogen, which are the same or different;
each $-R^{12}$, $-R^{12a}$, $-R^{13}$, $-R^{13a}$, $-R^{136}$ is independently selected from the group consisting of -H, and $C_{1-6}$ alkyl; wherein $C_{1-6}$ alkyl is optionally substituted with one or more halogen, which are the same or different;
optionally, one or more of the pairs $-R^1/-R^{1a}$, $-R^2/-R^{2a}$, $-R^3/-R^{3a}$, $-R^6/-R^{6a}$, $-R^7/-R^{7a}$ are joined together with the atom to which they are attached to form a $C_{3-10}$ cycloalkyl or a 3- to 10-membered heterocyclyl;
optionally, one or more of the pairs $-R^1/-R^2$, $-R^1/-R^3$, $-R^1/-R^4$, $-R^1/-R^5$, $-R^1/-R^6$, $-R^1/-R^7$, $-R^2/-R^3$, $-R^2/-R^4$, $-R^2/-R^5$, $-R^2/-R^6$, $-R^2/-R^7$, $-R^3/-R^4$, $-R^3/-R^5$, $-R^3/-R^6$, $-R^3/-R^7$, $-R^4/-R^5$, $-R^4/-R^6$, $-R^4/-R^7$, $-R^5/-R^6$, $-R^5/-R^7$, $-R^6/-R^7$ are joint together with the atoms to which they are attached to form a ring A;
A is selected from the group consisting of phenyl; naphthyl; indenyl; indanyl; tetralinyl; $C_{3-10}$ cycloalkyl; 3- to 10-membered heterocyclyl; and 8- to 11-membered heterobicyclyl;
wherein $-L^1-$ is substituted with at least one $-L^2-$ and wherein $-L^1-$ is optionally further substituted.

The optional further substituents of $-L^1-$ of formula (III) are preferably as described above.

Preferably $-L^1-$ of formula (III) is substituted with one moiety $-L^2-$.

In one embodiment $-L^1-$ of formula (III) is not further substituted.

In another embodiment $-L^1-$ has a structure as disclosed in EP1536334B1, WO2009/009712A1, WO2008/034122A1, WO2009/143412A2, WO2011/082368A2, and US8618124B2.

In certain embodiments $-L^1-$ has a structure as disclosed in US8946405B2 and US8754190B2. Accordingly, in certain embodiments $-L^1-$ is of formula (IV):

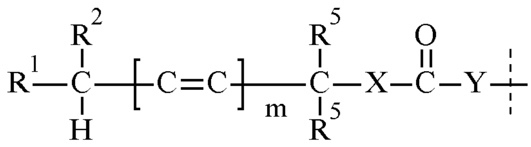

(IV),

wherein

the dashed line indicates attachment to -D through a functional group of -D selected from the group consisting of -OH, -SH and $-NH_2$;
m is 0 or 1;
at least one or both of $-R^1$ and $-R^2$ is/are independently of each other selected from the group consisting of -CN, $-NO_2$, optionally substituted aryl, optionally substituted heteroaryl, optionally substituted alkenyl, optionally substituted alkynyl, $-C(O)R^3$, $-S(O)R^3$, $-S(O)_2R^3$, and $-SR^4$,
one and only one of $-R^1$ and $-R^2$ is selected from the group consisting of -H, optionally substituted alkyl, optionally substituted arylalkyl, and optionally substituted heteroarylalkyl;
$-R^3$ is selected from the group consisting of -H, optionally substituted alkyl, optionally substituted aryl, optionally substituted arylalkyl, optionally substituted heteroaryl, optionally substituted heteroarylalkyl, $-OR^9$ and $-N(R^9)_2$;
$-R^4$ is selected from the group consisting of optionally substituted alkyl, optionally substituted aryl, optionally substituted arylalkyl, optionally substituted heteroaryl, and optionally substituted heteroarylalkyl;
each $-R^5$ is independently selected from the group consisting of -H, optionally substituted alkyl, optionally substituted alkenylalkyl, optionally substituted alkynylalkyl, optionally substituted aryl, optionally substituted arylalkyl, optionally substituted heteroaryl and optionally substituted heteroarylalkyl;
$-R^9$ is selected from the group consisting of -H and optionally substituted alkyl;
-Y- is absent and -X- is -O- or -S-; or
-Y- is $-N(Q)CH_2-$ and -X- is -O-;
Q is selected from the group consisting of optionally substituted alkyl, optionally substituted aryl, optionally substituted arylalkyl, optionally substituted heteroaryl and optionally substituted heteroarylalkyl;
optionally, $-R^1$ and $-R^2$ may be joined to form a 3 to 8-membered ring; and
optionally, both $-R^9$ together with the nitrogen to which they are attached form a heterocyclic ring;
wherein $-L^1-$ is substituted with at least one $-L^2-$ and wherein $-L^1-$ is optionally further substituted.

Only in the context of formula (IV) the terms used have the following meaning:
The term "alkyl" as used herein includes linear, branched or cyclic saturated hydrocarbon groups of 1 to 8 carbons, or in some embodiments 1 to 6 or 1 to 4 carbon atoms.

The term "alkoxy" includes alkyl groups bonded to oxygen, including methoxy, ethoxy, isopropoxy, cyclopropoxy, cyclobutoxy, and similar.

The term "alkenyl" includes non-aromatic unsaturated hydrocarbons with carbon-carbon double bonds.

The term "alkynyl" includes non-aromatic unsaturated hydrocarbons with carbon-carbon triple bonds.

The term "aryl" includes aromatic hydrocarbon groups of 6 to 18 carbons, preferably 6 to 10 carbons, including groups such as phenyl, naphthyl, and anthracenyl. The term "heteroaryl" includes aromatic rings comprising 3 to 15 carbons containing at least one N, O or S atom, preferably 3 to 7 carbons containing at least one N, O or S atom, including groups such as pyrrolyl, pyridyl, pyrimidinyl, imidazolyl, oxazolyl, isoxazolyl, thiazolyl, isothiazolyl, quinolyl, indolyl, indenyl, and similar.

In some instance, alkenyl, alkynyl, aryl or heteroaryl moieties may be coupled to the remainder of the molecule through an alkylene linkage. Under those circumstances, the substituent will be referred to as alkenylalkyl, alkynylalkyl, arylalkyl or heteroarylalkyl, indicating that an alkylene moiety is between the alkenyl, alkynyl, aryl or heteroaryl moiety and the molecule to which the alkenyl, alkynyl, aryl or heteroaryl is coupled.

The term "halogen" includes bromo, fluoro, chloro and iodo.

The term "heterocyclic ring" refers to a 4 to 8 membered aromatic or non-aromatic ring comprising 3 to 7 carbon atoms and at least one N, O, or S atom. Examples are piperidinyl, piperazinyl, tetrahydropyranyl, pyrrolidine, and tetrahydrofuranyl, as well as the exemplary groups provided for the term "heteroaryl" above.

When a ring system is optionally substituted, suitable substituents are selected from the group consisting of alkyl, alkenyl, alkynyl, or an additional ring, each optionally further substituted. Optional substituents on any group, including the above, include halo, nitro, cyano, -OR, -SR, $-NR_2$, -OCOR, -NRCOR, -COOR, $-CONR_2$, -SOR, $-SO_2R$, $-SONR_2$, $-SO_2NR_2$, wherein each R is independently alkyl, alkenyl, alkynyl, aryl or heteroaryl, or two R groups taken together with the atoms to which they are attached form a ring.

Preferably $-L^1-$ of formula (IV) is substituted with one moiety $-L^2-$.

In certain embodiments $-L^1-$ has a structure as disclosed in WO2013/036857A1. Accordingly, in certain embodiments $-L^1-$ is of formula (V):

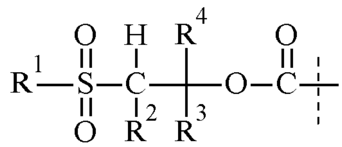

(V),

wherein

the dashed line indicates attachment to -D through an amine functional group of -D;

| | |
|---|---|
| $-R^1$ | is selected from the group consisting of optionally substituted $C_1$-$C_6$ linear, branched, or cyclic alkyl; optionally substituted aryl; optionally substituted heteroaryl; alkoxy; and $-NR^5{}_2$; |
| $-R^2$ | is selected from the group consisting of -H; optionally substituted $C_1$-$C_6$ alkyl; optionally substituted aryl; and optionally substituted heteroaryl; |
| $-R^3$ | is selected from the group consisting of -H; optionally substituted $C_1$-$C_6$ alkyl; optionally substituted aryl; and optionally substituted heteroaryl; |
| $-R^4$ | is selected from the group consisting of -H; optionally substituted $C_1$-$C_6$ alkyl; optionally substituted aryl; and optionally substituted heteroaryl; |
| each $-R^5$ | is independently of each other selected from the group consisting of -H; optionally substituted $C_1$-$C_6$ alkyl; optionally substituted aryl; and optionally substituted heteroaryl; or when taken together two $-R^5$ can be cycloalkyl or cycloheteroalkyl; |

wherein $-L^1-$ is substituted with at least one $-L^2-$ and wherein $-L^1-$ is optionally further substituted.

Only in the context of formula (V) the terms used have the following meaning:
"Alkyl", "alkenyl", and "alkynyl" include linear, branched or cyclic hydrocarbon groups of 1-8 carbons or 1-6 carbons or 1-4 carbons wherein alkyl is a saturated hydrocarbon, alkenyl includes one or more carbon-carbon double bonds and alkynyl includes one or more carbon-carbon triple bonds. Unless otherwise specified these contain 1-6 C.

"Aryl" includes aromatic hydrocarbon groups of 6-18 carbons, preferably 6-10 carbons, including groups such as phenyl, naphthyl, and anthracene. "Heteroaryl" includes aromatic rings comprising 3-15 carbons containing at least one N, O or S atom, preferably 3-7 carbons containing at least one N, O or S atom, including groups such as pyrrolyl, pyridyl, pyrimidinyl, imidazolyl, oxazolyl, isoxazolyl, thiszolyl, isothiazolyl, quinolyl, indolyl, indenyl, and similar.

The term "substituted" means an alkyl, alkenyl, alkynyl, aryl, or heteroaryl group comprising one or more substituent groups in place of one or more hydrogen atoms. Substituents may generally be selected from halogen including F, Cl, Br, and I; lower alkyl including linear, branched, and cyclic; lower haloalkyl including fluoroalkyl, chloroalkyl, bromoalkyl, and iodoalkyl; OH; lower alkoxy including linear, branched, and cyclic; SH; lower alkylthio including linear, branched and cyclic; amino, alkylamino, dialkylamino, silyl including alkylsilyl, alkoxysilyl, and arylsilyl; nitro; cyano; carbonyl; carboxylic acid, carboxylic ester, carboxylic amide, aminocarbonyl; aminoacyl; carbamate; urea; thiocarbamate; thiourea; ketne; sulfone; sulfonamide; aryl including phenyl, naphthyl, and anthracenyl; heteroaryl including 5-member heteroaryls including as pyrrole, imidazole, furan, thiophene, oxazole, thiazole, isoxazole, isothiazole, thiadiazole, triazole, oxadiazole, and tetrazole, 6-member heteroaryls including pyridine, pyrimidine, pyrazine, and fused heteroaryls including benzofuran, benzothiophene, benzoxazole, benzimidazole, indole, benzothiazole, benzisoxazole, and benzisothiazole.

Preferably $-L^1-$ of formula (V) is substituted with one moiety $-L^2-$.

In certain embodiments $-L^1-$ has a structure as disclosed in US7585837B2. Accordingly, in certain embodiments $-L^1-$ is of formula (VI):

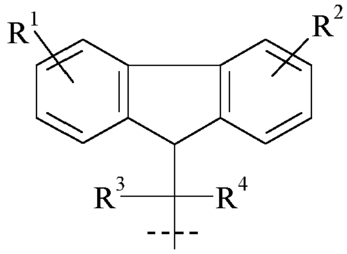

(VI),

wherein

the dashed line indicates attachment to -D through an amine functional group of -D;
$R^1$ and $R^2$ are independently selected from the group consisting of hydrogen, alkyl, alkoxy, alkoxyalkyl, aryl, alkaryl, aralkyl, halogen, nitro, $-SO_3H$, $-SO_2NHR^5$, amino, ammonium, carboxyl, $PO_3H_2$, and $OPO_3H_2$;
$R^3$, $R^4$, and $R^5$ are independently selected from the group consisting of hydrogen, alkyl, and aryl;
wherein $-L^1-$ is substituted with at least one $-L^2-$ and wherein $-L^1-$ is optionally further substituted.

Suitable substituents for formulas (VI) are alkyl (such as $C_{1-6}$ alkyl), alkenyl (such as $C_{2-6}$ alkenyl), alkynyl (such as $C_{2-6}$ alkynyl), aryl (such as phenyl), heteroalkyl, heteroalkenyl, heteroalkynyl, heteroaryl (such as aromatic 4 to 7 membered heterocycle) or halogen moieties.

Only in the context of formula (VI) the terms used have the following meaning:
The terms "alkyl", "alkoxy", "alkoxyalkyl", "aryl", "alkaryl" and "aralkyl" mean alkyl radicals of 1-8, preferably 1-4 carbon atoms, e.g. methyl, ethyl, propyl, isopropyl and butyl, and aryl radicals of 6-10 carbon atoms, e.g. phenyl and naphthyl. The term "halogen" includes bromo, fluoro, chloro and iodo.

Preferably $-L^1-$ of formula (VI) is substituted with one moiety $-L^2-$.

In certain embodiments $-L^1-$ has a structure as disclosed in WO2002/089789A1. Accordingly, in certain embodiments $-L^1-$ is of formula (VII):

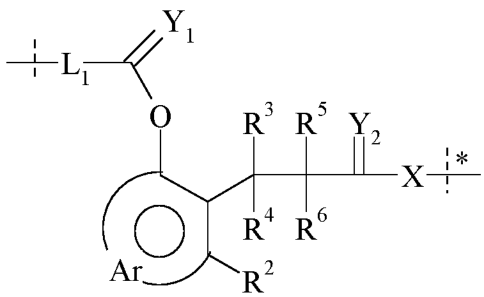

(VII),

wherein

the dashed line indicates attachment to -D through an amine functional group of -D;
$L_1$ is a bifunctional linking group,
$Y_1$ and $Y_2$ are independently O, S or $NR^7$;
$R^2$, $R^3$, $R^4$, $R^5$, $R^6$ and $R^7$ are independently selected from the group consisting of hydrogen, $C_{1-6}$ alkyls, $C_{3-12}$ branched alkyls, $C_{3-8}$ cycloalkyls, $C_{1-6}$ substituted alkyls, $C_{3-8}$ substituted cycloalkyls, aryls, substituted aryls, aralkyls, $C_{1-6}$ heteroalkyls, substituted $C_{1-6}$ heteroalkyls, $C_{1-6}$ alkoxy, phenoxy, and $C_{1-6}$ heteroalkoxy;
Ar is a moiety which when included in formula (VII) forms a multisubstituted aromatic hydrocarbon or a multi-substituted heterocyclic group;
X is a chemical bond or a moiety that is actively transported into a target cell, a hydrophobic moiety, or a combination thereof,
y is 0 or 1;
wherein $-L^1-$ is substituted with at least one $-L^2-$ and wherein $-L^1-$ is optionally further substituted.

Only in the context of formula (VII) the terms used have the following meaning:
The term "alkyl" shall be understood to include, e.g. straight, branched, substituted $C_{1-12}$ alkyls, including alkoxy, $C_{3-8}$ cycloalkyls or substituted cycloalkyls, etc.

The term "substituted" shall be understood to include adding or replacing one or more atoms contained within a functional group or compounds with one or more different atoms.

Substituted alkyls include carboxyalkyls, aminoalkyls, dialkylaminos, hydroxyalkyls and mercaptoalkyls; substtued cycloalkyls include moieties such as 4-chlorocyclohexyl; aryls include moieties such as napthyl; substituted aryls include moieties such as 3-bromo-phenyl; aralkyls include moieties such as toluyl; heteroalkyls include moieties such as ethylthiophene; substituted heteroalkyls include moieties such as 3-methoxythiophone; alkoxy includes moieities such as methoxy; and phenoxy includes moieties such as 3-nitrophenoxy. Halo- shall be understood to include fluoro, chloro, iodo and bromo.

Preferably -$L^1$- of formula (VII) is substituted with one moiety -$L^2$-.

In certain embodiments -$L^1$- comprises a substructure of formula (VIII)

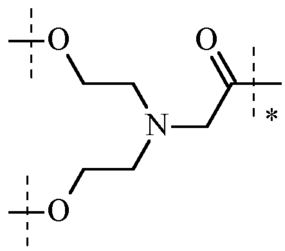

(VIII),

wherein

the dashed line marked with the asterisk indicates attachment to a nitrogen of -D by forming an amide bond;
the unmarked dashed lines indicate attachment to the remainder of -$L^1$-; and
wherein -$L^1$- is substituted with at least one -$L^2$- and wherein -$L^1$- is optionally further substituted.

Preferably -$L^1$- of formula (VIII) is substituted with one moiety -$L^2$-.

In one embodiment -$L^1$- of formula (VIII) is not further substituted.

In certain embodiments -$L^1$- comprises a substructure of formula (IX)

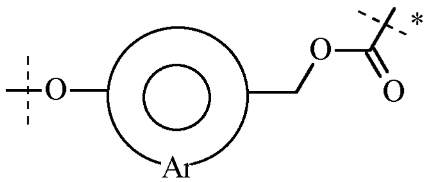

(IX),

wherein

the dashed line marked with the asterisk indicates attachment to a nitrogen of -D by forming a carbamate bond;
the unmarked dashed lines indicate attachment to the remainder of -$L^1$-; and
wherein -$L^1$- is substituted with at least one -$L^2$- and wherein -$L^1$- is optionally further substituted.

Preferably -$L^1$- of formula (IX) is substituted with one moiety -$L^2$-.

In one embodiment -$L^1$- of formula (IX) is not further substituted.

In certain embodiments -$L^1$- is of formula (IX-a):

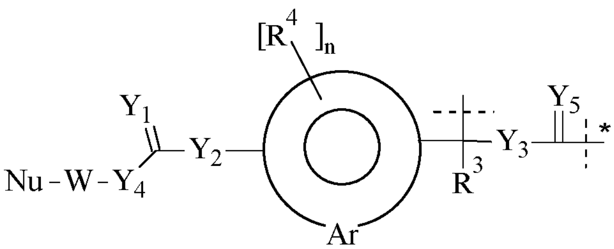

(IX-a),

wherein

the dashed line marked with the asterisk indicates attachment to a nitrogen of -D and the unmarked dashed line indicates attachment to $-L^2-$;
n is 0, 1, 2, 3, or 4;
$=Y_1$, $=Y_5$ are independently of each other selected from the group consisting of =O and =S;
$-Y_2-$ is selected from the group consisting of -O- and -S-;
$-Y_3-$ is selected from the group consisting of -O- and -S-;
$-Y_4-$ is selected from the group consisting of -O-, $-NR^5-$ and $-C(R^6R^{6a})-$;
$-R^3$, $-R^5$, $-R^6$, $-R^{6a}$ are independently of each other selected from the group consisting of -H, methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl;
$-R^4$ is selected from the group consisting of methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl;
-W- is selected from the group consisting of $C_{1-20}$ alkyl optionally interrupted by one or more groups selected from the group consisting of $C_{3-10}$ cycloalkyl, 8- to 30-membered carbopolycyclyl, 3- to 10-membered heterocyclyl, -C(O)-, $-C(O)N(R^7)-$, -O-, -S- and $-N(R^7)-$;
-Nu is a nucleophile selected from the group consisting of $-N(R^7R^{7a})$, $-N(R^7OH)$, $-N(R^7)-N(R^{7a}R^{7b})$, $-S(R^7)$,-COOH,

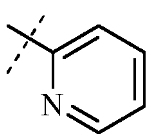, 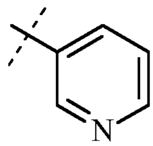, 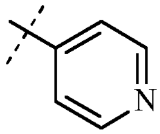, 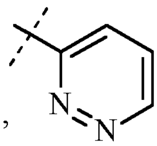,

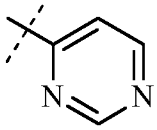, 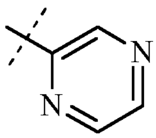, 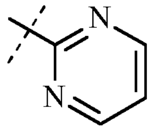, 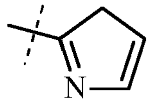,

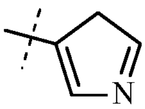, 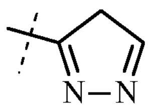, 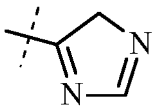 and 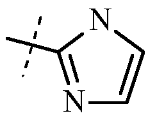;

-Ar- is selected from the group consisting of

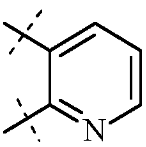, 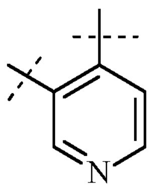, 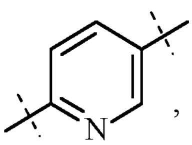, 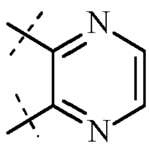, 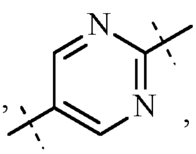,

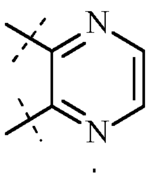, 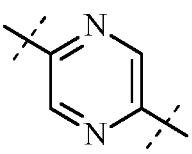, 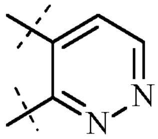, 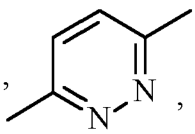, 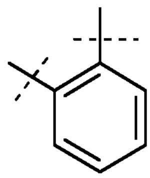,

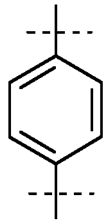, 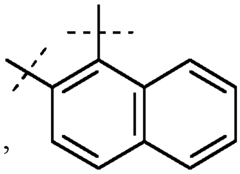, 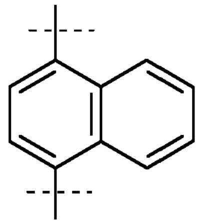, 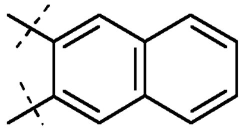,

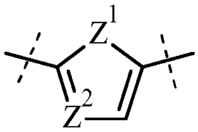, 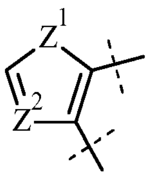, 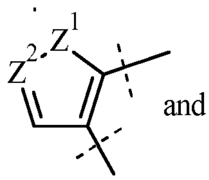 and 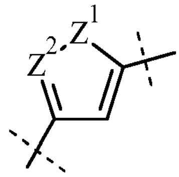;

wherein

dashed lines indicate attachment to the remainder of $-L^1-$,
$-Z^1-$ is selected from the group consisting of -O-, -S- and $-N(R^7)-$, and
$-Z^2-$ is $-N(R^7)-$; and

$-R^7$, $-R^{7a}$, $-R^{7b}$ are independently of each other selected from the group consisting of -H, $C_{1-6}$ alkyl, $C_{2-6}$ alkenyl and $C_{2-6}$ alkynyl;
wherein $-L^1-$ is optionally further substituted.

In one embodiment $-L^1-$ of formula (IX-a) is not further substituted.
In certain embodiments $-L^1-$ is of formula (IX-b):

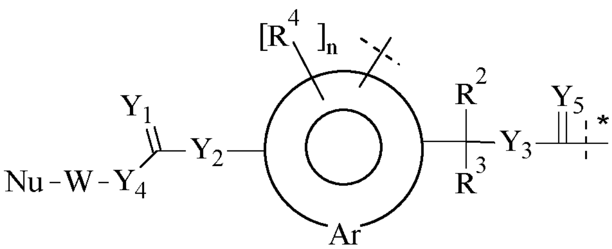

(IX-b),

wherein

the dashed line marked with the asterisk indicates attachment to a nitrogen of -D and the unmarked dashed line indicates attachment to $-L^2-$;
n is 0, 1, 2, 3, or 4;
$=Y_1$, $=Y_5$ are independently of each other selected from the group consisting of =O and =S;
$-Y_2-$ is selected from the group consisting of -O- and -S-;
$-Y_3-$ is selected from the group consisting of -O- and -S-;
$-Y_4-$ is selected from the group consisting of -O-, $-NR^5-$ and $-C(R^6R^{6a})-$;
$-R^2$, $-R^3$, $-R^5$, $-R^6$, $-R^{6a}$ are independently of each other selected from the group consisting of -H, methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl;
$-R^4$ is selected from the group consisting of methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl;
-W- is selected from the group consisting of $C_{1-20}$ alkyl optionally interrupted by one or more groups selected from the group consisting of $C_{3-10}$ cycloalkyl, 8- to 30-membered carbopolycyclyl, 3- to 10-membered heterocyclyl, -C(O)-, $-C(O)N(R^7)-$, -O-, -S- and $-N(R^7)-$;
-Nu is a nucleophile selected from the group consisting of $-N(R^7R^{7a})$, $-N(R^7OH)$, $-N(R^7)-N(R^{7a}R^{7b})$, $-S(R^7)$, -COOH,

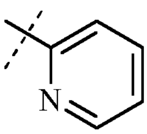 , 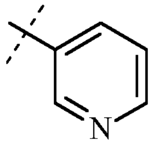 , 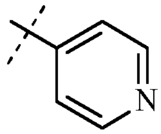 , 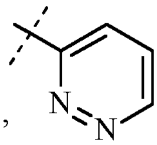 ,

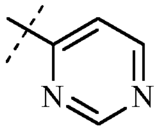 , 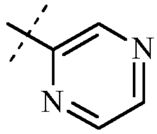 , 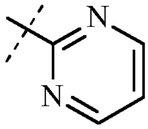 , 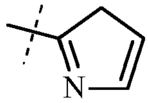 ,

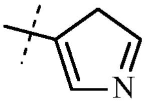 , 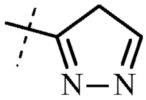 , 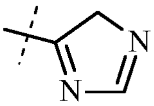 and 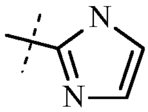 .

-Ar- is selected from the group consisting of

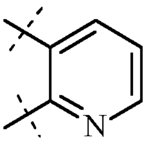 , 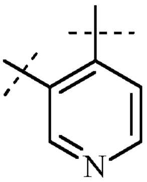 , 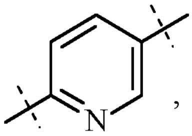 , 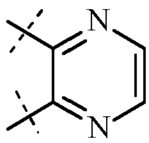 , 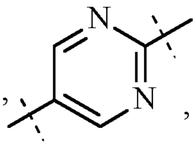 ,

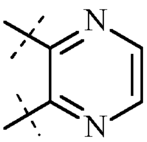 , 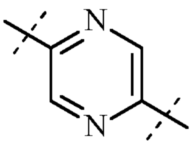 , 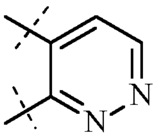 , 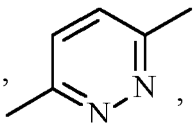 , 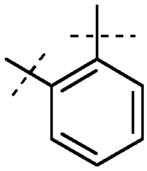 ,

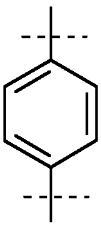 , 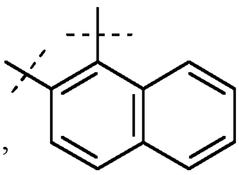 , 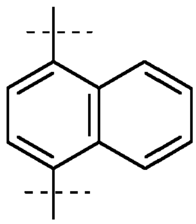 , 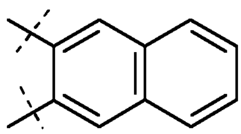 ,

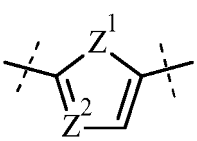 , 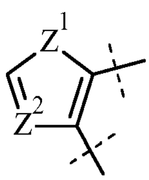 , 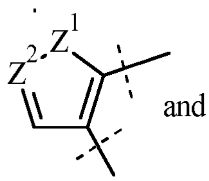 and 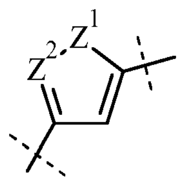 ;

wherein

dashed lines indicate attachment to the remainder of $-L^1-$,
$-Z^1-$ is selected from the group consisting of -O-, -S- and $-N(R^7)-$, and
$-Z^2-$ is $-N(R^7)-$; and

$-R^7$, $-R^{7a}$, $-R^{7b}$ are independently of each other selected from the group consisting of -H, $C_{1-6}$ alkyl, $C_{2-6}$ alkenyl and $C_{2-6}$ alkynyl;

wherein $-L^1-$ is optionally further substituted.

In one embodiment $-L^1-$ of formula (IX-b) is not further substituted.

In certain embodiments $-L^1-$ is of formula (X)

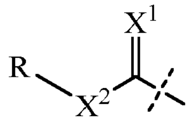
(X),

wherein

the dashed line indicates attachment to a nitrogen of an amine functional group of -D;
$=X^1$ is selected from the group consisting of =O, =S and =N;
$-X^2-$ is selected from the group consisting of -O-, -S- and -N-;
-R is $C_{1-50}$ alkyl, which $C_{1-50}$ alkyl is optionally interrupted by one or more groups selected from the group consisting of -T-, -C(O)O-, -O-, -C(O)-,
$-C(O)N(R^{z1})-$, $-S(O)_2N(R^{z1})-$, $-S(O)N(R^{z1})-$, $-S(O)_2-$, -S(O)-, $-N(R^{z1})S(O)_2N(R^{z1a})-$, -S-, $-N(R^{z1})-$, $-OC(OR^{z1})(R^{z1a})-$, $-N(R^{z1})C(O)N(R^{z1a})-$, and $-OC(O)N(R^{z1})-$; and which $C_{1-50}$ alkyl is optionally substituted with one or more $-R^{z2}$;
each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more $-R^{z2}$, which are the same or different;
each $-R^{z2}$ is independently selected from the group consisting of halogen, -CN, oxo (=O), $-COOR^{z3}$, $-OR^{z3}$, $-C(O)R^{z3}$, $-C(O)N(R^{z3}R^{z3a})$, $-S(O)_2N(R^{z3}R^{z3a})$, $-S(O)N(R^{z3}R^{z3a})$, $-S(O)_2R^{z3}$, $-S(O)R^{z3}$, $-N(R^{z3})S(O)_2N(R^{z3a}R^{z3b})$, $-SR^{z3}$, $-N(R^{z3}R^{z3a})$, $-NO_2$, $-OC(O)R^{z3}$, $-N(R^{z3})C(O)R^{z3a}$, $-N(R^{z3})S(O)2R^{z3a}$, $-N(R^{z3})S(O)R^{z3a}$, $-N(R^{z3})C(O)O\ R^{z3a}$, $-N(R^{z3})C(O)N(R^{z3a}R^{z3b})$, $-OC(O)N(R^{z3}R^{z3a})$, and $C_{1-6}$ alkyl; wherein $C_{1-6}$ alkyl is optionally substituted with one or more halogen, which are the same or different; and
each $-R^{z1}$, $-R^{z1a}$, $-R^{z3}$, $-R^{z3a}$ and $-R^{z3b}$ is independently selected from the group consisting of -H, and $C_{1-6}$ alkyl, wherein $C_{1-6}$ alkyl is optionally substituted with one or more halogen, which are the same or different;
wherein $-L^1-$ is substituted with at least one $-L^2-$ and wherein $-L^1-$ is optionally further substituted.

In certain embodiments $-L^1-$ is substituted with one $-L^2-$.

In one embodiment $-L^1-$ of formula (X) is not further substituted.

In certain embodiments $=X^1$ of formula (X) is selected from the group consisting of =N and =O. In certain embodiments $=X^1$ of formula (X) is =N. In certain embodiments $=X^1$ of formula (X) is =O.

In certain embodiments $-X^2-$ of formula (X) is selected from the group consisting of -N- and -O-. In certain embodiments $-X^2-$ of formula (X) is -N-. In certain embodiments $-X^2-$ of formula (X) is -O-.

In certain embodiments $=X^1$ of formula (X) is =N and $-X^2-$ of formula (X) is -O-. In certain embodiments $=X^1$ of formula (X) is =O and $-X^2-$ of formula (X) is -N-. In certain embodiment $=X^1$ of formula (X) is =N and $-X^2-$ of formula (X) is -N-. In certain embodiments $=X^1$ of formula (X) is =O and $-X^2-$ of formula (X) is -O-.

In certain embodiments -R of formula (X) is $C_{1-20}$ alkyl, which $C_{1-20}$ alkyl is optionally interrupted by one or more groups selected from the group consisting of -T-, -C(O)O-, -O-, -C(O)-,$-C(O)N(R^{z1})-$, $-S(O)_2N(R^{z1})-$, $-S(O)N(R^{z1})-$, $-S(O)_2-$, -S(O)-, -S-, $-N(R^{z1})-$, $-OC(OR^{z1})(R^{z1a})-$, $-N(R^{z1})C(O)N(R^{z1a})-$, and $-OC(O)N(R^{z1})-$; and which $C_{1-20}$ alkyl is optionally substituted with one or more $-R^{z2}$;

each $-R^{z1}$ and $-R^{z1a}$ is independently selected from the group consisting of -H, and $C_{1-6}$ alkyl, wherein $C_{1-6}$ alkyl is optionally substituted with one or more halogen, which are the same or different;
each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, wherein each T is independently optionally substituted with one or more $-R^{z2}$, which are the same or different;
each $-R^{z2}$ is independently selected from the group consisting of halogen, and $C_{1-6}$ alkyl; wherein $C_{1-6}$ alkyl is optionally substituted with one or more halogen, which are the same or different.

In certain embodiments the moiety of formula (X) is selected from the group consisting of formula (X-1), (X-2), (X-3), (X-4), (X-5), (X-6), (X-7), (X-8), (X-9), (X-10), (X-11) and (X-12)

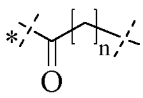 (X-1), 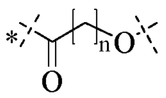 (X-2), 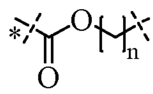 (X-3), 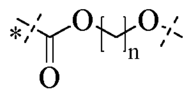 (X-4), 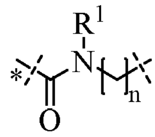 (X-5), 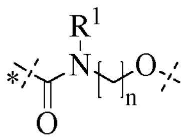 (X-6), 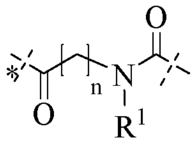 (X-7), 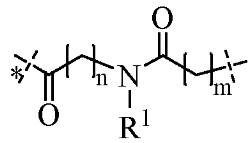 (X-8), 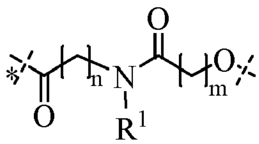 (X-9), 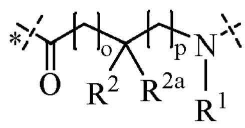 (X-10), 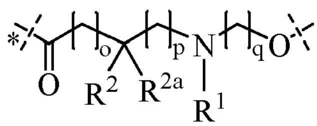 (X-11)

and

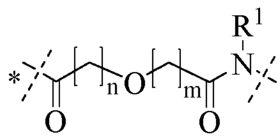 (X-12);

wherein

the dashed line marked with the asterisk indicates attachment to a nitrogen of an amine functional group of -D;
the unmarked dashed line indicates attachment to $-L^2-$;
$-R^1$ is selected from the group consisting of -H, $C_{1-10}$ alkyl, $C_{2-10}$ alkenyl and $C_{2-10}$ alkynyl;
$-R^2$ and $-R^{2a}$ are independently selected from the group consisting of -H, halogen, $C_{1-10}$ alkyl, $C_{2-10}$ alkenyl and $C_{2-10}$ alkynyl;
n is an integer selected from the group consisting of 1, 2, 3, 4, 5, 6, 7, 8, 9, 10, 11, 12, 13, 14, 15, 16, 17, 18, 19, 20, 21, 22, 23, 24 and 25;
m is an integer selected from the group consisting of 1, 2, 3, 4, 5, 6, 7, 8, 9, 10, 11, 12, 13, 14, 15, 16, 17, 18, 19, 20, 21, 22, 23, 24 and 25;
o is an integer selected from the group consisting of 0, 1, 2, 3, 4, 5, 6, 7, 8, 9 and 10;
p is an integer selected from the group consisting of 0, 1, 2, 3, 4, 5, 6, 7, 8, 9 and 10; and
q is an integer selected from the group consisting of 1, 2, 3, 4, 5, 6, 7, 8, 9, 10, 11, 12, 13, 14, 15, 16, 17, 18, 19, 20, 21, 22, 23, 24 and 25.

In certain embodiments at least one of $-R^2$ and $-R^{2a}$ of formula (X-10) and (X-11) is not -H.

In certain embodiments n of formula (X-1), (X-2), (X-3), (X-4), (X-5), (X-6), (X-7), (X-8), (X-9) or (X-12) is 1. In certain embodiments n of formula (X-1), (X-2), (X-3), (X-4), (X-5), (X-6), (X-7), (X-8), (X-9) or (X-12) is 2. In certain embodiments n of formula (X-1), (X-2), (X-3), (X-4), (X-5), (X-6), (X-7), (X-8), (X-9) or (X-12) is 3. In certain embodiments n of formula (X-1), (X-2), (X-3), (X-4), (X-5), (X-6), (X-7), (X-8), (X-9) or (X-12) is 4. In certain embodiments n of formula (X-1), (X-2), (X-3), (X-4), (X-5), (X-6), (X-7), (X-8), (X-9) or (X-12) is 5. In certain embodiments n of formula (X-1), (X-2), (X-3), (X-4), (X-5), (X-6), (X-7), (X-8), (X-9) or (X-12) is 6. In certain embodiments n of formula (X-1), (X-2), (X-3), (X-4), (X-5), (X-6), (X-7), (X-8), (X-9) or (X-12) is 7. In certain embodiments n of formula (X-1), (X-2), (X-3), (X-4), (X-5), (X-6), (X-7), (X-8), (X-9) or (X-12) is 8. In certain embodiments n of formula (X-1), (X-2), (X-3), (X-4), (X-5), (X-6), (X-7), (X-8), (X-9) or (X-12) is 9. In certain embodiments n of formula (X-1), (X-2), (X-3), (X-4), (X-5), (X-6), (X-7), (X-8), (X-9) or (X-12) is 10.

In certain embodiments m of formula (X-8), (X-9) or (X-12) is 1. In certain embodiments m of formula (X-8), (X-9) or (X-12) is 2. In certain embodiments m of formula (X-8), (X-9) or (X-12) is 3. In certain embodiments m of formula (X-8), (X-9) or (X-12) is 4. In certain embodiments m of formula (X-8), (X-9) or (X-12) is 5. In certain embodiments m of formula (X-8), (X-9) or (X-12) is 6. In certain embodiments m of formula (X-8), (X-9) or (X-12) is 7. In certain embodiments m of formula (X-8), (X-9) or (X-12) is 8. In certain embodiments m of formula (X-8), (X-9) or (X-12) is 9. In certain embodiments m

of formula (X-8), (X-9) or (X-12) is 10.

In certain embodiments o of formula (X-10) or (X-11) is 0. In certain embodiments o of formula (X-10) or (X-11) is 1. In certain embodiments o of formula (X-10) or (X-11) is 2. In certain embodiments o of formula (X-10) or (X-11) is 3. In certain embodiments o of formula (X-10) or (X-11) is 4. In certain embodiments o of formula (X-10) or (X-11) is 5. In certain embodiments o of formula (X-10) or (X-11) is 6. In certain embodiments o of formula (X-10) or (X-11) is 7. In certain embodiments o of formula (X-10) or (X-11) is 8. In certain embodiments o of formula (X-10) or (X-11) is 9. In certain embodiments o of formula (X-10) or (X-11) is 10.

In certain embodiments p of formula (X-10) or (X-11) is 0. In certain embodiments p of formula (X-10) or (X-11) is 1. In certain embodiments p of formula (X-10) or (X-11) is 2. In certain embodiments p of formula (X-10) or (X-11) is 3. In certain embodiments p of formula (X-10) or (X-11) is 4. In certain embodiments p of formula (X-10) or (X-11) is 5. In certain embodiments p of formula (X-10) or (X-11) is 6. In certain embodiments p of formula (X-10) or (X-11) is 7. In certain embodiments p of formula (X-10) or (X-11) is 8. In certain embodiments p of formula (X-10) or (X-11) is 9. In certain embodiments p of formula (X-10) or (X-11) is 10.

In certain embodiments q of formula (X-11) is 1. In certain embodiments q of formula (X-11) is 2. In certain embodiments q of formula (X-11) is 3. In certain embodiments q of formula (X-11) is 4. In certain embodiments q of formula (X-11) is 5. In certain embodiments q of formula (X-11) is 6. In certain embodiments q of formula (X-11) is 7. In certain embodiments q of formula (X-11) is 8. In certain embodiments q of formula (X-11) is 9. In certain embodiments q of formula (X-11) is 10.

In certain embodiments $-R^1$ of formula (X-5), (X-6), (X-7), (X-8), (X-9), (X-10), (X-11) or (X-12) is -H. In certain embodiments $-R^1$ of formula (X-5), (X-6), (X-7), (X-8), (X-9), (X-10), (X-11) or (X-12) is $C_{1-10}$ alkyl, such as methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl or 3,3-dimethylpropyl. In certain embodiments $-R^1$ of formula (X-5), (X-6), (X-7), (X-8), (X-9), (X-10), (X-11) or (X-12) is $C_{2-10}$ alkenyl. In certain embodiments $-R^1$ of formula (X-5), (X-6), (X-7), (X-8), (X-9), (X-10), (X-11) or (X-12) is $C_{2-10}$ alkynyl.

In certain embodiments $-R^2$ of formula (X-10) or (X-11) is -H. In certain embodiments $-R^2$ of formula (X-10) or (X-11) is halogen, such as fluoro or chloro. In certain embodiments $-R^2$ of formula (X-10) or (X-11) is $C_{1-10}$ alkyl, such as methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl or 3,3-dimethylpropyl. In certain embodiments $-R^2$ of formula (X-10) or (X-11) is $C_{2-10}$ alkenyl, such as $C_2$ alkenyl, $C_3$ alkenyl, $C_4$ alkenyl, $C_5$ alkenyl or $C_6$ alkenyl. In certain embodiments $-R^2$ of formula (X-10) or (X-11) is $C_{2-10}$ alkynyl, such as $C_2$ alkynyl, $C_3$ alkynyl, $C_4$ alkynyl, $C_5$ alkynyl or $C_6$ alkynyl.

In certain embodiments $-R^{2a}$ of formula (X-10) or (X-11) is -H. In certain embodiments $-R^{2a}$ of formula (X-10) or (X-11) is halogen. In certain embodiments $-R^{2a}$ of formula (X-10) or (X-11) is $C_{1-10}$ alkyl, such as methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl or 3,3-dimethylpropyl. In certain embodiments $-R^{2a}$ of formula (X-10) or (X-11) is $C_{2-10}$ alkenyl, such as $C_2$ alkenyl, $C_3$ alkenyl, $C_4$ alkenyl, $C_5$ alkenyl or $C_6$ alkenyl. In certain embodiments $-R^{2a}$ of formula (X-10) or (X-11) is $C_{2-10}$ alkynyl, such as $C_2$ alkynyl, $C_3$ alkynyl, $C_4$ alkynyl, $C_5$ alkynyl or $C_6$ alkynyl.

In certain embodiments at least one of $-R^2$ and $-R^{2a}$ of formula (X-10) and (X-11) is not -H.

In certain embodiments $-L^1-$ is of formula (X-1). In certain embodiments $-L^1-$ is of formula (X-1) with n = 1. In certain embodiments $-L^1-$ is of formula (X-1) with n = 2. In certain embodiments $-L^1-$ is of formula (X-1) with n = 3. In certain embodiments $-L^1-$ is of formula (X-1) with n = 4. In certain embodiments $-L^1-$ is of formula (X-1) with n = 5.

In certain embodiments $-L^1-$ is of formula (X-2). In certain embodiments $-L^1-$ is of formula (X-2) with n = 1. In certain embodiments $-L^1-$ is of formula (X-2) with n = 2. In certain embodiments $-L^1-$ is of formula (X-2) with n = 3. In certain embodiments $-L^1-$ is of formula (X-2) with n = 4. In certain embodiments $-L^1-$ is of formula (X-2) with n = 5.

In certain embodiments $-L^1-$ is of formula (X-3). In certain embodiments $-L^1-$ is of formula (X-3) with n = 1. In certain embodiments $-L^1-$ is of formula (X-3) with n = 2. In certain embodiments $-L^1-$ is of formula (X-3) with n = 3. In certain embodiments $-L^1-$ is of formula (X-3) with n = 4. In certain embodiments $-L^1-$ is of formula (X-3) with n = 5.

In certain embodiments $-L^1-$ is of formula (X-4). In certain embodiments $-L^1-$ is of formula (X-4) with n = 1. In certain embodiments $-L^1-$ is of formula (X-4) with n = 2. In certain embodiments $-L^1-$ is of formula (X-4) with n = 3. In certain embodiments $-L^1-$ is of formula (X-4) with n = 4. In certain embodiments $-L^1-$ is of formula (X-4) with n = 5.

In certain embodiments $-L^1-$ is of formula (X-5). In certain embodiments $-L^1-$ is of formula (X-5) and $-R^1$ is -H. In certain embodiments $-L^1-$ is of formula (X-5) and $-R^1$ is methyl. In certain embodiments $-L^1-$ is of formula (X-5) and $-R^1$ is ethyl. In certain embodiments $-L^1-$ is of formula (X-5) and n is 1. In certain embodiments $-L^1-$ is of formula (X-5) and n is 2. In certain embodiments $-L^1-$ is of formula (X-5) and n is 3. In certain embodiments $-L^1-$ is of formula (X-5), $-R^1$ is -H and n is 1. In certain embodiments $-L^1-$ is of formula (X-5), $-R^1$ is -H and n is 2. In certain embodiments $-L^1-$ is of formula (X-5), $-R^1$ is -H and n is 3. In certain embodiments $-L^1-$ is of formula (X-5), $-R^1$ is methyl and n is 1. In certain embodiments $-L^1-$ is of formula (X-5), $-R^1$ is methyl and n is 2. In certain embodiments $-L^1-$ is of formula (X-5), $-R^1$ is methyl and n is 3.

In certain embodiments $-L^1-$ is of formula (X-6). In certain embodiments $-L^1-$ is of formula (X-6) and $-R^1$ is -H. In

certain embodiments $-L^1-$ is of formula (X-6) and $-R^1$ is methyl. In certain embodiments $-L^1-$ is of formula (X-6) and $-R^1$ is ethyl. In certain embodiments $-L^1-$ is of formula (X-6) and n is 1. In certain embodiments $-L^1-$ is of formula (X-6) and n is 2. In certain embodiments $-L^1-$ is of formula (X-6) and n is 3. In certain embodiments $-L^1-$ is of formula (X-6), $-R^1$ is -H and n is 1. In certain embodiments $-L^1-$ is of formula (X-6), $-R^1$ is -H and n is 2. In certain embodiments $-L^1-$ is of formula (X-6), $-R^1$ is -H and n is 3. In certain embodiments $-L^1-$ is of formula (X-6), $-R^1$ is methyl and n is 1. In certain embodiments $-L^1-$ is of formula (X-6), $-R^1$ is methyl and n is 2. In certain embodiments $-L^1-$ is of formula (X-6), $-R^1$ is methyl and n is 3.

In certain embodiments $-L^1-$ is of formula (X-7). In certain embodiments $-L^1-$ is of formula (X-7) and $-R^1$ is -H. In certain embodiments $-L^1-$ is of formula (X-7) and $-R^1$ is methyl. In certain embodiments $-L^1-$ is of formula (X-7) and $-R^1$ is ethyl. In certain embodiments $-L^1-$ is of formula (X-7) and n is 1. In certain embodiments $-L^1-$ is of formula (X-7) and n is 2. In certain embodiments $-L^1-$ is of formula (X-7) and n is 3. In certain embodiments $-L^1-$ is of formula (X-7), $-R^1$ is -H and n is 1. In certain embodiments $-L^1-$ is of formula (X-7), $-R^1$ is -H and n is 2. In certain embodiments $-L^1-$ is of formula (X-7), $-R^1$ is -H and n is 3. In certain embodiments $-L^1-$ is of formula (X-7), $-R^1$ is methyl and n is 1. In certain embodiments $-L^1-$ is of formula (X-7), $-R^1$ is methyl and n is 2. In certain embodiments $-L^1-$ is of formula (X-7), $-R^1$ is methyl and n is 3.

In certain embodiments $-L^1-$ is of formula (X-8). In certain embodiments $-L^1-$ is of formula (X-8) and $-R^1$ is -H. In certain embodiments $-L^1-$ is of formula (X-8) and $-R^1$ is methyl. In certain embodiments $-L^1-$ is of formula (X-8) and $-R^1$ is ethyl. In certain embodiments $-L^1-$ is of formula (X-8) and n is 1. In certain embodiments $-L^1-$ is of formula (X-8) and n is 2. In certain embodiments $-L^1-$ is of formula (X-8) and n is 3. In certain embodiments $-L^1-$ is of formula (X-8) and m is 1. In certain embodiments $-L^1-$ is of formula (X-8) and m is 2. In certain embodiments $-L^1-$ is of formula (X-8) and m is 3. In certain embodiments $-L^1-$ is of formula (X-8), $-R^1$ is -H, n is 1 and m is 1. In certain embodiments $-L^1-$ is of formula (X-8), $-R^1$ is -H, n is 1 and m is 2. In certain embodiments $-L^1-$ is of formula (X-8), $-R^1$ is -H, n is 1 and m is 3. In certain embodiments $-L^1-$ is of formula (X-8), $-R^1$ is -H, n is 2 and m is 1. In certain embodiments $-L^1-$ is of formula (X-8), $-R^1$ is -H, n is 2 and m is 2. In certain embodiments $-L^1-$ is of formula (X-8), $-R^1$ is -H, n is 2 and m is 3. In certain embodiments $-L^1-$ is of formula (X-8), $-R^1$ is -H, n is 3 and m is 1. In certain embodiments $-L^1-$ is of formula (X-8), $-R^1$ is -H, n is 3 and m is 2. In certain embodiments $-L^1-$ is of formula (X-8), $-R^1$ is -H, n is 3 and m is 3.

In certain embodiments $-L^1-$ is of formula (X-9). In certain embodiments $-L^1-$ is of formula (X-9) and $-R^1$ is -H. In certain embodiments $-L^1-$ is of formula (X-9) and $-R^1$ is methyl. In certain embodiments $-L^1-$ is of formula (X-9) and $-R^1$ is ethyl. In certain embodiments $-L^1-$ is of formula (X-9) and n is 1. In certain embodiments $-L^1-$ is of formula (X-9) and n is 2. In certain embodiments $-L^1-$ is of formula (X-9) and n is 3. In certain embodiments $-L^1-$ is of formula (X-9) and m is 1. In certain embodiments $-L^1-$ is of formula (X-9) and m is 2. In certain embodiments $-L^1-$ is of formula (X-9) and m is 3. In certain embodiments $-L^1-$ is of formula (X-9), $-R^1$ is -H, n is 1 and m is 1. In certain embodiments $-L^1-$ is of formula (X-9), $-R^1$ is -H, n is 1 and m is 2. In certain embodiments $-L^1-$ is of formula (X-9), $-R^1$ is -H, n is 1 and m is 3. In certain embodiments $-L^1-$ is of formula (X-9), $-R^1$ is -H, n is 2 and m is 1. In certain embodiments $-L^1-$ is of formula (X-9), $-R^1$ is -H, n is 2 and m is 2. In certain embodiments $-L^1-$ is of formula (X-9), $-R^1$ is -H, n is 2 and m is 3. In certain embodiments $-L^1-$ is of formula (X-9), $-R^1$ is -H, n is 3 and m is 1. In certain embodiments $-L^1-$ is of formula (X-9), $-R^1$ is -H, n is 3 and m is 2. In certain embodiments $-L^1-$ is of formula (X-9), $-R^1$ is -H, n is 3 and m is 3.

In certain embodiments $-L^1-$ is of formula (X-10). In certain embodiments $-R^1$ of formula (X-10) is -H. In certain embodiments o of formula (X-10) is 0. In certain embodiments o of formula (X-10) is 1. In certain embodiments o of formula (X-10) is 2. In certain embodiments o of formula (X-10) is 3. In certain embodiments p of formula (X-10) is 0. In certain embodiments p of formula (X-10) is 1. In certain embodiments p of formula (X-10) is 2. In certain embodiments p of formula (X-10) is 3. In certain embodiments $-R^2$ of formula (X-10) is -H. In certain embodiments $-R^2$ of formula (X-10) is halogen, such as fluor. In certain embodiments $-R^2$ of formula (X-10) is methyl. In certain embodiments $-R^2$ of formula (X-10) is ethyl. In certain embodiments $-R^2$ of formula (X-10) is n-propyl. In certain embodiments $-R^2$ of formula (X-10) is isopropyl. In certain embodiments $-R^2$ of formula (X-10) is 2-methylpropyl. In certain embodiments $-R^2$ of formula (X-10) is 2-methylpropyl. In certain embodiments $-R^2$ of formula (X-10) is 1-methylpropyl. In certain embodiments $-R^{2a}$ of formula (X-10) is -H. In certain embodiments both $-R^2$ and $-R^{2a}$ of formula (X-10) are methyl. In certain embodiments $-R^2$ of formula (X-10) is fluor and $-R^{2a}$ of formula (X-10) is -H. In certain embodiments $-R^2$ of formula (X-10) is isopropyl and $-R^{2a}$ of formula (X-10) is -H. In certain embodiments $-R^2$ of formula (X-10) is 2-methylpropyl and $-R^{2a}$ of formula (X-10) is -H.

In certain embodiments $-L^1-$ is of formula (X-11). In certain embodiments $-R^1$ of formula (X-11) is -H. In certain embodiments $-R^1$ of formula (X-11) is methyl. In certain embodiments $-R^1$ of formula (X-11) is ethyl. In certain embodiments o of formula (X-11) is 0. In certain embodiments o of formula (X-11) is 1. In certain embodiments o of formula (X-11) is 2. In certain embodiments p of formula (X-11) is 0. In certain embodiments p of formula (X-11) is 1. In certain embodiments p of formula (X-11) is 2. In certain embodiments $-R^2$ of formula (X-11) is -H. In certain embodiments $-R^2$ of formula (X-11) is halogen, such as fluor. In certain embodiments $-R^2$ of formula (X-11) is methyl. In certain embodiments $-R^2$ of formula (X-11) is ethyl. In certain embodiments $-R^2$ of formula (X-11) is n-propyl. In certain embodiments $-R^2$ of formula (X-11) is isopropyl. In certain embodiments $-R^2$ of formula (X-11) is 2-methylpropyl. In certain embodiments $-R^2$ of formula (X-11) is 2-methylpropyl. In certain embodiments $-R^2$ of formula (X-11) is 1-methylpropyl. In certain embodiments $-R^{2a}$ of formula (X-11) is -H. In certain embodiments both $-R^2$ and $-R^{2a}$ of formula (X-11) are methyl. In certain embodiments $-R^2$ of formula (X-11) is fluor and $-R^{2a}$ of formula (X-11) is -H. In certain embodiments $-R^2$ of formula (X-11) is isopropyl and $-R^{2a}$ of formula

(X-11) is -H. In certain embodiments -$R^2$ of formula (X-11) is 2-methylpropyl and -$R^{2a}$ of formula (X-11) is -H. In certain embodiments q of formula (X-11) is 1. In certain embodiments q of formula (X-11) is 2. In certain embodiments q of formula (X-11) is 3.

In certain embodiments -$L^1$- is of formula (X-12). In certain embodiments -$L^1$- is of formula (X-12) and n is 1. In certain embodiment -$L^1$- is of formula (X-12) and n is 2. In certain embodiments $L^1$- is of formula (X-12) and n is 3. In certain embodiments -$L^1$- is of formula (X-12) and m is 1. In certain embodiment $L^1$- is of formula (X-12) and m is 2. In certain embodiments $L^1$- is of formula (X-12) and m is 3. In certain embodiments -$L^1$- is of formula (X-12) and both n and m are 1. In certain embodiments -$L^1$- is of formula (X-12) and -$R^1$ is -H. In certain embodiments $L^1$- is of formula (X-12) and -$R^1$ is methyl. In certain embodiments $L^1$- is of formula (X-12) and -$R^1$ is ethyl.

In certain embodiments -$L^1$- is selected from the group consisting of

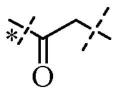 (X-a1), 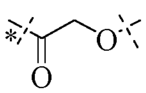 (X-a2), 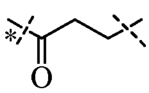 (X-a3), 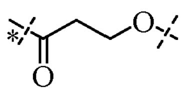 (X-a4), 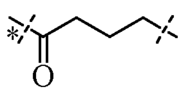 (X-a5), 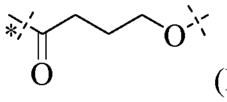 (X-a6), 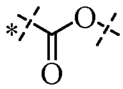 (X-a7), 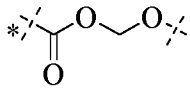 (X-a8), 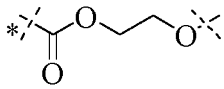 (X-a9), 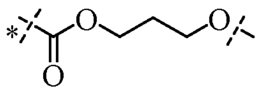 (X-a10), 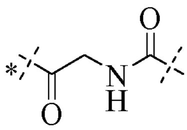 (X-a11), 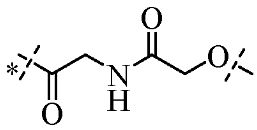 (X-a12), 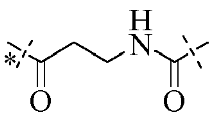 (X-a13), 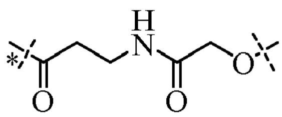 (X-a14), 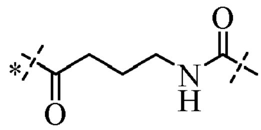 (X-a15), 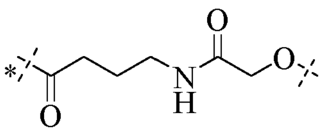 (X-a16), 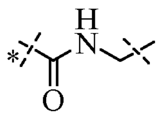 (X-a17), 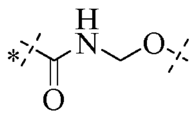 (X-a18), 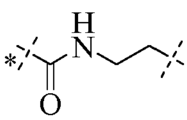 (X-a19), 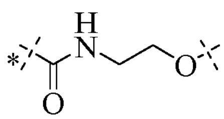 (X-a20), 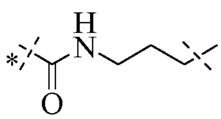 (X-a21), 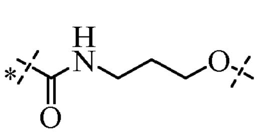 (X-a22), 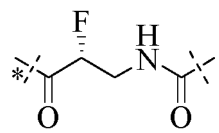 (X-a23), 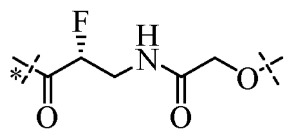 (X-a24), 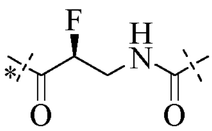 (X-a25), 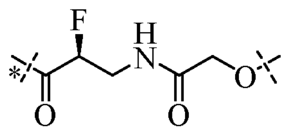 (X-a26), 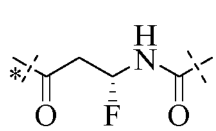 (X-a27),

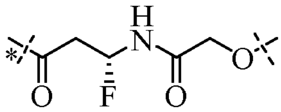 (X-a28), 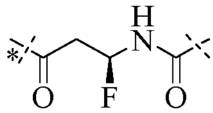 (X-a29), 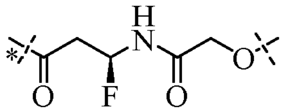 (X-a30),

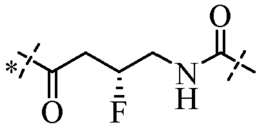 (X-a31), 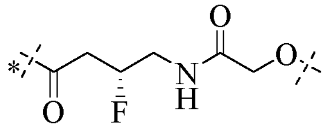 (X-a32), 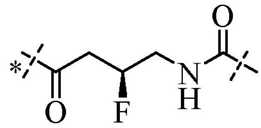 (X-a33),

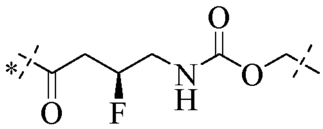 (X-a34), 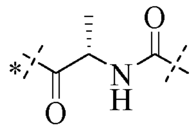 (X-a35), 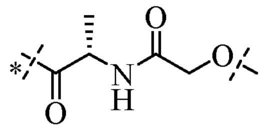 (X-a36),

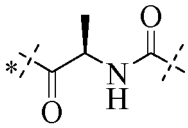 (X-a37), 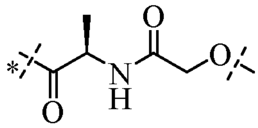 (X-a38), 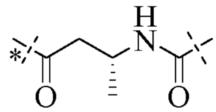 (X-a39),

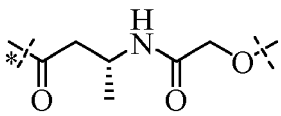 (X-a40), 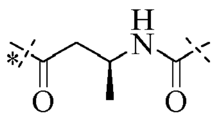 (X-a41), 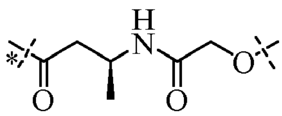 (X-a42),

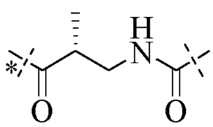 (X-a43), 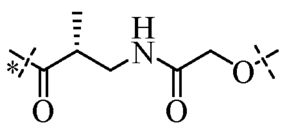 (X-a44), 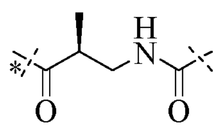 (X-a45),

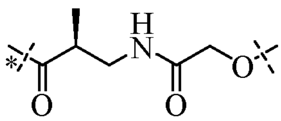 (X-a46), 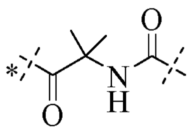 (X-a47), 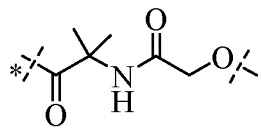 (X-a48),

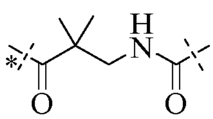 (X-a49), 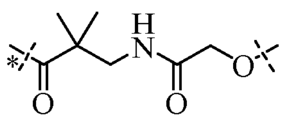 (X-a50), 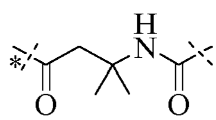 (X-a51),

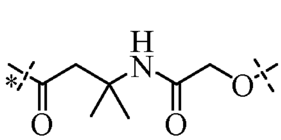 (X-a52), 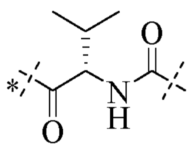 (X-a53), 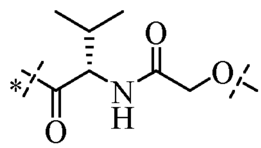 (X-a54),

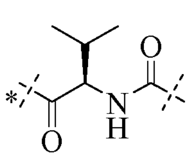 (X-a55), 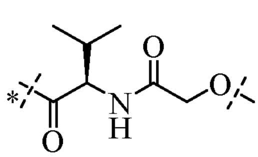 (X-a56), 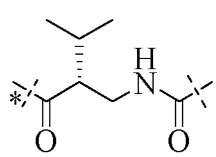 (X-a57),

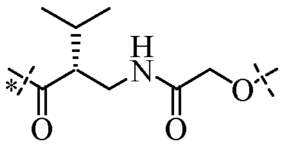 (X-a58), 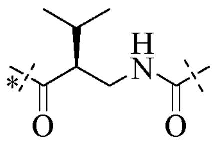 (X-a59), 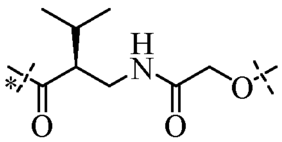 (X-a60),

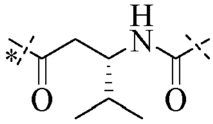 (X-a61), 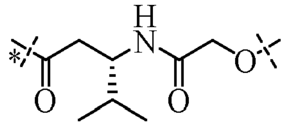 (X-a62), 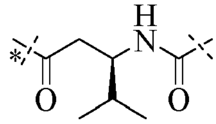 (X-a63),

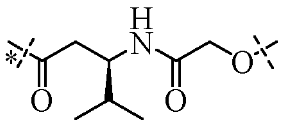 (X-a64), 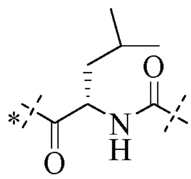 (X-a65), 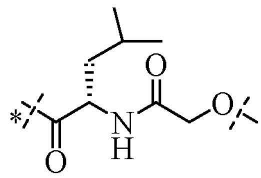 (X-a66),

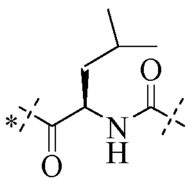 (X-a67), 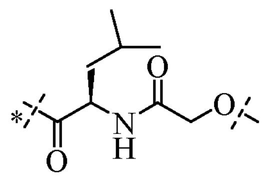 (X-a68), 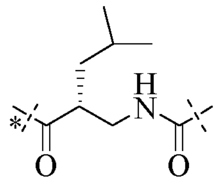 (X-a69),

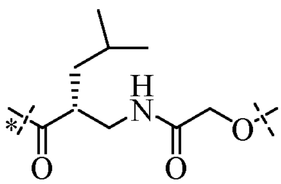 (X-a70), 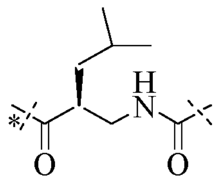 (X-a71), 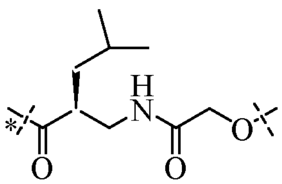 (X-a72),

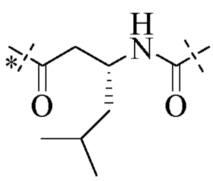 (X-a73), 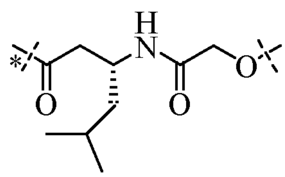 (X-a74), 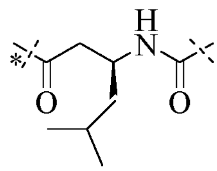 (X-a75),

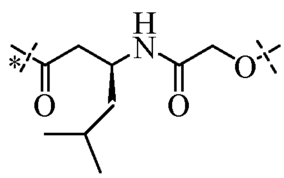 (X-a76), 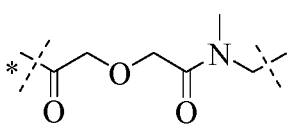 (X-a77) and 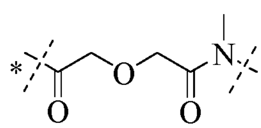

(X-a78);

wherein

the dashed line marked with the asterisk indicates attachment to a nitrogen of an amine functional group of -D ; and the unmarked dashed line indicates attachment to $-L^2-$.

In certain embodiments $-L^1-$ is of formula (X-a1). In certain embodiments $-L^1-$ is of formula (X-a2). In certain embodiments $-L^1-$ is of formula (X-a3). In certain embodiments $-L^1-$ is of formula (X-a4). In certain embodiments $-L^1-$ is of

formula (X-a5). In certain embodiments $-L^1-$ is of formula (X-a6). In certain embodiments $-L^1-$ is of formula (X-a7). In certain embodiments $-L^1-$ is of formula (X-a8). In certain embodiments $-L^1-$ is of formula (X-a9). In certain embodiments $-L^1-$ is of formula (X-a10). In certain embodiments $-L^1-$ is of formula (X-a11). In certain embodiments $-L^1-$ is of formula (X-a12). In certain embodiments $-L^1-$ is of formula (X-a13). In certain embodiments $-L^1-$ is of formula (X-a14). In certain embodiments $-L^1-$ is of formula (X-a15). In certain embodiments $-L^1-$ is of formula (X-a16). In certain embodiments $-L^1-$ is of formula (X-a17). In certain embodiments $-L^1-$ is of formula (X-a18). In certain embodiments $-L^1-$ is of formula (X-a19). In certain embodiments $-L^1-$ is of formula (X-a20). In certain embodiments $-L^1-$ is of formula (X-a21). In certain embodiments $-L^1-$ is of formula (X-a22). In certain embodiments $-L^1-$ is of formula (X-a23). In certain embodiments $-L^1-$ is of formula (X-24). In certain embodiments $-L^1-$ is of formula (X-a25). In certain embodiments $-L^1-$ is of formula (X-a26). In certain embodiments $-L^1-$ is of formula (X-a27). In certain embodiments $-L^1-$ is of formula (X-a28). In certain embodiments $-L^1-$ is of formula (X-a29). In certain embodiments $-L^1-$ is of formula (X-a30). In certain embodiments $-L^1-$ is of formula (X-a31). In certain embodiments $-L^1-$ is of formula (X-a32). In certain embodiments $-L^1-$ is of formula (X-a33). In certain embodiments $-L^1-$ is of formula (X-a34). In certain embodiments $-L^1-$ is of formula (X-a35). In certain embodiments $-L^1-$ is of formula (X-a36). In certain embodiments $-L^1-$ is of formula (X-a37). In certain embodiments $-L^1-$ is of formula (X-a38). In certain embodiments $-L^1-$ is of formula (X-a39). In certain embodiments $-L^1-$ is of formula (X-a40). In certain embodiments $-L^1-$ is of formula (X-a41). In certain embodiments $-L^1-$ is of formula (X-a42). In certain embodiments $-L^1-$ is of formula (X-a43). In certain embodiments $-L^1-$ is of formula (X-a44). In certain embodiments $-L^1-$ is of formula (X-a45). In certain embodiments $-L^1-$ is of formula (X-a46). In certain embodiments $-L^1-$ is of formula (X-a47). In certain embodiments $-L^1-$ is of formula (X-a48). In certain embodiments $-L^1-$ is of formula (X-a49). In certain embodiments $-L^1-$ is of formula (X-a50). In certain embodiments $-L^1-$ is of formula (X-a51). In certain embodiments $-L^1-$ is of formula (X-a52). In certain embodiments $-L^1-$ is of formula (X-a53). In certain embodiments $-L^1-$ is of formula (X-a54). In certain embodiments $-L^1-$ is of formula (X-a55). In certain embodiments $-L^1-$ is of formula (X-a56). In certain embodiments $-L^1-$ is of formula (X-a57). In certain embodiments $-L^1-$ is of formula (X-a58). In certain embodiments $-L^1-$ is of formula (X-a59). In certain embodiments $-L^1-$ is of formula (X-a60). In certain embodiments $-L^1-$ is of formula (X-a61). In certain embodiments $-L^1-$ is of formula (X-a62). In certain embodiments $-L^1-$ is of formula (X-a63). In certain embodiments $-L^1-$ is of formula (X-a64). In certain embodiments $-L^1-$ is of formula (X-a65). In certain embodiments $-L^1-$ is of formula (X-a66). In certain embodiments $-L^1-$ is of formula (X-a67). In certain embodiments $-L^1-$ is of formula (X-a68). In certain embodiments $-L^1-$ is of formula (X-a69). In certain embodiments $-L^1-$ is of formula (X-a70). In certain embodiments $-L^1-$ is of formula (X-a71). In certain embodiments $-L^1-$ is of formula (X-a72). In certain embodiments $-L^1-$ is of formula (X-a73). In certain embodiments $-L^1-$ is of formula (X-a74). In certain embodiments $-L^1-$ is of formula (X-a75). In certain embodiments $-L^1-$ is of formula (X-a76). In certain embodiments $-L^1-$ is of formula (X-a77). In certain embodiments $-L^1-$ is of formula (X-a78).

In certain embodiments release half-life, i.e. the time in which half of all moieties -D are released from $-L^1-$, is pH independent, in particular independent for a pH ranging from about 6.8 to about 7.4. Such pH-independent release is advantageous, because pH in tumor tissue may vary and such pH-independence allows for a more uniform and thus more predictable drug release.

It was surprisingly found that moieties $-L^1-$ of formula (X-a11) and (X-a12) have a release half-life that is independent of pH for a pH ranging from 6.8 to 7.4.

In certain embodiments the moiety $-L^1$-D is of formula (X-b1)

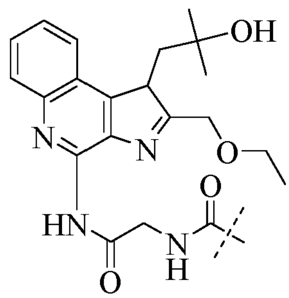

(X-b1),

wherein the dashed line indicates attachment to $-L^2-$.

In certain embodiments the moiety $-L^1$-D is of formula (X-b2)

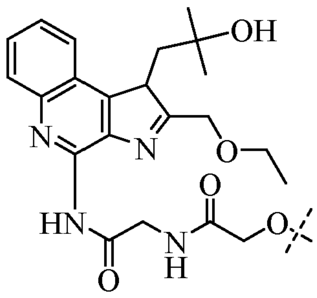

(X-b2),

wherein the dashed line indicates attachment to $-L^2-$.

In certain embodiments the moiety $-L^1-D$ is of formula (X-b5)

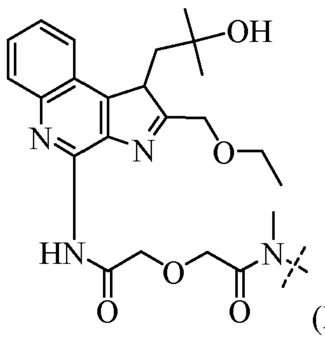

(X-b5),

wherein the dashed line indicates attachment to $-L^2-$.

In certain embodiments the moiety $-L^1-D$ is of formula (X-b6)

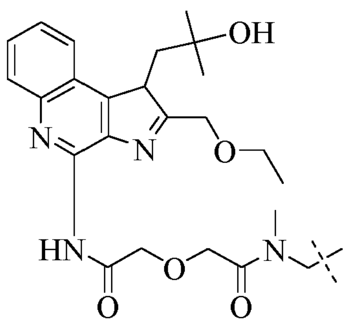

(X-b6),

wherein the dashed line indicates attachment to $-L^2-$.

In certain embodiments $-L^1-$ is of formula (XI)

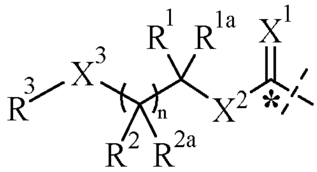

(XI),

wherein

the dashed line indicates the attachment to a $\pi$-electron-pair-donating heteroaromatic N of -D;
n is an integer selected from the group consisting of 0, 1, 2, 3 and 4;
$=X^1$ is selected from the group consisting of =O, =S and $=N(R^4)$;
$-X^2-$ is selected from the group consisting of -O-, -S-, $-N(R^5)-$ and $-C(R^6)(R^{6a})-$;
$-X^3-$ is selected from the group consisting of

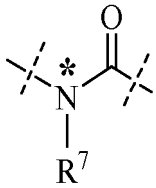 , 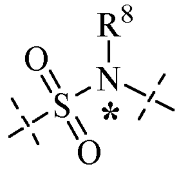 , 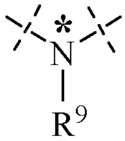 ,

$-C(R^{10})(R^{10a})-$, $-C(R^{11})(R^{11a})-C(R^{12})(R^{12a})-$, -O- and -C(O)-;
$-R^1$, $-R^{1a}$, $-R^6$, $-R^{6a}$, $-R^{10}$, $-R^{10a}$, $-R^{11}$, $-R^{11a}$, $-R^{12}$, $-R^{12a}$ and each of $-R^2$ and $-R^{2a}$ are independently selected from the group consisting of -H, -C(O)OH, halogen, -CN, -OH, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more $-R^{13}$, which are the same or different; and wherein $C_{1-6}$ alkyl, $C_{2-6}$ alkenyl and $C_{2-6}$ alkynyl are optionally interrupted by one or more groups selected from the group consisting of -T-, -C(O)O-, -O-, -C(O)-, $-C(O)N(R^{14})-$, $-S(O)_2N(R^{14})-$, $-S(O)N(R^{14})-$, $-S(O)_2-$, -S(O)-, $-N(R^{14})S(O)_2N(R^{14a})-$, -S-, $-N(R^{14})-$, $-OC(OR^{14})(R^{14a})-$, $-N(R^{14})C(O)N(R^{14a})-$ and $-OC(O)N(R^{14})-$;
$-R^3$, $-R^4$, $-R^5$, $-R^7$, $-R^8$ and $-R^9$ are independently selected from the group consisting of -H, -T, -CN, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more $-R^{13}$, which are the same or different; and wherein $C_{1-6}$ alkyl, $C_{2-6}$ alkenyl and $C_{2-6}$ alkynyl are optionally interrupted by one or more groups selected from the group consisting of -T-, -C(O)O-, -O-, -C(O)-, $-C(O)N(R^{14})-$, $-S(O)_2N(R^{14})-$, $-S(O)N(R^{14})-$, $-S(O)_2-$, -S(O)-, $-N(R^{14})S(O)_2N(R^{14a})-$, -S-, $-N(R^{14})-$, $-OC(OR^{14})(R^{14a})-$, $-N(R^{14})C(O)N(R^{14a})-$ and $-OC(O)N(R^{14})-$;

each T is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl; wherein each T is independently optionally substituted with one or more $-R^{13}$, which are the same or different;
wherein $-R^{13}$ is selected from the group consisting of -H, $-NO_2$, $-OCH_3$, -CN, $-N(R^{14})(R^{14a})$, -OH, -C(O)OH and $C_{1-6}$ alkyl; wherein $C_{1-6}$ alkyl is optionally substituted with one or more halogen, which are the same or different;
wherein $-R^{14}$ and $-R^{14a}$ are independently selected from the group consisting of -H and $C_{1-6}$ alkyl; wherein $C_{1-6}$ alkyl is optionally substituted with one or more halogen, which are the same or different;

optionally, one or more of the pairs $-R^1/-R^{1a}$, $-R^2/-R^{2a}$, two adjacent $R^2$, $-R^6/-R^{6a}$, $-R^{10}/-R^{10a}$, $-R^{11}/-R^{11a}$ and $-R^{12}/-R^{12a}$ are joined together with the atom to which they are attached to form a $C_{3-10}$ cycloalkyl, 3- to 10-membered heterocyclyl or an 8- to 11-membered heterobicyclyl;
optionally, one or more of the pairs $-R^1/-R^2$, $-R^1/-R^5$, $-R^1/-R^6$, $-R^1/-R^9$, $-R^1/-R^{10}$, $-R^3/-R^{6a}$, $-R^4/-R^5$, $-R^{4a}/-R^5$, $-R^4/-R^6$, $-R^5/-R^{10}$, $-R^6/-R^{10}$ and $-R^{4a}/-R^6$ are joined together with the atoms to which they are attached to form a ring -A-;
wherein -A- is selected from the group consisting of phenyl, naphthyl, indenyl, indanyl, tetralinyl, $C_{3-10}$ cycloalkyl, 3- to 10-membered heterocyclyl and 8- to 11-membered heterobicyclyl;
optionally, $-R^1$ and an adjacent $-R^2$ form a carbon-carbon double bond provided that n is selected from the group consisting of 1, 2, 3 and 4;
optionally, two adjacent $-R^2$ form a carbon-carbon double bond provided that n is selected from the group consisting of 2, 3 and 4;
provided that if $-X^2-$ is $-N(R^5)-$, $-X^3-$ is selected from the group consisting of

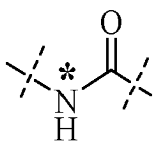 , 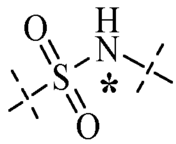 and 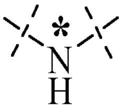 ,

and the distance between the nitrogen atom marked with an asterisk and the carbon atom marked with an asterisk in formula (XI) is 5, 6 or 7 atoms and if present the carbon-carbon double bond formed between $-R^1$ and $-R^2$ or two adjacent $-R^2$ is in a cis configuration; and
wherein $-L^1-$ is substituted with $-L^2-$ and wherein $-L^1-$ is optionally further substituted.

It is understood that two adjacent $-R^2$ in formula (XI) can only exist if n is at least 2.

It is understood that the expression "distance between the nitrogen atom marked with an asterisk and the carbon atom marked with an asterisk" refers to the total number of atoms in the shortest distance between the nitrogen and carbon atoms marked with the asterisk and also includes the nitrogen and carbon atoms marked with the asterisk. For example, in

the structure below, n is 1 and the distance between the nitrogen marked with an asterisk and the carbon marked with an asterisk is 5:

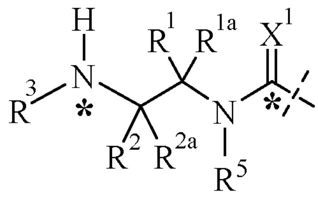

and in the structure below, n is 2, $-R^1$ and $-R^{1a}$ form a cyclohexal and the distance between the nitrogen marked with an asterisk and the carbon marked with an asterisk is 6:

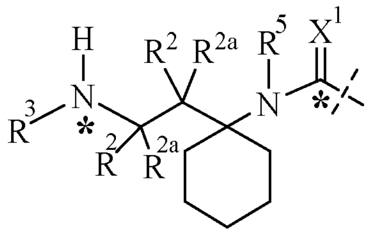

The optional further substituents of $-L^1-$ of formula (XI) are as described elsewhere herein.

In certain embodiments $-L^1-$ of formula (XI) is not further substituted.

In certain embodiments $=X^1$ of formula (XI) is =O. In certain embodiments $=X^1$ of formula (XI) is =S. In certain embodiments $=X^1$ of formula (XI) is $=N(R^4)$.

In certain embodiments $-X^2-$ of formula (XI) is -O-. In certain embodiments $-X^2-$ of formula (XI) is -S-. In certain embodiments $-X^2-$ of formula (XI) is $-N(R^5)-$. In certain embodiments $-X^2-$ of formula (XI) is $-C(R^6)(R^{6a})-$.

In certain embodiments $-X^3-$ of formula (XI) is

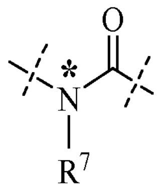

In certain embodiments $-X^3-$ of formula (XI) is

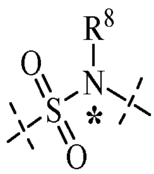

In certain embodiments $-X^3-$ of formula (XI) is

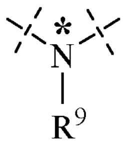

In certain embodiments $-X^3-$ of formula (XI) is $-C(R^{10})(R^{10a})-$. In certain embodiments $-X^3-$ of formula (XI) is $-C(R^{11})(R^{11a})-C(R^{12})(R^{12a})-$. In certain embodiments $-X^3-$ of formula (XI) is -O-. In certain embodiments $-X^3-$ of formula (XI) is -C(O)-.

In certain embodiments $-X^2-$ of formula (XI) is $-N(R^5)-$, $-X^3-$ is

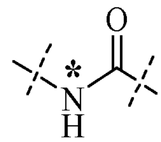

and the distance between the nitrogen atom marked with an asterisk and the carbon atom marked with an asterisk in formula (XI) is 5 atoms.

In certain embodiments -$X^2$- of formula (XI) is -N($R^5$)-, -$X^3$- is

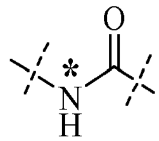

and the distance between the nitrogen atom marked with an asterisk and the carbon atom marked with an asterisk in formula (XI) is 6 atoms.

In certain embodiments -$X^2$- of formula (XI) is -N($R^5$)-, -$X^3$- is

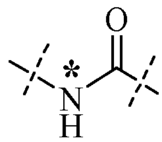

and the distance between the nitrogen atom marked with an asterisk and the carbon atom marked with an asterisk in formula (XI) is 7 atoms.

In certain embodiments -$X^2$- of formula (XI) is -N($R^5$)-, -$X^3$- is

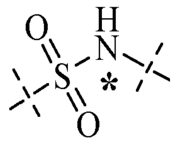

and the distance between the nitrogen atom marked with an asterisk and the carbon atom marked with an asterisk in formula (XI) is 5 atoms.

In certain embodiments -$X^2$- of formula (XI) is -N($R^5$)-, -$X^3$- is

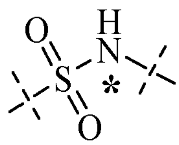

and the distance between the nitrogen atom marked with an asterisk and the carbon atom marked with an asterisk in formula (XI) is 6 atoms.

In certain embodiments -$X^2$- of formula (XI) is -N($R^5$)-, -$X^3$- is

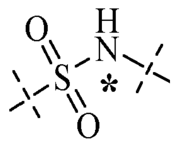

and the distance between the nitrogen atom marked with an asterisk and the carbon atom marked with an asterisk in formula (XI) is 7 atoms.

In certain embodiments -$X^2$- of formula (XI) is -N($R^5$)-, -$X^3$- is

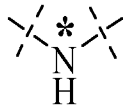

and the distance between the nitrogen atom marked with an asterisk and the carbon atom marked with an asterisk in formula (XI) is 5 atoms.

In certain embodiments $-X^2-$ of formula (XI) is $-N(R^5)-$, $-X^3-$ is

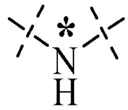

and the distance between the nitrogen atom marked with an asterisk and the carbon atom marked with an asterisk in formula (XI) is 6 atoms.

In certain embodiments $-X^2-$ of formula (XI) is $-N(R^5)-$, $-X^3-$ is

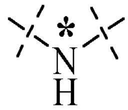

and the distance between the nitrogen atom marked with an asterisk and the carbon atom marked with an asterisk in formula (XI) is 7 atoms.

In certain embodiments $-R^1$, $-R^{1a}$, $-R^6$, $-R^{6a}$, $-R^{10}$, $-R^{10a}$, $-R^{11}$, $-R^{11a}$, $-R^{12}$, $-R^{12a}$ and each of $-R^2$ and $-R^{2a}$ of formula (XI) are independently selected from the group consisting of -H, -C(O)OH, halogen, -CN, -OH, $C_{1-6}$ alkyl, $C_{2-6}$ alkenyl and $C_{2-6}$ alkynyl.

In certain embodiments $-R^1$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments $-R^1$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments $-R^1$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments $-R^1$ of formula (XI) is selected from the group consisting of -H, -C(O)OH, -OH and $C_{1-6}$ alkyl. In certain embodiments $-R^1$ of formula (XI) is -H. In certain embodiments $-R^1$ of formula (XI) is -C(O)OH. In certain embodiments $-R^1$ of formula (XI) is halogen. In certain embodiments $-R^1$ of formula (XI) is -F. In certain embodiments $-R^1$ of formula (XI) is -CN. In certain embodiments $-R^1$ of formula (XI) is -OH. In certain embodiments $-R^1$ of formula (XI) is $C_{1-6}$ alkyl. In certain embodiments $-R^1$ of formula (XI) is $C_{2-6}$ alkenyl. In certain embodiments $-R^1$ of formula (XI) is $C_{2-6}$ alkynyl. In certain embodiments $-R^1$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments $-R^{1a}$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments $-R^{1a}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments $-R^{1a}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments $-R^{1a}$ of formula (XI) is selected from the group consisting of -H, -C(O)OH, -OH and $C_{1-6}$ alkyl. In certain embodiments $-R^{1a}$ of formula (XI) is -H. In certain embodiments $-R^{1a}$ of formula (XI) is -C(O)OH. In certain embodiments $-R^{1a}$ of formula (XI) is halogen. In certain embodiments $-R^{1a}$ of formula (XI) is -F. In certain embodiments $-R^{1a}$ of formula (XI) is -CN. In certain embodiments $-R^{1a}$ of formula (XI) is -OH. In certain embodiments $-R^{1a}$ of formula (XI) is $C_{1-6}$ alkyl. In certain embodiments $-R^{1a}$ of formula (XI) is $C_{2-6}$ alkenyl. In certain embodiments $-R^{1a}$ of formula (XI) is $C_{2-6}$ alkynyl. In certain embodiments $-R^{1a}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments $-R^6$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments $-R^6$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments $-R^6$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments $-R^6$ of formula (XI) is selected from the group consisting of -H, -C(O)OH, -OH and $C_{1-6}$ alkyl. In certain embodiments $-R^6$ of formula (XI) is -H. In certain embodiments $-R^6$ of formula (XI) is -C(O)OH. In certain embodiments $-R^6$ of formula (XI) is halogen. In certain embodiments $-R^6$ of formula (XI) is -F. In certain embodiments $-R^6$ of formula (XI) is

-CN. In certain embodiments -$R^6$ of formula (XI) is -OH. In certain embodiments -$R^6$ of formula (XI) is $C_{1-6}$ alkyl. In certain embodiments -$R^6$ of formula (XI) is $C_{2-6}$ alkenyl. In certain embodiments -$R^6$ of formula (XI) is $C_{2-6}$ alkynyl. In certain embodiments -$R^6$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^{6a}$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments -$R^{6a}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments -$R^{6a}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments -$R^{6a}$ of formula (XI) is selected from the group consisting of -H, -C(O)OH, -OH and $C_{1-6}$ alkyl. In certain embodiments -$R^{6a}$ of formula (XI) is -H. In certain embodiments -$R^{6a}$ of formula (XI) is -C(O)OH. In certain embodiments -$R^{6a}$ of formula (XI) is halogen. In certain embodiments -$R^{6a}$ of formula (XI) is -F. In certain embodiments -$R^{6a}$ of formula (XI) is -CN. In certain embodiments -$R^{6a}$ of formula (XI) is -OH. In certain embodiments -$R^{6a}$ of formula (XI) is $C_{1-6}$ alkyl. In certain embodiments -$R^{6a}$ of formula (XI) is $C_{2-6}$ alkenyl. In certain embodiments -$R^{6a}$ of formula (XI) is $C_{2-6}$ alkynyl. In certain embodiments -$R^{6a}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^{10}$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments -$R^{10}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments -$R^{10}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments -$R^{10}$ of formula (XI) is selected from the group consisting of -H, -C(O)OH, -OH and $C_{1-6}$ alkyl. In certain embodiments -$R^{10}$ of formula (XI) is -H. In certain embodiments -$R^{10}$ of formula (XI) is -C(O)OH. In certain embodiments -$R^{10}$ of formula (XI) is halogen. In certain embodiments -$R^{10}$ of formula (XI) is -F. In certain embodiments -$R^{10}$ of formula (XI) is -CN. In certain embodiments -$R^{10}$ of formula (XI) is -OH. In certain embodiments -$R^{10}$ of formula (XI) is $C_{1-6}$ alkyl. In certain embodiments -$R^{10}$ of formula (XI) is $C_{2-6}$ alkenyl. In certain embodiments -$R^{10}$ of formula (XI) is $C_{2-6}$ alkynyl. In certain embodiments -$R^{10}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^{10a}$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments -$R^{10a}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments -$R^{10a}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments -$R^{10a}$ of formula (XI) is selected from the group consisting of -H, -C(O)OH, -OH and $C_{1-6}$ alkyl. In certain embodiments -$R^{10a}$ of formula (XI) is -H. In certain embodiments -$R^{10a}$ of formula (XI) is -C(O)OH. In certain embodiments -$R^{10a}$ of formula (XI) is halogen. In certain embodiments -$R^{10a}$ of formula (XI) is -F. In certain embodiments -$R^{10a}$ of formula (XI) is -CN. In certain embodiments -$R^{10a}$ of formula (XI) is -OH. In certain embodiments -$R^{10a}$ of formula (XI) is $C_{1-6}$ alkyl. In certain embodiments -$R^{10a}$ of formula (XI) is $C_{2-6}$ alkenyl. In certain embodiments -$R^{10a}$ of formula (XI) is $C_{2-6}$ alkynyl. In certain embodiments -$R^{10a}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^{11}$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments -$R^{11}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments -$R^{11}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments -$R^{11}$ of formula (XI) is selected from the group consisting of -H, -C(O)OH, -OH and $C_{1-6}$ alkyl. In certain embodiments -$R^{11}$ of formula (XI) is -H. In certain embodiments -$R^{11}$ of formula (XI) is -C(O)OH. In certain embodiments -$R^{11}$ of formula (XI) is halogen. In certain embodiments -$R^{11}$ of formula (XI) is -F. In certain embodiments -$R^{11}$ of formula (XI) is -CN. In certain embodiments -$R^{11}$ of formula (XI) is -OH. In certain embodiments -$R^{11}$ of formula (XI) is $C_{1-6}$ alkyl. In certain embodiments -$R^{11}$ of formula (XI) is $C_{2-6}$ alkenyl. In certain embodiments -$R^{11}$ of formula (XI) is $C_{2-6}$ alkynyl. In certain embodiments -$R^{11}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^{11a}$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments -$R^{11a}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments -$R^{11a}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments -$R^{11a}$ of formula (XI) is selected from the group consisting of -H, -C(O)OH, -OH and $C_{1-6}$ alkyl. In certain

embodiments -$R^{11a}$ of formula (XI) is -H. In certain embodiments -$R^{11a}$ of formula (XI) is -C(O)OH. In certain embodiments -$R^{11a}$ of formula (XI) is halogen. In certain embodiments -$R^{11a}$ of formula (XI) is -F. In certain embodiments -$R^{11a}$ of formula (XI) is -CN. In certain embodiments -$R^{11a}$ of formula (XI) is -OH. In certain embodiments -$R^{11a}$ of formula (XI) is $C_{1-6}$ alkyl. In certain embodiments -$R^{11a}$ of formula (XI) is $C_{2-6}$ alkenyl. In certain embodiments -$R^{11a}$ of formula (XI) is $C_{2-6}$ alkynyl. In certain embodiments -$R^{11a}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^{12}$ of formula (XI) is selected from the group consisting of -H, -C(O)OH, halogen, -CN, -OH, $C_{1-6}$ alkyl, $C_{2-6}$ alkenyl and $C_{2-6}$ alkynyl. In certain embodiments -$R^{12}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments -$R^{12}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments -$R^{12}$ of formula (XI) is selected from the group consisting of -H, -C(O)OH, -OH and $C_{1-6}$ alkyl. In certain embodiments -$R^{12}$ of formula (XI) is -H. In certain embodiments -$R^{12}$ of formula (XI) is -C(O)OH. In certain embodiments -$R^{12}$ of formula (XI) is halogen. In certain embodiments -$R^{12}$ of formula (XI) is -F. In certain embodiments -$R^{12}$ of formula (XI) is -CN. In certain embodiments -$R^{12}$ of formula (XI) is -OH. In certain embodiments -$R^{12}$ of formula (XI) is $C_{1-6}$ alkyl. In certain embodiments -$R^{12}$ of formula (XI) is $C_{2-6}$ alkenyl. In certain embodiments -$R^{12}$ of formula (XI) is $C_{2-6}$ alkynyl. In certain embodiments -$R^{12}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^{12a}$ of formula (XI) is selected from the group consisting of -H,-C(O)OH, halogen, -CN, -OH, $C_{1-6}$ alkyl, $C_{2-6}$ alkenyl and $C_{2-6}$ alkynyl. In certain embodiments -$R^{12a}$ of formula (XI) is selected from the group consisting of -H, -C(O)OH, -CN, -OH, $C_{1-6}$ alkyl, $C_{2-6}$ alkenyl and $C_{2-6}$ alkynyl. In certain embodiments -$R^{12a}$ of formula (XI) is selected from the group consisting of -H, -C(O)OH, halogen, -OH, $C_{1-6}$ alkyl, $C_{2-6}$ alkenyl and $C_{2-6}$ alkynyl. In certain embodiments -$R^{12a}$ of formula (XI) is selected from the group consisting of -H, -C(O)OH, -OH and $C_{1-6}$ alkyl. In certain embodiments -$R^{12a}$ of formula (XI) is -H. In certain embodiments -$R^{12a}$ of formula (XI) is -C(O)OH. In certain embodiments -$R^{12a}$ of formula (XI) is halogen. In certain embodiments -$R^{12a}$ of formula (XI) is -F. In certain embodiments -$R^{12a}$ of formula (XI) is -CN. In certain embodiments -$R^{12a}$ of formula (XI) is -OH. In certain embodiments -$R^{12a}$ of formula (XI) is $C_{1-6}$ alkyl. In certain embodiments -$R^{12a}$ of formula (XI) is $C_{2-6}$ alkenyl. In certain embodiments -$R^{12a}$ of formula (XI) is $C_{2-6}$ alkynyl. In certain embodiments -$R^{12a}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments each of -$R^2$ of formula (XI) is independently selected from the group consisting of -H, -C(O)OH, halogen, -CN, -OH, $C_{1-6}$ alkyl, $C_{2-6}$ alkenyl and $C_{2-6}$ alkynyl. In certain embodiments each of -$R^2$ of formula (XI) is independently selected from the group consisting of -H, -C(O)OH, -CN, -OH, $C_{1-6}$ alkyl, $C_{2-6}$ alkenyl and $C_{2-6}$ alkynyl. In certain embodiments each of -$R^2$ of formula (XI) is independently selected from the group consisting of -H, -C(O)OH, halogen, -OH, $C_{1-6}$ alkyl, $C_{2-6}$ alkenyl and $C_{2-6}$ alkynyl. In certain embodiments each of -$R^2$ of formula (XI) is independently selected from the group consisting of -H, -C(O)OH, -OH and $C_{1-6}$ alkyl. In certain embodiments each of -$R^2$ of formula (XI) is -H. In certain embodiments each of -$R^2$ of formula (XI) is -C(O)OH. In certain embodiments each of -$R^2$ of formula (XI) is halogen. In certain embodiments each of -$R^2$ of formula (XI) is -F. In certain embodiments each of -$R^2$ of formula (XI) is -CN. In certain embodiments each of -$R^2$ of formula (XI) is -OH. In certain embodiments each of -$R^2$ of formula (XI) is $C_{1-6}$ alkyl. In certain embodiments each of -$R^2$ of formula (XI) is $C_{2-6}$ alkenyl. In certain embodiments each of -$R^2$ of formula (XI) is $C_{2-6}$ alkynyl. In certain embodiments each of -$R^2$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments each of -$R^{2a}$ of formula (XI) is independently selected from the group consisting of -H, -C(O)OH, halogen, -CN, -OH, $C_{1-6}$ alkyl, $C_{2-6}$ alkenyl and $C_{2-6}$ alkynyl. In certain embodiments each of -$R^{2a}$ of formula (XI) is independently selected from the group consisting of -H, -C(O)OH, -CN, -OH, $C_{1-6}$ alkyl, $C_{2-6}$ alkenyl and $C_{2-6}$ alkynyl. In certain embodiments each of -$R^{2a}$ of formula (XI) is independently selected from the group consisting of -H, -C(O)OH, halogen, -OH, $C_{1-6}$ alkyl, $C_{2-6}$ alkenyl and $C_{2-6}$ alkynyl. In certain embodiments each of -$R^{2a}$ of formula (XI) is independently selected from the group consisting of -H, -C(O)OH, -OH and $C_{1-6}$ alkyl. In certain embodiments each of -$R^{2a}$ of formula (XI) is -H. In certain embodiments each of -$R^{2a}$ of formula (XI) is -C(O)OH. In certain embodiments each of -$R^{2a}$ of formula (XI) is halogen. In certain embodiments each of -$R^{2a}$ of formula (XI) is -F. In certain embodiments each of -$R^{2a}$ of formula (XI) is -CN. In certain embodiments each of - $R^{2a}$ of formula (XI) is -OH. In certain embodiments each of -$R^{2a}$ of formula (XI) is $C_{1-6}$ alkyl. In certain embodiments each of -$R^{2a}$ of formula (XI) is $C_{2-6}$ alkenyl. In certain embodiments each of -$R^{2a}$ of formula (XI) is $C_{2-6}$ alkynyl. In certain embodiments each of -$R^{2a}$ of formula (XI) is selected from the group consisting of -H, methyl, ethyl, n-propyl, iso-propyl, n-butyl, sec-butyl, iso-butyl, tert-butyl, n-pentyl, 1,1-dimethylpropyl, 2,2-dimethylpropyl, 3-methylbutyl, 1-methylbutyl and 1-ethylpropyl.

In certain embodiments -$R^3$, -$R^4$, -$R^5$, -$R^7$, -$R^8$ and -$R^9$ of formula (XI) are independently selected from the group

consisting of -H, -T, -CN, $C_{1-6}$ alkyl, $C_{2-6}$ alkenyl and $C_{2-6}$ alkynyl. In certain embodiments -$R^3$, -$R^4$, -$R^5$, -$R^7$, -$R^8$ and -$R^9$ of formula (XI) are independently selected from the group consisting of -H, -T, -CN, $C_{1-6}$ alkyl and $C_{2-6}$ alkenyl. In certain embodiments-$R^3$, -$R^4$, -$R^5$, -$R^7$, -$R^8$ and -$R^9$ of formula (XI) are independently selected from the group consisting of -H, -T, -CN and $C_{1-6}$ alkyl. In certain embodiments -$R^3$, -$R^4$, -$R^5$, -$R^7$, -$R^8$ and -$R^9$ of formula (XI) are independently selected from the group consisting of -H, -T and $C_{1-6}$ alkyl. In certain embodiments -$R^3$, -$R^4$, -$R^5$, -$R^7$, -$R^8$ and -$R^9$ of formula (XI) are independently selected from the group consisting of -H and $C_{1-6}$ alkyl.

In certain embodiments -$R^3$ of formula (XI) is selected from the group consisting of -H, -T, -CN, $C_{1-6}$ alkyl, $C_{2-6}$ alkenyl and $C_{2-6}$ alkynyl. In certain embodiments -$R^3$ of formula (XI) is -H. In certain embodiments -$R^3$ of formula (XI) is -T. In certain embodiments -$R^3$ of formula (XI) is -CN. In certain embodiments -$R^3$ of formula (XI) is $C_{1-6}$ alkyl. In certain embodiments -$R^3$ of formula (XI) is $C_{2-6}$ alkenyl. In certain embodiments -$R^3$ of formula (XI) is $C_{2-6}$ alkynyl.

In certain embodiments -$R^4$ of formula (XI) is selected from the group consisting of -H, -T, -CN, $C_{1-6}$ alkyl, $C_{2-6}$ alkenyl and $C_{2-6}$ alkynyl. In certain embodiments -$R^4$ of formula (XI) is -H. In certain embodiments -$R^4$ of formula (XI) is -T. In certain embodiments -$R^4$ of formula (XI) is -CN. In certain embodiments -$R^4$ of formula (XI) is $C_{1-6}$ alkyl. In certain embodiments -$R^4$ of formula (XI) is $C_{2-6}$ alkenyl. In certain embodiments -$R^4$ of formula (XI) is $C_{2-6}$ alkynyl.

In certain embodiments -$R^5$ of formula (XI) is selected from the group consisting of -H, -T, -CN, $C_{1-6}$ alkyl, $C_{2-6}$ alkenyl and $C_{2-6}$ alkynyl. In certain embodiments -$R^5$ of formula (XI) is -H. In certain embodiments -$R^5$ of formula (XI) is -T. In certain embodiments -$R^5$ of formula (XI) is -CN. In certain embodiments -$R^5$ of formula (XI) is $C_{1-6}$ alkyl. In certain embodiments -$R^5$ of formula (XI) is $C_{2-6}$ alkenyl. In certain embodiments -$R^5$ of formula (XI) is $C_{2-6}$ alkynyl.

In certain embodiments -$R^7$ of formula (XI) is selected from the group consisting of -H, -T, -CN, $C_{1-6}$ alkyl, $C_{2-6}$ alkenyl and $C_{2-6}$ alkynyl. In certain embodiments -$R^7$ of formula (XI) is -H. In certain embodiments -$R^7$ of formula (XI) is -T. In certain embodiments -$R^7$ of formula (XI) is -CN. In certain embodiments -$R^7$ of formula (XI) is $C_{1-6}$ alkyl. In certain embodiments -$R^7$ of formula (XI) is $C_{2-6}$ alkenyl. In certain embodiments -$R^7$ of formula (XI) is $C_{2-6}$ alkynyl.

In certain embodiments -$R^8$ of formula (XI) is selected from the group consisting of -H, -T, -CN, $C_{1-6}$ alkyl, $C_{2-6}$ alkenyl and $C_{2-6}$ alkynyl. In certain embodiments -$R^8$ of formula (XI) is -H. In certain embodiments -$R^8$ of formula (XI) is -T. In certain embodiments -$R^8$ of formula (XI) is -CN. In certain embodiments -$R^8$ of formula (XI) is $C_{1-6}$ alkyl. In certain embodiments -$R^8$ of formula (XI) is $C_{2-6}$ alkenyl. In certain embodiments -$R^8$ of formula (XI) is $C_{2-6}$ alkynyl.

In certain embodiments -$R^9$ of formula (XI) is selected from the group consisting of -H, -T, -CN, $C_{1-6}$ alkyl, $C_{2-6}$ alkenyl and $C_{2-6}$ alkynyl. In certain embodiments -$R^9$ of formula (XI) is -H. In certain embodiments -$R^9$ of formula (XI) is -T. In certain embodiments -$R^9$ of formula (XI) is -CN. In certain embodiments -$R^9$ of formula (XI) is $C_{1-6}$ alkyl. In certain embodiments -$R^9$ of formula (XI) is $C_{2-6}$ alkenyl. In certain embodiments -$R^9$ of formula (XI) is $C_{2-6}$ alkynyl.

In certain embodiments T of formula (XI) is selected from the group consisting of phenyl, naphthyl, indenyl, indanyl, tetralinyl, $C_{3-10}$ cycloalkyl, 3- to 10-membered heterocyclyl and 8-to 11-membered heterobicyclyl. In certain embodiments T of formula (XI) is phenyl. In certain embodiments T of formula (XI) is naphthyl. In certain embodiments T of formula (XI) is indenyl. In certain embodiments T of formula (XI) is indanyl. In certain embodiments T of formula (XI) is tetralinyl. In certain embodiments T of formula (XI) is $C_{3-10}$ cycloalkyl. In certain embodiments T of formula (XI) is 3- to 10-membered heterocyclyl. In certain embodiments T of formula (XI) is 8- to 11-membered heterobicyclyl.

In certain embodiments T of formula (XI) is substituted with one or more -$R^{13}$, which are the same or different.

In certain embodiments T of formula (XI) is substituted with one -$R^{13}$.

In certain embodiments T of formula (XI) is not substituted with -$R^{13}$.

In certain embodiments -$R^{13}$ of formula (XI) is selected from the group consisting of -H, -$NO_2$, -$OCH_3$, -CN, -N($R^{14}$)($R^{14a}$), -OH, -C(O)OH and $C_{1-6}$ alkyl.

In certain embodiments -$R^{13}$ of formula (XI) is -H. In certain embodiments -$R^{13}$ of formula (XI) is -$NO_2$. In certain embodiments -$R^{13}$ of formula (XI) is -$OCH_3$. In certain embodiments -$R^{13}$ of formula (XI) is -CN. In certain embodiments -$R^{13}$ of formula (XI) is -N($R^{14}$)($R^{14a}$). In certain embodiments -$R^{13}$ of formula (XI) is -OH. In certain embodiments -$R^{13}$ of formula (XI) is -C(O)OH. In certain embodiments -$R^{13}$ of formula (XI) is $C_{1-6}$ alkyl.

In certain embodiments -$R^{14}$ and -$R^{14a}$ of formula (XI) are independently selected from the group consisting of -H and $C_{1-6}$ alkyl. In certain embodiments -$R^{14}$ of formula (XI) is -H. In certain embodiments -$R^{14}$ of formula (XI) is $C_{1-6}$ alkyl. In certain embodiments -$R^{14a}$ of formula (XI) is -H. In certain embodiments -$R^{14a}$ of formula (XI) is $C_{1-6}$ alkyl.

In certain embodiments n of formula (XI) is selected from the group consisting of 0, 1, 2 and 3. In certain embodiments n of formula (XI) is selected from the group consisting of 0, 1 and 2. In certain embodiments n of formula (XI) is selected from the group consisting of 0 and 1. In certain embodiments n of formula (XI) is 0. In certain embodiments n of formula (I) is 1. In certain embodiments n of formula (XI) is 2. In certain embodiments n of formula (I) is 3. In certain embodiments n of formula (XI) is 4.

In certain embodiments -$L^1$- of formula (XI) is connected to -D through a linkage selected from the group consisting of amide, carbamate, dithiocarbamate, O-thiocarbamate, S-thiocarbamate, urea, thiourea, thioamide, amidine and guanidine. It is understood that some of these linkages may not be reversible *per se,* but that in the present invention neighboring groups present in -$L^1$-, such as for example amide, primary amine, secondary amine and tertiary amine, render these linkages reversible.

In certain embodiments $-L^1-$ of formula (XI) is conjugated to -D through an amide linkage, i.e. $=X^1$ is =O and $-X^2-$ is $-C(R^6)(R^{6a})-$.
In certain embodiments $-L^1-$ of formula (XI) is conjugated to -D through a carbamate linkage, i.e. $=X^1$ is =O and $-X^2-$ is -O-.
In certain embodiments $-L^1-$ of formula (XI) is conjugated to -D through a dithiocarbamate linkage, i.e. $=X^1$ is =S and $-X^2-$ is -S-.
In certain embodiments $-L^1-$ of formula (XI) is conjugated to -D through an O-thiocarbamate linkage, i.e. $=X^1$ is =S and $-X^2-$ is -O-.
In certain embodiments $-L^1-$ of formula (XI) is conjugated to -D through a S-thiocarbamate linkage, i.e. $=X^1$ is =O and $-X^2-$ is -S-.
In certain embodiments $-L^1-$ of formula (XI) is conjugated to -D through a urea linkage, i.e. $=X^1$ is =O and $-X^2-$ is $-N(R^5)-$.
In certain embodiments $-L^1-$ of formula (XI) is conjugated to -D through a thiourea linkage, i.e. $=X^1$ is =S and $-X^2-$ is $-N(R^5)-$.
In certain embodiments $-L^1-$ of formula (XI) is conjugated to -D through a thioamide linkage, i.e. $=X^1$ is =S and $-X^2-$ is $-C(R^6)(R^{6a})-$.
In certain embodiments $-L^1-$ of formula (XI) is conjugated to -D through an amidine linkage, i.e. $=X^1$ is $=N(R^4)$ and $-X^2-$ is $-C(R^6)(R^{6a})-$.
In certain embodiments $-L^1-$ of formula (XI) is conjugated to -D through a guanidine linkage, i.e. $=X^1$ is $=N(R^4)$ and $-X^2-$ is $-N(R^5)-$.
In certain embodiments $-L^1-$ is of formula (XI'):

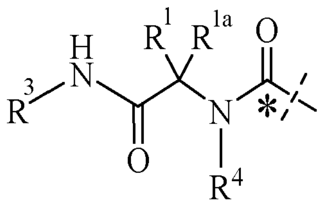

(XI′),

wherein the dashed line indicates the attachment to a $\pi$-electron-pair-donating heteroaromatic N of -D; and

$-R^1$, $-R^{1a}$, $-R^3$ and $-R^4$ are used as defined in formula (XI).

In certain embodiments $-R^1$ and $-R^{1a}$ of formula (XI') are both -H.
In certain embodiments $-R^1$ of formula (XI') is -H and $-R^{1a}$ of formula (XI') is $C_{1-6}$ alkyl.
In certain embodiments $-R^3$ of formula (XI') is $C_{1-6}$ alkyl.
In certain embodiments $-R^4$ of formula (XI') is methyl.
In certain embodiments $-R^4$ of formula (XI') is ethyl.
In certain embodiments $-L^1-$ is of formula (XII)

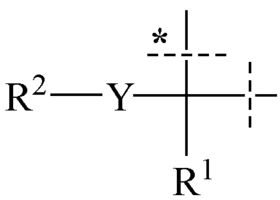

(XII)

wherein

the dashed line marked with an asterisk indicates the attachment to $-L^2-$;
the unmarked dashed line indicates the attachment to a $\pi$-electron-pair-donating heteroaromatic N of -D;
-Y- is selected from the group consisting of $-N(R^3)-$, -O- and -S-;
$-R^1$, $-R^2$ and $-R^3$ are independently selected from the group consisting of -H, -T, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more $-R^4$, which are the same or different; and wherein $C_{1-6}$ alkyl, $C_{2-6}$ alkenyl and $C_{2-6}$ alkynyl are optionally interrupted by one or more groups selected from the group consisting of -T-, -C(O)O-, -O-, -C(O)-, $-C(O)N(R^5)-$, $-S(O)_2N(R^5)-$, $-S(O)N(R^5)-$,

-$S(O)_2$-, -S(O)-, -$N(R^5)S(O)_2N(R^{5a})$-, -S-, -$N(R^5)$, -$OC(OR^5)(R^{5a})$-, -$N(R^5)C(O)N(R^{5a})$- and -$OC(O)N(R^5)$-;

each T is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl, wherein each T is independently optionally substituted with one or more -$R^4$, which are the same or different;
wherein -$R^4$, -$R^5$ and -$R^{5a}$ are independently selected from the group consisting of -H and $C_{1-6}$ alkyl; wherein $C_{1-6}$ alkyl is optionally substituted with one or more halogen, which are the same or different; and

wherein -$L^1$- is substituted with -$L^2$- and wherein -$L^1$- is optionally further substituted.

The optional further substituents of -$L^1$- of formula (XII) are as described elsewhere herein.
In certain embodiments -$L^1$- of formula (XII) is not further substituted.
In certain embodiments -Y- of formula (XII) is -$N(R^3)$-.
In certain embodiments -Y- of formula (XII) is -O-.
In certain embodiments -Y- of formula (XII) is -S-.
In certain embodiments -$R^1$, -$R^2$ and -$R^3$ of formula (XII) are independently selected from the group consisting of -H, -T, $C_{1-6}$ alkyl, $C_{2-6}$ alkenyl and $C_{2-6}$ alkynyl.
In certain embodiments -$R^1$ of formula (XII) is independently selected from the group consisting of -H, -T, $C_{1-6}$ alkyl, $C_{2-6}$ alkenyl and $C_{2-6}$ alkynyl. In certain embodiments -$R^1$ of formula (XII) is -H. In certain embodiments -$R^1$ of formula (XII) is -T. In certain embodiments -$R^1$ of formula (XII) is $C_{1-6}$ alkyl. In certain embodiments -$R^1$ of formula (XII) is $C_{2-6}$ alkenyl. In certain embodiments -$R^1$ of formula (XII) is $C_{2-6}$ alkynyl.
In certain embodiments -$R^2$ of formula (XII) is independently selected from the group consisting of -H, -T, $C_{1-6}$ alkyl, $C_{2-6}$ alkenyl and $C_{2-6}$ alkynyl. In certain embodiments -$R^2$ of formula (XII) is -H. In certain embodiments -$R^2$ of formula (XII) is -T. In certain embodiments -$R^2$ of formula (XII) is $C_{1-6}$ alkyl. In certain embodiments -$R^2$ of formula (XII) is $C_{2-6}$ alkenyl.
In certain embodiments -$R^2$ of formula (XII) is $C_{2-6}$ alkynyl.
In certain embodiments -$R^3$ of formula (XII) is independently selected from the group consisting of -H, -T, $C_{1-6}$ alkyl, $C_{2-6}$ alkenyl and $C_{2-6}$ alkynyl. In certain embodiments -$R^3$ of formula (XII) is -H. In certain embodiments -$R^3$ of formula (XII) is -T. In certain embodiments -$R^3$ of formula (XII) is $C_{1-6}$ alkyl. In certain embodiments -$R^3$ of formula (XII) is $C_{2-6}$ alkenyl. In certain embodiments -$R^3$ of formula (XII) is $C_{2-6}$ alkynyl.
In certain embodiments T of formula (XII) is selected from the group consisting of phenyl, naphthyl, indenyl, indanyl, tetralinyl, $C_{3-10}$ cycloalkyl, 3- to 10-membered heterocyclyl and 8-to 11- heterobicyclyl. In certain embodiments T of formula (XII) is phenyl. In certain embodiments T of formula (XII) is naphthyl. In certain embodiments T of formula (XII) is indenyl. In certain embodiments T of formula (XII) is indanyl. In certain embodiments T of formula (XII) is tetralinyl. In certain embodiments T of formula (XII) is $C_{3-10}$ cycloalkyl. In certain embodiments T of formula (XII) is 3- to 10-membered heterocyclyl. In certain embodiments T of formula (XII) is 8- to 11-heterobicyclyl.
In certain embodiments T of formula (XII) is substituted with one or more -$R^4$.
In certain embodiments T of formula (XII) is substituted with one -$R^4$.
In certain embodiments T of formula (XII) is not substituted with -$R^4$.
In certain embodiments -$R^4$, -$R^5$ and -$R^{5a}$ of formula (XII) are independently selected from the group consisting of -H and $C_{1-6}$ alkyl.
In certain embodiments -$R^4$ of formula (XII) is selected from the group consisting of -H and $C_{1-6}$ alkyl. In certain embodiments -$R^4$ of formula (XII) is -H. In certain embodiments -$R^4$ of formula (XII) is $C_{1-6}$ alkyl.
In certain embodiments -$R^5$ of formula (XII) is selected from the group consisting of -H and $C_{1-6}$ alkyl. In certain embodiments -$R^5$ of formula (XII) is -H. In certain embodiments -$R^5$ of formula (XII) is $C_{1-6}$ alkyl.
In certain embodiments -$R^{5a}$ of formula (XII) is selected from the group consisting of -H and $C_{1-6}$ alkyl. In certain embodiments -$R^{5a}$ of formula (XII) is -H. In certain embodiments -$R^{5a}$ of formula (XII) is $C_{1-6}$ alkyl.
In certain embodiments -$L^1$- of formula (XII) is connected to -D through a heminal linkage.
In certain embodiments -$L^1$- of formula (XII) is connected to -D through an aminal linkage.
In certain embodiments -$L^1$- of formula (XII) is connected to -D through a hemithioaminal linkage.
A moiety -$L^1$- suitable for drugs D that when bound to -$L^1$- comprise an electron-donating heteroaromatic $N^+$ moiety or a quaternary ammonium cation and becomes a moiety -$D^+$ upon linkage with -$L^1$- is of formula (XIII)

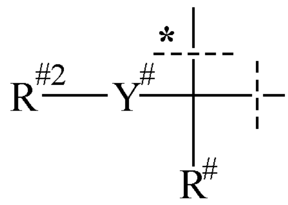

(XIII)

wherein

the dashed line marked with an asterisk indicates the attachment to $-L^2-$, the unmarked dashed line indicates the attachment to the $N^+$ of $-D^+$;

$-Y^{\#}-$ is selected from the group consisting of $-N(R^{\#3})-$, -O- and -S-;

$-R^{\#1}$, $-R^{\#2}$ and $-R^{\#3}$ are independently selected from the group consisting of -H, $-T^{\#}$, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more $-R^{\#4}$, which are the same or different; and wherein $C_{1-6}$ alkyl, $C_{2-6}$ alkenyl and $C_{2-6}$ alkynyl are optionally interrupted by one or more groups selected from the group consisting of $-T^{\#}-$, -C(O)O-, -O-, -C(O)-, $-C(O)N(R^{\#5})-$, $-S(O)_2N(R^{\#5})-$, $-S(O)N(R^{\#5})-$, $-S(O)_2-$, -S(O)-, $-N(R^{\#5})S(O)_2N(R^{\#5a})-$, -S-, $-N(R^{\#5})$, $-OC(OR^{\#5})(R^{\#5a})-$, $-N(R^{\#5})C(O)N(R^{\#5a})-$ and $-OC(O)N(R^{\#5})-$;

each $T^{\#}$ is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl, wherein each $T^{\#}$ is independently optionally substituted with one or more $-R^{\#4}$, which are the same or different; and

wherein $-R^{\#4}$, $-R^{\#5}$ and $-R^{\#5a}$ are independently selected from the group consisting of -H and $C_{1-6}$ alkyl; wherein $C_{1-6}$ alkyl is optionally substituted with one or more halogen, which are the same or different; and

each $-L^1-$ is substituted with $-L^2-$ and optionally further substituted.

It is understood that in certain embodiments $-D^+$ may comprise both an electron-donating heteroaromatic $N^+$ and a quaternary ammonium cation and analogously the corresponding D may comprise both an electron-donating heteroaromatic N and a tertiary amine. It is also understood that if D is conjugated to $-L^1-$, then $-D^+$ and $-L^1-$ form a quaternary ammonium cation, for which there may be a counter anion. Examples of counter anions include, but are not limited to, chloride, bromide, acetate, bicarbonate, sulfate, bisulfate, nitrate, carbonate, alkyl sulfonate, aryl sulfonate and phosphate.

Such drug moiety $-D^+$ comprises at least one, such as one, two, three, four, five, six, seven, eight, nine or ten electron-donating heteroaromatic $N^+$ or quaternary ammonium cations and analogously the corresponding released drug D comprises at least one, such as one, two, three, four, five, six, seven, eight, nine or ten electron-donating heteroaromatic N or tertiary amines. Examples of chemical structures including heteroaromatic nitrogens i.e. $N^+$ or N, that donate an electron to the aromatic $\pi$-system include, but are not limited to, pyridine, pyridazine, pyrimidine, quinoline, quinazoline, quinoxaline, pyrazole, imidazole, isoindazole, indazole, purine, tetrazole, triazole and triazine. For example, in the imidazole ring below the heteroaromatic nitrogen which donates one electron to the aromatic $\pi$-system is marked with "§":

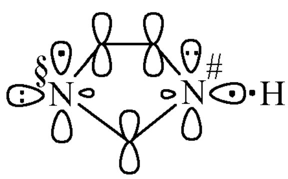

.

Such electron-donating heteroaromatic nitrogen atoms do not comprise heteroaromatic nitrogen atoms which donate one electron pair (i.e. not one electron) to the aromatic $\pi$-system, such as for example the nitrogen that is marked with "#" in the abovementioned imidazole ring structure. The drug D may exist in one or more tautomeric forms, such as with one hydrogen atom moving between at least two heteroaromatic nitrogen atoms. In all such cases, the linker moiety is covalently and reversibly attached at a heteroaromatic nitrogen that donates an electron to the aromatic $\pi$-system.

In certain embodiments - $Y^{\#}$- of formula (XIII) is $-N(R^{\#3})-$. In certain embodiments $-Y^{\#}-$ of formula (XI) is -O-. In certain embodiments $-Y^{\#}-$ of formula (XI) is -S-.

In certain embodiments $-R^{\#1}$, $-R^{\#2}$ and $-R^{\#3}$ of formula (XIII) are independently selected from the group consisting of -H, $-T^{\#}$, $C_{1-6}$ alkyl, $C_{2-6}$ alkenyl and $C_{2-6}$ alkynyl.

In certain embodiments $-R^{\#1}$ of formula (XIII) is independently selected from the group consisting of -H, $-T^{\#}$, $C_{1-6}$

alkyl, $C_{2-6}$ alkenyl and $C_{2-6}$ alkynyl. In certain embodiments -$R^{\#1}$ of formula (XIII) is -H. In certain embodiments -$R^{\#1}$ of formula (XIII) is -$T^{\#}$. In certain embodiments -$R^{\#1}$ of formula (XI) is $C_{1-6}$ alkyl. In certain embodiments -$R^{\#1}$ of formula (XIII) is $C_{2-6}$ alkenyl. In certain embodiments -$R^{\#1}$ of formula (XIII) is $C_{2-6}$ alkynyl.

In certain embodiments -$R^{\#2}$ of formula (XIII) is independently selected from the group consisting of -H, -$T^{\#}$, $C_{1-6}$ alkyl, $C_{2-6}$ alkenyl and $C_{2-6}$ alkynyl. In certain embodiments -$R^{\#2}$ of formula (XI) is -H. In certain embodiments -$R^2$ of formula (XIII) is -$T^{\#}$. In certain embodiments -$R^{\#2}$ of formula (XI) is $C_{1-6}$ alkyl. In certain embodiments -$R^{\#2}$ of formula (XIII) is $C_{2-6}$ alkenyl. In certain embodiments -$R^{\#2}$ of formula (XIII) is $C_{2-6}$ alkynyl.

In certain embodiments, -$R^{\#3}$ of formula (XIII) is independently selected from the group consisting of -H, -$T^{\#}$, $C_{1-6}$ alkyl, $C_{2-6}$ alkenyl and $C_{2-6}$ alkynyl. In certain embodiments -$R^{\#3}$ of formula (XIII) is -H. In certain embodiments -$R^{\#3}$ of formula (XIII) is -$T^{\#}$. In certain embodiments, -$R^{\#3}$ is $C_{1-6}$ alkyl. In certain embodiments -$R^{\#3}$ of formula (XIII) is $C_{2-6}$ alkenyl. In certain embodiments -$R^{\#3}$ of formula (XIII) is $C_{2-6}$ alkynyl.

In certain embodiments $T^{\#}$ of formula (XIII) is selected from the group consisting of phenyl, naphthyl, indenyl, indanyl, tetralinyl, $C_{3-10}$ cycloalkyl, 3- to 10-membered heterocyclyl and 8-to 11- heterobicyclyl. In certain embodiments $T^{\#}$ of formula (XIII) is phenyl. In certain embodiments $T^{\#}$ of formula (XIII) is naphthyl. In certain embodiments $T^{\#}$ of formula (XIII) is indenyl. In certain embodiments $T^{\#}$ of formula (XIII) is indanyl. In certain embodiments $T^{\#}$ of formula (XIII) is tetralinyl. In certain embodiments $T^{\#}$ of formula (XIII) is $C_{3-10}$ cycloalkyl. In certain embodiments $T^{\#}$ of formula (XIII) is 3- to 10-membered heterocyclyl. In certain embodiments $T^{\#}$ of formula (XIII) is 8- to 11-heterobicyclyl. In certain embodiments $T^{\#}$ of formula (XIII) is substituted with one or more -$R^4$.

In certain embodiments $T^{\#}$ of formula (XIII) is substituted with one -$R^4$.

In certain embodiments $T^{\#}$ of formula (XIII) is not substituted with -$R^4$.

In certain embodiments -$R^{\#4}$, -$R^{\#5}$ and -$R^{\#5a}$ of formula (XIII) are independently selected from the group consisting of -H and $C_{1-6}$ alkyl.

In certain embodiments -$R^{\#4}$ of formula (XIII) is selected from the group consisting of -H and $C_{1-6}$ alkyl. In certain embodiments -$R^{\#4}$ of formula (XIII) is -H. In certain embodiments -$R^{\#4}$ of formula (XIII) is $C_{1-6}$ alkyl.

In certain embodiments -$R^{\#5}$ of formula (XIII) is selected from the group consisting of -H and $C_{1-6}$ alkyl. In certain embodiments -$R^5$ of formula (XIII) is -H. In certain embodiments -$R^{\#5}$ of formula (XIII) is $C_{1-6}$ alkyl.

In certain embodiments -$R^{\#5a}$ of formula (XIII) is selected from the group consisting of -H and $C_{1-6}$ alkyl. In certain embodiments -$R^{\#5a}$ of formula (XIII) is -H. In certain embodiments -$R^{\#5a}$ of formula (XIII) is $C_{1-6}$ alkyl.

A moiety -$L^1$- suitable for drugs D that when bound to -$L^1$- comprise an electron-donating heteroaromatic $N^+$ moiety or a quaternary ammonium cation and becomes a moiety -$D^+$ upon linkage with -$L^1$- is of formula (XIV)

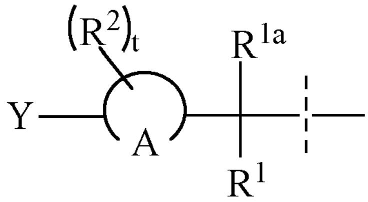

(XIV)

wherein

the dashed line indicates the attachment to the $N^+$ of -$D^+$;
t is selected from the group consisting of 0, 1, 2, 3, 4, 5 and 6;
-A- is a ring selected from the group consisting of monocyclic or bicyclic aryl and heteroaryl, provided that -A- is connected to -Y and -$C(R^1)(R^{1a})$- via carbon atoms;
wherein said monocyclic or bicyclic aryl and heteroaryl are optionally substituted with one or more -$R^2$, which are the same or different;
-$R^1$, -$R^{1a}$ and each -$R^2$ are independently selected from the group consisting of -H, -C(O)OH, -halogen, -$NO_2$, -CN, -OH, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more -$R^3$, which are the same or different; and wherein $C_{1-6}$ alkyl, $C_{2-6}$ alkenyl and $C_{2-6}$ alkynyl are optionally interrupted by one or more groups selected from the group consisting of -T-, -C(O)O-, -O-, -C(O)-, -C(O)N($R^4$)-, -$S(O)_2N(R^4)$-, -S(O)N($R^4$)-, -$S(O)_2$-, -S(O)-, -$N(R^4)S(O)_2N(R^{4a})$-, -S-, -N($R^4$)-, -$OC(OR^4)(R^{4a})$-, -$N(R^4)C(O)N(R^{4a})$- and -OC(O)N($R^4$)-;

each -T- is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl, wherein each -T- is independently optionally substituted with one or more -$R^3$, which are the same or different;
wherein -$R^3$ is selected from the group consisting of -H, -$NO_2$, -$OCH_3$, -CN, -$N(R^4)(R^{4a})$, -OH, -C(O)OH and $C_{1-6}$

alkyl; wherein $C_{1-6}$ alkyl is optionally substituted with one or more halogen, which are the same or different; wherein $-R^4$ and $-R^{4a}$ are independently selected from the group consisting of -H and $C_{1-6}$ alkyl; wherein $C_{1-6}$ alkyl is optionally substituted with one or more halogen, which are the same or different;

-Y is selected from the group consisting of:

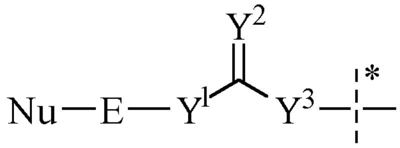,
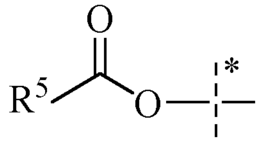,
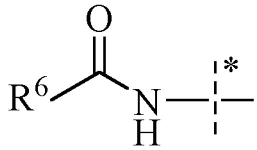,
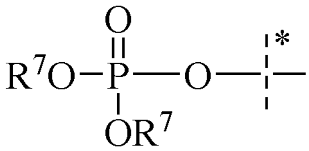,
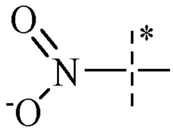,
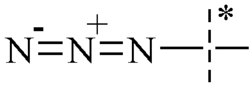,
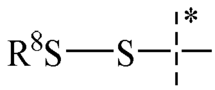,
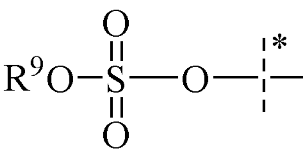,
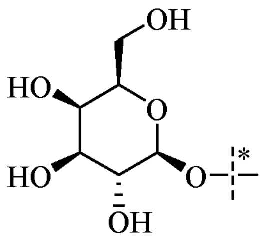,
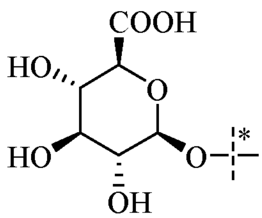,
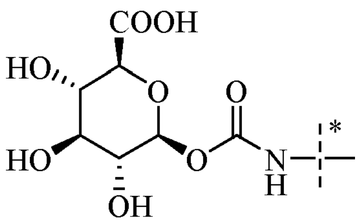

and a peptidyl moiety; wherein

the dashed line marked with an asterisk indicates the attachment to -A-;
-Nu is a nucleophile;
$-Y^1-$ is selected from the group consisting of -O-, $-C(R^{10})(R^{10a})-$, $-N(R^{11})-$ and -S-;
$=Y^2$ is selected from the group consisting of =O, =S and $=N(R^{12})$;
$-Y^3-$ is selected from the group consisting of -O-, -S- and $-N(R^{13})$;
-E- is selected from the group consisting of $C_{1-6}$ alkyl, $C_{2-6}$ alkenyl, $C_{2-6}$ alkynyl and -Q-; wherein $C_{1-6}$ alkyl, $C_{2-6}$ alkenyl, $C_{2-6}$ alkynyl are optionally substituted with one or more $-R^{14}$, which are the same or different;
$-R^5$, $-R^6$, each $-R^7$, $-R^8$, $-R^9$, $-R^{10}$, $-R^{10a}$ $-R^{11}$, $-R^{12}$ and $-R^{13}$ are independently selected from the group consisting of $C_{1-20}$ alkyl, $C_{2-20}$ alkenyl, $C_{2-20}$ alkynyl and -Q; wherein $C_{1-20}$ alkyl, $C_{2-20}$ alkenyl and $C_{2-20}$ alkynyl are optionally substituted with one or more $-R^{14}$, which are the same or different; and wherein $C_{1-10}$ alkyl, $C_{2-10}$ alkenyl and $C_{2-10}$ alkynyl are optionally interrupted by one or more groups selected from the group consisting of Q, -C(O)O-, -O-, -C(O)-, $-C(O)N(R^{15})-$, $-S(O)_2N(R^{15})$, $-S(O)N(R^{15})-$, $-S(O)_2-$, -S(O)-, $-N(R^{15})S(O)_2N(R^{15a})-$, -S-, $-N(R^{15})-$, $-OC(OR^{15})R^{15a}-$, $-N(R^{15})C(O)N(R^{15a})-$ and $-OC(O)N(R^{15})-$;

each Q is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl, wherein each Q is independently optionally substituted with one or more $-R^{14}$, which are the same or different;
wherein $-R^{14}$, $-R^{15}$ and $-R^{15a}$ are independently selected from the group consisting of -H and $C_{1-6}$ alkyl; wherein $C_{1-6}$ alkyl is optionally substituted with one or more halogen, which are the same or different; and

each -$L^1$- is substituted with -$L^2$- and optionally further substituted.

It is understood that in certain embodiments -$D^+$ may comprise both an electron-donating heteroaromatic $N^+$ and a quaternary ammonium cation and analogously the corresponding D may comprise both an electron-donating heteroaromatic N and a tertiary amine. It is also understood that if D is conjugated to -$L^1$-, then -$D^+$ and -$L^1$- form a quaternary ammonium cation, for which there may be a counter anion. Examples of counter anions include, but are not limited to, chloride, bromide, acetate, bicarbonate, sulfate, bisulfate, nitrate, carbonate, alkyl sulfonate, aryl sulfonate and phosphate.

The optional further substituents of -$L^1$- of formula (XIV) are as described elsewhere herein.

In certain embodiments -$L^1$- of formula (XIV) is not further substituted.

Such drug moiety -$D^+$ comprises at least one, such as one, two, three, four, five, six, seven, eight, nine or ten electron-donating heteroaromatic $N^+$ or quaternary ammonium cations and analogously the corresponding released drug D comprises at least one, such as one, two, three, four, five, six, seven, eight, nine or ten electron-donating heteroaromatic N or tertiary amines. Examples of chemical structures including heteroaromatic nitrogens i.e. $N^+$ or N, that donate an electron to the aromatic $\pi$-system include, but are not limited to, pyridine, pyridazine, pyrimidine, quinoline, quinazoline, quinoxaline, pyrazole, imidazole, isoindazole, indazole, purine, tetrazole, triazole and triazine. For example, in the imidazole ring below the heteroaromatic nitrogen which donates one electron to the aromatic $\pi$-system is marked with "§" .

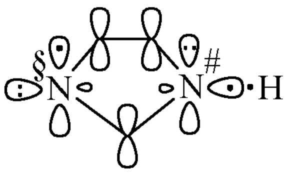.

Such electron-donating heteroaromatic nitrogen atoms do not comprise heteroaromatic nitrogen atoms which donate one electron pair (i.e. not one electron) to the aromatic $\pi$-system, such as for example the nitrogen that is marked with "#" in the abovementioned imidazole ring structure. The drug D may exist in one or more tautomeric forms, such as with one hydrogen atom moving between at least two heteroaromatic nitrogen atoms. **In** all such cases, the linker moiety is covalently and reversibly attached at a heteroaromatic nitrogen that donates an electron to the aromatic $\pi$-system.

As used herein, the term "monocyclic or bicyclic aryl" means an aromatic hydrocarbon ring system which may be monocyclic or bicyclic, wherein the monocyclic aryl ring consists of at least 5 ring carbon atoms and may comprise up to 10 ring carbon atoms and wherein the bicylic aryl ring consists of at least 8 ring carbon atoms and may comprise up to 12 ring carbon atoms. Each hydrogen atom of a monocyclic or bicyclic aryl may be replaced by a substituent as defined below.

As used herein, the term "monocyclic or bicyclic heteroaryl" means a monocyclic aromatic ring system that may comprise 2 to 6 ring carbon atoms and 1 to 3 ring heteroatoms or a bicyclic aromatic ring system that may comprise 3 to 9 ring carbon atoms and 1 to 5 ring heteroatoms, such as nitrogen, oxygen and sulfur. Examples for monocyclic or bicyclic heteroaryl groups include, but are not limited to, benzofuranyl, benzothiophenyl, furanyl, imidazolyl, indolyl, azaindolyl, azabenzimidazolyl, benzoxazolyl, benzthiazolyl, benzthiadiazolyl, benzotriazolyl, tetrazinyl, tetrazolyl, isothiazolyl, oxazolyl, isoxazolyl, pyrazinyl, pyrazolyl, pyridazinyl, pyridinyl, pyrimidinyl, pyrrolyl, quinolinyl, quinazolinyl, quinoxalinyl, triazolyl, thiazolyl and thiophenyl. Each hydrogen atom of a monocyclic or bicyclic heteroaryl may be replaced by a substituent as defined below.

As used herein, the term "nucleophile" refers to a reagent or functional group that forms a bond to its reaction partner, i.e. the electrophile by donating both bonding electrons.

In certain embodiments t of formula (XIV) is 0. In certain embodiments t of formula (XIV) is 1. In certain embodiments t of formula (XIV) is 2. In certain embodiments t of formula (XIV) is3. In certain embodiments t of formula (XIV) is 4. In certain embodiments t of formula (XIV) is 5. In certain embodiments t of formula (XIV) is 6.

In certain embodiments -A- of formula (XIV) is a ring selected from the group consisting of monocyclic or bicyclic aryl and heteroaryl. In certain embodiments -A- of formula (XIV) is substituted with one or more -$R^2$ which are the same or different. In certain embodiments -A-of formula (XIV) is not substituted with -$R^2$. In certain embodiments -A- of formula (XIV) is selected from the group consisting of:

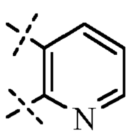, 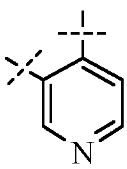, 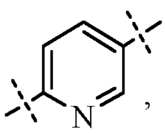, 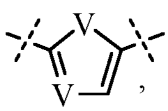,

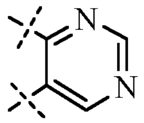, 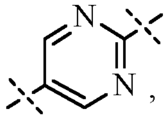, 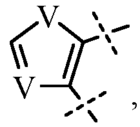,

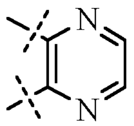, 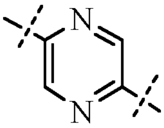, 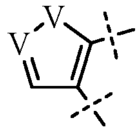,

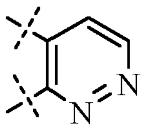, 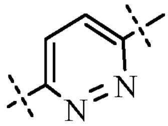, 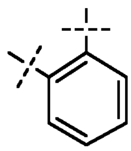, 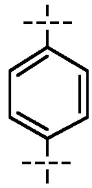,

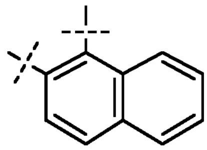, 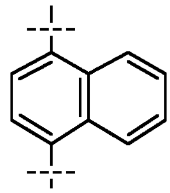 and 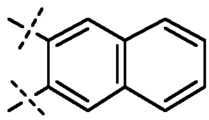;

wherein each V is independently selected from the group consisting of O, S and N.

In certain embodiments $-R^1$, $-R^{1a}$ and each $-R^2$ of formula (XIV) are independently selected from the group consisting of -H, -C(O)OH, -halogen, -CN, $-NO_2$, -OH, $C_{1-6}$ alkyl, $C_{2-6}$ alkenyl and $C_{2-6}$ alkynyl. In certain embodiments $-R^1$ of formula (XIV) is -H. In certain embodiments $-R^1$ of formula (XIV) is -C(O)OH. In certain embodiments $-R^1$ of formula (XIV) is -halogen. In certain embodiments $-R^1$ of formula (XIV) is -F. In certain embodiments $-R^1$ of formula (XIV) is -CN. In certain embodiments $-R^1$ of formula (XIV) is $-NO_2$. In certain embodiments $-R^1$ of formula (XIV) is -OH. In certain embodiments $-R^1$ of formula (XIV) is $C_{1-6}$ alkyl. In certain embodiments $-R^1$ of formula (XIV) is $C_{2-6}$ alkenyl. In certain embodiments $-R^1$ is $C_{2-6}$ alkynyl. In certain embodiments $-R^{1a}$ of formula (XIV) is -H. In certain embodiments $-R^{1a}$ of formula (XIV) is -C(O)OH. In certain embodiments $-R^{1a}$ of formula (XIV) is -halogen. In certain embodiments $-R^{1a}$ of formula (XIV) is -F. In certain embodiments $-R^{1a}$ of formula (XIV) is -CN. In certain embodiments $-R^{1a}$ of formula (XIV) is $-NO_2$. In certain embodiments $-R^{1a}$ of formula (XIV) is -OH. In certain embodiments $-R^{1a}$ of formula (XIV) is $C_{1-6}$ alkyl. In certain embodiments $-R^{1a}$ of formula (XIV) is $C_{2-6}$ alkenyl. In certain embodiments $-R^{1a}$ of formula (XIV) is $C_{2-6}$ alkynyl.

In certain embodiments each of $-R^2$ of formula (XIV) is independently selected from the group consisting of -H, -C(O)OH, -halogen, -CN, $-NO_2$, -OH, $C_{1-6}$ alkyl, $C_{2-6}$ alkenyl and $C_{2-6}$ alkynyl. In certain embodiments each of $-R^2$ of formula (XIV) is -H. In certain embodiments each of $-R^2$ of formula (XIV) is -C(O)OH. In certain embodiments each of $-R^2$ of formula (XIV) is - halogen. In certain embodiments each of $-R^2$ of formula (XIV) is -F. In certain embodiments each of $-R^2$ of formula (XIV) is -CN. In certain embodiments each of $-R^2$ of formula (XIV) is - $NO_2$. In certain embodiments each of $-R^2$ of formula (XIV) is -OH. In certain embodiments each of $-R^2$ of formula (XIV) is $C_{1-6}$ alkyl. In certain embodiments each of $-R^2$ of formula (XIV) is $C_{2-6}$ alkenyl. In certain embodiments each of $-R^2$ of formula (XIV) is $C_{2-6}$ alkynyl.

In certain embodiments T of formula (XIV) is selected from the group consisting of phenyl, naphthyl, indenyl, indanyl, tetralinyl, $C_{3-10}$ cycloalkyl, 3- to 10-membered heterocyclyl and 8-to 11-membered heterobicyclyl. In certain embodiments T of formula (XIV) is phenyl. In certain embodiments T of formula (XIV) is naphthyl. In certain embodiments T of formula (XIV) is indenyl. In certain embodiments T of formula (XIV) is indanyl. In certain embodiments T of formula (XIV) is tetralinyl. In certain embodiments T of formula (XIV) is $C_{3-10}$ cycloalkyl. In certain embodiments T of formula (XIV) is 3- to 10-membered heterocyclyl. In certain embodiments T of formula (XIV) is 8- to 11-membered heterobicyclyl.

In certain embodiments T of formula (XIV) is substituted with one or more $-R^3$, which are the same or different. In certain embodiments T of formula (XIV) is substituted with one $-R^3$. In certain embodiments T of formula (XIV) is not substituted with $-R^3$.

In certain embodiments $-R^3$ of formula (XIV) is selected from the group consisting of -H, $-NO_2$, $-OCH_3$, -CN, $-N(R^4)(R^{4a})$, -OH, -C(O)OH and $C_{1-6}$ alkyl. In certain embodiments $-R^3$ of formula (XIV) is -H. In certain embodiments $-R^3$ of formula (XIV) is $-NO_2$. In certain embodiments - $R^3$ of formula (XIV) is $-OCH_3$. In certain embodiments $-R^3$ of formula (XIV) is -CN. In certain embodiments $-R^3$ of formula (XIV) is $-N(R^4)(R^{4a})$. In certain embodiments $-R^3$ of formula (XIV) is

-OH. In certain embodiments $-R^3$ of formula (XIV) is -C(O)OH. In certain embodiments $-R^3$ of formula (XIV) is $C_{1-6}$ alkyl. In certain embodiments $-R^4$ and $-R^{4a}$ of formula (XIV) are independently selected from the group consisting of -H and $C_{1-6}$ alkyl. In certain embodiments $-R^4$ of formula (XIV) is -H. In certain embodiments $-R^4$ is $C_{1-6}$ alkyl. In certain embodiments $-R^{4a}$ of formula (XIV) is -H. In certain embodiments $-R^{4a}$ of formula (XIV) is $C_{1-6}$ alkyl.

In certain embodiments -Y of formula (XIV) is

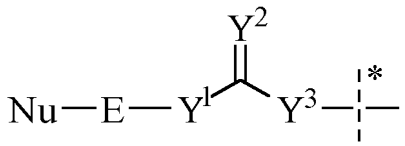

wherein -Nu, -E, $-Y^1$-, $=Y^2$ and $-Y^3$- are as defined elsewhere herein and the dashed line marked with an asterisk indicates the attachment to -A- of formula (XIV).

In certain embodiments -Nu of formula (XIV) is a nucleophile selected from the group consisting of primary, secondary, tertiary amine and amide. In certain embodiments -Nu of formula (XIV) is a primary amine. In certain embodiments -Nu of formula (XIV) is a secondary amine. In certain embodiments -Nu of formula (XIV) is a tertiary amine. In certain embodiments -Nu of formula (XIV) is an amide.

In certain embodiments $-Y^1$- of formula (XIV) is selected from the group consisting of -O-, $-C(R^{10})(R^{10a})$-, $-N(R^{11})$- and -S-. In certain embodiments $-Y^1$- of formula (XIV) is -O-. In certain embodiments $-Y^1$- of formula (XIV) is $-C(R^{10})(R^{10a})$-. In certain embodiments $-Y^1$- of formula (XIV) is $-N(R^{11})$-. In certain embodiments $-Y^1$- is -S-.

In certain embodiments $=Y^2$ of formula (XIV) is selected from the group consisting of =O, =S and $=N(R^{12})$. In certain embodiments $=Y^2$ of formula (XIV) is =O. In certain embodiments $=Y^2$ of formula (XIV) is =S. In certain embodiments $=Y^2$ of formula (XIV) is $=N(R^{12})$.

In certain embodiments $-Y^3$- of formula (XIV) is selected from the group consisting of -O-, -Sand $-N(R^{13})$. In certain embodiments $-Y^3$- of formula (XIV) is -O-. In certain embodiments $-Y^3$-of formula (XIV) is -S-. In certain embodiments $-Y^3$- of formula (XIV) is $-N(R^{13})$.

In certain embodiments $-Y^1$- of formula (XIV) is $-N(R^{11})$-, $=Y^2$ of formula (XIV) is =O and $-Y^3$-is -O-.

In certain embodiments $-Y^1$- of formula (XIV) is $-N(R^{11})$-, $=Y^2$ of formula (XIV) is =O, $-Y^3$-of formula (XIV) is -O- and -Nu of formula (XIV) is $-N(CH_3)_2$.

In certain embodiments -E- of formula (XIV) is selected from the group consisting of $C_{1-6}$ alkyl, $C_{2-6}$ alkenyl, $C_{2-6}$ alkynyl and -Q-. In certain embodiments -E- of formula (XIV) is $C_{1-6}$ alkyl. In certain embodiments -E- of formula (XIV) is $C_{2-6}$ alkenyl. In certain embodiments -E- of formula (XIV) is $C_{2-6}$ alkynyl. In certain embodiments -E- of formula (XIV) is -Q-.

In certain embodiments Q of formula (XIV) is selected from the group consisting of phenyl, naphthyl, indenyl, indanyl, tetralinyl, $C_{3-10}$ cycloalkyl, 3- to 10-membered heterocyclyl and 8-to 11-membered heterobicyclyl. In certain embodiments Q of formula (XIV) is phenyl. In certain embodiments Q of formula (XIV) is naphthyl. In certain embodiments Q of formula (XIV) is indenyl. In certain embodiments Q of formula (XIV) is indanyl. In certain embodiments Q of formula (XIV) is tetralinyl. In certain embodiments Q of formula (XIV) is $C_{3-10}$ cycloalkyl. In certain embodiments Q of formula (XIV) is 3- to 10-membered heterocyclyl. In certain embodiments Q of formula (XIV) is 8- to 11-membered heterobicyclyl. In certain embodiments Q of formula (XIV) is substituted with one or more $-R^{14}$. In certain embodiments Q of formula (XIV) is not substituted with $-R^{14}$.

In certain embodiments $-R^5$, $-R^6$, each $-R^7$, $-R^8$, $-R^9$, $-R^{10}$, $-R^{10a}$, $-R^{11}$, $-R^{12}$ and $-R^{13}$ of formula (XIV) are independently selected from the group consisting of $C_{1-20}$ alkyl, $C_{2-20}$ alkenyl, $C_{2-20}$ alkynyl and -Q.

In certain embodiments $-R^5$ of formula (XIV) is $C_{1-20}$ alkyl. In certain embodiments $-R^5$ of formula (XIV) is $C_{2-20}$ alkenyl. In certain embodiments $-R^5$ of formula (XIV) is $C_{2-20}$ alkynyl. In certain embodiments $-R^5$ of formula (XIV) is -Q.

In certain embodiments $-R^6$ of formula (XIV) is $C_{1-20}$ alkyl. In certain embodiments $-R^6$ of formula (XIV) is $C_{2-20}$ alkenyl. In certain embodiments $-R^6$ of formula (XIV) is $C_{2-20}$ alkynyl. In certain embodiments $-R^6$ is -Q.

In certain embodiments each of $-R^7$ of formula (XIV) is independently selected from the group consisting of $C_{1-20}$ alkyl, $C_{2-20}$ alkenyl, $C_{2-20}$ alkynyl and -Q. In certain embodiments each of $-R^7$ of formula (XIV) is $C_{1-20}$ alkyl. In certain embodiments each of $-R^7$ of formula (XIV) is $C_{2-20}$ alkenyl. In certain embodiments each of $-R^7$ of formula (XIV) is $C_{2-20}$ alkynyl. In certain embodiments each of $-R^7$ of formula (XIV) is -Q.

In certain embodiments $-R^8$ of formula (XIV) is $C_{1-20}$ alkyl. In certain embodiments $-R^8$ of formula (XIV) is $C_{2-20}$ alkenyl. In certain embodiments $-R^8$ of formula (XIV) is $C_{2-20}$ alkynyl. In certain embodiments $-R^8$ of formula (XIV) is -Q.

In certain embodiments $-R^9$ of formula (XIV) is $C_{1-20}$ alkyl. In certain embodiments $-R^9$ of formula (XIV) is $C_{2-20}$ alkenyl. In certain embodiments $-R^9$ of formula (XIV) is $C_{2-20}$ alkynyl. In certain embodiments $-R^9$ of formula (XIV) is -Q.

In certain embodiments $-R^{10}$ of formula (XIV) is $C_{1-20}$ alkyl. In certain embodiments $-R^{10}$ of formula (XIV) is $C_{2-20}$ alkenyl. In certain embodiments $-R^{10}$ of formula (XIV) is $C_{2-20}$ alkynyl. In certain embodiments $-R^{10}$ of formula (XIV) is -Q.

In certain embodiments $-R^{10a}$ of formula (XIV) is $C_{1-20}$ alkyl. In certain embodiments $-R^{10a}$ of formula (XIV) is

$C_{2-20}$ alkenyl. In certain embodiments $-R^{10a}$ of formula (XIV) is $C_{2-20}$ alkynyl. In certain embodiments $-R^{10a}$ of formula (XIV) is -Q.

In certain embodiments $-R^{11}$ of formula (XIV) is $C_{1-20}$ alkyl. In certain embodiments $-R^{11}$ of formula (XIV) is $C_{2-20}$ alkenyl. In certain embodiments $-R^{11}$ of formula (XIV) is $C_{2-20}$ alkynyl. In certain embodiments $-R^{11}$ of formula (XIV) is -Q.

In certain embodiments $-R^{12}$ of formula (XIV) is $C_{1-20}$ alkyl. In certain embodiments $-R^{12}$ of formula (XIV) is $C_{2-20}$ alkenyl. In certain embodiments $-R^{12}$ of formula (XIV) is $C_{2-20}$ alkynyl. In certain embodiments $-R^{12}$ of formula (XIV) is -Q.

In certain embodiments $-R^{13}$ of formula (XIV) is $C_{1-20}$ alkyl. In certain embodiments $-R^{13}$ of formula (XIV) is $C_{2-20}$ alkenyl. In certain embodiments $-R^{13}$ of formula (XIV) is $C_{2-20}$ alkynyl. In certain embodiments $-R^{13}$ of formula (XIV) is -Q.

In certain embodiments $-R^{14}$, $-R^{15}$ and $-R^{15a}$ of formula (XIV) are selected from the group consisting of -H and $C_{1-6}$ alkyl.

In certain embodiments $-R^{14}$ of formula (XIV) is -H. In certain embodiments $-R^{14}$ of formula (XIV) is $C_{1-6}$ alkyl.

In certain embodiments $-R^{15}$ of formula (XIV) is -H. In certain embodiments $-R^{15}$ of formula (XIV) is $C_{1-6}$ alkyl.

In certain embodiments $-R^{15a}$ of formula (XIV) is -H. In certain embodiments $-R^{15a}$ of formula (XIV) is $C_{1-6}$ alkyl.

In certain embodiments -Y of formula (XIV) is

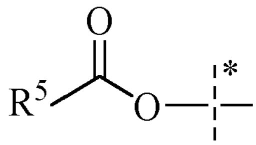
,

wherein $-R^5$ is as defined above and the dashed line marked with an asterisk indicates the attachment to -A-.

In certain embodiments -Y of formula (XIV) is

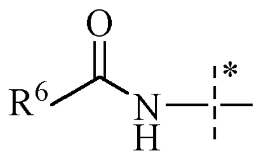
,

wherein $-R^6$ is as defined above and the dashed line marked with an asterisk indicates the attachment to -A-.

In certain embodiments $-R^6$ of formula (XIV) is of formula (XIVa):

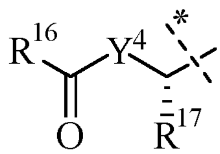
(XIVa),

wherein $-Y^4-$ is selected from the group consisting of $C_{3-10}$ cycloalkyl, 3- to 10-membered heterocyclyl and 8- to 11-membered heterobicyclyl, which are optionally substituted with one or more $-R^{18}$ which are the same or different; $-R^{16}$ and $-R^{17}$ are independently selected from the group consisting of -H, $C_{1-10}$ alkyl, $C_{2-10}$ alkenyl and $C_{2-10}$ alkynyl; wherein $C_{1-10}$ alkyl, $C_{2-10}$ alkenyl and $C_{2-10}$ alkynyl are optionally substituted with one or more $-R^{18}$ which are the same or different; and wherein $C_{1-10}$ alkyl, $C_{2-10}$ alkenyl and $C_{2-10}$ alkynyl are optionally interrupted by one or more groups selected from the group consisting of -A'-, -C(O)O-, -O-, -C(O)-, $-C(O)N(R^{19})-$, $-S(O)_2N(R^{19})$, $-S(O)N(R^{19})-$, $-S(O)_2-$, -S(O)-, $-N(R^{19})S(O)_2N(R^{19a})-$, -S-, $-N(R^{19})-$, $-OC(OR^{19})R^{19a}-$, $-N(R^{19})C(0)N(R^{19a})-$, $-OC(O)N(R^{19})-$ and $-N(R^{19})C(NH)N(R^{19a})-$;

each A' is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl, wherein each A' is independently optionally substituted with one or more $-R^{18}$ which are the same or different;

wherein $-R^{18}$, $-R^{19}$ and $-R^{19a}$ are independently selected from the group consisting of -H and $C_{1-6}$ alkyl; wherein $C_{1-6}$ alkyl is optionally substituted with one or more halogen, which are the same or different; and

wherein the dashed line marked with an asterisk indicates the attachment to the rest of -Y.

In certain embodiments $-Y^4-$ of formula (XIVa) is selected from the group consisting of $C_{3-10}$ cycloalkyl, 3- to 10-membered heterocyclyl and 8- to 11-membered heterobicyclyl. In certain embodiments $-Y^4-$ of formula (XIVa) is $C_{3-10}$ cycloalkyl. In certain embodiments $-Y^4-$ of formula (XIVa) is 3- to 10-membered heterocyclyl. In certain embodiments $-Y^4-$ of formula (XIVa) is 8- to 11-membered heterobicyclyl. In certain embodiments $-Y^4-$ of formula (XIVa) is substituted with one or more $-R^{18}$ which are the same or different. In certain embodiments - $Y^4-$ of formula (XIVa) is not substituted with $-R^{18}$.

In certain embodiments $-R^{16}$ and $-R^{17}$ of formula (XIVa) are selected from the group consisting of $C_{1-10}$ alkyl, $C_{2-10}$ alkenyl and $C_{2-10}$ alkynyl. In certain embodiments $-R^{16}$ of formula (XIVa) is $C_{1-10}$ alkyl. In certain embodiments $-R^{16}$ of formula (XIVa) is $C_{2-10}$ alkenyl. In certain embodiments $-R^{16}$ of formula (XIVa) is $C_{2-10}$ alkynyl. In certain embodiments $-R^{17}$ of formula (XIVa) is $C_{1-10}$ alkyl. In certain embodiments $-R^{17}$ of formula (XIVa) is $C_{2-10}$ alkenyl. In certain embodiments $-R^{17}$ of formula (XIVa) is $C_{2-10}$ alkynyl.

In certain embodiments A' of formula (XIVa) is selected from the group consisting of phenyl, naphthyl, indenyl, indanyl, tetralinyl, $C_{3-10}$ cycloalkyl, 3- to 10-membered heterocyclyl and 8-to 11-membered heterobicyclyl. In certain embodiments A' of formula (XIVa) is phenyl. In certain embodiments A' of formula (XIVa) is naphthyl. In certain embodiments A' of formula (XIVa) is indenyl. In certain embodiments A' of formula (XIVa) is indanyl. In certain embodiments A' of formula (XIVa) is tetralinyl. In certain embodiments A' of formula (XIVa) is $C_{3-10}$ cycloalkyl. In certain embodiments A' of formula (XIVa) is 3- to 10-membered heterocyclyl. In certain embodiments A' of formula (XIVa) is 8- to 11-membered heterobicyclyl.

In certain embodiments A' of formula (XIVa) is substituted with one or more $-R^{18}$, which are the same or different. In certain embodiments A' of formula (XIVa) is not substituted with - $R^{18}$.

In certain embodiments $-R^{18}$, $-R^{19}$ and $-R^{19a}$ of formula (XIVa) are selected from the group consisting of -H and $C_{1-6}$ alkyl.

In certain embodiments $-R^{18}$ of formula (XIVa) is -H. In certain embodiments $-R^{18}$ of formula (XIVa) is $C_{1-6}$ alkyl. In certain embodiments $-R^{19}$ of formula (XIVa) is -H. In certain embodiments $-R^{19}$ of formula (XIVa) is $C_{1-6}$ alkyl. In certain embodiments $-R^{19a}$ of formula (XIVa) is -H. In certain embodiments $-R^{19a}$ of formula (XIVa) is $C_{1-6}$ alkyl.

In certain embodiments $-R^6$ of formula (XIV) is of formula (XIVb):

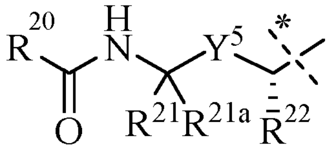
(XIVb),

**wherein** $-Y^5-$ is selected from the group consisting of -Q'-, $C_{1-10}$ alkyl, $C_{2-10}$ alkenyl and $C_{2-10}$ alkynyl; wherein $C_{1-10}$ alkyl, $C_{2-10}$ alkenyl and $C_{2-10}$ alkynyl are optionally substituted with one or more $-R^{23}$, which are the same or different; and wherein $C_{1-10}$ alkyl, $C_{2-10}$ alkenyl and $C_{2-10}$ alkynyl are optionally interrupted by one or more groups selected from the group consisting of -Q'-, -C(O)O-, -O-, -C(O)-, $-C(O)N(R^{24})-$, $-S(O)_2N(R^{24})$, $-S(O)N(R^{24})-$, $-S(O)_2-$, -S(O)-, $-N(R^{24})S(O)_2N(R^{24a})-$, -S- $-N(R^{24})-$, $-OC(OR^{24})R^{24a}-$, $-N(R^{24})C(O)N(R^{24a})-$, $-OC(O)N(R^{24})-$ and $-N(R^{24})C(NH)N(R^{24a})-$;

$-R^{20}$, $-R^{21}$, $-R^{21a}$ and $-R^{22}$ are independently selected from the group consisting of -H, $C_{1-10}$ alkyl, $C_{2-10}$ alkenyl and $C_{2-10}$ alkynyl; wherein $C_{1-10}$ alkyl, $C_{2-10}$ alkenyl and $C_{2-10}$ alkynyl are optionally substituted with one or more $-R^{23}$ which are the same or different; and wherein $C_{1-10}$ alkyl, $C_{2-10}$ alkenyl and $C_{2-10}$ alkynyl are optionally interrupted by one or more groups selected from the group consisting of -Q'-, -C(O)O-, -O-, -C(O)-, $-C(O)N(R^{24})-$, $-S(O)_2N(R^{24})$, $-S(O)N(R^{24})-$, $-S(O)_2-$, -S(O)- $-N(R^{24})S(O)_2N(R^{24a})-$, $-S--N(R^{24})-$, $-OC)(OR^{24})R^{24a}-$, $-N(R^{24})C(O)N(R^{24a})-$, $-OC(O)N(R^{24})-$ and $-N(R^{24})C(NH)N(R^{24a})-$;

each Q' is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl, wherein each Q' is independently optionally substituted with one or more $-R^{23}$, which are the same or different;

wherein $-R^{23}$, $-R^{24}$ and $-R^{24a}$ are independently selected from the group consisting of -H and $C_{1-6}$ alkyl; wherein $C_{1-6}$ alkyl is optionally substituted with one or more halogen, which are the same or different;

optionally, the pair $-R^{21}/-R^{21a}$ is joined together with the atoms to which is attached to form a $C_{3-10}$ cycloalkyl, 3- to 10-membered heterocyclyl or an 8- to 11-membered heterobicyclyl; and

wherein the dashed line marked with an asterisk indicates the attachment to the rest of -Y.

In certain embodiments $-Y^5-$ of formula (XIVb) is selected from the group consisting of -Q'-, $C_{1-10}$ alkyl, $C_{2-10}$ alkenyl and $C_{2-10}$ alkynyl. In certain embodiments $-Y^5-$ of formula (XIVb) is -Q'-. In certain embodiments $-Y^5-$ of formula (XIVb) is $C_{1-10}$ alkyl. In certain embodiments - $Y^5-$ of formula (XIVb) is $C_{2-10}$ alkenyl. In certain embodiments $-Y^5-$ of formula (XIVb) is $C_{2-10}$ alkynyl.

In certain embodiments Q' of formula (XIVb) is selected from the group consisting of phenyl, naphthyl, indenyl, indanyl, tetralinyl, $C_{3-10}$ cycloalkyl, 3- to 10-membered heterocyclyl and 8-to 11-membered heterobicyclyl. In certain embodiments Q' of formula (XIVb) is phenyl. In certain embodiments Q' of formula (XIVb) is naphthyl. In certain embodiments Q' of formula (XIVb) is indenyl. In certain embodiments Q' of formula (XIVb) is indanyl. In certain embodiments Q' of formula (XIVb) is $C_{3-10}$ cycloalkyl. In certain embodiments Q' of formula (XIVb) is 3- to 10-membered heterocyclyl. In certain embodiments Q' of formula (XIVb) is 8-to 11-membered heterobicyclyl. In certain embodiments Q' of formula (XIVb) is substituted with one or more $-R^{23}$ which are the same or different. In certain embodiments Q' of formula (XIVb) is not substituted with $-R^{23}$.

In certain embodiments $-R^{20}$, $-R^{21}$, $-R^{21a}$ and $-R^{22}$ of formula (XIVb) are selected from the group consisting of -H, $C_{1-10}$ alkyl, $C_{2-10}$ alkenyl and $C_{2-10}$ alkynyl. In certain embodiments $-R^{20}$ of formula (XIVb) is -H. In certain embodiments $-R^{20}$ of formula (XIVb) is $C_{1-10}$ alkyl. In certain embodiments $-R^{20}$ of formula (XIVb) is $C_{2-10}$ alkenyl. In certain embodiments $-R^{20}$ of formula (XIVb) is $C_{2-10}$ alkynyl. In certain embodiments $-R^{21}$ of formula (XIVb) is -H. In certain embodiments $-R^{21}$ of formula (XIVb) is $C_{1-10}$ alkyl. In certain embodiments $-R^{21}$ of formula (XIVb) is $C_{2-10}$ alkenyl. In certain embodiments $-R^{21}$ of formula (XIVb) is $C_{2-10}$ alkynyl. In certain embodiments $-R^{21a}$ of formula (XIVb) is -H. In certain embodiments $-R^{21a}$ of formula (XIVb) is $C_{1-10}$ alkyl. In certain embodiments $-R^{21a}$ of formula (XIVb) is $C_{2-10}$ alkenyl. In certain embodiments $-R^{21a}$ of formula (XIVb) is $C_{2-10}$ alkynyl. In certain embodiments $-R^{22}$ of formula (XIVb) is -H. In certain embodiments $-R^{22}$ of formula (XIVb) is $C_{1-10}$ alkyl. In certain embodiments $-R^{22}$ of formula (XIVb) is $C_{2-10}$ alkenyl. In certain embodiments $-R^{22}$ of formula (XIVb) is $C_{2-10}$ alkynyl.

In certain embodiments $-R^{23}$, $-R^{24}$ and $-R^{24a}$ of formula (XIVb) are selected from the group consisting of -H and $C_{1-6}$ alkyl. In certain embodiments $-R^{23}$ of formula (XIVb) is -H. In certain embodiments $-R^{23}$ of formula (XIVb) is $C_{1-6}$ alkyl. In certain embodiments $-R^{24}$ of formula (XIVb) is -H. In certain embodiments $-R^{24}$ of formula (XIVb) is $C_{1-6}$ alkyl. In certain embodiments $-R^{24a}$ of formula (XIVb) is -H. In certain embodiments $-R^{24a}$ of formula (XIVb) is $C_{1-6}$ alkyl.

In certain embodiments the pair $-R^{21}/-R^{21a}$ of formula (XIVb) is joined together with the atoms to which is attached to form a $C_{3-10}$ cycloalkyl.

In certain embodiments $-R^6$ of formula (XIVb) is of formula (XIVc):

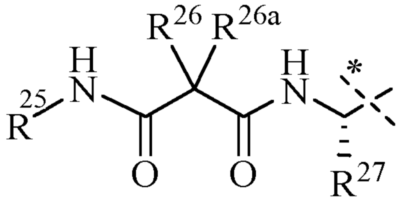
(XIVc),

wherein

- $R^{25}$, $-R^{26}$, $-R^{26a}$ and $-R^{27}$ are independently selected from the group consisting of -H, $C_{1-10}$ alkyl, $C_{2-10}$ alkenyl and $C_{2-10}$ alkynyl; wherein $C_{1-10}$ alkyl, $C_{2-10}$ alkenyl and $C_{2-10}$ alkynyl are optionally substituted with one or more $-R^{28}$ which are the same or different; and wherein $C_{1-10}$ alkyl, $C_{2-10}$ alkenyl and $C_{2-10}$ alkynyl are optionally interrupted by one or more groups selected from the group consisting of -Q*-, -C(O)O-, -O-, -C(O)-, $-C(O)N(R^{29})-$, $-S(O)_2N(R^{29})$, $-S(O)N(R^{29})-$, $-S(O)_2-$, -S(O)-, $-N(R^{29})S(O)_2N(R^{29a})-$, -S-, $-N(R^{29})-$, $-OC(OR^{29})R^{29a}-$, $-N(R^{29})C(O)N(R^{29a})-$, $-OC(O)N(R^{29})-$ and $-N(R^{29})C(NH)N(R^{29a})-$;

  each Q* is independently selected from the group consisting of phenyl, naphthyl, indenyl, in7danyl, tetralinyl, $C_{3-10}$ cycloalkyl, 3- to 10-membered heterocyclyl and 8- to 11-membered heterobicyclyl, wherein each Q* is independently optionally substituted with one or more $-R^{28}$, which are the same or different;
  wherein $-R^{28}$, $-R^{29}$ and $-R^{29a}$ are independently selected from the group consisting **of -H** and $C_{1-6}$ alkyl; wherein $C_{1-6}$ alkyl is optionally substituted with one or more halogen, which are the same or different;

  optionally, the pair $-R^{26}/-R^{26a}$ is joined together with the atoms to which is attached to form a $C_{3-10}$ cycloalkyl, 3- to 10-membered heterocyclyl or an 8- to 11-membered heterobicyclyl; and

  wherein the dashed line marked with an asterisk indicates the attachment to the rest of -Y.

**In** certain embodiments **$-R^{25}$,** $-R^{26}$, $-R^{26a}$ and $-R^{27}$ of formula (XIVc) are selected from the group consisting of **-H,** $C_{1-10}$ alkyl, $C_{2-10}$ alkenyl and $C_{2-10}$ alkynyl. **In** certain embodiments **$-R^{25}$** of formula (XIVc) is **-H. In** certain embodiments **$-R^{25}$** of formula (XIVc) is $C_{1-10}$ alkyl. **In** certain embodiments **$-R^{25}$** of formula (XIVc) is $C_{2-10}$ alkenyl. **In** certain embodiments **$-R^{25}$** of formula (XIVc) is $C_{2-10}$ alkynyl. **In** certain embodiments $-R^{26}$ of formula (XIVc) is **-H. In** certain embodiments $-R^{26}$ of

formula (XIVc) is $C_{1-10}$ alkyl. **In** certain embodiments -$R^{26}$ of formula (XIVc) is $C_{2-10}$ alkenyl. **In** certain embodiments -$R^{26}$ of formula (XIVc) is $C_{2-10}$ alkynyl. **In** certain embodiments -$R^{26a}$ of formula (XIVc) is **-H. In** certain embodiments -$R^{26a}$ of formula (XIVc) is $C_{1-10}$ alkyl. **In** certain embodiments -$R^{26a}$ of formula (XIVc) is $C_{2-10}$ alkenyl. **In** certain embodiments -$R^{26a}$ of formula (XIVc) is $C_{2-10}$ alkynyl. **In** certain embodiments -$R^{27}$ of formula (XIVc) is **-H. In** certain embodiments -$R^{27}$ of formula (XIVc) is $C_{1-10}$ alkyl. **In** certain embodiments -$R^{27}$ of formula (XIVc) is $C_{2-10}$ alkenyl. **In** certain embodiments -$R^{27}$ of formula (XIVc) is $C_{2-10}$ alkynyl.

**In** certain embodiments Q* of formula (XIVc) is selected from the group consisting of phenyl, naphthyl, indenyl, indanyl, tetralinyl, $C_{3-10}$ cycloalkyl, 3- to 10-membered heterocyclyl and 8-to 11-membered heterobicyclyl. **In** certain embodiments Q* of formula (XIVc) is phenyl. **In** certain embodiments Q* of formula (XIVc) is naphthyl. **In** certain embodiments Q* of formula (XIVc) is indenyl. **In** certain embodiments Q* of formula (XIVc) is indanyl. **In** certain embodiments Q* of formula (XIVc) is tetralinyl. **In** certain embodiments Q* of formula (XIVc) is $C_{3-10}$ cycloalkyl. **In** certain embodiments Q* of formula (XIVc) is 3- to 10-membered heterocyclyl. **In** certain embodiments Q* of formula (XIVc) is 8- to 11-membered heterobicyclyl. **In** certain embodiments Q* of formula (XIVc) is substituted with one or more -$R^{28}$, which are the same or different. **In** certain embodiments Q* of formula (XIVc) is not substituted with -$R^{28}$.

In certain embodiments -$R^{28}$, -$R^{29}$ and -$R^{29a}$ of formula (XIVc) are selected from the group consisting of -H and $C_{1-6}$ alkyl. In certain embodiments -$R^{28}$ of formula (XIVc) is -H. In certain embodiments -$R^{28}$ of formula (XIVc) is $C_{1-6}$ alkyl. In certain embodiments -$R^{29}$ of formula (XIVc)is -H. In certain embodiments -$R^{29}$ of formula (XIVc) is $C_{1-6}$ alkyl. In certain embodiments -$R^{29a}$ of formula (XIVc) is -H. In certain embodiments -$R^{29a}$ of formula (XIVc) is $C_{1-6}$ alkyl.

In certain embodiments the pair -$R^{26}$/-$R^{26a}$ of formula (XIVc) is joined together with the atoms to which is attached to form a $C_{3-10}$ cycloalkyl. In certain embodiments the pair -$R^{26}$/-$R^{26a}$ of formula (XIVc) is joined together with the atoms to which is attached to form a cyclobutyl.

In certain embodiments -Y of formula (XIV) is

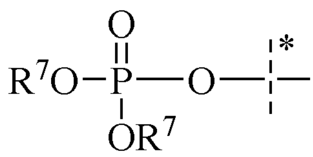

,

wherein each -$R^7$ is as defined above and the dashed line marked with an asterisk indicates the attachment to -A-. It is understood that in this instance the release of the drug D may be triggered by an enzyme, such as phosphatase.

In certain embodiments -Y of formula (XIV) is

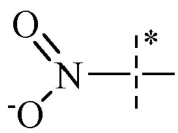

,

wherein the dashed line marked with an asterisk indicates the attachment to -A-.

In certain embodiments -Y of formula (XIV) is

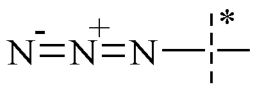

,

wherein the dashed line marked with an asterisk indicates the attachment to -A-.

In certain embodiments -Y of formula (XIV) is

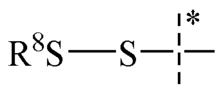

,

wherein -$R^8$ is as defined above and the dashed line marked with an asterisk indicates the attachment to -A-.

In certain embodiments -Y of formula (XIV) is

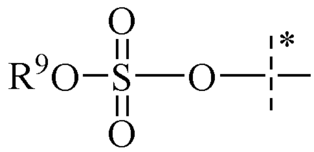,

wherein -$R^9$ is as defined above and the dashed line marked with an asterisk indicates the attachment to -A-. It is understood that in this instance the release of the drug D may be triggered by an enzyme, such as sulfatase.

In certain embodiments -Y of formula (XIV) is

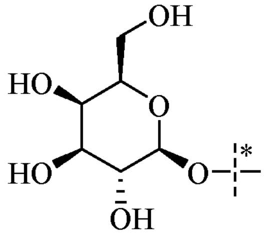,

wherein the dashed line marked with an asterisk indicates the attachment to -A-. It is understood that in this instance the release of the drug D may be triggered by an enzyme, such as α-galactosidase.

In certain embodiments -Y of formula (XIV) is

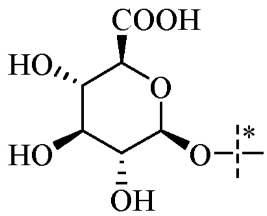,

wherein the dashed line marked with an asterisk indicates the attachment to -A-. It is understood that in this instance the release of the drug D may be triggered by an enzyme, such as β-glucuronidase.

In certain embodiments -Y of formula (XIV)is

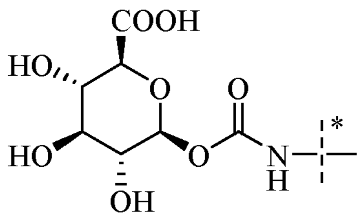,

wherein the dashed line marked with an asterisk indicates the attachment to -A-. It is understood that in this instance the release of the drug D may be triggered by an enzyme, such as β-glucuronidase.

In certain embodiments -Y of formula (XIV) is a peptidyl moiety.

It is understood that if -Y of formula (XIV) is a peptidyl moiety, then the release of the drug D may be triggered by an enzyme, such as protease. In certain embodiments the protease is selected from the group consisting of cathepsin B and cathepsin K. In certain embodiments the protease is cathepsin B. In certain embodiments the protease is cathepsin K.

In certain embodiments -Y of formula (XIV) is a peptidyl moiety, such as a dipeptidyl, tripeptidyl, tetrapeptidyl, pentapeptidyl or hexapeptidyl moiety. In certain embodiments -Y of formula (XIV) is a dipeptidyl moiety. In certain embodiments -Y of formula (XIV) is a tripeptidyl moiety. In certain embodiments -Y of formula (XIV) is a tetrapeptidyl moiety. In certain embodiments -Y of formula (XIV) is a pentapeptidyl moiety. In certain embodiments - Y of formula (XIV) is a hexapeptidyl moiety.

In certain embodiments -Y of formula (XIV) is a peptidyl moiety selected from the group consisting of:

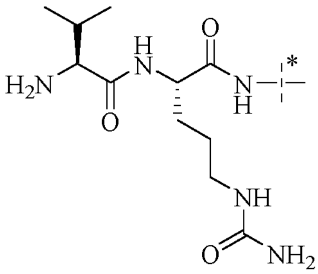, 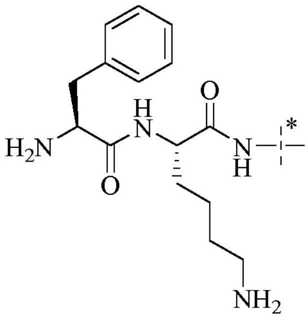 and 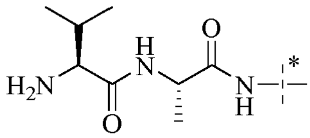,

wherein the dashed line marked with an asterisk indicates the attachment to -A-.

In certain embodiments -Y of formula (XIV) is

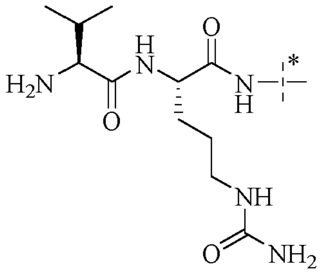.

In certain embodiments -Y of formula (XIV) is

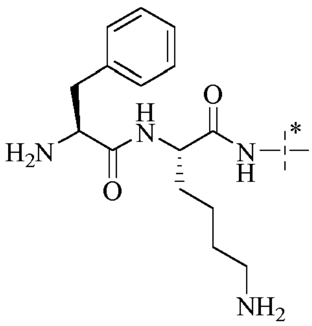.

In certain embodiments -Y of formula (XIV) is

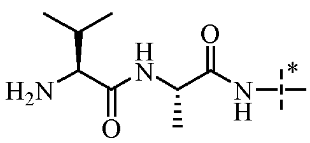.

In certain embodiments one hydrogen given by -$R^{1a}$ of formula (XIV) is replaced by -$L^2$- and -$L^1$- is of formula (XIV'):

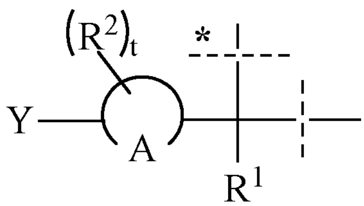

(XIV')

wherein

the unmarked dashed line indicates the attachment to the $N^+$ of $-D^+$, the dashed line marked with an asterisk indicates the attachment to $-L^2-$; and

$-R^1$, -Ar-, -Y, $R^2$ and t are defined as in formula (XIV).

In certain embodiments one hydrogen given by $-R^2$ of formula (XIV) is replaced by $-L^2-$ and $-L^1-$ is of formula (XIV''):

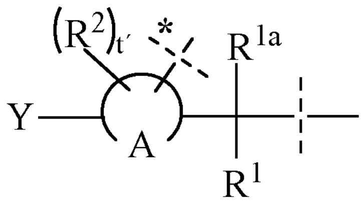

(XIV'')

wherein

the unmarked dashed line indicates the attachment to the $N^+$ of $-D^+$, the dashed line marked with an asterisk indicates the attachment to $-L^2-$;

- $R^1$, -Ar-, -Y and $R^2$ are defined as in formula (XIV); and

t' is selected from the group consisting of 0, 1, 2, 3, 4 and 5.

In certain embodiments t' of formula (XIV'') is 0. In certain embodiments t' of formula (XIV'') is 1. In certain embodiments t' of formula (XIV'') is 2. In certain embodiments t' of formula (XIV'') is 3. In certain embodiments t' of formula (XIV'') is 4. In certain embodiments t' of formula (XIV'') is 5.

In certain embodiments $-L^1-$ is of formula (XV):

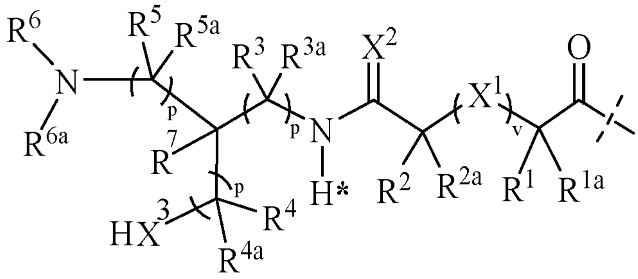

(XV),

wherein

the dashed line indicates the attachment to the nitrogen of the primary or secondary amine of -D;
v is selected from the group consisting of 0 or 1;
$-X^1-$ is selected from the group consisting of $-C(R^8)(R^{8a})-$, $-N(R^9)-$ and -O-;
$=X^2$ is selected from the group consisting of =O and $=N(R^{10})$;
$-X^3$ is selected from the group consisting of -O, -S and -Se;
each p is independently selected from the group consisting of 0 or 1, provided that at most one p is 0;
$-R^6$, $-R^{6a}$, $-R^{10}$ are independently selected from the group consisting of -H, $-C(R^{11})(R^{11a})(R^{11b})$ and -T;
$-R^9$ is selected from the group consisting of $-C(R^{11})(R^{11a})(R^{11b})$ and -T;
$-R^1$, $-R^{1a}$, $-R^2$, $-R^{2a}$, $-R^3$, $-R^{3a}$, $-R^4$, $-R^{4a}$, $-R^5$, $-R^{5a}$, $-R^7$, $-R^8$ $-R^{8a}$, $-R^{11}$, $-R^{11a}$ and $-R^{11b}$ are independently selected from the group consisting of -H, halogen, -CN, $-C(O)OR^{12}$, $-OR^{12}$, $-C(O)R^{12}$, $-C(O)N(R^{12})(R^{12a})$, $-S(O)_2N(R^{12})(R^{12a})$, $-S(O)N(R^{12})(R^{12a})$, $-S(O)_2R^{12}$, $-S(O)R^{12}$, $-N(R^{12})S(O)_2N(R^{12a})(R^{12b})$, $-SR^{12}$, $-NO_2$, $-N(R^{12})C(O)OR^{12a}$, $-N(R^{12})C(O)N(R^{12a})(R^{12b})$, $-OC(O)N(R^{12})(R^{12a})$, -T, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more $-R^{13}$, which are the same or different; and wherein $C_{1-6}$ alkyl, $C_{2-6}$ alkenyl and $C_{2-6}$ alkynyl are optionally interrupted by one or more groups selected from the group consisting of -T-, -C(O)O-, -O-, -C(O)-, $-C(O)N(R^{14})-$, $-S(O)_2N(R^{14})-$, $-S(O)N(R^{14})-$, $-S(O)_2-$, -S(O)-, $-N(R^{14})S(O)_2N(R^{14a})-$, -S-, $-N(R^{14})-$, $-OC(OR^{14})(R^{14a})-$, $-N(R^{14})C(O)N(R^{14a})-$ and $-OC(O)N(R^{14})-$;
$-R^{12}$, $-R^{12a}$, $-R^{126}$ are independently selected from the group consisting of -H, -T, $C_{1-6}$ alkyl, $C_{2-6}$ alkenyl and $C_{2-6}$ alkynyl; wherein -T, $C_{1-6}$ alkyl, $C_{2-6}$ alkenyl and $C_{2-6}$ alkynyl are optionally substituted with one or more $-R^{13}$, which are

the same or different and wherein $C_{1-6}$ alkyl, $C_{2-6}$ alkenyl and $C_{2-6}$ alkynyl are optionally interrupted by one or more groups selected from the group consisting of -T-, -C(O)O-, -O-, -C(O)-, $-C(O)N(R^{14})-$, $-S(O)_2N(R^{14})-$, $-S(O)N(R^{14})-$, $-S(O)_2-$, -S(O)-, $-N(R^{14})S(O)_2N(R^{14a})-$, -S-, $-N(R^{14})-$, $-OC(OR^{14})(R^{14a})-$, $-N(R^{14})C(O)N(R^{14a})-$ and $-OC(O)N(R^{14})-$; wherein each T is independently selected from the group consisting of phenyl, naphthyl, indenyl, indanyl, tetralinyl, $C_{3-10}$ cycloalkyl, 3- to 10-membered heterocyclyl and 8- to 11-membered heterobicyclyl; wherein each T is independently optionally substituted with one or more $-R^{13}$, which are the same or different;

$-R^{13}$ is selected from the group consisting of halogen, -CN, oxo, $-C(O)OR^{15}$, $-OR^{15}$, $-C(O)R^{15}$, $-C(O)N(R^{15})(R^{15a})$, $-S(O)_2N(R^{15})(R^{15a})$, $-S(O) N(R^{15})(R^{15a})$, $-S(O)_2R^{15}$, $-S(O)R^{15}$, $-N(R^{15})S(O)_2N(R^{15a})(R^{15b})$, $-SR^{15}$, $-N(R^{15})(R^{15a})$, $-NO_2$, $-OC(O)R^{15}$, $-N(R^{15})C(O)R^{15a}$, $-N(R^{15})S(O)_2R^{15a,}$ $-N(R^{15})S(O)R^{15a}$, $-N(R^{15})C(O)OR^{15a}$, $-N(R^{15})C(O)N(R^{15a})(R^{15b})$, $-OC(O)N(R^{15})(R^{15a})$ and $C_{1-6}$ alkyl; wherein $C_{1-6}$ alkyl is optionally substituted with one or more halogen, which are the same or different;

wherein $-R^{14}$, $-R^{14a}$, $-R^{15}$, $-R^{15a}$ and $-R^{15b}$ are independently selected from the group consisting of -H and $C_{1-6}$ alkyl; wherein $C_{1-6}$ alkyl is optionally substituted with one or more halogen, which are the same or different;

optionally, one or more of the pairs $-R^1/-R^{1a}$, $-R^2/-R2^a$ , $-R^3/-R^{3a}$, $-R^4/-R^{4a}$, $-R^5/-R^{5a}$ or $-R^8/-R^{8a}$ are joined together with the atom to which they are attached to form a $C_{3-10}$ cycloalkyl, 3- to 10-membered heterocyclyl or an 8- to 11-membered heterobicyclyl;

optionally, one or more of the pairs $-R^1/-R^2$, $-R^1/-R^8$, $-R^1/-R^9$, $-R^2/-R^9$ or $-R^2/-R^{10}$ are joined together with the atoms to which they are attached to form a ring -A-;

wherein -A- is selected from the group consisting of phenyl, naphthyl, indenyl, indanyl, tetralinyl, $C_{3-10}$ cycloalkyl, 3- to 10-membered heterocyclyl and 8- to 11-membered heterobicyclyl;

optionally, one or more of the pairs $-R^3/-R^6$, $-R^4/-R^6$, $-R^5/-R^6$, $-R^6/-R^{6a}$ or $-R^6/-R^7$ form together with the atoms to which they are attached a ring -A'-;

wherein -A'- is selected from the group consisting of 3- to 10-membered heterocyclyl and 8- to 11-membered heterobicyclyl; and

wherein $-L^1-$ is substituted with at least one $-L^2-$ and wherein $-L^1-$ is optionally further substituted.

The optional further substituents of $-L^1-$ of formula (XV) are preferably as described above.

Preferably $-L^1-$ of formula (XV) is substituted with one moiety $-L^2-$.

In one embodiment $-L^1-$ of formula (XV) is not further substituted.

In the conjugates of the present invention $-L^2-$ is a chemical bond or a spacer moiety. In certain embodiments $-L^2-$ does not comprise a reversible linkage, i.e. all linkages in $-L^2-$ are stable linkages. In certain embodiments $-L^1-$ is connected to $-L^2-$ via a stable linkage. In certain embodiments $-L^2-$ is connected to -Z via a stable linkage.

In certain embodiments $-L^2-$ is a chemical bond.

In certain embodiments $-L^2-$ is a spacer moiety.

In certain embodiments $-L^2-$ is a spacer moiety selected from the group consisting of -T-, -C(O)O-, -O-, -C(O)-, $-C(O)N(R^{y1})-$, $-S(O)_2N(R^{y1})-$, $-S(O)N(R^{y1})-$, $-S(O)_2-$, -S(O)-, $-N(R^{y1})S(O)_2N(R^{y1a})-$, -S-, $-N(R^{y1})-$, $-OC(OR^{y1})(R^{y1a})-$, $-N(R^{y1})C(O)N(R^{y1a})-$, $-OC(O)N(R^{y1})-$, $C_{1-50}$ alkyl, $C_{2-50}$ alkenyl, and $C_{2-50}$ alkynyl; wherein -T-, $C_{1-50}$ alkyl, $C_{2-50}$ alkenyl, and $C_{2-50}$ alkynyl are optionally substituted with one or more $-R^{y2}$, which are the same or different and wherein $C_{1-50}$ alkyl, $C_{2-50}$ alkenyl, and $C_{2-50}$ alkynyl are optionally interrupted by one or more groups selected from the group consisting of -T-, -C(O)O-, -O-, -C(O)-, $-C(O)N(R^{y3})-$, $-S(O)_2N(R^{y3})-$, $-S(O)N(R^{y3})-$, $-S(O)_2-$, -S(O)-, $-N(R^{y3})S(O)_2N(R^{y3a})-$, -S-, $-N(R^{y3})-$, $-OC(OR^{y3})(R^{y3a})-$, $-N(R^{y3})C(O)N(R^{y3a})-$, and $-OC(O)N(R^{y3})-$;

$-R^{y1}$ and $-R^{y1a}$ are independently of each other selected from the group consisting of -H, -T, $C_{1-50}$ alkyl, $C_{2-50}$ alkenyl, and $C_{2-50}$ alkynyl; wherein -T, $C_{1-50}$ alkyl, $C_{2-50}$ alkenyl, and $C_{2-50}$ alkynyl are optionally substituted with one or more $-R^{y2}$, which are the same or different, and wherein $C_{1-50}$ alkyl, $C_{2-50}$ alkenyl, and $C_{2-50}$ alkynyl are optionally interrupted by one or more groups selected from the group consisting of -T-, -C(O)O-, -O-, -C(O)-, $-C(O)N(R^{y4})-$, $-S(O)_2N(R^{y4})-$, $-S(O)N(R^{y4})-$, $-S(O)_2-$, -S(O)-, $-N(R^{y4})S(O)_2N(R^{y4a})-$, -S-, $-N(R^{y4})-$, $-OC(OR^{y4})(R^{y4a})-$, $-N(R^{y4})C(O)N(R^{y4a})-$, and $-OC(O)N(R^{y4})-$;

each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more $-R^{y2}$, which are the same or different;

each $-R^{y2}$ is independently selected from the group consisting of halogen, -CN, oxo (=O), $-COOR^{y5}$, $-OR^{y5}$, $-C(O)R^{y5}$, $-C(O)N(R^{y5}R^{y5a})$, $-S(O)_2N(R^{y5}R^{y5a})$, $-S(O)N(R^{y5}R^{y5a})$, $-S(O)_2R^{y5}$, $-S(O)R^{y5}$, $-N(R^{y5})S(O)_2N(R^{y5a}R^{y5b})$, $-SR^{y5}$, $-N(R^{y5}R^{y5a})$, $-NO_2$, $-OC(O)R^{y5}$, $-N(R^{y5})C(O)R^{y5a}$, $-N(R^{y5})S(O)_2R^{y5a,}$ $-N(R^{y5})S(O)R^{y5a}$, $-N(R^{y5})C(O)OR^{y5a}$, $-N(R^{y5})C(0)N(R^{y5a}R^{y5b})$, $-OC(O)N(R^{y5}R^{y5a})$, and $C_{1-6}$ alkyl; wherein $C_{1-6}$ alkyl is optionally substituted with one

or more halogen, which are the same or different; and

each $-R^{y3}$, $-R^{y3a}$, $-R^{y4}$, $-R^{y4a}$, $-R^{y5}$, $-R^{y5a}$ and $-R^{y5b}$ is independently selected from the group consisting of -H, and $C_{1-6}$ alkyl, wherein $C_{1-6}$ alkyl is optionally substituted with one or more halogen, which are the same or different.

In certain embodiments $-L^2-$ is a spacer moiety selected from -T-, -C(O)O-, -O-, -C(O)-, $-C(O)N(R^{y1})-$, $-S(O)_2N(R^{y1})-$, $-S(O)N(R^{y1})-$, $-S(O)_2-$, -S(O)-, $-N(R^{y1})S(O)_2N(R^{y1a})-$, -S-, $-N(R^{y1})-$, $-OC(OR^{y1})(R^{y1a})-$, $-N(R^{y1})C(O)N(R^{y1a})-$, $-OC(O)N(R^{y1})-$, $C_{1-50}$ alkyl, $C_{2-50}$ alkenyl, and $C_{2-50}$ alkynyl; wherein -T-, $C_{1-20}$ alkyl, $C_{2-20}$ alkenyl, and $C_{2-20}$ alkynyl are optionally substituted with one or more $-R^{y2}$, which are the same or different and wherein $C_{1-20}$ alkyl, $C_{2-20}$ alkenyl, and $C_{2-20}$ alkynyl are optionally interrupted by one or more groups selected from the group consisting of -T-, -C(O)O-, -O-, -C(O)-, $-C(O)N(R^{y3})-$, $-S(O)_2N(R^{y3})-$, $-S(O)N(R^{y3})-$, $-S(O)_2-$, -S(O)-, $-N(R^{y3})S(O)_2N(R^{y3a})-$, -S-, $-N(R^{y3})-$, $-OC(OR^{y3})(R^{y3a})-$, $-N(R^{y3})C(O)N(R^{y3a})-$, and $-OC(O)N(R^{y3})-$;

$-R^{y1}$ and $-R^{y1a}$ are independently of each other selected from the group consisting of -H, -T, $C_{1-10}$ alkyl, $C_{2-10}$ alkenyl, and $C_{2-10}$ alkynyl; wherein -T, $C_{1-10}$ alkyl, $C_{2-10}$ alkenyl, and $C_{2-10}$ alkynyl are optionally substituted with one or more $-R^{y2}$, which are the same or different, and wherein $C_{1-10}$ alkyl, $C_{2-10}$ alkenyl, and $C_{2-10}$ alkynyl are optionally interrupted by one or more groups selected from the group consisting of -T-, -C(O)O-, -O-, -C(O)-, $-C(O)N(R^{y4})-$, $-S(O)_2N(R^{y4})-$, $-S(O)N(R^{y4})$_, $-S(O)_2-$, -S(O)-, $-N(R^{y4})S(O)_2N(R^{y4a})-$, -S-, $-N(R^{y4})-$, $-OC(OR^{y4})(R^{y4a})-$, $-N(R^{y4})C(O)N(R^{y4a})-$, and $-OC(O)N(R^{y4})-$;

each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more $-R^{y2}$, which are the same or different;

$-R^{y2}$ is selected from the group consisting of halogen, -CN, oxo (=O), $-COOR^{y5}$, $-OR^{y5}$, $-C(O)R^{y5}$, $-C(O)N(R^{y5}R^{y5a})$, $-S(O)_2N(R^{y5}R^{y5a})$, $-S(O)N(R^{y5}R^{y5a})$, $-S(O)_2R^{y5}$, $-S(O)R^{y5}$, $-N(R^{y5})S(O)_2N(R^{y5a}R^{y5b})$, $-SR^{y5}$, $-N(R^{y5}R^{y5a})$, $-NO_2$, $-OC(O)R^{y5}$, $-N(R^{y5})$ $C(O)R^{y5a}$, $-N(R^{y5})S(O)_2R^{y5a}$, $-N(R^{y5})S(O)R^{y5a}$, $-N(R^{y5})C(O)OR^{y5a}$, $-N(R^{y5})C(0)N(R^{y5a}R^{y5b})$, $-OC(O)N(R^{y5}R^{y5a})$, and $C_{1-6}$ alkyl; wherein $C_{1-6}$ alkyl is optionally substituted with one or more halogen, which are the same or different; and

each $-R^{y3}$, $-R^{y3a}$, $-R^{y4}$, $-R^{y4a}$, $-R^{y5}$, $-R^{y5a}$ and $-R^{y5b}$ is independently of each other selected from the group consisting of -H, and $C_{1-6}$ alkyl; wherein $C_{1-6}$ alkyl is optionally substituted with one or more halogen, which are the same or different.

In certain embodiments $-L^2-$ is a spacer moiety selected from the group consisting of -T-, -C(O)O-, -O-, -C(O)-, $-C(O)N(R^{y1})-$, $-S(O)_2N(R^{y1})-$, $-S(O)N(R^{y1})-$, $-S(O)_2-$, -S(O)-, $-N(R^{y1})S(O)_2N(R^{y1a})-$, -S-, $-N(R^{y1})-$, $-OC(OR^{y1})(R^{y1a})-$, $-N(R^{y1})C(O)N(R^{y1a})-$, $-OC(O)N(R^{y1})-$, $C_{1-50}$ alkyl, $C_{2-50}$ alkenyl, and $C_{2-50}$ alkynyl; wherein -T-, $C_{1-50}$ alkyl, $C_{2-50}$ alkenyl, and $C_{2-50}$ alkynyl are optionally substituted with one or more $-R^{y2}$, which are the same or different and wherein $C_{1-50}$ alkyl, $C_{2-50}$ alkenyl, and $C_{2-50}$ alkynyl are optionally interrupted by one or more groups selected from the group consisting of -T-, -C(O)O-, -O-, -C(O)-, $-C(O)N(R^{y3})-$, $-S(O)_2N(R^{y3})-$, $-S(O)N(R^{y3})-$, $-S(O)_2-$, -S(O)-, $-N(R^{y3})S(O)_2N(R^{y3a})-$, -S-, $-N(R^{y3})-$, $-OC(OR^{y3})(R^{y3a})-$, $-N(R^{y3})C(O)N(R^{y3a})-$, and $-OC(O)N(R^{y3})-$;

$-R^{y1}$ and $-R^{y1a}$ are independently selected from the group consisting of -H, -T, $C_{1-10}$ alkyl, $C_{2-10}$ alkenyl, and $C_{2-10}$ alkynyl;

each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl;

each $-R^{y2}$ is independently selected from the group consisting of halogen, and $C_{1-6}$ alkyl; and

each $-R^{y3}$, $-R^{y3a}$, $-R^{y4}$, $-R^{y4a}$, $-R^{y5}$, $-R^{y5a}$ and $-R^{y5b}$ is independently of each other selected from the group consisting of -H, and $C_{1-6}$ alkyl; wherein $C_{1-6}$ alkyl is optionally substituted with one or more halogen, which are the same or different.

In certain embodiments $-L^2-$ is a $C_{1-20}$ alkyl chain, which is optionally interrupted by one or more groups independently selected from -O-, -T- and $-C(O)N(R^{y1})-$; and which $C_{1-20}$ alkyl chain is optionally substituted with one or

more groups independently selected from -OH, -T and -C(O)N($R^{y6}R^{y6a}$); wherein -$R^{y1}$, -$R^{y6}$, -$R^{y6a}$ are independently selected from the group consisting of H and $C_{1-4}$ alkyl and wherein T is selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl.

In certain embodiments -$L^2$- has a molecular weight ranging from 14 g/mol to 750 g/mol.

In certain embodiments -$L^2$- comprises a moiety selected from

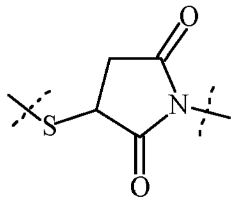

In certain embodiments -$L^2$- has a chain lengths of 1 to 20 atoms.

As used herein the term "chain length" with regard to the moiety -$L^2$- refers to the number of atoms of -$L^2$- present in the shortest connection between -$L^1$- and -Z.

In certain embodiments -$L^2$- is of formula (A-1)

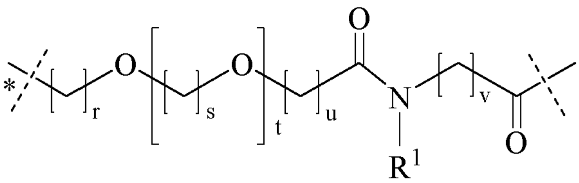

(A-1),

wherein

the dashed line marked with the asterisk indicates attachment to -$L^1$-,
the unmarked dashed line indicates attachment to Z,
r is selected from the group consisting of 1, 2, 3, 4, 5, 6, 7, 8, 9 and 10;
s is selected from the group consisting of 1, 2, 3, 4, 5, 6, 7, 8, 9 and 10;
t is selected from the group consisting of 0, 1, 2, 3, 4, 5, 6, 7, 8, 9 and 10;
u is selected from the group consisting of 1, 2, 3, 4, 5, 6, 7, 8, 9 and 10;
v is selected from the group consisting of 1, 2, 3, 4, 5, 6, 7, 8, 9 and 10; and
-$R^1$ is selected from the group consisting of -H, $C_{1-10}$ alkyl, $C_{2-10}$ alkenyl and $C_{2-10}$ alkynyl.

In certain embodiments r of formula (A-1) is 1. In certain embodiments r of formula (A-1) is 2. In certain embodiments r of formula (A-1) is 3. In certain embodiments r of formula (A-1) is 4. In certain embodiments r of formula (A-1) is 5. In certain embodiments r of formula (A-1) is 6. In certain embodiments r of formula (A-1) is 7. In certain embodiments r of formula (A-1) is 8. In certain embodiments r of formula (A-1) is 9. In certain embodiments r of formula (A-1) is 10.

In certain embodiments s of formula (A-1) is 1. In certain embodiments s of formula (A-1) is 2. In certain embodiments s of formula (A-1) is 3. In certain embodiments s of formula (A-1) is 4. In certain embodiments s of formula (A-1) is 5. In certain embodiments s of formula (A-1) is 6. In certain embodiments s of formula (A-1) is 7. In certain embodiments s of formula (A-1) is 8. In certain embodiments s of formula (A-1) is 9. In certain embodiments s of formula (A-1) is 10.

In certain embodiments t of formula (A-1) is 1. In certain embodiments t of formula (A-1) is 2. In certain embodiments t of formula (A-1) is 3. In certain embodiments t of formula (A-1) is 4. In certain embodiments t of formula (A-1) is 5. In certain embodiments t of formula (A-1) is 6. In certain embodiments t of formula (A-1) is 7. In certain embodiments t of formula (A-1) is 8. In certain embodiments t of formula (A-1) is 9. In certain embodiments t of formula (A-1) is 10.

In certain embodiments u of formula (A-1) is 1. In certain embodiments u of formula (A-1) is 2. In certain embodiments u of formula (A-1) is 3. In certain embodiments u of formula (A-1) is 4. In certain embodiments u of formula (A-1) is 5. In certain embodiments u of formula (A-1) is 6. In certain embodiments u of formula (A-1) is 7. In certain embodiments u of formula (A-1) is 8. In certain embodiments u of formula (A-1) is 9. In certain embodiments u of formula (A-1) is 10.

In certain embodiments v of formula (A-1) is 1. In certain embodiments v of formula (A-1) is 2. In certain embodiments v of formula (A-1) is 3. In certain embodiments v of formula (A-1) is 4. In certain embodiments v of formula (A-1) is 5. In certain embodiments v of formula (A-1) is 6. In certain embodiments v of formula (A-1) is 7. In certain embodiments v of formula (A-1) is 8. In certain embodiments v of formula (A-1) is 9. In certain embodiments v of formula (A-1) is 10.

In certain embodiments $-R^1$ of formula (A-1) is -H. In certain embodiments $-R^1$ of formula (A-1) is methyl. In certain embodiments $-R^1$ of formula (A-1) is ethyl. In certain embodiments $-R^1$ of formula (A-1) is n-propyl. In certain embodiments $-R^1$ of formula (A-1) is isopropyl. In certain embodiments $-R^1$ of formula (A-1) is n-butyl. In certain embodiments $-R^1$ of formula (A-1) is isobutyl. In certain embodiments $-R^1$ of formula (A-1) is sec-butyl. In certain embodiments $-R^1$ of formula (A-1) is tert-butyl. In certain embodiments $-R^1$ of formula (A-1) is n-pentyl. In certain embodiments $-R^1$ of formula (A-1) is 2-methylbutyl. In certain embodiments $-R^1$ of formula (A-1) is 2,2-dimethylpropyl. In certain embodiments $-R^1$ of formula (A-1) is n-hexyl. In certain embodiments $-R^1$ of formula (A-1) is 2-methylpentyl. In certain embodiments $-R^1$ of formula (A-1) is 3-methylpentyl. In certain embodiments $-R^1$ of formula (A-1) is 2,2-dimethylbutyl. In certain embodiments $-R^1$ of formula (A-1) is 2,3-dimethylbutyl. In certain embodiments $-R^1$ of formula (A-1) is 3,3-dimethylpropyl.

In certain embodiments r of formula (A-1) is 1, s of formula (A-1) is 2, t of formula (A-1) is 2, u of formula (A-1) is 1, v of formula (A-1) is 2 and $-R^1$ of formula (A-1) is -H.

In certain embodiments r of formula (A-1) is 1, s of formula (A-1) is 2, t of formula (A-1) is 3, u of formula (A-1) is 1, v of formula (A-1) is 2 and $-R^1$ of formula (A-1) is -H.

In certain embodiments r of formula (A-1) is 1, s of formula (A-1) is 2, t of formula (A-1) is 4, u of formula (A-1) is 1, v of formula (A-1) is 2 and $-R^1$ of formula (A-1) is -H.

In certain embodiments r of formula (A-1) is 1, s of formula (A-1) is 2, t of formula (A-1) is 5, u of formula (A-1) is 1, v of formula (A-1) is 2 and $-R^1$ of formula (A-1) is -H. Z is a hydrogel.

In certain embodiments such hydrogel Z comprises a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(alkylene glycols), such as poly(ethylene glycols) and poly(propylene glycol), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

In certain embodiments Z is a poly(alkylene glycol)-based hydrogel, such as a poly(propylene glycol)-based hydrogel or a poly(ethylene glycol)-based (PEG-based) hydrogel, or a hyaluronic acid-based hydrogel.

In certain embodiments Z is a PEG-based hydrogel. Suitable hydrogels are known in the art. Examples are WO2006/003014, WO2011/012715 and WO2014/056926.

In certain embodiments such PEG-based hydrogel comprises a plurality of backbone moieties that are crosslinked via crosslinker moieties $-CL^p-$. Optionally, there is a spacer moiety $-SP^1-$ between a backbone moiety and a crosslinker moiety. In certain embodiments such spacer $-SP^1-$ is defined as described above for $-L^2-$.

In certain embodiments the backbone moieties have a molecular weight ranging from 1 kDa to 20 kDa.

In certain embodiments the backbone moieties are of formula (pA)

$$B^*\text{-}(A\text{-}Hyp)_x \quad (pA),$$

wherein

B* is a branching core,
A is a PEG-based polymer,
Hyp is a branched moiety,
x is an integer of from 3 to 16;
and wherein each backbone moiety is connected to one or more crosslinker moieties and to one or more moieties $-L^2-$, which crosslinker moieties and moieties $-L^2-$ are connected to Hyp, either directly or through a spacer moiety.

In certain embodiments B* of formula (pA) is selected from the group consisting of polyalcohol moieties and

polyamine moieties. In certain embodiments B* of formula (pA) is a polyalcohol moiety. In certain embodiments B* of formula (pA) is a polyamine moiety.

In certain embodiments the polyalcohol moieties for B* of formula (pA) are selected from the group consisting of a pentaerythritol moiety, tripentaerythritol moiety, hexaglycerine moiety, sucrose moiety, sorbitol moiety, fructose moiety, mannitol moiety and glucose moiety. In certain embodiments B* of formula (pA) is a pentaerythritol moiety, i.e. a moiety of formula

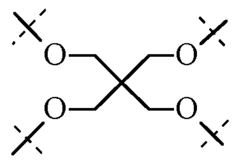
,

wherein dashed lines indicate attachment to -A-.

In certain embodiments the polyamine moieties for B* of formula (pA) is selected from the group consisting of an ornithine moiety, diaminobutyric acid moiety, trilysine moiety, tetralysine moiety, pentalysine moiety, hexalysine moiety, heptalysine moiety, octalysine moiety, nonalysine moiety, decalysine moiety, undecalysine moiety, dodecalysine moiety, tridecalysine moiety, tetradecalysine moiety and pentadecalysine moiety. In certain embodiments B* of formula (pA) is selected from the group consisting of an ornithine moiety, diaminobutyric acid moiety and a trilysine moiety.

A backbone moiety of formula (pA) may consist of the same or different PEG-based moieties -A- and each moiety -A- may be chosen independently. In certain embodiments all moieties -A- present in a backbone moiety of formula (pA) have the same structure. It is understood that the phrase "have the same structure" with regard to polymeric moieties, such as with regard to the PEG-based polymer -A-, means that the number of monomers of the polymer, such as the number of ethylene glycol monomers, may vary due to the polydisperse nature of polymers. In certain embodiments the number of monomer units does not vary by more than a factor of 2 between all moieties -A- of a hydrogel.

In certain embodiments each -A- of formula (pA) has a molecular weight ranging from 0.3 kDa to 40 kDa; e.g. from 0.4 to 30 kDa, from 0.4 to 25 kDa, from 0.4 to 20 kDa, from 0.4 to 15 kDa, from 0.4 to 10 kDa or from 0.4 to 5 kDa. In certain embodiments each -A- has a molecular weight from 0.4 to 5 kDa. In certain embodiments -A- has a molecular weight of about 0.5 kDa. In certain embodiments -A- has a molecular weight of about 1 kDa. In certain embodiments -A- has a molecular weight of about 2 kDa. In certain embodiments -A- has a molecular weight of about 3 kDa. In certain embodiments -A- has a molecular weight of about 5 kDa.

In certain embodiments -A- of formula (pA) is of formula (pB-i)

$-(CH_2)_{n1}(OCH_2CH_2)_nX-$ (pB-i),

wherein

n1 is 1 or 2;
n is an integer ranging from 3 to 250, such as from 5 to 200, such as from 8 to 150 or from 10 to 100; and
X is a chemical bond or a linkage covalently linking A and Hyp.

In certain embodiments -A- of formula (pA) is of formula (pB-ii)

$-(CH_2)_{n1}(OCH_2CH_2)_n-(CH_2)_{n2}X-$ (pB-ii),

wherein

n1 is 1 or 2;
n is an integer ranging from 3 to 250, such as from 5 to 200, such as from 8 to 150 or from 10 to 100;
n2 is 0 or 1; and
X is a chemical bond or a linkage covalently linking A and Hyp.

In certain embodiments -A- of formula (pA) is of formula (pB-i')

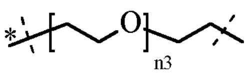
(pB-i'),

wherein

the dashed line marked with the asterisk indicates attachment to B*,
the unmarked dashed line indicates attachment to -Hyp; and
n3 is an integer ranging from 10 to 50.

In certain embodiments n3 of formula (pB-i') is 25. In certain embodiments n3 of formula (pB-i') is 26. In certain embodiments n3 of formula (pB-i') is 27. In certain embodiments n3 of formula (pB-i') is 28. In certain embodiments n3 of formula (pB-i') is 29. In certain embodiments n3 of formula (pB-i') is 30.

In certain embodiments a moiety B*-$(A)_4$ is of formula (pB-a)

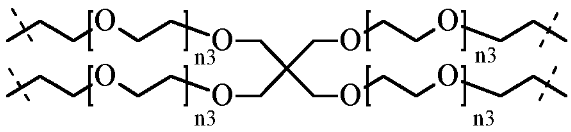
(pB-a),

wherein

dashed lines indicate attachment to Hyp; and
each n3 is independently an integer selected from 10 to 50.

**In** certain embodiments n3 of formula (pB-a) is 25. **In** certain embodiments n3 of formula (pB-a) is 26. **In** certain embodiments n3 of formula (pB-a) is 27. **In** certain embodiments n3 of formula (B-a) is 28. **In** certain embodiments n3 of formula (pB-a) is 29. **In** certain embodiments n3 of formula (pB-a) is 30.

A backbone moiety of formula (pA) may consist of the same or different dendritic moieties -Hyp and that each -Hyp can be chosen independently. **In** certain embodiments all moieties -Hyp present in a backbone moiety of formula (pA) have the same structure.

**In** certain embodiments each -Hyp of formula (pA) has a molecular weight ranging from 0.3 kDa to 5 kDa.

**In** certain embodiments -Hyp is selected from the group consisting of a moiety of formula (pHyp-i)

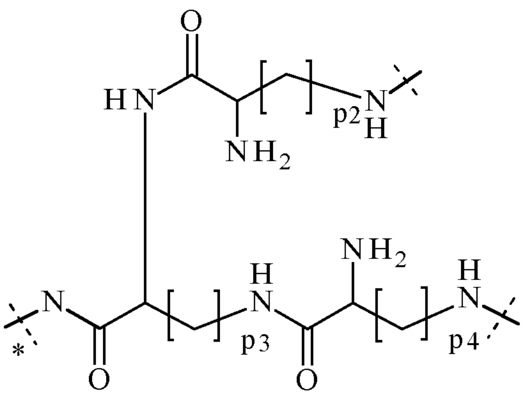
(pHyp-i),

wherein

the dashed line marked with the asterisk indicates attachment to -A-,
the unmarked dashed lines indicate attachment to a spacer moiety -$SP^1$-, a crosslinker moiety -$CL^p$- or to -$L^2$-; and
p2, p3 and p4 are identical or different and each is independently of the others an integer from 1 to 5;

a moiety of formula (pHyp-ii)

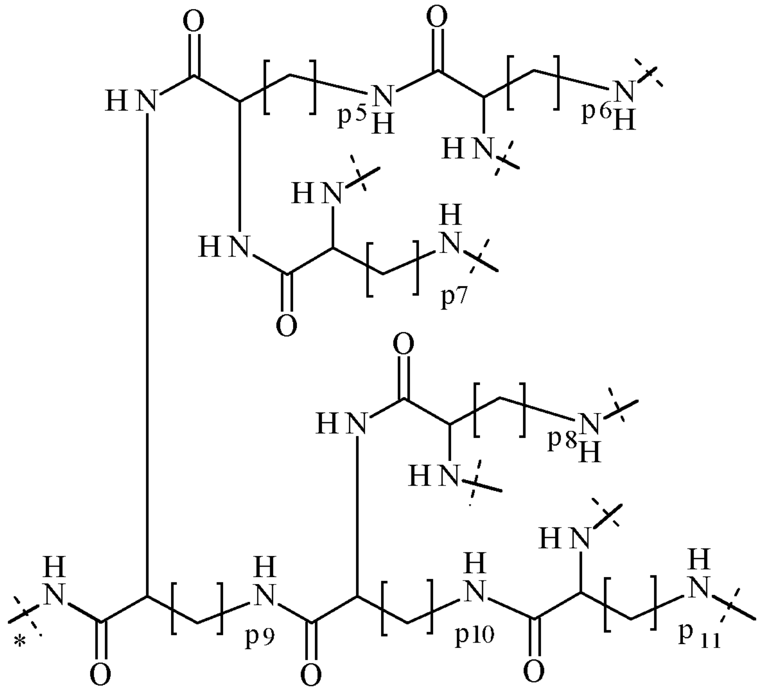

(pHyp-ii),

wherein

the dashed line marked with the asterisk indicates attachment to -A-,
the unmarked dashed lines indicate attachment to a spacer moiety $-SP^1-$, a crosslinker moiety $-CL^p-$ or to $-L^2-$; and
p5 to p11 are identical or different and each is independently of the others an integer from 1 to 5;

a moiety of formula (pHyp-iii)

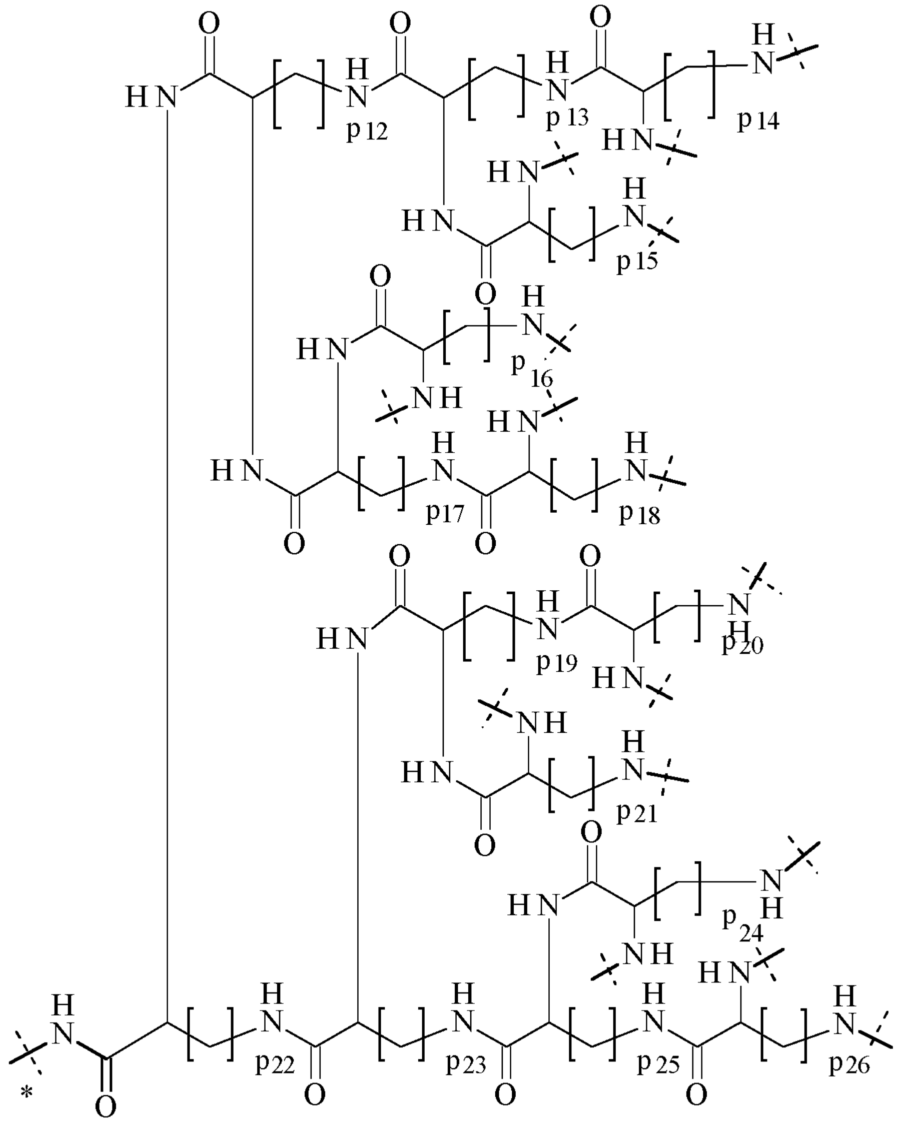

(pHyp-iii),

wherein

the dashed line marked with the asterisk indicates attachment to -A-,
the unmarked dashed lines indicate attachment to a spacer moiety -$SP^1$-, a crosslinker moiety -$CL^p$- or to -$L^2$-; and
p12 to p26 are identical or different and each is independently of the others an integer from 1 to 5; and

a moiety of formula (pHyp-iv)

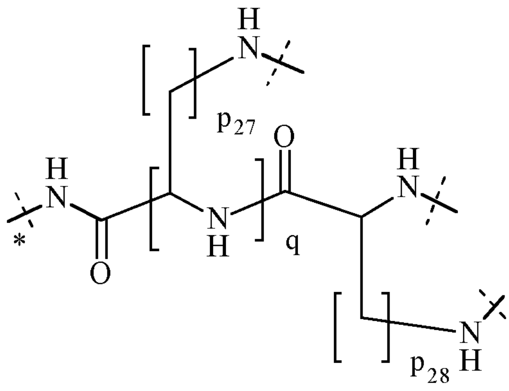

(pHyp-iv),

wherein

the dashed line marked with the asterisk indicates attachment to -A-,
the unmarked dashed lines indicate attachment to a spacer moiety $-SP^1-$, a crosslinker moiety $-CL^p-$ or to $-L^2-$;
p27 and p28 are identical or different and each is independently of the other an integer from 1 to 5; and
q is an integer from 1 to 8;

wherein the moieties (pHyp-i) to (pHyp-iv) may at each chiral center be in either R- or S-configuration.

In certain embodiments all chiral centers of a moiety (pHyp-i), (pHyp-ii), (pHyp-iii) or (pHyp-iv) are in the same configuration. In certain embodiments all chiral centers of a moiety (pHyp-i), (pHyp-ii), (pHyp-iii) or (pHyp-iv) are in R-configuration. In certain embodiments all chiral centers of a moiety (pHyp-i), (pHyp-ii), (pHyp-iii) or (pHyp-iv) are in S-configuration.

In certain embodiments p2, p3 and p4 of formula (pHyp-i) are 4.

In certain embodiments p5 to p11 of formula (pHyp-ii) are 4.

In certain embodiments p12 to p26 of formula (pHyp-iii) are 4.

In certain embodiments q of formula (pHyp-iv) is 2 or 6. In certain embodiments q of formula (pHyp-iv) q is 6.

In certain embodiments p27 and p28 of formula (pHyp-iv) are 4.

In certain embodiments -Hyp of formula (pA) comprises a branched polypeptide moiety.

In certain embodiments -Hyp of formula (pA) comprises a lysine moiety. In certain embodiments each -Hyp of formula (pA) is independently selected from the group consisting of a trilysine moiety, tetralysine moiety, pentalysine moiety, hexalysine moiety, heptalysine moiety, octalysine moiety, nonalysine moiety, decalysine moiety, undecalysine moiety, dodecalysine moiety, tridecalysine moiety, tetradecalysine moiety, pentadecalysine moiety, hexadecalysine moiety, heptadecalysine moiety, octadecalysine moiety and nonadecalysine moiety.

In certain embodiments -Hyp comprises 3 lysine moieties. In certain embodiments -Hyp comprises 7 lysine moieties. In certain embodiments -Hyp comprises 15 lysine moieties. In certain embodiments -Hyp comprises heptalysinyl.

In certain embodiments x of formula (pA) is 3. In certain embodiments x of formula (pA) is 4. In certain embodiments x of formula (pA) is 6. In certain embodiments x of formula (pA) is 8.

In certain embodiments the backbone moieties are of formula (pC1)

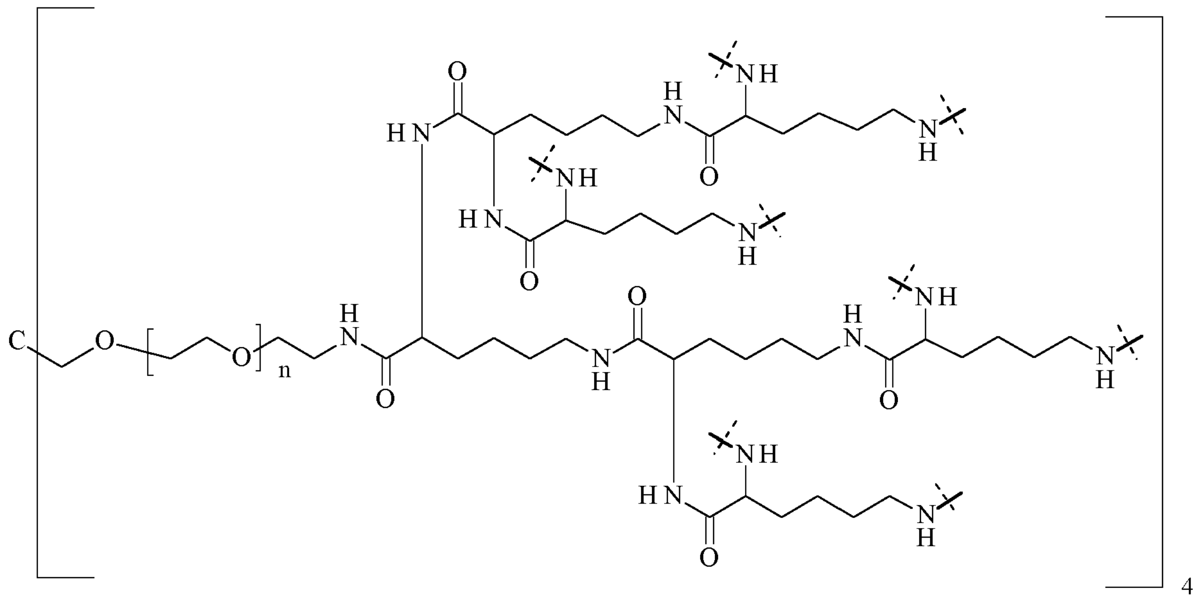

(pC1),

wherein

dashed lines indicate attachment to a spacer moiety -SP1-, a crosslinker moiety -CLp- or to -L2-; and
n ranges from 10 to 40.

In certain embodiments n of formula (pC1) is about 28.
In certain embodiments the backbone moiety is of formula (pC2)

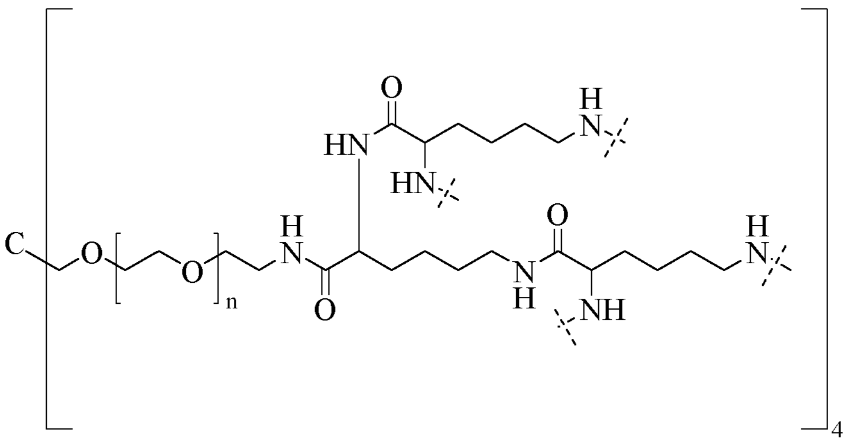

(pC2),

wherein

dashed lines indicate attachment to a spacer moiety -SP1-, a crosslinker moiety -CLp- or to -L2-; and
n ranges from 10 to 40.

In certain embodiments there is no spacer moiety -SP1- between a backbone moiety and a crosslinker moiety -CLp-, i.e. -CLp- is directly linked to -Hyp.
The crosslinker -CLp- of the PEG-based hydrogel is in certain embodiments poly(alkylene glycol) (PAG)-based. In certain embodiments the crosslinker is poly(propylene glycol)-based. In certain embodiments the crosslinker -CLp- is PEG-based.
In certain embodiments such PAG-based crosslinker moiety -CLp- is of formula (pD)

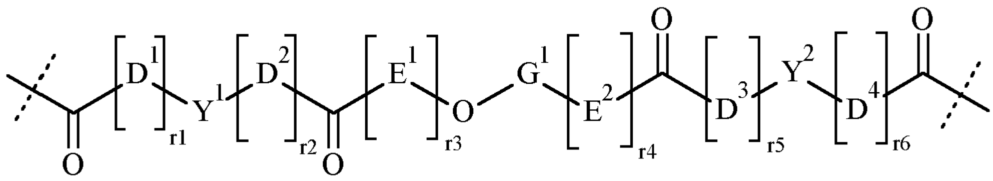

(pD),

wherein

dashed lines indicate attachment to a backbone moiety or to a spacer moiety -$SP^1$-;
-$Y^1$- is of formula

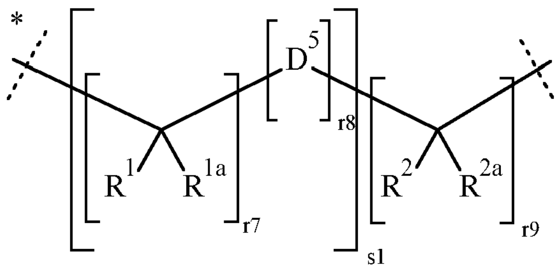

,

wherein the dashed line marked with the asterisk indicates attachment to -$D^1$- and the unmarked dashed line indicates attachment to -$D^2$-;
-$Y^2$- is of formula

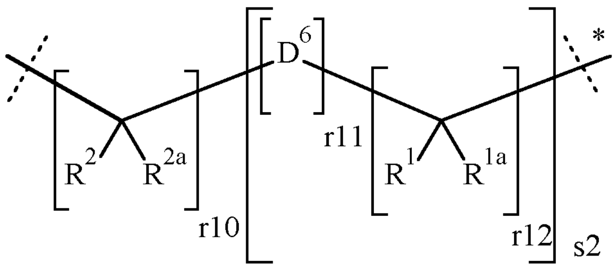

,

wherein the dashed line marked with the asterisk indicates attachment to -$D^4$- and the unmarked dashed line indicates attachment to -$D^3$-;
-$E^1$- is of formula

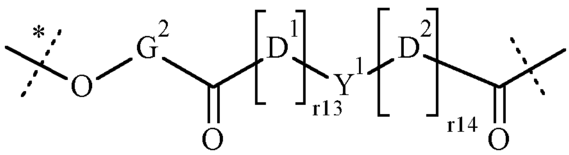

,

wherein the dashed line marked with the asterisk indicates attachment to -(C=O)- and the unmarked dashed line indicates attachment to -O-;
-$E^2$- is of formula

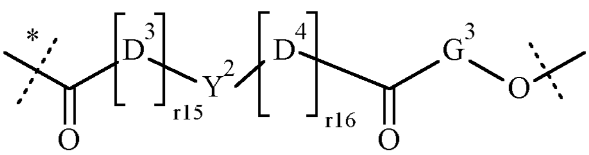

,

wherein the dashed line marked with the asterisk indicates attachment to $-G^1-$ and the unmarked dashed line indicates attachment to -(C=O)-;
$-G^1-$ is of formula

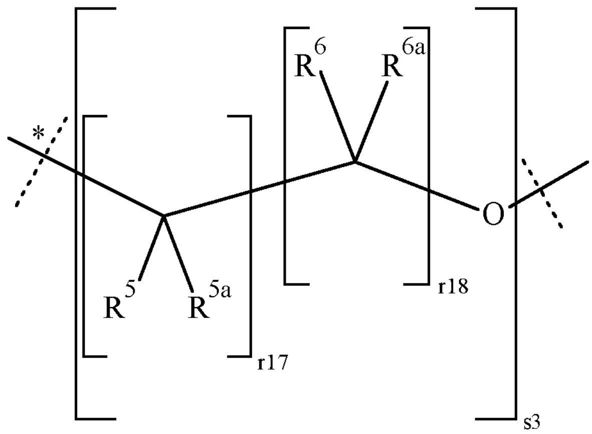

,

wherein the dashed line marked with the asterisk indicates attachment to -O- and the unmarked dashed line indicates attachment to $-E^2-$;
$-G^2-$ is of formula

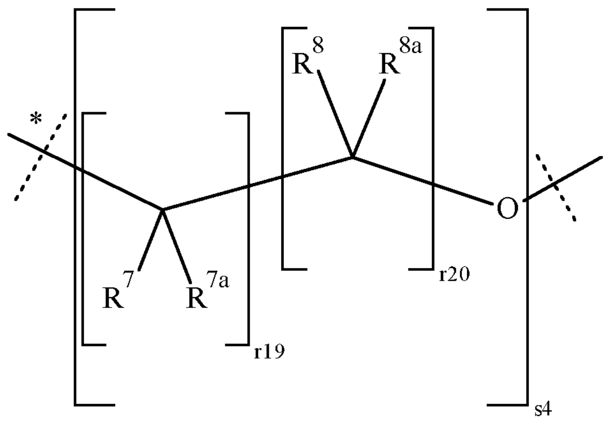

,

wherein the dashed line marked with the asterisk indicates attachment to -O- and the unmarked dashed line indicates attachment to -(C=O)-;
$-G^3-$ is of formula

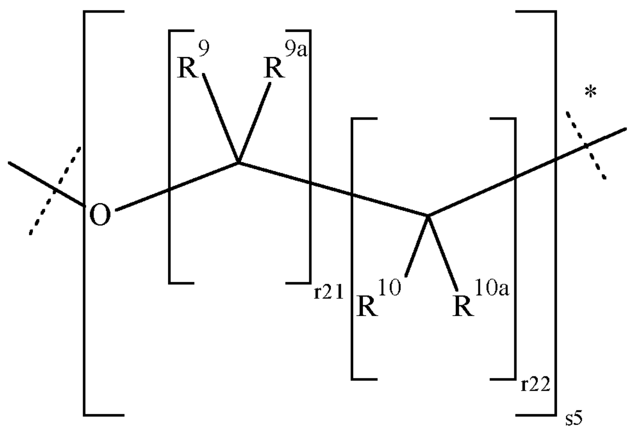

,

wherein the dashed line marked with the asterisk indicates attachment to -O- and the unmarked dashed line

indicates attachment to -(C=O)-;
$-D^1-$, $-D^2-$, $-D^3-$, $-D^4-$, $-D^5-$ and $-D^6-$ are identical or different and each is independently of the others selected from the group comprising -O-, $-NR^{11}-$, $-N^+R^{12}R^{12a}-$, -S-, -(S=O)-, $-(S(O)_2)-$, -C(O)-, $-P(O)R^{13}-$, $-P(O)(OR^{13})$ and $-CR^{14}R^{14a}-$;
$-R^1$, $-R^{1a}$, $-R^2$, $-R^{2a}$, $-R^3$, $-R^{3a}$, $-R^4$, $-R^{4a}$, $-R^5$, $-R^{5a}$, $-R^6$, $-R^{6a}$, $-R^7$, $-R^{7a}$, $-R^8$, $-R^{8a}$, $-R^9$, $-R^{9a}$, $-R^{10}$, $-R^{10a}$, $-R^{11}$, $-R^{12}$, $-R^{12a}$, $-R^{13}$, $-R^{14}$ and $-R^{14a}$ are identical or different and each is independently of the others selected from the group consisting of -H and $C_{1-6}$ alkyl;
optionally, one or more of the pairs $-R^1/-R^{1a}$, $-R^2/-R^{2a}$, $-R^3/-R^{3a}$, $-R^4/-R^{4a}$, $-R^1/-R^2$, $-R^3/-R^4$, $-R^{1a}/-R^{2a}$, $-R^{3a}/-R^{4a}$, $-R^{12}/-R^{12a}$, and $-R^{14}/-R^{14a}$ form a chemical bond or are joined together with the atom to which they are attached to form a $C_{3-8}$ cycloalkyl or to form a ring A or are joined together with the atom to which they are attached to form a 4- to 7-membered heterocyclyl or 8- to 11-membered heterobicyclyl or adamantyl;
A is selected from the group consisting of phenyl, naphthyl, indenyl, indanyl and tetralinyl;
r1, r2, r5, r6, r13, r14, r15 and r16 are independently 0 or 1;
r3, r4, r7, r8, r9, r10, r11, r12 are independently 0, 1, 2, 3, or 4;
r17, r18, r19, r20, r21 and r22 are independently 1, 2, 3, 4, 5, 6, 7, 8, 9 or 10;
s1, s2, s4, s5 are independently 1, 2, 3, 4, 5 or 6; and
s3 ranges from 1 to 900.

In certain embodiments s3 ranges from 1 to 500. In certain embodiments s3 ranges from 1 to 200.

In certain embodiments r1 of formula (pD) is 0. In certain embodiments r1 of formula (pD) is 1. In certain embodiments r2 of formula (pD) is 0. In certain embodiments r2 of formula (pD) is 1. In certain embodiments r5 of formula (pD) is 0. In certain embodiments r5 of formula (pD) is 1.

In certain embodiments r1, r2, r5 and r6 of formula (pD) are 0.

In certain embodiments r6 of formula (pD) is 0. In certain embodiments r6 of formula (pD) is 1. In certain embodiments r13 of formula (pD) is 0. In certain embodiments r13 of formula (pD) is 1. In certain embodiments r14 of formula (pD) is 0. In certain embodiments r14 of formula (pD) is 1. In certain embodiments r15 of formula (pD) is 0. In certain embodiments r15 of formula (pD) is 1. In certain embodiments r16 of formula (pD) is 0. In certain embodiments r16 of formula (pD) is 1.

In certain embodiments r3 of formula (pD) is 1. In certain embodiments r3 of formula (pD) is 2. In certain embodiments r4 of formula (pD) is 1. In certain embodiments r4 of formula (pD) is 2. In certain embodiments r3 and r4 of formula (pD) are both 1. In certain embodiments r3 and r4 of formula (pD) are both 2. In certain embodiments r3 and r4 of formula (pD) are both 3.

In certain embodiments r7 of formula (pD) is 0. In certain embodiments r7 of formula (pD) is 1. In certain embodiments r7 of formula (pD) is 2. In certain embodiments r8 of formula (pD) is 0. In certain embodiments r8 of formula (pD) is 1. In certain embodiments r8 of formula (pD) is 2. In certain embodiments r9 of formula (pD) is 0. In certain embodiments r9 of formula (pD) is 1. In certain embodiments r9 of formula (pD) is 2. In certain embodiments r10 of formula (pD) is 0. In certain embodiments r10 of formula (pD) is 1. In certain embodiments r10 of formula (pD) is 2. In certain embodiments r11 of formula (pD) is 0. In certain embodiments r11 of formula (pD) is 1. In certain embodiments r11 of formula (pD) is 2. In certain embodiments r12 of formula (pD) is 0. In certain embodiments r12 of formula (pD) is 1. In certain embodiments r12 of formula (pD) is 2.

In certain embodiments r17 of formula (pD) is 1. In certain embodiments r18 of formula (pD) is 1. In certain embodiments r19 of formula (pD) is 1. In certain embodiments r20 of formula (pD) is 1. In certain embodiments r21 of formula (pD) is 1.

In certain embodiments s1 of formula (pD) is 1. In certain embodiments s1 of formula (pD) is 2. In certain embodiments s2 of formula (pD) is 1. In certain embodiments s2 of formula (pD) is 2. In certain embodiments s4 of formula (pD) is 1. In certain embodiments s4 of formula (pD) is 2.

In certain embodiments s3 of formula (pD) ranges from 5 to 500. In certain embodiments s3 of formula (pD) ranges from 10 to 250. In certain embodiments s3 of formula (pD) ranges from 12 to 150. In certain embodiments s3 of formula (pD) ranges from 15 to 100. In certain embodiments s3 of formula (pD) ranges from 18 to 75. In certain embodiments s3 of formula (pD) ranges from 20 to 50.

In certain embodiments $-R^1$ of formula (pD) is -H. In certain embodiments $-R^1$ of formula (pD) is methyl. In certain embodiments $-R^1$ of formula (pD) is ethyl. In certain embodiments $-R^{1a}$ of formula (pD) is -H. In certain embodiments $-R^{1a}$ of formula (pD) is methyl. In certain embodiments $-R^{1a}$ of formula (pD) is ethyl. In certain embodiments $-R^2$ of formula (pD) is -H. In certain embodiments $-R^2$ of formula (pD) is methyl. In certain embodiments $-R^2$ of formula (pD) is ethyl. In certain embodiments $-R^{2a}$ of formula (pD) is -H. In certain embodiments $-R^{2a}$ of formula (pD) is methyl. In certain embodiments $-R^{2a}$ of formula (pD) is ethyl. In certain embodiments $-R^3$ of formula (pD) is -H. In certain embodiments $-R^3$ of formula (pD) is methyl. In certain embodiments $-R^3$ of formula (pD) is ethyl. In certain embodiments $-R^{3a}$ of formula (pD) is -H. In certain embodiments $-R^{3a}$ of formula (pD) is methyl. In certain embodiments $-R^{3a}$ of formula (pD) is ethyl. In certain embodiments

$-R^4$ of formula (pD) is -H. In certain embodiments $-R^4$ of formula (pD) is methyl. In certain embodiments $-R^4$ of formula (pD) is methyl. In certain embodiments $-R^{4a}$ of formula (pD) is -H. In certain embodiments $-R^{4a}$ of formula (pD) is methyl. In certain embodiments $-R^{4a}$ of formula (pD) is ethyl. In certain embodiments $-R^5$ of formula (pD) is -H. In certain embodiments $-R^5$ of formula (pD) is methyl. In certain embodiments $-R^5$ of formula (pD) is ethyl. In certain embodiments $-R^{5a}$ of formula (pD) is -H. In certain embodiments $-R^{5a}$ of formula (pD) is methyl. In certain embodiments $-R^{5a}$ of formula (pD) is ethyl. In certain embodiments $-R^6$ of formula (pD) is -H. In certain embodiments $-R^6$ of formula (pD) is methyl. In certain embodiments $-R^6$ of formula (pD) is ethyl. In certain embodiments $-R^{6a}$ of formula (pD) is -H. In certain embodiments $-R^{6a}$ of formula (pD) is methyl. In certain embodiments $-R^{6a}$ of formula (pD) is ethyl. In certain embodiments $-R^7$ of formula (pD) is -H. In certain embodiments $-R^7$ of formula (pD) is methyl. In certain embodiments $-R^7$ of formula (pD) is ethyl. In certain embodiments $-R^8$ of formula (pD) is -H. In certain embodiments $-R^8$ of formula (pD) is methyl. In certain embodiments $-R^8$ of formula (pD) is ethyl. In certain embodiments $-R^{8a}$ of formula (pD) is -H. In certain embodiments $-R^{8a}$ of formula (pD) is methyl. In certain embodiments $-R^{8a}$ of formula (pD) is ethyl. In certain embodiments $-R^9$ of formula (pD) is -H. In certain embodiments $-R^9$ of formula (pD) is methyl. In certain embodiments $-R^9$ of formula (pD) is ethyl. In certain embodiments $-R^{9a}$ of formula (pD) is -H. In certain embodiments $-R^{9a}$ of formula (pD) is methyl. In certain embodiments $-R^{9a}$ of formula (pD) is ethyl. In certain embodiments $-R^{9a}$ of formula (pD) is -H. In certain embodiments $-R^{9a}$ of formula (pD) is methyl. In certain embodiments $-R^{9a}$ of formula (pD) is ethyl. In certain embodiments $-R^{10}$ of formula (pD) is -H. In certain embodiments $-R^{10}$ of formula (pD) is methyl. In certain embodiments $-R^{10}$ of formula (pD) is ethyl. In certain embodiments $-R^{10a}$ of formula (pD) is -H. In certain embodiments $-R^{10a}$ of formula (pD) is methyl. In certain embodiments $-R^{10a}$ of formula (pD) is ethyl. In certain embodiments $-R^{11}$ of formula (pD) is -H. In certain embodiments $-R^{11}$ of formula (pD) is methyl. In certain embodiments $-R^{11}$ of formula (pD) is ethyl. In certain embodiments $-R^{12}$ of formula (pD) is -H. In certain embodiments $-R^{12}$ of formula (pD) is methyl. In certain embodiments $-R^{12}$ of formula (pD) is ethyl. In certain embodiments $-R^{12a}$ of formula (pD) is -H. In certain embodiments $-R^{12a}$ of formula (pD) is methyl. In certain embodiments $-R^{12a}$ of formula (pD) is ethyl. In certain embodiments $-R^{13}$ of formula (pD) is -H. In certain embodiments $-R^{13}$ of formula (pD) is methyl. In certain embodiments $-R^{13}$ of formula (pD) is ethyl. In certain embodiments $-R^{14}$ of formula (pD) is -H. In certain embodiments $-R^{14}$ of formula (pD) is methyl. In certain embodiments $-R^{14}$ of formula (pD) is ethyl. In certain embodiments $-R^{14a}$ of formula (pD) is -H. In certain embodiments $-R^{14a}$ of formula (pD) is methyl. In certain embodiments $-R^{14a}$ of formula (pD) is ethyl.

In certain embodiments $-D^1-$ of formula (pD) is -O-. In certain embodiments $-D^1-$ of formula (pD) is $-NR^{11}-$. In certain embodiments $-D^1-$ of formula (pD) is $-N^+R^{12}R^{12a}-$. In certain embodiments $-D^1-$ of formula (pD) is -S-. In certain embodiments $-D^1-$ of formula (pD) is -(S=O). In certain embodiments $-D^1-$ of formula (pD) is $-(S(O)_2)-$. In certain embodiments $-D^1-$ of formula (pD) is -C(O)-. In certain embodiments $-D^1-$ of formula (pD) is - $P(O)R^{13}-$. In certain embodiments $-D^1-$ of formula (pD) is $-P(O)(OR^{13})-$. In certain embodiments $-D^1-$ of formula (pD) is $-CR^{14}R^{14a}-$.

In certain embodiments $-D^2-$ of formula (pD) is -O-. In certain embodiments $-D^2-$ of formula (pD) is $-NR^{11}-$. In certain embodiments $-D^2-$ of formula (pD) is $-N^+R^{12}R^{12a}-$. In certain embodiments $-D^2-$ of formula (pD) is -S-. In certain embodiments $-D^2-$ of formula (pD) is -(S=O). In certain embodiments $-D^2-$ of formula (pD) is $-(S(O)_2)-$. In certain embodiments $-D^2-$ of formula (pD) is -C(O)-. In certain embodiments $-D^2-$ of formula (pD) is - $P(O)R^{13}-$. In certain embodiments $-D^2-$ of formula (pD) is $-P(O)(OR^{13})-$. In certain embodiments $-D^2-$ of formula (pD) is $-CR^{14}R^{14a}-$.

In certain embodiments $-D^3-$ of formula (pD) is -O-. In certain embodiments $-D^3-$ of formula (pD) is $-NR^{11}-$. In certain embodiments $-D^3-$ of formula (pD) is $-N^+R^{12}R^{12a}-$. In certain embodiments $-D^3-$ of formula (pD) is -S-. In certain embodiments $-D^3-$ of formula (pD) is -(S=O). In certain embodiments $-D^3-$ of formula (pD) is $-(S(O)_2)-$. In certain embodiments $-D^3-$ of formula (pD) is -C(O)-. In certain embodiments $-D^3-$ of formula (pD) is $-P(O)R^{13}-$. In certain embodiments $-D^3-$ of formula (pD) is $-P(O)(OR^{13})-$. In certain embodiments $-D^3-$ of formula (pD) is $-CR^{14}R^{14a}-$.

In certain embodiments $-D^4-$ of formula (pD) is -O-. In certain embodiments $-D^4-$ of formula (pD) is $-NR^{11}-$. In certain embodiments $-D^4-$ of formula (pD) is $-N^+R^{12}R^{12a}-$. In certain embodiments $-D^4-$ of formula (pD) is -S-. In certain embodiments $-D^4-$ of formula (pD) is -(S=O). In certain embodiments $-D^4-$ of formula (pD) is $-(S(O)_2)-$. In certain embodiments $-D^4-$ of formula (pD) is -C(O)-. In certain embodiments $-D^4-$ of formula (pD) is - $P(O)R^{13}-$. In certain embodiments $-D^4-$ of formula (pD) is $-P(O)(OR^{13})-$. In certain embodiments $-D^4-$ of formula (pD) is $-CR^{14}R^{14a}-$.

In certain embodiments $-D^5-$ of formula (pD) is -O-. In certain embodiments $-D^5-$ of formula (pD) is $-NR^{11}-$. In certain embodiments $-D^5-$ of formula (pD) is $-N^+R^{12}R^{12a}-$. In certain embodiments $-D^5-$ of formula (pD) is -S-. In certain embodiments $-D^5-$ of formula (pD) is -(S=O)-. In certain embodiments $-D^5-$ of formula (pD) is $-(S(O)_2)-$. In certain embodiments - $D^5-$ of formula (pD) is -C(O)-. In certain embodiments $-D^5-$ of formula (pD) is $-P(O)R^{13}-$. In certain embodiments $-D^5-$ of formula (pD) is $-P(O)(OR^{13})-$. In certain embodiments $-D^5-$ of formula (pD) is $-CR^{14}R^{14a}-$.

In certain embodiments $-D^6-$ of formula (pD) is -O-. In certain embodiments $-D^6-$ of formula (pD) is $-NR^{11}-$. In certain embodiments $-D^6-$ of formula (pD) is $-N^+R^{12}R^{12a}-$. In certain embodiments $-D^6-$ of formula (pD) is -S-. In certain embodiments $-D^6-$ of formula (pD) is -(S=O). In certain embodiments $-D^6-$ of formula (pD) is $-(S(O)_2)-$. In certain embodiments $-D^6-$ of formula (pD) is -C(O)-. In certain embodiments $-D^6-$ of formula (pD) is - $P(O)R^{13}-$. In certain embodiments $-D^6-$ of formula (pD) is $-P(O)(OR^{13})-$. In certain embodiments $-D^6-$ of formula (pD) is $-CR^{14}R^{14a}-$.

In one embodiment $-CL^p-$ is of formula (pE)

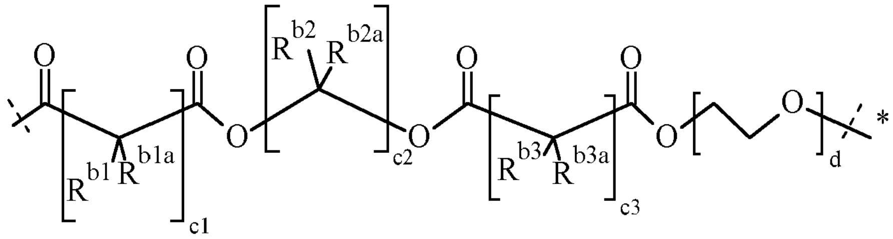

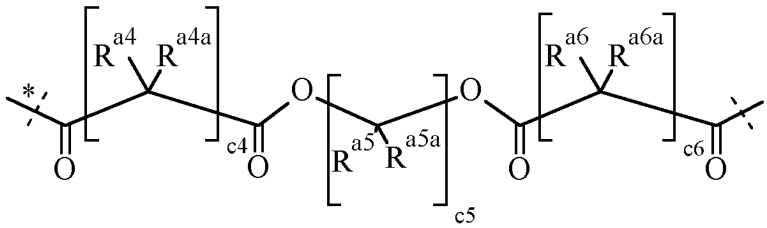

(pE),

wherein

dashed lines marked with an asterisk indicate the connection point between the upper and the lower substructure, unmarked dashed lines indicate attachment to a backbone moiety or to a spacer moiety $-SP^1-$;

$-R^{b1}$, $-R^{b1a}$, $-R^{b2}$, $-R^{b2a}$, $-R^{b3}$, $-R^{b3a}$, $-R^{b4}$, $-R^{b4a}$, $-R^{b5}$, $-R^{b5a}$, $-R^{b6}$ and $-R^{b6}$ are independently selected from the group consisting of -H and $C_{1-6}$ alkyl;

c1, c2, c3, c4, c5 and c6 are independently selected from the group consisting of 1, 2, 3, 4, 5 and 6;

d is an integer ranging from 2 to 250.

In certain embodiments d of formula (pE) ranges from 3 to 200. In certain embodiments d of formula (pE) ranges from 4 to 150. In certain embodiments d of formula (pE) ranges from 5 to 100. In certain embodiments d of formula (pE) ranges from 10 to 50. In certain embodiments d of formula (pE) ranges from 15 to 30. In certain embodiments d of formula (pE) is about 23.

In certain embodiments $-R^{b1}$ and $-R^{b1a}$ of formula (pE) are -H. In certain embodiments $-R^{b1}$ and $-R^{b1a}$ of formula (pE) are -H. In certain embodiments $-R^{b2}$ and $-R^{b2a}$ of formula (pE) are -H. In certain embodiments $-R^{b3}$ and-$R^{b3a}$ of formula (pE) are -H. In certain embodiments $-R^{b4}$ and $-R^{b4a}$ of formula (pE) are -H. In certain embodiments $-R^{b5}$ and $-R^{b5a}$ of formula (pE) are -H. In certain embodiments $-R^{b6}$ and $-R^{b6a}$ of formula (pE) are -H.

In certain embodiments $-R^{b1}$, $-R^{b1a}$, $-R^{b2}$, $-R^{b2a}$, $-R^{b3}$, $-R^{b3a}$, $-R^{b4}$, $-R^{b4a}$, $-R^{b5}$, $-R^{b5a}$, $-R^{b6}$ and $-R^{b6}$ of formula (pE) are all -H.

In certain embodiments c1 of formula (pE) is 1. In certain embodiments c1 of formula (pE) is 2. In certain embodiments c1 of formula (pE) is 3. In certain embodiments c1 of formula (pE) is 4. In certain embodiments c1 of formula (pE) is 5. In certain embodiments c1 of formula (pE) is 6.

In certain embodiments c2 of formula (pE) is 1. In certain embodiments c2 of formula (pE) is 2. In certain embodiments c2 of formula (pE) is 3. In certain embodiments c2 of formula (pE) is 4. In certain embodiments c2 of formula (pE) is 5. In certain embodiments c2 of formula (pE) is 6.

In certain embodiments c3 of formula (pE) is 1. In certain embodiments c3 of formula (pE) is 2. In certain embodiments c3 of formula (pE) is 3. In certain embodiments c3 of formula (pE) is 4. In certain embodiments c3 of formula (pE) is 5. In certain embodiments c3 of formula (pE) is 6.

In certain embodiments c4 of formula (pE) is 1. In certain embodiments c4 of formula (pE) is 2. In certain embodiments c4 of formula (pE) is 3. In certain embodiments c4 of formula (pE) is 4. In certain embodiments c4 of formula (pE) is 5. In certain embodiments c4 of formula (pE) is 6.

In certain embodiments c5 of formula (pE) is 1. In certain embodiments c5 of formula (pE) is 2. In certain embodiments c5 of formula (pE) is 3. In certain embodiments c5 of formula (pE) is 4. In certain embodiments c5 of formula (pE) is 5. In certain embodiments c5 of formula (pE) is 6.

In certain embodiments c6 of formula (pE) is 1. In certain embodiments c6 of formula (pE) is 2. In certain embodiments c6 of formula (pE) is 3. In certain embodiments c6 of formula (pE) is 4. In certain embodiments c6 of formula (pE) is 5. In certain embodiments c6 of formula (pE) is 6.

In certain embodiments a crosslinker moiety $-CL^p-$ is of formula (pE-i)

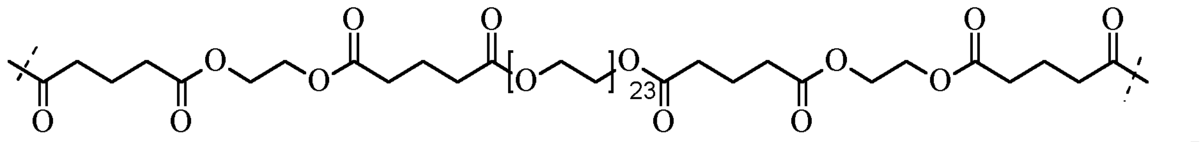

(pE-i),

wherein
dashed lines indicate attachment to a backbone moiety or to a spacer moiety $-SP^1-$.

In certain embodiments -Z is a hyaluronic acid-based hydrogel. Such hyaluronic acid-based hydrogels are known in the art, such as for example from WO2018/175788.

If -Z is a hyaluronic acid-based hydrogel, a conjugate of the present invention is in certain embodiments a conjugate comprising crosslinked hyaluronic acid strands to which a plurality of drug moieties is covalently and reversibly conjugated, wherein the conjugate comprises a plurality of connected units selected from the group consisting of

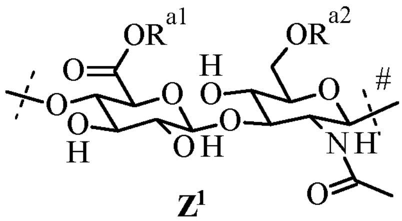

$Z^1$ ,

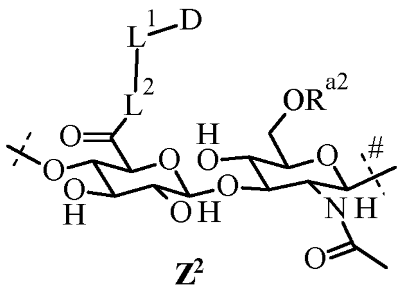

$Z^2$

and

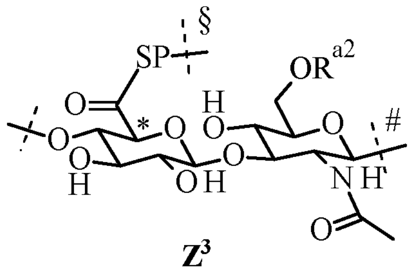

$Z^3$ ,

wherein

an unmarked dashed line indicates a point of attachment to an adjacent unit at a dashed line marked with # or to a hydrogen;
a dashed line marked with # indicates a point of attachment to an adjacent unit at an unmarked dashed line or to a hydroxyl;
a dashed line marked with § indicates a point of connection between at least two units $Z^3$ via a moiety -CL-;
each -D, $-L^1-$, and $-L^2$ are used as defined above;
each -CL- is independently a moiety connecting at least two units $Z^3$ and wherein there is at least one degradable bond in the direct connection between any two carbon atoms marked with the * connected by a moiety -CL-;
each -SP- is independently absent or a spacer moiety;
each $-R^{a1}$ is independently selected from the group consisting of -H, $C_{1-4}$ alkyl, an ammonium ion, a tetrabutylammonium ion, a cetyl methylammonium ion, an alkali metal ion and an alkaline earth metal ion;
each $-R^{a2}$ is independently selected from the group consisting of -H and $C_{1-10}$ alkyl;
wherein
all units $Z^1$ present in the conjugate may be the same or different;
all units $Z^2$ present in the conjugate may be the same or different;
all units $Z^3$ present in the conjugate may be the same or different;
at least one unit $Z^3$ is present per hyaluronic acid strand which is connected to at least one unit $Z^3$ on a different

hyaluronic acid strand; and
the conjugate comprises at least one moiety $-L^2-L^1-D$.

The presence of at least one degradable bond between the carbon atom marked with the * of a first moiety $Z^3$ and the direct connection to the carbon atom marked with the * of a second moiety $Z^3$ ensures that after cleavage of all such degradable bonds the hyaluronic acid strands present in said conjugate are no longer crosslinked, which allows clearance of the hyaluronic acid network

It is understood that in case a degradable bond is located in a ring structure present in the direct connection of the carbon atom marked with the * of a first moiety $Z^3$ and the carbon atom marked with the * of a second moiety $Z^3$ such degradable bond is not sufficient to allow complete cleavage and accordingly one or more additional degradable bonds are present in the direct connection of the carbon atom marked with the * of a first moiety $Z^3$ and the carbon atom marked with the * of a second moiety $Z^3$.

It is understood that the phrase "a dashed line marked with § indicates a point of connection between at least two units $Z^3$ via a moiety -CL-" refers to the following structure

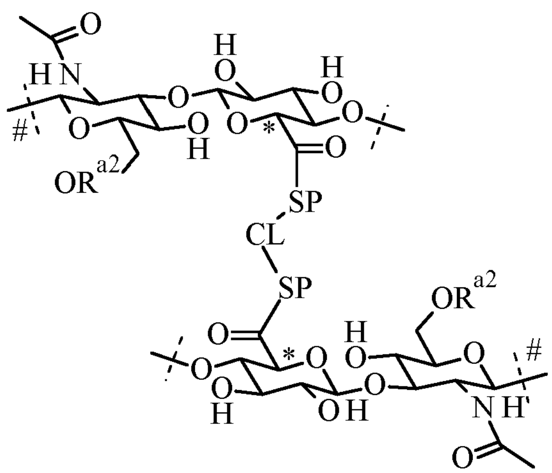

if -CL- is for example connected to two units $Z^3$, which two moieties $Z^3$ are connected at the position indicated with § via a moiety -CL-.

It is understood that no three-dimensionally crosslinked hydrogel can be formed if all hyaluronic acid strands of the present conjugate comprise only one unit $Z^3$, which is connected to only one unit $Z^3$ on a different hyaluronic acid strand. However, if a first unit $Z^3$ is connected to more than one unit $Z^3$ on a different strand, i.e. if -CL- is branched, such first unit $Z^3$ may be crosslinked to two or more other units $Z^3$ on two or more different hyaluronic acid strands. Accordingly, the number of units $Z^3$ per hyaluronic acid strand required for a crosslinked hyaluronic acid hydrogel depends on the degree of branching of -CL-. In certain embodiments at least 30% of all hyaluronic acid strands present in the conjugate are connected to at least two other hyaluronic acid strands. It is understood that it is sufficient if the remaining hyaluronic acid strands are connected to only one other hyaluronic acid strand.

It is understood that such hydrogel also comprises partly reacted or unreacted units and that the presence of such moieties cannot be avoided. In certain embodiments the sum of such partly reacted or unreacted units is no more than 25% of the total number of units present in the conjugate, such as no more than 10%, such as no more than 15% or such as no more than 10%.

Furthermore, it is understood that in addition to units $Z^1$, $Z^2$ and $Z^3$, partly reacted and unreacted units a conjugate may also comprise units that are the result of cleavage of the reversible bond between -D and $-L^1-$ or of one or more of the degradable bonds present in the direct connection between any two carbon atoms marked with the * connected by a moiety -CL-, i.e. units resulting from degradation of the conjugate.

In certain embodiments each strand present in the conjugates of the present invention comprises at least 20 units, such as from 20 to 2500 units, from 25 to 2200 units, from 50 to 2000 units, from 75 to 100 units, from 75 to 100 units, from 80 to 560 units, from 100 to 250 units, from 200 to 800 units, from 20 to 1000, from 60 to 1000, from 60 to 400 or from 200 to 600 units.

In certain embodiments the moieties -CL- present in the conjugates of the present invention have different structures. In certain embodiments the moieties -CL- present in the conjugates of the present invention have the same structure.

In general, any moiety that connects at least two other moieties is suitable for use as a moiety -CL-, which may also be referred to as a "crosslinker moiety".

The at least two units $Z^3$ that are connected via a moiety -CL- may either be located on the same hyaluronic acid strand or on different hyaluronic acid strands.

The moiety -CL- may be linear or branched. In certain embodiments -CL- is linear. In certain embodiments -CL- is branched.

In certain embodiments -CL- connects two units $Z^3$. In certain embodiments -CL- connects three units $Z^3$. In certain embodiments -CL- connects four units $Z^3$. In certain embodiments -CL- connects five units $Z^3$. In certain embodiments -CL- connects six units $Z^3$. In certain embodiments -CL- connects seven units $Z^3$. In certain embodiments -CL- connects eight units $Z^3$. In certain embodiments -CL- connects nine units $Z^3$.

If -CL- connects two units $Z^3$ -CL- may be linear or branched. If -CL- connects more than two units $Z^3$ -CL- is branched.

A branched moiety -CL- comprises at least one branching point from which at least three branches extend, which branches may also be referred to as "arms". Such branching point may be selected from the group consisting of

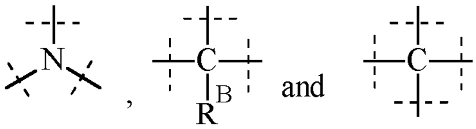

, and ,

wherein

dashed lines indicate attachment to an arm; and

$-R^B$ is selected from the group consisting of -H, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more $-R^{B1}$, which are the same or different, and wherein $C_{1-6}$ alkyl, $C_{2-6}$ alkenyl and $C_{2-6}$ alkynyl are optionally interrupted with -C(O)O-, -O-, -C(O)-, $-C(O)N(R^{B2})-$, $-S(O)_2N(R^{B2})-$, $-S(O)N(R^{B2})-$, $-S(O)_2-$, -S(O)-, $-N(R^{B2})S(O)_2N(R^{B2a})-$, -S-, $-N(R^{B2})-$, $-OC(OR^{B2})(R^{B2a})-$, $-N(R^{B2})C(O)N(R^{B2a})-$, and $-OC(O)N(R^{B2})-$; wherein $-R^{B1}$, $-R^{B2}$ and $-R^{B2a}$ are selected from -H, $C_{1-6}$ alkyl, $C_{2-6}$ alkenyl and $C_{2-6}$ alkynyl.

In certain embodiments $-R^B$ is selected from the group consisting of -H, methyl and ethyl.

A branched moiety -CL- may comprise a plurality of branching points, such as 1, 2, 3, 4, 5, 6, 7 or more branching points, which may be the same or different.

If a moiety -CL- connects three units $Z^3$, such moiety -CL- comprises at least one branching point from which at least three arms extend.

If a moiety -CL- connects four units $Z^3$, such moiety -CL- may comprise one branching point from which four arms extend. However, alternative geometries are possible, such as at least two branching points from which at least three arms each extend. The larger the number of connected units $Z^3$, the larger the number of possible geometries is.

In a first embodiment at least 70%, such as at least 75%, such as at least 80%, such as at least 85%, such as at least 90% or such as at least 95% of the number of hyaluronic acid strands of the conjugate of the present invention comprise at least one moiety $Z^2$ and at least one moiety $Z^3$. In such embodiment units $Z^2$ and $Z^3$ can be found in essentially all hyaluronic acid strands present in the conjugates of the present invention.

Accordingly, a conjugate of this first embodiment comprises crosslinked hyaluronic acid strands to which a plurality of drug moieties are covalently and reversibly conjugated, wherein the conjugate comprises a plurality of connected units selected from the group consisting of

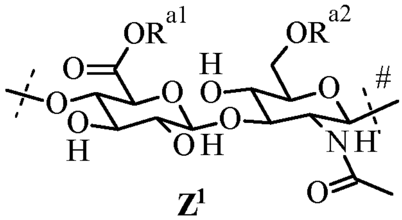

$Z^1$ ,

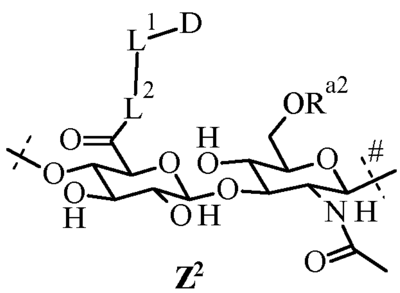

$Z^2$

and

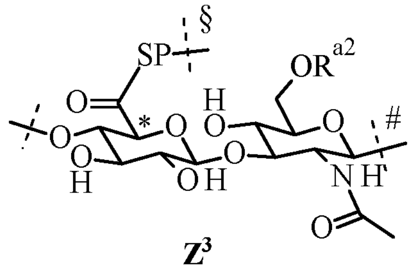

$Z^3$ ,

wherein

an unmarked dashed line indicates a point of attachment to an adjacent unit at a dashed line marked with # or to a hydrogen;
a dashed line marked with # indicates a point of attachment to an adjacent unit at an unmarked dashed line or to a hydroxyl;
a dashed line marked with § indicates a point of connection between at least two units $Z^3$ via a moiety -CL-;
-D, $-L^1-$, $-L^2-$, are used as defined above;

wherein

all units $Z^1$ present in the conjugate may be the same or different;
all units $Z^2$ present in the conjugate may be the same or different;
all units $Z^3$ present in the conjugate may be the same or different;
the number of $Z^1$ units ranges from 1% to 98% of the total number of units present in the conjugate;
the number of $Z^2$ units ranges from 1% to 98% of the total number of units present in the conjugate, provided at least one unit $Z^2$ is present in the conjugate;
the number of $Z^3$ units ranges from 1% to 97% of the total number of units present in the conjugate, provided that at least one unit $Z^3$ is present per strand; and
wherein at least 70% of all hyaluronic acid strands comprise at least one moiety $Z^2$ and at least one moiety $Z^3$.

In a conjugate according to this first embodiment the number of units $Z^2$ ranges from 1 to 70% of all units present in the conjugate, such as from 2 to 15%, from 2 to 10%, from 16 to 39, from 40 to 65%, or from 50 to 60% of all units present in the conjugate.
In a conjugate according to this first embodiment the number of units $Z^3$ ranges from 1 to 30% of all units present in the conjugate, such as from 2 to 5%, from 5 to 20%, from 10 to 18%, or from 14 to 18% of all units present in the conjugate.
In a conjugate according to this first embodiment the number of units $Z^1$ ranges from 10 to 97% of all units present in the conjugate, such as from 20 to 40%, such as from 25 to 35%, such as from 41 to 95%, such as from 45 to 90%, such as from 50 to 70% of all units present in the conjugate.
Each degradable bond present in the direct connection between any two carbon atoms marked with the * connected by a moiety -CL- may be different or all such degradable bonds present in the conjugate may be the same.
Each direct connection between two carbon atoms marked with the * connected by a moiety -CL- may have the same or a different number of degradable bonds.
In certain embodiments the number of degradable bonds present in the conjugate of the present invention between all combinations of two carbon atoms marked with the * connected by a moiety -CL- is the same and all such degradable bonds have the same structure.
In the first embodiment the at least one degradable bond present in the direct connection between any two carbon atoms marked with the * connected by a moiety -CL- may be selected from the group consisting of ester, carbonate, sulfate, phosphate bonds, carbamate and amide bonds. It is understood that carbamates and amides are not reversible per se, and that in this context neighboring groups render these bonds reversible. In certain embodiments there is one degradable bond selected from the group consisting of ester, carbonate, sulfate, phosphate bonds, carbamate and amide bonds in the direct connection between any two carbon atoms marked with the * connected by a moiety -CL-. In certain embodiments there are two degradable bonds selected from the group consisting of ester, carbonate, sulfate, phosphate bonds, carbamate and amide bonds in the direct connection between any two carbon atoms marked with the * connected by a moiety -CL-, which degradable bonds may be the same or different. In certain embodiments there are three degradable bonds selected from the group consisting of ester, carbonate, sulfate, phosphate bonds, carbamate and amide bonds in the direct connection between any two carbon atoms marked with the * connected by a moiety -CL-, which degradable bonds may be the same or different. In certain embodiments there are four degradable bonds selected from the group

consisting of ester, carbonate, sulfate, phosphate bonds, carbamate and amide bonds in the direct connection between any two carbon atoms marked with the * connected by a moiety -CL-, which degradable bonds may be the same or different. In certain embodiments there are five degradable bonds selected from the group consisting of ester, carbonate, sulfate, phosphate bonds, carbamate and amide bonds in the direct connection between any two carbon atoms marked with the * connected by a moiety -CL-, which degradable bonds may be the same or different. In certain embodiments there are six degradable bonds selected from the group consisting of ester, carbonate, sulfate, phosphate bonds, carbamate and amide bonds in the direct connection between any two carbon atoms marked with the * connected by a moiety -CL-, which degradable bonds may be the same or different. It is understood that if more than two units $Z^3$ are connected by -CL- there are more than two carbons marked with * that are connected and thus there is more than one shortest connection with at least one degradable bond present. Each shortest connection may have the same or different number of degradable bonds.

In certain embodiments the at least one degradable bond, such as one, two, three, four, five, six degradable bonds, are located within -CL-.

In certain embodiments the at least one degradable bond present in the direct connection between any two carbon atoms marked with * connected by a moiety -CL- is one ester bond. In other embodiments the at least one degradable bond are two ester bonds. In other embodiments the at least one degradable bond are three ester bonds. In other embodiments the at least one degradable bond are four ester bonds. In other embodiments the at least one degradable bond are five ester bonds. In other embodiments the at least one degradable bond are six ester bonds.

In certain embodiments the at least one degradable bond present in the direct connection between any two carbon atoms marked with * connected by a moiety -CL- is one carbonate bond. In other embodiments the at least one degradable bond are two carbonate bonds. In other embodiments the at least one degradable bond are three carbonate bonds. In other embodiments the at least one degradable bond are four carbonate bonds. In other embodiments the at least one degradable bond are five carbonate bonds. In other embodiments the at least one degradable bond are six carbonate bonds.

In certain embodiments the at least one degradable bond present in the direct connection between any two carbon atoms marked with * connected by a moiety -CL- is one phosphate bond. In other embodiments the at least one degradable bond are two phosphate bonds. In other embodiments the at least one degradable bond are three phosphate bonds. In other embodiments the at least one degradable bond are four phosphate bonds. In other embodiments the at least one degradable bond are five phosphate bonds. In other embodiments the at least one degradable bond are six phosphate bonds.

In certain embodiments the at least one degradable bond present in the direct connection between any two carbon atoms marked with * connected by a moiety -CL- is one sulfate bond. In other embodiments the at least one degradable bond are two sulfate bonds. In other embodiments the at least one degradable bond are three sulfate bonds. In other embodiments the at least one degradable bond are four sulfate bonds. In other embodiments the at least one degradable bond are five sulfate bonds. In other embodiments the at least one degradable bond are six sulfate bonds.

In certain embodiments the at least one degradable bond present in the direct connection between any two carbon atoms marked with * connected by a moiety -CL- is one carbamate bond. In other embodiments the at least one degradable bond are two carbamate bonds. In other embodiments the at least one degradable bond are three carbamate bonds. In other embodiments the at least one degradable bond are four carbamate bonds. In other embodiments the at least one degradable bond are five carbamate bonds. In other embodiments the at least one degradable bond are six carbamate bonds.

In certain embodiments the at least one degradable bond present in the direct connection between any two carbon atoms marked with * connected by a moiety -CL- is one amide bond. In other embodiments the at least one degradable bond are two amide bonds. In other embodiments the at least one degradable bond are three amide bonds. In other embodiments the at least one degradable bond are four amide bonds. In other embodiments the at least one degradable bond are five amide bonds. In other embodiments the at least one degradable bond are six amide bonds.

It was found that a high degree of derivatization of the disaccharide units of hyaluronic acid, meaning that the number of units $Z^1$ is less than 80% of all units present in the conjugate, interferes with degradation of the hydrogel by certain hyaluronidases. This has the effect that less degradation by hyaluronidases occurs and that chemical cleavage of the degradable bonds becomes more relevant. This renders degradation of the conjugate more predictable. The reason for this is that the level of enzymes, such as hyaluronidases, exhibits inter-patient variability and may vary between different administration sites, whereas chemical cleavage predominantly depends on temperature and pH which are more stable parameters and thus chemical cleavage tends to be more predictable.

In some embodiments -CL- is $C_{1-50}$ alkyl, which is optionally interrupted by one or more atoms or groups selected from the group consisting of -T-, -C(O)O-, -O-, -C(O)-, -C(O)N($R^{c1}$)-, -S(O)$_2$-, -S(O)-, -S-, -N($R^{c1}$)-, -OC(O$R^{c1}$)($R^{c1a}$)- and -OC(O)N($R^{c1}$)-;

wherein -T- is selected from the group consisting of phenyl, naphthyl, indenyl, indanyl, tetralinyl, $C_{3-10}$ cycloalkyl, 3- to

10-membered heterocyclyl, and 8- to 11-membered heterobicyclyl; and
$-R^{c1}$ and $-R^{c1a}$ are selected from the group consisting of -H and $C_{1-6}$ alkyl.

In certain embodiments -CL- is a moiety of formula (A)

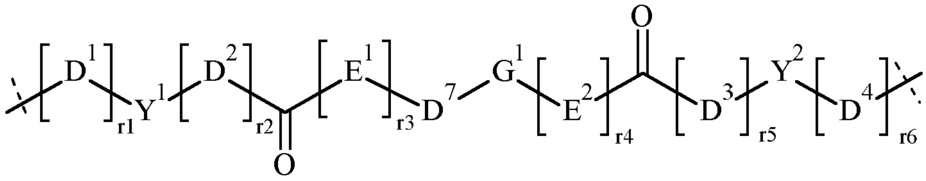

(A),

wherein

-$Y^1$- is of formula

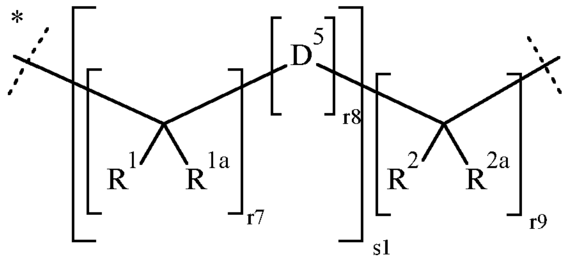

,

wherein the dashed line marked with the asterisk indicates attachment to -$D^1$- and the unmarked dashed line indicates attachment to -$D^2$-;
-$Y^2$- is of formula

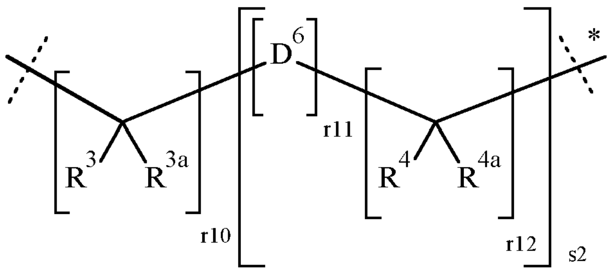

,

wherein the dashed line marked with the asterisk indicates attachment to -$D^4$- and the unmarked dashed line indicates attachment to -$D^3$-;
-$E^1$- is of formula

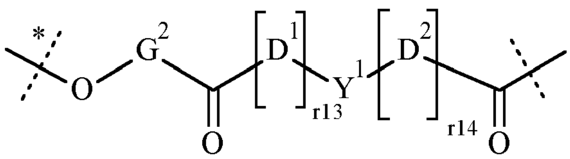

,

wherein the dashed line marked with the asterisk indicates attachment to -(C=O)- and the unmarked dashed line indicates attachment to -O-;
-$E^2$- is of formula

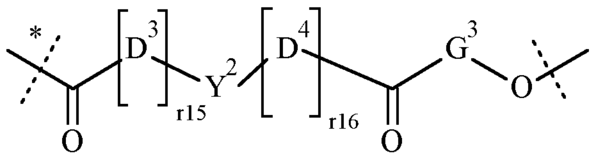

,

wherein the dashed line marked with the asterisk indicates attachment to -$G^1$- and the unmarked dashed line indicates attachment to -(C=O)-;
-$G^1$- is of formula

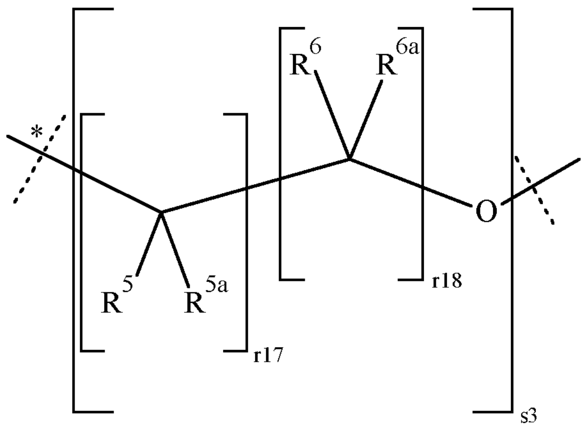

,

wherein the dashed line marked with the asterisk indicates attachment to -O- and the unmarked dashed line indicates attachment to -$E^2$-;
-$G^2$- is of formula

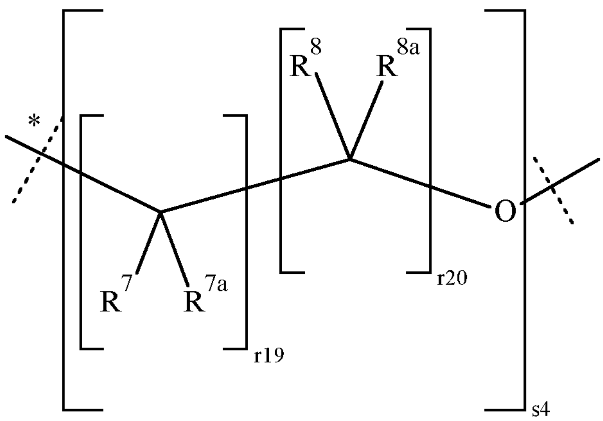

,

wherein the dashed line marked with the asterisk indicates attachment to -O- and the unmarked dashed line indicates attachment to -(C=O)-;
-$G^3$- is of formula

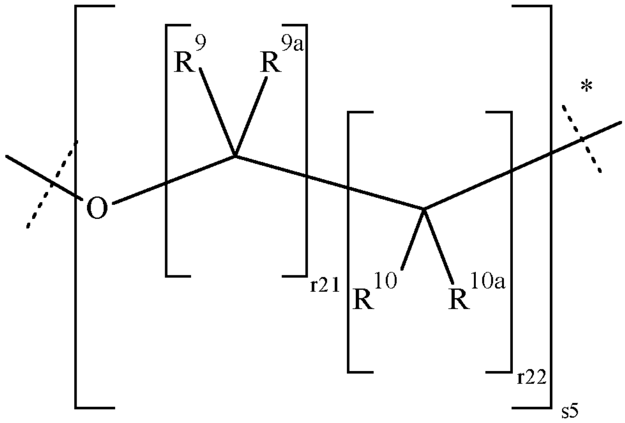
(C-vii),

wherein the dashed line marked with the asterisk indicates attachment to -O- and the unmarked dashed line indicates attachment to -(C=O)-;
$-D^1-$, $-D^2-$, $-D^3-$, $-D^4-$, $-D^5-$, $-D^6-$ and $-D^7-$ are identical or different and each is independently of the others selected from the group comprising -O-, $-NR^{11}-$, $-N^+R^{12}R^{12a}-$, -S-, -(S=O)-, $-(S(O)_2)$, -C(O)-, $-P(O)R^{13}$ and $-CR^{14}R^{14a}-$;
$-R^1$, $-R^{1a}$, $-R^2$, $-R^{2a}$, $-R^3$, $-R^{3a}$, $-R^4$, $-R^{4a}$, $-R^5$, $-R^{5a}$, $-R^6$, $-R^{6a}$, $-R^7$, $-R^{7a}$, $-R^8$, $-R^{8a}$, $-R^9$, $-R^{9a}$, $-R^{10}$, $-R^{10a}$, $-R^{11}$, $-R^{12}$, $-R^{12a}$, $-R^{13}$, $-R^{14}$ and $-R^{14a}$ are identical or different and each is independently of the others selected from the group comprising -H and $C_{1-6}$ alkyl;
optionally, one or more of the pairs $-R^1/-R^{1a}$, $-R^2/-R^{2a}$, $-R^3/-R^{3a}$, $-R^4/-R^{4a}$, $-R^1/-R^2$, $-R^3/-R^4$, $-R^{1a}/-R^{2a}$, $-R^{3a}/-R^{4a}$, $-R^{12}/-R^{12a}$, and $-R^{14}/-R^{14a}$ form a chemical bond or are joined together with the atom to which they are attached to form a $C_{3-8}$ cycloalkyl or to form a ring A or are joined together with the atom to which they are attached to form a 4- to 7-membered heterocyclyl or 8- to 11-membered heterobicyclyl or adamantyl;
A is selected from the group consisting of phenyl, naphthyl, indenyl, indanyl and tetralinyl;
r1, r2, r5, r6, r13, r14, r15 and r16 are independently 0 or 1;
r3, r4, r7, r8, r9, r10, r11, r12 are independently 0, 1, 2, 3, or 4;
r17, r18, r19, r20, r21 and r22 are independently 1, 2, 3, 4, 5, 6, 7, 8, 9 or 10; and
s1, s2, s4, s5 are independently 1, 2, 3, 4, 5 or 6.
s3 ranges from 1 to 200, preferably from 1 to 100 and more preferably from 1 to 50

In certain embodiments r1 of formula (A) is 0. In certain embodiments r1 of formula (A) is 1. In certain embodiments r2 of formula (A) is 0. In certain embodiments r2 of formula (A) is 1. In certain embodiments r5 of formula (A) is 0. In certain embodiments r5 of formula (A) is 1. In certain embodiments r6 of formula (A) is 0. In certain embodiments r6 of formula (A) is 1. In certain embodiments r13 of formula (A) is 0. In certain embodiments r13 of formula (A) is 1. In certain embodiments r14 of formula (A) is 0. In certain embodiments r14 of formula (A) is 1. In certain embodiments r15 of formula (A) is 0. In certain embodiments r15 of formula (A) is 1. In certain embodiments r16 of formula (A) is 0. In certain embodiments r16 of formula (A) is 1.

In certain embodiments r3 of formula (A) is 0. In certain embodiments r3 of formula (A) is 1. In certain embodiments r4 of formula (A) is 0. In certain embodiments r4 of formula (A) is 1. In certain embodiments r3 of formula (A) and r4 of formula (A) are both 0.

In certain embodiments r7 of formula (A) is 0. In certain embodiments r7 of formula (A) is 1. In certain embodiments r7 of formula (A) is 2. In certain embodiments r8 of formula (A) is 0. In certain embodiments r8 of formula (A) is 1. In certain embodiments r8 of formula (A) of formula (A) is 2. In certain embodiments r9 of formula (A) is 0. In certain embodiments r9 of formula (A) is 1. In certain embodiments r9 of formula (A) is 2. In certain embodiments r10 of formula (A) is 0. In certain embodiments r10 of formula (A) is 1. In certain embodiments r10 of formula (A) is 2. In certain embodiments r11 of formula (A) is 0. In certain embodiments r11 of formula (A) is 1. In certain embodiments r11 of formula (A) is 2. In certain embodiments r12 of formula (A) is 0. In certain embodiments r12 of formula (A) is 1. In certain embodiments r12 of formula (A) is 2.

In certain embodiments r17 of formula (A) is 1. In certain embodiments r18 of formula (A) is 1. In certain embodiments r19 of formula (A) is 1. In certain embodiments r20 of formula (A) is 1. In certain embodiments r21 of formula (A) is 1.

In certain embodiments s1 of formula (A) is 1. In certain embodiments s1 of formula (A) is 2. In certain embodiments s2 of formula (A) is 1. In certain embodiments s2 of formula (A) is 2. In certain embodiments s4 of formula (A) is 1. In certain embodiments s4 of formula (A) is 2.

In certain embodiments s3 of formula (A) ranges from 1 to 100. In certain embodiments s3 of formula (A) ranges

from 1 to 75. In certain embodiments s3 of formula (A) ranges from 2 to 50. In certain embodiments s3 of formula (A) ranges from 2 to 40. In certain embodiments s3 of formula (A) ranges from 3 to 30. In certain embodiments s3 of formula (A) is about 3.

In certain embodiments $-R^1$ of formula (A) is -H. In certain embodiments $-R^1$ of formula (A) is methyl. In certain embodiments $-R^1$ of formula (A) is ethyl. In certain embodiments $-R^{1a}$ of formula (A) is -H. In certain embodiments $-R^{1a}$ of formula (A) is methyl. In certain embodiments $-R^{1a}$ of formula (A) is ethyl. In certain embodiments $-R^2$ of formula (A) is -H. In certain embodiments $-R^2$ of formula (A) is methyl. In certain embodiments $-R^2$ of formula (A) is ethyl. In certain embodiments $-R^{2a}$ of formula (A) is -H. In certain embodiments $-R^{2a}$ of formula (A) is methyl. In certain embodiments $-R^{2a}$ of formula (A) is ethyl. In certain embodiments $-R^3$ of formula (A) is -H. In certain embodiments $-R^3$ of formula (A) is methyl. In certain embodiments $-R^3$ of formula (A) is ethyl. In certain embodiments $-R^{3a}$ of formula (A) is -H. In certain embodiments $-R^{3a}$ of formula (A) is methyl. In certain embodiments $-R^{3a}$ of formula (A) is ethyl. In certain embodiments $-R^4$ of formula (A) is -H. In certain embodiments $-R^4$ of formula (A) is methyl. In certain embodiments $-R^4$ of formula (A) is methyl. In certain embodiments $-R^{4a}$ of formula (A) is -H. In certain embodiments $-R^{4a}$ of formula (A) is methyl. In certain embodiments $-R^{4a}$ of formula (A) is ethyl. In certain embodiments $-R^5$ of formula (A) is -H. In certain embodiments $-R^5$ of formula (A) is methyl. In certain embodiments $-R^5$ of formula (A) is ethyl. In certain embodiments $-R^{5a}$ of formula (A) is -H. In certain embodiments $-R^{5a}$ of formula (A) is methyl. In certain embodiments $-R^{5a}$ of formula (A) is ethyl. In certain embodiments $-R^6$ of formula (A) is -H. In certain embodiments $-R^6$ of formula (A) is methyl. In certain embodiments $-R^6$ of formula (A) is ethyl. In certain embodiments $-R^{6a}$ of formula (A) is -H. In certain embodiments $-R^{6a}$ of formula (A) is methyl. In certain embodiments $-R^{6a}$ of formula (A) is ethyl. In certain embodiments $-R^7$ of formula (A) is -H. In certain embodiments $-R^7$ of formula (A) is methyl. In certain embodiments $-R^7$ of formula (A) is ethyl. In certain embodiments $-R^8$ of formula (A) is -H. In certain embodiments $-R^8$ of formula (A) is methyl. In certain embodiments $-R^8$ of formula (A) is ethyl. In certain embodiments $-R^{8a}$ of formula (A) is -H. In certain embodiments $-R^{8a}$ of formula (A) is methyl. In certain embodiments $-R^{8a}$ of formula (A) is ethyl. In certain embodiments $-R^9$ of formula (A) is -H. In certain embodiments $-R^9$ of formula (A) is methyl. In certain embodiments $-R^9$ of formula (A) is ethyl. In certain embodiments $-R^{9a}$ of formula (A) is -H. In certain embodiments $-R^{9a}$ of formula (A) is methyl. In certain embodiments $-R^{9a}$ of formula (A) is ethyl. In certain embodiments $-R^{9a}$ of formula (A) is -H. In certain embodiments $-R^{9a}$ of formula (A) is methyl. In certain embodiments $-R^{9a}$ of formula (A) is ethyl. In certain embodiments $-R^{10}$ of formula (A) is -H. In certain embodiments $-R^{10}$ of formula (A) is methyl. In certain embodiments $-R^{10}$ of formula (A) is ethyl. In certain embodiments $-R^{10a}$ of formula (A) is -H. In certain embodiments $-R^{10a}$ of formula (A) is methyl. In certain embodiments $-R^{10a}$ of formula (A) is ethyl. In certain embodiments $-R^{11}$ of formula (A) is -H. In certain embodiments $-R^{11}$ of formula (A) is methyl. In certain embodiments $-R^{11}$ of formula (A) is ethyl. In certain embodiments $-R^{12}$ of formula (A) is -H. In certain embodiments $-R^{12}$ of formula (A) is methyl. In certain embodiments $-R^{12}$ of formula (A) is ethyl. In certain embodiments $-R^{12a}$ of formula (A) is -H. In certain embodiments $-R^{12a}$ of formula (A) is methyl. In certain embodiments $-R^{12a}$ of formula (A) is ethyl. In certain embodiments $-R^{13}$ of formula (A) is -H. In certain embodiments $-R^{13}$ of formula (A) is methyl. In certain embodiments $-R^{13}$ of formula (A) is ethyl In certain embodiments $-R^{14}$ of formula (A) is -H. In certain embodiments $-R^{14}$ of formula (A) is methyl. In certain embodiments $-R^{14}$ of formula (A) is ethyl. In certain embodiments $-R^{14a}$ of formula (A) is -H. In certain embodiments $-R^{14a}$ of formula (A) is methyl. In certain embodiments $-R^{14a}$ of formula (A) is ethyl.

In certain embodiments $-D^1-$ of formula (A) is -O-. In certain embodiments $-D^1-$ of formula (A) is $-NR^{11}-$. In certain embodiments $-D^1-$ of formula (A) is $-N^+R^{12}R^{12a}-$. In certain embodiments $-D^1-$ of formula (A) is -S-. In certain embodiments $-D^1-$ of formula (A) is -(S=O). In certain embodiments $-D^1-$ of formula (A) is $-(S(O)_2)-$. In certain embodiments $-D^1-$ of formula (A) is -C(O)-. In certain embodiments $-D^1-$ of formula (A) is $-P(O)R^{13}-$. In certain embodiments $-D^1-$ of formula (A) is $-P(O)(OR^{13})-$. In certain embodiments $-D^1-$ of formula (A) is $-CR^{14}R^{14a}-$.

In certain embodiments $-D^2-$ of formula (A) is -O-. In certain embodiments $-D^2-$ of formula (A) is $-NR^{11}-$. In certain embodiments $-D^2-$ of formula (A) is $-N^+R^{12}R^{12a}-$. In certain embodiments $-D^2-$ of formula (A) is -S-. In certain embodiments $-D^2-$ of formula (A) is -(S=O). In certain embodiments $-D^2-$ of formula (A) is $-(S(O)_2)-$. In certain embodiments $-D^2-$ of formula (A) is -C(O)-. In certain embodiments $-D^2-$ of formula (A) is $-P(O)R^{13}-$. In certain embodiments $-D^2-$ of formula (A) is $-P(O)(OR^{13})-$. In certain embodiments $-D^2-$ of formula (A) is $-CR^{14}R^{14a}-$.

In certain embodiments $-D^3-$ of formula (A) is -O-. In certain embodiments $-D^3-$ of formula (A) is $-NR^{11}-$. In certain embodiments $-D^3-$ of formula (A) is $-N^+R^{12}R^{12a}-$. In certain embodiments $-D^3-$ of formula (A) is -S-. In certain embodiments $-D^3-$ of formula (A) is -(S=O). In certain embodiments $-D^3-$ of formula (A) is $-(S(O)_2)-$. In certain embodiments $-D^3-$ of formula (A) is -C(O)-. In certain embodiments $-D^3-$ of formula (A) is $-P(O)R^{13}-$. In certain embodiments $-D^3-$ of formula (A) is $-P(O)(OR^{13})-$. In certain embodiments $-D^3-$ of formula (A) is $-CR^{14}R^{14a}-$.

In certain embodiments $-D^4-$ of formula (A) is -O-. In certain embodiments $-D^4-$ of formula (A) is $-NR^{11}-$. In certain embodiments $-D^4-$ of formula (A) is $-N^+R^{12}R^{12a}-$. In certain embodiments $-D^4-$ of formula (A) is -S-. In certain embodiments $-D^4-$ of formula (A) is -(S=O). In certain embodiments $-D^4-$ of formula (A) is $-(S(O)_2)-$. In certain embodiments $-D^4-$ of formula (A) is -C(O)-. In certain embodiments $-D^4-$ of formula (A) is $-P(O)R^{13}-$. In certain embodiments $-D^4-$ of formula (A) is $-P(O)(OR^{13})-$. In certain embodiments $-D^4-$ of formula (A) is $-CR^{14}R^{14a}-$.

In certain embodiments $-D^5-$ of formula (A) is -O-. In certain embodiments $-D^5-$ of formula (A) is $-NR^{11}-$. In certain

embodiments $-D^5-$ of formula (A) is $-N^+R^{12}R^{12a}-$. In certain embodiments $-D^5-$ of formula (A) is -S-. In certain embodiments $-D^5-$ of formula (A) is -(S=O)-. In certain embodiments $-D^5-$ of formula (A) is $-(S(O)_2)-$. In certain embodiments $-D^5-$ of formula (A) is -C(O)-. In certain embodiments $-D^5-$ of formula (A) is $-P(O)R^{13}-$. In certain embodiments $-D^5-$ of formula (A) is $-P(O)(OR^{13})-$. In certain embodiments $-D^5-$ of formula (A) is $-CR^{14}R^{14a}-$.

In certain embodiments $-D^6-$ of formula (A) is -O-. In certain embodiments $-D^6-$ of formula (A) is $-NR^{11}-$. In certain embodiments $-D^6-$ of formula (A) is $-N^+R^{12}R^{12a}-$. In certain embodiments $-D^6-$ of formula (A) is -S-. In certain embodiments $-D^6-$ of formula (A) is -(S=O). In certain embodiments $-D^6-$ of formula (A) is $-(S(O)_2)-$. In certain embodiments $-D^6-$ of formula (A) is -C(O)-. In certain embodiments $-D^6-$ of formula (A) is $-P(O)R^{13}-$. In certain embodiments $-D^6-$ of formula (A) is $-P(O)(OR^{13})-$. In certain embodiments $-D^6-$ of formula (A) is $-CR^{14}R^{14a}-$.

In certain embodiments $-D^7-$ of formula (A) is -O-. In certain embodiments $-D^7-$ of formula (A) is $-NR^{11}-$. In certain embodiments $-D^7-$ of formula (A) is $-N^+R^{12}R^{12a}-$. In certain embodiments $-D^7-$ of formula (A) is -S-. In certain embodiments $-D^7-$ of formula (A) is -(S=O). In certain embodiments $-D^7-$ of formula (A) is $-(S(O)_2)-$. In certain embodiments $-D^7-$ of formula (A) is -C(O)-. In certain embodiments $-D^7-$ of formula (A) is $-P(O)R^{13}-$. In certain embodiments $-D^7-$ of formula (A) is $-P(O)(OR^{13})-$. In certain embodiments $-D^7-$ of formula (A) is $-CR^{14}R^{14a}-$.

In certain embodiments -CL- is of formula (B)

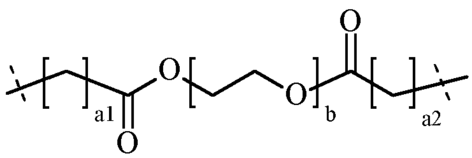

(B),

wherein

a1 and a2 are independently selected from the group consisting of a1 and a2 are independently selected from the group consisting of 1, 2, 3, 4, 5, 6, 7, 8, 9, 10, 11, 12, 13 and 14; and
b is an integer ranging from 1 to 50.

In certain embodiments a1 and a2 of formula (B) are different. In certain embodiments a1 and a2 of formula (B) are the same.

In certain embodiments a1 of formula (B) is 1. In certain embodiments a1 of formula (B) is 2. In certain embodiments a1 of formula (B) is 3. In certain embodiments a1 of formula (B) is 4. In certain embodiments a1 of formula (B) is 5. In certain embodiments a1 of formula (B) is 6. In certain embodiments a1 of formula (B) is 7. In certain embodiments a1 of formula (B) is 8. In certain embodiments a1 of formula (B) is 9. In certain embodiments a1 of formula (B) is 10.

In certain embodiments a2 of formula (B) is 1. In certain embodiments a2 of formula (B) is 2. In certain embodiments a2 of formula (B) is 3. In certain embodiments a2 of formula (B) is 4. In certain embodiments a2 of formula (B) is 5. In certain embodiments a2 of formula (B) is 6. In certain embodiments a2 of formula (B) is 7. In certain embodiments a2 of formula (B) is 8. In certain embodiments a2 of formula (B) is 9. In certain embodiments a2 of formula (B) is 10.

In certain embodiments b of formula (B) ranges from 1 to 500. In certain embodiments b of formula (B) ranges from 2 to 250. In certain embodiments b of formula (B) ranges from 3 to 100. In certain embodiments b of formula (B) ranges from 3 to 50. In certain embodiments b of formula (B) ranges from 3 to 25. In certain embodiments b of formula (B) is 3. In certain embodiments b of formula (B) is 25.

In certain embodiments -CL- is of formula (B-i)

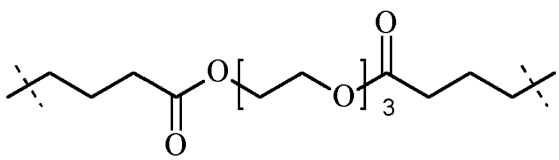

(B-i).

In certain embodiments -CL- is of formula (C)

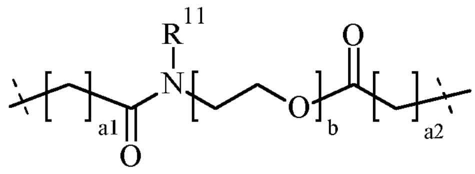

(C),

wherein

a1 and a2 are independently selected from the group consisting of 1, 2, 3, 4, 5, 6, 7, 8, 9, 10, 11, 12, 13 and 14;
b is an integer ranging from 1 to 50; and
$-R^{11}$ is selected from the group comprising -H and $C_{1-6}$ alkyl.

In certain embodiments a1 and a2 of formula (C) are different. In certain embodiments a1 and a2 of formula (B) are the same.

In certain embodiments a1 of formula (C) is 1. In certain embodiments a1 of formula (C) is 2. In certain embodiments a1 of formula (C) is 3. In certain embodiments a1 of formula (C) is 4. In certain embodiments a1 of formula (C) is 5. In certain embodiments a1 of formula (C) is 6. In certain embodiments a1 of formula (C) is 7. In certain embodiments a1 of formula (C) is 8. In certain embodiments a1 of formula (C) is 9. In certain embodiments a1 of formula (C) is 10.

In certain embodiments a2 of formula (C) is 1. In certain embodiments a2 of formula (C) is 2. In certain embodiments a2 of formula (C) is 3. In certain embodiments a2 of formula (C) is 4. In certain embodiments a2 of formula (C) is 5. In certain embodiments a2 of formula (C) is 6. In certain embodiments a2 of formula (C) is 7. In certain embodiments a2 of formula (C) is 8. In certain embodiments a2 of formula (C) is 9. In certain embodiments a2 of formula (C) is 10. In certain embodiments b of formula (C) ranges from 1 to 500. In certain embodiments b of formula (C) ranges from 2 to 250. In certain embodiments b of formula (C) ranges from 3 to 100. In certain embodiments b of formula (C) ranges from 3 to 50. In certain embodiments b of formula (C) ranges from 3 to 25. In certain embodiments b of formula (C) is 3. In certain embodiments b of formula (C) is 25.

In certain embodiments $-R^{11}$ of formula (C) is -H. In certain embodiments $-R^{11}$ of formula (C) is methyl. In certain embodiments $-R^{11}$ of formula (C) is ethyl. In certain embodiments $-R^{11}$ of formula (C) is n-propyl. In certain embodiments $-R^{11}$ of formula (C) is isopropyl. In certain embodiments $-R^{11}$ of formula (C) is n-butyl. In certain embodiments $-R^{11}$ of formula (C) is isobutyl. In certain embodiments $-R^{11}$ of formula (C) is sec-butyl. In certain embodiments $-R^{11}$ of formula (C) is tert-butyl. In certain embodiments $-R^{11}$ of formula (C) is n-pentyl. In certain embodiments $-R^{11}$ of formula (C) is 2-methylbutyl. In certain embodiments $-R^{11}$ of formula (C) is 2,2-dimethylpropyl. In certain embodiments $-R^{11}$ of formula (C) is n-hexyl. In certain embodiments $-R^{11}$ of formula (C) is 2-methylpentyl. In certain embodiments $-R^{11}$ of formula (C) is 3-methylpentyl. In certain embodiments $-R^{11}$ of formula (C) is 2,2-dimethylbutyl. In certain embodiments $-R^{11}$ of formula (C) is 2,3-dimethylbutyl. In certain embodiments $-R^{11}$ of formula (C) is 3,3-dimethylpropyl.

In certain embodiments -CL- is of formula (C-i)

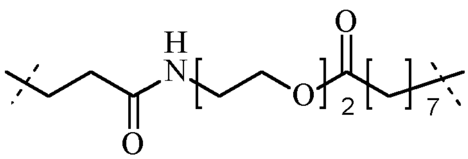

(C-i).

In a second embodiment the moiety -CL- is selected from the group consisting of wherein

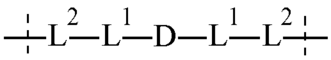 (C-i), 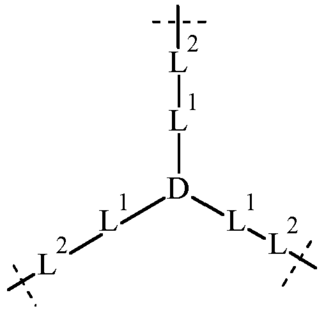 (C-ii),

each dashed line indicates attachment to a unit $Z^3$; and
$-L^1-$, $-L^2-$ and -D are used as defined for $Z^2$.

It is understood that in formula (C-i) two functional groups of the drug are conjugated to one moiety $-L^1-$ each and that in formula (C-ii) three functional groups of the drug are conjugated to one moiety $-L^1-$ each. The moiety -CL- of formula (C-i) connects two moieties $Z^3$ and the moiety -CL- of formula (C-ii) connects three moieties $Z^3$, which may be on the same or different hyaluronic acid strand. In this embodiment -CL- comprises at least two degradable bonds, if -CL- is of formula (C-i) or at least three degradable bonds, if -CL- is of formula (C-ii), namely the degradable bonds that connect D with a moiety $-L^1-$. A conjugate may only comprise moieties -CL- of formula (C-i), may only comprise moieties -CL- of formula (C-ii) or may comprise moieties -CL- of formula (C-i) and formula (C-ii).

Accordingly, a conjugate of this second embodiment comprises crosslinked hyaluronic acid strands to which a plurality of drug moieties are covalently and reversibly conjugated, wherein the conjugate comprises a plurality of connected units selected from the group consisting of

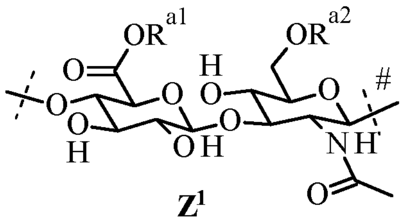

$Z^1$ ,

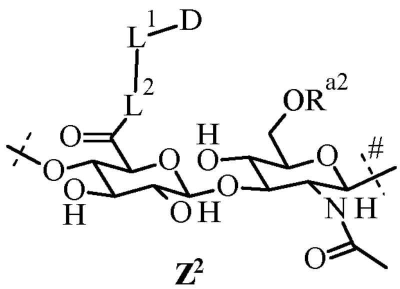

$Z^2$

and

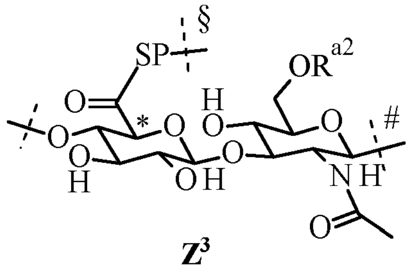

$Z^3$ ,

wherein

an unmarked dashed line indicates a point of attachment to an adjacent unit at a dashed line marked with # or to a hydrogen;
a dashed line marked with # indicates a point of attachment to an adjacent unit at an unmarked dashed line or to a hydroxyl;
a dashed line marked with § indicates a point of connection between at least two units $Z^3$ via a moiety -CL-;
each -CL- comprises at least one degradable bond between the two carbon atoms marked with the * connected by a moiety -CL- and each -CL- is independently selected from the group consisting of formula (C-i) and (C-ii)

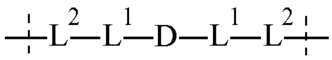 (C-i), 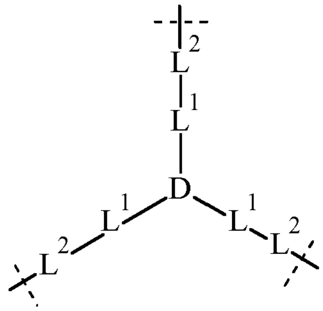 (C-ii),

wherein
dashed lines indicate attachment to a unit $Z^3$;
-D, $-L^1-$, $-L^2-$ , -SP-, $-R^{a1}$ and $-R^{a2}$ are used as defined for $Z^1$, $Z^2$ and $Z^3$;

wherein

all units $Z^1$ present in the conjugate may be the same or different;
all units $Z^2$ present in the conjugate may be the same or different;
all units $Z^3$ present in the conjugate may be the same or different;
the number of $Z^1$ units ranges from 1% to 98% of the total number of units present in the conjugate;
the number of $Z^2$ units ranges from 0% to 98% of the total number of units present in the conjugate;
the number of $Z^3$ units ranges from 1% to 97% of the total number of units present in the conjugate, provided that at least one unit $Z^3$ is present per strand which is connected to at least one unit $Z^3$ on a different hyaluronic acid strand.

It is understood that such hydrogel according to the second embodiment also comprises partly reacted or unreacted units and that the presence of such moieties cannot be avoided. In certain embodiments the sum of such partly reacted or unreacted units is no more than 25% of the total number of units present in the conjugate, such as no more than 10%, such as no more than 15% or such as no more than 10%.

In a conjugate according to this second embodiment the number of units $Z^2$ ranges from 0 to 70% of all units present in the conjugate, such as from 2 to 15%, from 2 to 10%, from 16 to 39, from 40 to 65%, or from 50 to 60% of all units present in the conjugate.

In a conjugate according to this second embodiment the number of units $Z^3$ ranges from 1 to 30% of all units present in the conjugate, such as from 2 to 5%, from 5 to 20%, from 10 to 18%, or from 14 to 18% of all units present in the conjugate.

In a conjugate according to this second embodiment the number of units $Z^1$ ranges from 10 to 97% of all units present in the conjugate, such as from 20 to 40%, such as from 25 to 35%, such as from 41 to 95%, such as from 45 to 90%, such as from 50 to 70% of all units present in the conjugate.

More specific embodiments for -D, $-L^1-$, $-L^2-$, -SP-, $-R^{a1}$ and $-R^{a2}$ of the second embodiment are as described elsewhere herein.

In a third embodiment the moiety -CL- is a moiety

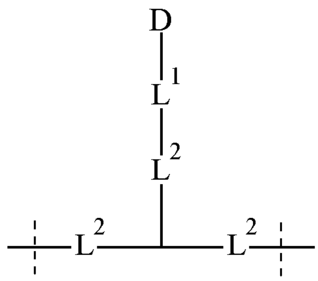 (D-i),

wherein
each dashed line indicates attachment to a unit $Z^3$.

It is understood that a moiety -CL- of formula (D-i) comprises at least one branching point, which branching point may be selected from the group consisting of

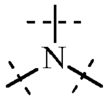, 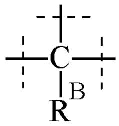 and 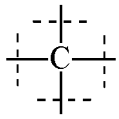,

wherein

dashed lines indicate attachment to an arm; and
$-R^B$ is selected from the group consisting of -H, $C_{1-6}$ alkyl, $C_{2-6}$ alkenyl and $C_{2-6}$ alkynyl; wherein $C_{1-6}$ alkyl, $C_{2-6}$ alkenyl and $C_{2-6}$ alkynyl are optionally substituted with one or more $-R^{B1}$, which are the same or different, and wherein $C_{1-6}$ alkyl, $C_{2-6}$ alkenyl and $C_{2-6}$ alkynyl are optionally interrupted with -C(O)O-, -O-, -C(O)-, $-C(O)N(R^{B2})-$, $-S(O)_2N(R^{B2})-$, $-S(O)N(R^{B2})-$, $-S(O)_2-$, -S(O)-, $-N(R^{B2})S(O)_2N(R^{B2a})-$, -S-, $-N(R^{B2})-$, $-OC(OR^{B2})(R^{B2a})-$, $-N(R^{B2})C(O)N(R^{B2a})-$, and $-OC(O)N(R^{B2})-$; wherein $-R^{B1}$, $-R^{B2}$ and $-R^{B2a}$ are selected from -H, $C_{1-6}$ alkyl, $C_{2-6}$ alkenyl and $C_{2-6}$ alkynyl.

In certain embodiments $-R^B$ is selected from the group consisting of -H, methyl and ethyl.

Accordingly, a conjugate of the third embodiment comprises crosslinked hyaluronic acid strands to which a plurality of drug moieties are covalently and reversibly conjugated, wherein the conjugate comprises a plurality of connected units selected from the group consisting of

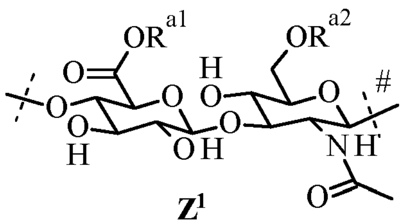

$\mathbf{Z^1}$,

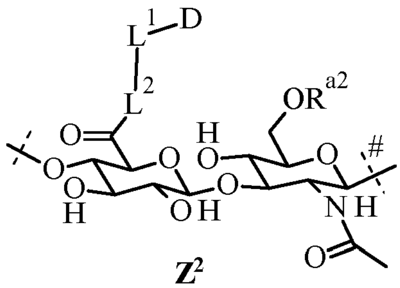

$\mathbf{Z^2}$

and

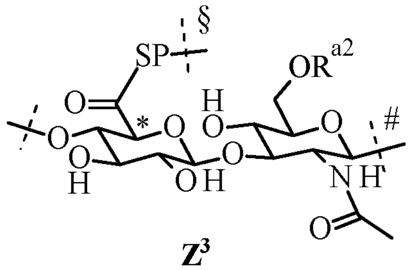

$\mathbf{Z^3}$,

wherein

an unmarked dashed line indicates a point of attachment to an adjacent unit at a dashed line marked with # or to a hydrogen;
a dashed line marked with # indicates a point of attachment to an adjacent unit at an unmarked dashed line or to a hydroxyl;
a dashed line marked with § indicates a point of connection between two units $Z^3$ via a moiety -CL-;
each -CL- comprises at least one degradable bond between the two carbon atoms marked with the * connected by a moiety -CL- and each -CL- is independently of formula (D-i)

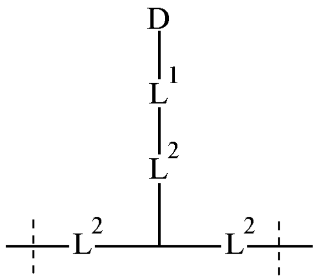

(D-i),

wherein
dashed lines indicate attachment to a unit $Z^3$;
-D, $-L^1-$, $-L^2-$ , -SP-, $-R^{a1}$ and $-R^{a2}$ are used as defined for $Z^1$, $Z^2$ and $Z^3$;

wherein

all units $Z^1$ present in the conjugate may be the same or different;
all units $Z^2$ present in the conjugate may be the same or different;
all units $Z^3$ present in the conjugate may be the same or different;
the number of units $Z^1$ ranges from 1% to 99% of the total number of units present in the conjugate;
the number of units $Z^2$ ranges from 0% to 98% of the total number of units present in the conjugate; and
the number of units $Z^3$ ranges from 1% to 97% of the total number of units present in the conjugate, provided that at least one unit $Z^3$ is present per strand.

It is understood that such hydrogel according to the third embodiment also comprises partly reacted or unreacted units and that the presence of such moieties cannot be avoided. In certain embodiments the sum of such partly reacted or unreacted units is no more than 25% of the total number of units present in the conjugate, such as no more than 10%, such as no more than 15% or such as no more than 10%.
In a conjugate according to this third embodiment the number of units $Z^2$ ranges from 0 to 70% of all units present in the conjugate, such as from 2 to 15%, from 2 to 10%, from 16 to 39, from 40 to 65%, or from 50 to 60% of all units present in the conjugate.
In a conjugate according to this third embodiment the number of units $Z^3$ ranges from 1 to 30% of all units present in the conjugate, such as from 2 to 5%, from 5 to 20%, from 10 to 18%, or from 14 to 18% of all units present in the conjugate.
In a conjugate according to this third embodiment the number of units $Z^1$ ranges from 10 to 97% of all units present in the conjugate, such as from 20 to 40%, such as from 25 to 35%, such as from 41 to 95%, such as from 45 to 90%, such as from 50 to 70% of all units present in the conjugate.
In this third embodiment -CL- comprises a moiety $-L^2- L^1-D$, so the presence of units $Z^2$ is optional in this embodiment. In certain embodiment no units $Z^2$ are present in the third embodiment. In certain embodiments the conjugate according to the third embodiment also comprises units $Z^2$. The presence of units $Z^2$ may have the effect that in case of a high drug loading is desired, which in this embodiment also means a high degree of crosslinking, an undesired high degree of crosslinking can be avoided by the presence of units $Z^2$.
More specific embodiments for -D, $-L^1-$, $-L^2-$, -SP-, $-R^{a1}$ and $-R^{a2}$ of the second embodiment are as described elsewhere herein.
-SP- is absent or a spacer moiety. In certain embodiments -SP- does not comprise a reversible linkage, i.e. all linkages in -SP- are stable linkages.
In certain embodiments -SP- is absent.
In certain embodiments -SP- is a spacer moiety.
In certain embodiments -SP- does not comprise a degradable bond, i.e. all bonds of -SP- are stable bonds. In certain embodiments at least one of the at least one degradable bond in the direct connection between two carbon atoms marked with the * connected by a moiety -CL- is provided by -SP-.
In certain embodiments -SP- is a spacer moiety selected from the group consisting of -T-, $C_{1-50}$ alkyl, $C_{2-50}$ alkenyl, and $C_{2-50}$ alkynyl; wherein -T-, $C_{1-50}$ alkyl, $C_{2-50}$ alkenyl, and $C_{2-50}$ alkynyl are optionally substituted with one or more $-R^{y2}$, which are the same or different and wherein $C_{1-50}$ alkyl, $C_{2-50}$ alkenyl, and $C_{2-50}$ alkynyl are optionally interrupted by one or more groups selected from the group consisting of -T-, -C(O)O-, -O-, -C(O)-, $-C(O)N(R^{y3})-$, $-S(O)_2N(R^{y3})-$, $-S(O)N(R^{y3})-$, $-S(O)_2-$, -S(O)-, $-N(R^{y3})S(O)_2N(R^{y3a})-$, -S-, $-N(R^{y3})-$, $-OC(OR^{y3})(R^{y3a})-$, $-N(R^{y3})C(O)N(R^{y3a})-$, and $-OC(O)N(R^{y3})-$;

$-R^{y1}$ and $-R^{y1a}$ are independently of each other selected from the group consisting of -H, -T, $C_{1-50}$ alkyl, $C_{2-50}$ alkenyl,

and $C_{2-50}$ alkynyl; wherein -T, $C_{1-50}$ alkyl, $C_{2-50}$ alkenyl, and $C_{2-50}$ alkynyl are optionally substituted with one or more $-R^{y2}$, which are the same or different, and wherein $C_{1-50}$ alkyl, $C_{2-50}$ alkenyl, and $C_{2-50}$ alkynyl are optionally interrupted by one or more groups selected from the group consisting of -T-, -C(O)O-, -O-, -C(O)-, $-C(O)N(R^{y4})-$, $-S(O)_2N(R^{y4})-$, $-S(O)N(R^{y4})-$, $-S(O)_2-$, -S(O)-, $-N(R^{y4})S(O)_2N(R^{y4a})-$, -S-, $-N(R^{y4})-$, $-OC(OR^{y4})(R^{y4a})-$, $-N(R^{y4})C(O)N(R^{y4a})-$, and $-OC(O)N(R^{y4})-$;

each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more $-R^{y2}$, which are the same or different;

each $-R^{y2}$ is independently selected from the group consisting of halogen, -CN, oxo (=O), $-COOR^{y5}$, $-OR^{y5}$, $-C(O)R^{y5}$, $-C(O)N(R^{y5}R^{y5a})$, $-S(O)_2N(R^{y5}R^{y5a})$, $-S(O)N(R^{y5}R^{y5a})$, $-S(O)_2R^{y5}$, $-S(O)R^{y5}$, $-N(R^{y5})S(O)_2N(R^{y5a}R^{y5b})$, $-SR^{y5}$, $-N(R^{y5}R^{y5a})$, $-NO_2$, $-OC(O)R^{y5}$, $-N(R^{y5})C(O)R^{y5a}$, $-N(R^{y5})S(O)_2R^{y5a}$, $-N(R^{y5})S(O)R^{y5a}$, $-N(R^{y5})C(O)OR^{y5a}$, $-N(R^{y5})C(O)N(R^{y5a}R^{y5b})$, $-OC(O)N(R^{y5}R^{y5a})$, and $C_{1-6}$ alkyl; wherein $C_{1-6}$ alkyl is optionally substituted with one or more halogen, which are the same or different; and

each $-R^{y3}$, $-R^{y3a}$, $-R^{y4}$, $-R^{y4a}$, $-R^{y5}$, $-R^{y5a}$ and $-R^{y5b}$ is independently selected from the group consisting of -H, and $C_{1-6}$ alkyl, wherein $C_{1-6}$ alkyl is optionally substituted with one or more halogen, which are the same or different.

In certain embodiments -SP- is a spacer moiety selected from the group consisting of -T-, $C_{1-50}$ alkyl, $C_{2-50}$ alkenyl, and $C_{2-50}$ alkynyl; wherein -T-, $C_{1-20}$ alkyl, $C_{2-20}$ alkenyl, and $C_{2-20}$ alkynyl are optionally substituted with one or more $-R^{y2}$, which are the same or different and wherein $C_{1-20}$ alkyl, $C_{2-20}$ alkenyl, and $C_{2-20}$ alkynyl are optionally interrupted by one or more groups selected from the group consisting of -T-, -C(O)O-, -O-, -C(O)-, $-C(O)N(R^{y3})-$, $-S(O)_2N(R^{y3})-$, $-S(O)N(R^{y3})-$, $-S(O)_2-$, -S(O)-, $-N(R^{y3})S(O)_2N(R^{y3a})-$, -S-, $-N(R^{y3})-$, $-OC(OR^{y3})(R^{y3a})-$, $-N(R^{y3})C(O)N(R^{y3a})-$, and $-OC(O)N(R^{y3})-$;

$-R^{y1}$ and $-R^{y1a}$ are independently of each other selected from the group consisting of -H, -T, $C_{1-10}$ alkyl, $C_{2-10}$ alkenyl, and $C_{2-10}$ alkynyl; wherein -T, $C_{1-10}$ alkyl, $C_{2-10}$ alkenyl, and $C_{2-10}$ alkynyl are optionally substituted with one or more $-R^{y2}$, which are the same or different, and wherein $C_{1-10}$ alkyl, $C_{2-10}$ alkenyl, and $C_{2-10}$ alkynyl are optionally interrupted by one or more groups selected from the group consisting of -T-, -C(O)O-, -O-, -C(O)-, $-C(O)N(R^{y4})-$, $-S(O)_2N(R^{y4})-$, $-S(O)N(R^{y4})-$, $-S(O)_2-$, -S(O)-, $-N(R^{y4})S(O)_2N(R^{y4a})-$, -S-, $-N(R^{y4})-$, $-OC(OR^{y4})(R^{y4a})-$, $-N(R^{y4})C(O)N(R^{y4a})-$, and $-OC(O)N(R^{y4})-$;

each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more $-R^{y2}$, which are the same or different;

$-R^{y2}$ is selected from the group consisting of halogen, -CN, oxo (=O), $-COOR^{y5}$, $-OR^{y5}$, $-C(O)R^{y5}$, $-C(O)N(R^{y5}R^{y5a})$, $-S(O)_2N(R^{y5}R^{y5a})$, $-S(O)N(R^{y5}R^{y5a})$, $-S(O)_2R^{y5}$, $-S(O)R^{y5}$, $-N(R^{y5})S(O)_2N(R^{y5a}R^{y5b})$, $-SR^{y5}$, $-N(R^{y5}R^{y5a})$, $-NO_2$, $-OC(O)R^{y5}$, $-N(R^{y5})C(O)R^{y5a}$, $-N(R^{y5})S(O)_2R^{y5a}$, $-N(R^{y5})S(O)R^{y5a}$, $-N(R^{y5})C(O)OR^{y5a}$, $-N(R^{y5})C(O)N(R^{y5a} R^{y5b})$, $-OC(O)N(R^{y5}R^{y5a})$, and $C_{1-6}$ alkyl; wherein $C_{1-6}$ alkyl is optionally substituted with one or more halogen, which are the same or different; and

each $-R^{y3}$, $-R^{y3a}$, $-R^{y4}$, $-R^{y4a}$, $-R^{y5}$, $-R^{y5a}$ and $-R^{y5b}$ is independently of each other selected from the group consisting of -H, and $C_{1-6}$ alkyl; wherein $C_{1-6}$ alkyl is optionally substituted with one or more halogen, which are the same or different.

In certain embodiments -SP- is a spacer moiety selected from the group consisting of -T-, $C_{1-50}$ alkyl, $C_{2-50}$ alkenyl, and $C_{2-50}$ alkynyl; wherein -T-, $C_{1-50}$ alkyl, $C_{2-50}$ alkenyl, and $C_{2-50}$ alkynyl are optionally substituted with one or more $-R^{y2}$, which are the same or different and wherein $C_{1-50}$ alkyl, $C_{2-50}$ alkenyl, and $C_{2-50}$ alkynyl are optionally interrupted by one or more groups selected from the group consisting of -T-, -C(O)O-, -O-, -C(O)-, $-C(O)N(R^{y3})-$, $-S(O)_2N(R^{y3})-$, $-S(O)N(R^{y3})-$, $-S(O)_2-$, -S(O)-, $-N(R^{y3})S(O)_2N(R^{y3a})-$, -S-, $-N(R^{y3})-$, $-OC(OR^{y3})(R^{y3a})-$, $-N(R^{y3})C(O)N(R^{y3a})-$, and $-OC(O)N(R^{y3})-$;

$-R^{y1}$ and $-R^{y1a}$ are independently selected from the group consisting of -H, -T, $C_{1-10}$ alkyl, $C_{2-10}$ alkenyl, and $C_{2-10}$ alkynyl;

each T is independently selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl;

each $-R^{y2}$ is independently selected from the group consisting of halogen and $C_{1-6}$ alkyl; and

each $-R^{y3}$, $-R^{y3a}$, $-R^{y4}$, $-R^{y4a}$, $-R^{y5}$, $-R^{y5a}$ and $-R^{y5b}$ is independently of each other selected from the group consisting of -H, and $C_{1-6}$ alkyl; wherein $C_{1-6}$ alkyl is optionally substituted with one or more halogen, which are the same or different.

In certain embodiments -SP- is a $C_{1-20}$ alkyl chain, which is optionally interrupted by one or more groups independently selected from -O-, -T-, $-N(R^{y3})-$ and $-C(O)N(R^{y1})-$; and which $C_{1-20}$ alkyl chain is optionally substituted with one or more groups independently selected from -OH, -T, $-N(R^{y3})-$ and $-C(O)N(R^{y6}R^{y6a})$; wherein $-R^{y1}$, $-R^{y6}$, $-R^{y6a}$ are independently selected from the group consisting of H and $C_{1-4}$ alkyl, wherein T is selected from the group consisting of phenyl, naphthyl, indenyl, indanyl, tetralinyl, $C_{3-10}$ cycloalkyl, 3- to 10-membered heterocyclyl, 8- to 11-membered heterobicyclyl, 8-to 30-membered carbopolycyclyl, and 8- to 30-membered heteropolycyclyl, provided that -SP- is attached to $-X^{0E}-$ and $-X^{0F}-$ via a carbon atom of -SP-.

In certain embodiments -SP- has a molecular weight ranging from 14 g/mol to 750 g/mol.

In certain embodiments -SP- has a chain length ranging from 1 to 20 atoms.

In certain embodiments all moieties -SP- of a conjugate are identical.

In certain embodiments -SP- is a $C_{1-10}$ alkyl. In certain embodiments -SP- is a $C_1$ alkyl. In certain embodiments -SP- is a $C_2$ alkyl. In certain embodiments -SP- is a $C_3$ alkyl. In certain embodiments -SP- is a $C_4$ alkyl. In certain embodiments -SP- is a $C_5$ alkyl. In certain embodiments -SP- is a $C_6$ alkyl. In certain embodiments -SP- is a $C_7$ alkyl. In certain embodiments -SP- is a $C_8$ alkyl. In certain embodiments -SP- is a $C_9$ alkyl. In certain embodiments -SP- is a $C_{10}$ alkyl.

In certain embodiments at least 25% of the amount of PRRA in the form of the water-insoluble controlled-release PRRA administered via intra-tumoral administration remains local in the tumor 3 days after administration.

The term "amount of PRRA administered" in this context refers to the total combined amount of both free PRRA that was released from the conjugate and the PRRA still covalently conjugated in the conjugate.

As used herein the term "local" refers to an area restricted to the injected tissue or organ, specifically the total volume around the site of administration of the conjugate within 3 times the radius (r) from the injection site in any direction, wherein r is the distance in centimeters (cm) calculated from the volume (*V*) of conjugate injected in cubic centimeters ($cm^3$) following the spheroid equation $V = \left(\frac{4}{3}\right) \times \pi r^3$. For example, if 0.5 $cm^3$ of conjugate is injected into a given tissue, a sample aiming to capture the total injected material containing the total volume within 1.47 cm in any direction of, and including, the injection site would be measured for drug levels, i.e. the amount of PRRA present.

Suitable measurements are known to the person skilled in the art. In order to obtain the total amount of both free PRRA that was released from the conjugate and to measure PRRA still covalently conjugated, the PRRA still first needs to be released. This may be done by using suitable procedures, such as incubation at release-accelerating conditions, such as increased temperatures or changes in pH. In order to separately measure the free and conjugated PRRA in tissue, the tissue may be first weighed and then dissociated in a fashion that does not disrupt the conjugated PRRA and allows for separation of the free PRRA from the conjugate PRRA for measurement and then, separately, the PRRA may be released from the conjugate and measured.

At least 25% of the total amount of PRRA administered remains in such tissue after 3 days, such as at least 30%, at least 35%, at least 40% or at least 45%. It is understood that the total amount of PRRA present in the tissue after 3 days does not exceed 100%. In certain embodiments at least 25% of the total amount of PRRA administered remains in such tissue after 7 days, such as at least 30%, at least 35%, at least 40% or at least 45%. In certain embodiments at least 25% of the total amount of PRRA administered remains in such tissue after 10 days, such as at least 30%, at least 35%, at least 40% or at least 45%. In certain embodiments at least 25% of the total amount of PRRA administered remains in such tissue after 14 days, such as at least 30%, at least 35%, at least 40% or at least 45%. In certain embodiments at least 25% of the total amount of PRRA administered remains in such tissue after 21 days, such as at least 30%, at least 35%, at least 40% or at least 45%. In certain embodiments at least 25% of the total amount of PRRA administered remains in such tissue after 28 days, such as at least 30%, at least 35%, at least 40% or at least 45%. In certain embodiments at least 25% of the total amount of PRRA administered remains in such tissue after 35 days, such as at least 30%, at least 35%, at least 40% or at least 45%. In certain embodiments at least 25% of the total amount of PRRA administered remains in such tissue after 42 days, such as at least 30%, at least 35%, at least 40% or at least 45%. In certain embodiments at least 25% of the total amount of PRRA administered remains in such tissue after 49 days, such as at least 30%, at least 35%, at least 40% or at

least 45%. In certain embodiments at least 25% of the total amount of PRRA administered remains in such tissue after 56 days, such as at least 30%, at least 35%, at least 40% or at least 45%.

In certain embodiments the one or more additional drug is IL-2. In certain embodiments said IL-2 is administered systemically. It is understood that such IL-2 drug may be administered in the form of free or unmodified IL-2 or as a controlled-release form of IL-2. In certain embodiments such IL-2 drug is administered in the form of free or unmodified IL-2. In certain embodiments such IL-2 drug is administered as a controlled-release form of IL-2. Embodiments for such IL-2 are as described elsewhere herein.

In certain embodiments intra-tumoral administration of the water-insoluble controlled-release PRRA and systemic administration of IL-2 induces a more than 1.5-fold, such as more than 2-fold, 3-fold, 4-fold or 5-fold, increase in the percent of antigen-presenting cell subsets in tumor-draining lymph nodes 7 days following said administration compared to intra-tumoral administration of an equimolar amount of the same water-insoluble controlled-release PRRA alone or of an equimolar amount of the same IL-2 alone. It is understood that the IL-2 may be in the form of free or unmodified IL-2 or as a controlled-release form of IL-2. Administration of the PRRA and the IL-2 may occur simultaneously or consecutive with either one given first, followed by administration of the second one.

In certain embodiments the one or more additional drug is a TKI. In certain embodiments said TKI is administered systemically. It is understood that such TKI drug may be administered in the form of free or unmodified TKI or as a controlled-release form of TKI. In certain embodiments such TKI drug is administered in the form of free or unmodified TKI. In certain embodiments such TKI drug is administered as a controlled-release form of TKI. Embodiments for such TKI are as described elsewhere herein.

In certain embodiments the one or more additional drug is an inhibitor of CTLA-4. In certain embodiments said inhibitor of CTLA-4 is administered systemically. It is understood that such inhibitor of CTLA-4 drug may be administered in the form of free or unmodified inhibitor of CTLA-4 or as a controlled-release form of inhibitor of CTLA-4. In certain embodiments such inhibitor of CTLA-4 drug is administered in the form of free or unmodified inhibitor of CTLA-4. In certain embodiments such inhibitor of CTLA-4 drug is administered as a controlled-release form of inhibitor of CTLA-4. Embodiments for such inhibitor of CTLA-4 are as described elsewhere herein.

In certain embodiments intra-tumoral administration of the water-insoluble controlled-release PRRA and systemic administration of IL-2 induces a more than 1.5-fold, such as more than 1.8-fold, 2-fold, 2.5-fold, 2.8-fold, 3-fold, 3.5-fold, 4-fold, 4.5-fold or 5-fold, increase in the percent of CD8 T cells in tumor-draining lymph nodes 7 days following said administration compared to intra-tumoral administration of an equimolar amount of the same water-insoluble controlled-release PRRA alone. It is understood that the IL-2 may be in the form of free or unmodified IL-2 or as a controlled-release form of IL-2. Administration of the PRRA and the IL-2 may occur simultaneously or consecutive with either one given first, followed by administration of the second one.

In certain embodiments intra-tumoral administration of the water-insoluble controlled-release PRRA of the present invention and systemic administration of IL-2 induces a more than 1.5-fold, such as more than 1.5-fold, 1.7-fold, 2-fold, 2.2-fold, 2.5-fold, 3-fold, 3.5-fold, 4-fold or 5-fold, increase in the percent of CD8 T cells in the peripheral blood 4 days following said administration compared to either treatment with vehicle alone or compared to with treatment with intra-tumoral administration of the water-insoluble controlled-release PRRA alone. It is understood that the IL-2 may be in the form of free or unmodified IL-2 or as a controlled-release form of IL-2, as described elsewhere herein. Administration of the water-insoluble controlled-release PRRA of the present invention and the IL-2 may occur simultaneously or consecutive with either one given first, followed by administration of the second one.

In certain embodiments intra-tumoral administration of the water-insoluble controlled-release PRRA and systemic administration of IL-2 induces a more than 1.25-fold, such as more than 1.5-fold, 1.7-fold, 2-fold, 2.2-fold, 2.5-fold, 3-fold, 3.5-fold, 4-fold or 5-fold, increase in the expression of markers of memory in CD8 T cells in tumor-draining lymph nodes 7 days following said administration compared to intra-tumoral administration of an equimolar amount of the same water-insoluble controlled-release PRRA alone. It is understood that the IL-2 may be in the form of free or unmodified IL-2 or as a controlled-release form of IL-2. Administration of the PRRA and the IL-2 may occur simultaneously or consecutive with either one given first, followed by administration of the second one.

## Materials and methods

### Chemicals

All materials were obtained from commercial vendors except where stated otherwise.

### RP-HPLC purification:

Preparative RP-HPLC purifications were performed with a Waters 600 controller with a 2487 Dual Absorbance Detector or an Agilent Infinity 1260 preparative system using a Waters XBridge BEH300 Prep C18 10 μm, 150 x 30 mm

column as stationary phase. Products were detected at 215 nm or 320 nm.

**Flash Chromatography:**

Flash chromatography purifications were performed on an Isolera One system or an Isolera Four system from Biotage AB, Sweden, using Biotage KP-Sil silica cartridges. Products were detected at 215 nm, 254 nm or 280 nm.

**RP-LPLC purification:**

Low pressure RP chromatography purifications were performed on an Isolera One system or an Isolera Four system from Biotage AB, Sweden, using Biotage SNAP C18 cartridges. Products were detected at 215 nm.

**Analytical methods**

**Analytical UPLC-MS analysis:**

Analytical ultra-performance LC (UPLC)-MS was performed on a Waters Acquity system or an Agilent 1290 Infinity II equipped with a Waters BEH300 C18 column (2.1 x 50 mm, 1.7 μm particle size or 2.1 x 100 mm, 1.7 μm particle size); solvent A: water containing 0.05% TFA (v/v), solvent B: acetonitrile containing 0.04% TFA (v/v) coupled to a Waters Micromass ZQ or coupled to an Agilent Single Quad MS system.

**Amine content determination on the PEG-hydrogel beads:**

Amino group content of the PEG-hydrogel was determined by conjugation of an Fmoc-amino acid to the free amino groups on the hydrogel and subsequent Fmoc-determination as described by Gude, M., J. Ryf, et al. (2002) Letters in Peptide Science 9(4): 203-206.

**Content determination of conjugated Resiquimod in hydrogel suspensions**

The Resiquimod content of a hydrogel suspension was determined by incubating a sample of the hydrogel suspension with an equal volume of 1M NaOH at 37 °C for 16-20 h. After pH adjustment with 1M HCl, the Resiquimod content was determined by HPLC (detection at 320 nm) against a calibration curve obtained from at least 4 different calibration standards.

**Example 1: Synthesis of linker reagent 6**

**Step 1:**

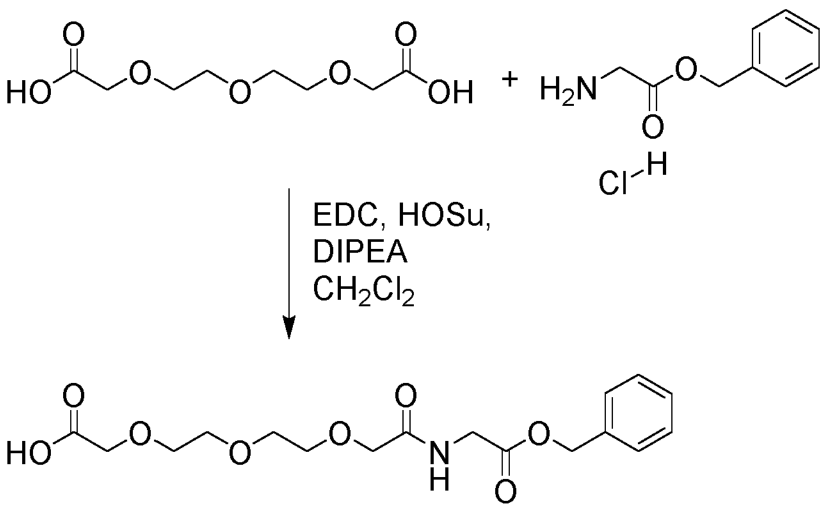


**1**

In a 250 mL round bottom flask, 3,6,9-trioxaundecanedioic acid (9.45 g; 29.79 mmol; 10.01 eq.) and glycine benzyl ester hydrochloride (600.00 mg; 2.98 mmol; 1.00 eq.) were dissolved in anhydrous dichloromethane (50.00 mL). HOSu (858.20 mg; 7.46 mmol; 2.51 eq.) and EDC (1.15 g; 5.98 mmol; 2.01 eq.) were added, resulting in a turbid mixture which became clear upon addition of DIPEA (4.16 mL; 23.80 mmol; 8.00 eq.). The solution was stirred at room temperature for 3.5 h.

The solvent was evaporated, and the residue was dissolved in acetonitrile/water 1:1 (v/v, 0.1% TFA, 10 mL). The crude product was purified by RP-LPLC using a gradient (10-35 %) of acetonitrile (0.1 % TFA) in water (0.1 % TFA). Product fractions were pooled and lyophilized.

Yield: 1.07 g (97.36 %) of a colorless oil
*m*/*z* = 370.40 $[M+H]^+$

**Step 2:**

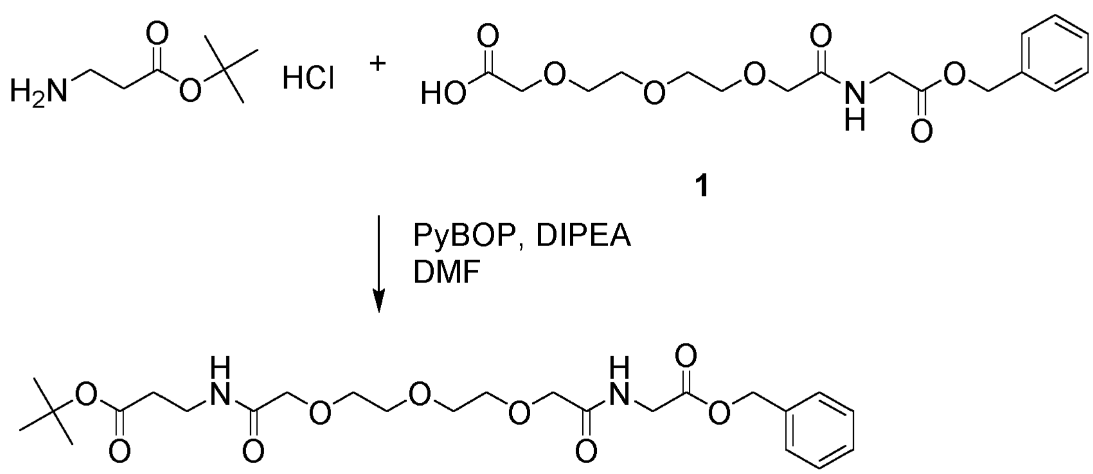


1

2

Compound **1** (525.30 mg; 1.42 mmol; 1.00 eq.) and PyBOP (740.08 mg; 1.42 mmol; 1.00 eq.) were dissolved in anhydrous DMF (5.00 mL). β-Alanine *tert.-butylester* hydrochloride (258.35 mg; 1.42 mmol; 1.00 eq.) and DIPEA (496.77 μL; 2.84 mmol; 2.00 eq.) were added successively, and the solution was stirred at room temperature for 4.5 h. The reaction was quenched by addition of 1N HCl (2.2 mL). The mixture was diluted with DCM (100 mL) and washed with 0.1 N HCl (3x50 mL), aqueous saturated $NaHCO_3$ (3x50 mL) and brine (50 mL). The organic phase was dried over $Na_2SO_4$, filtered, and the solvent was evaporated. The crude product obtained in this way was purified by flash chromatography on silica using a gradient (10-100 %) of acetonitrile in DCM. Product fractions were pooled, concentrated under reduced pressure and dried *in vacuo.*

Yield: 495.10 mg (70.11 %) of a colorless oil
*m*/*z* = 497.49 $[M+H]^+$

**Step 3:**

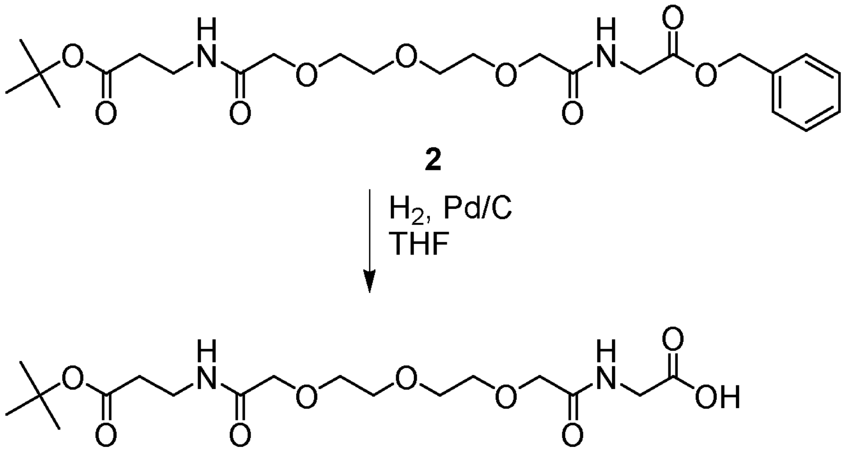


2

3

Compound 2 (495.10 mg; 1.00 mmol; 1.00 eq.) was dissolved in anhydrous THF (10.00 mL). Palladium on activated charcoal (10 % wt, 21.22 mg; 0.20 mmol; 0.20 eq.) was added to the solution, and the reaction mixture was stirred at room temperature under an atmosphere of hydrogen for 1 h. The reaction mixture was filtered, volatiles were evaporated under reduced pressure, and the residue was dried *in vacuo.* 354 mg of the residue were submitted to purification by preparative RP-HPLC using a gradient (0-50 %) of acetonitrile (0.1 % TFA) in water (0.1 % TFA). Product fractions were pooled and lyophilized.

Yield: 307.00 mg of a colorless oil
*m*/*z* = 407.44 $[M+H]^+$

**Step** 4: **Resiquimod coupling**

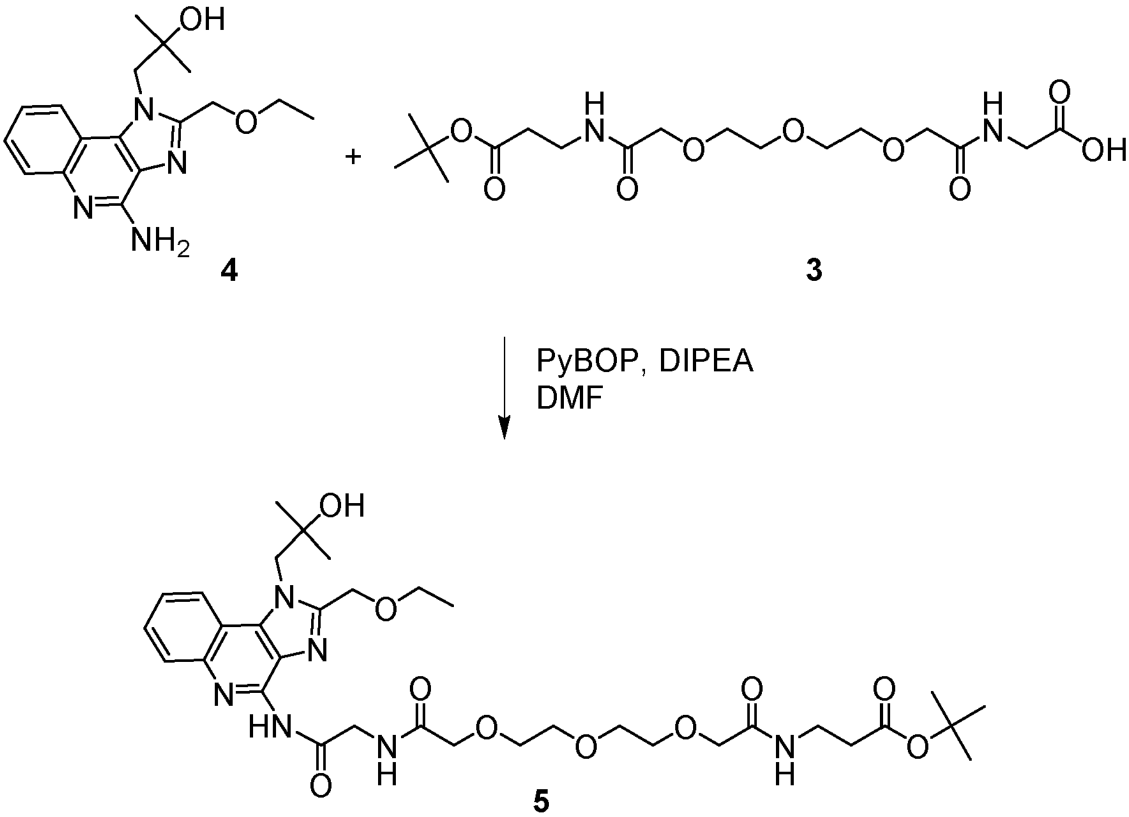


4

3

5

Resiquimod **4** (32.50 mg; 103.38 µmol; 1.00 eq.) was added to a solution of protected linker reagent **3** (76.00 mg; 186.99 µmol; 1.80 eq.) in anhydrous DMF (0.40 mL). PyBOP (98.00 mg; 188.32 µmol; 1.81 eq.) and DIPEA (160.00 µL; 918.58 µmol; 8.84 eq.) were added. After 18 h at r.t., the reaction was quenched with AcOH (160 µL) and 2 mL of 30 mM phosphate buffer (pH 8.2) which contained 20% Acetonitrile were added, resulting in ca 2.7 mL of crude product solution. The product was purified by preparative RP-HPLC using a gradient (25-45 %) of acetonitrile in 30 mM sodium phosphate buffer (pH 8.2). Product fractions were pooled and transferred in a separation funnel. The aqueous phase was extracted with ethyl acetate (60 ml, 30 ml, 30 ml) and the combined organic phases were dried ($MgSO_4$), filtered, concentrated under reduced pressure and dried *in vacuo.*

Yield: 61.4 mg (84 %).
*m*/*z* = 703.65 $[M+H]^+$

**Step 5: Deprotection**

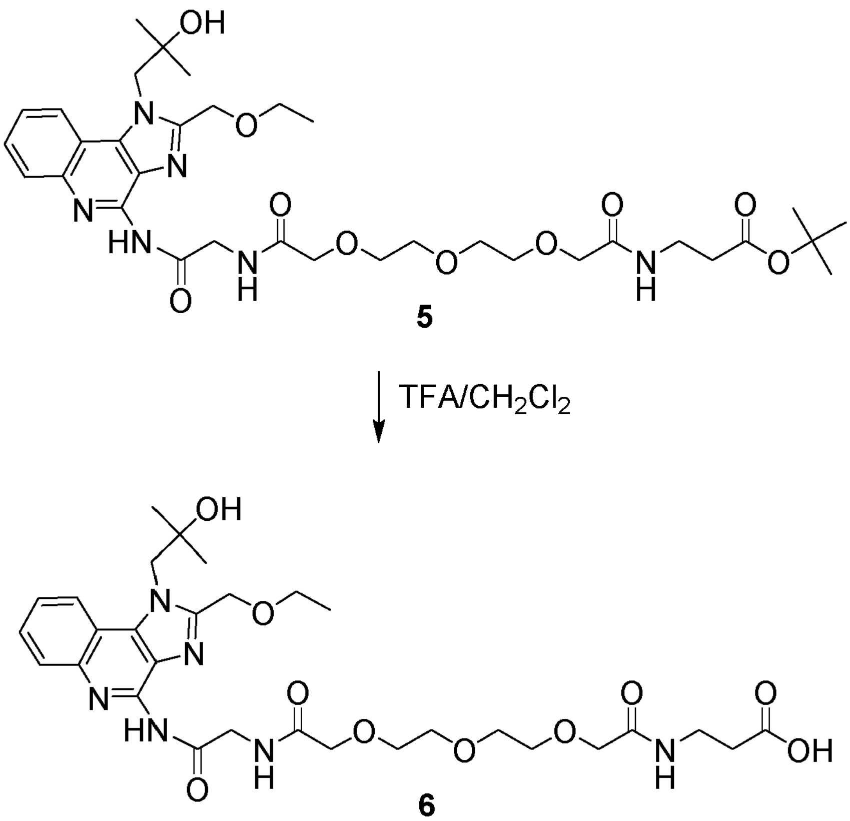


**5**

**6**

Compound 5 (64.00 mg; 0.09 mmol; 1.00 eq.) was dissolved in anhydrous dichloromethane (2.00 mL) and trifluoroacetic acid (2.00 mL). After 2 h, the reaction solution was concentrated under reduced pressure. To the residue was added 1 mL of 30 mM pH 8.2 phosphate buffer containing 20 % of acetonitrile. The resulting emulsion was purified by preparative RP-HPLC using a gradient (5-50 %) of acetonitrile in water. Pooled fractions were lyophilized. The residue (43.7 mg, 74 %) was dissolved in DMF anhydrous (2.18 mL) to result in a solution with a content of 21.8 mg/ml.

Yield: 43.7 mg (74 %)
*m*/*z* = 647.59 $[M+H]^+$

**Example 2: Synthesis of PEG-hydrogel beads containing free amino groups (0.075 mmol/g)**

**Step 1: Synthesis of backbone reagent 7**

Backbone reagent 7 was synthesized as HCl salt using L-lysine building blocks, analogously to an earlier described procedure (WO2013/053856, example 1, compound **1g** therein):

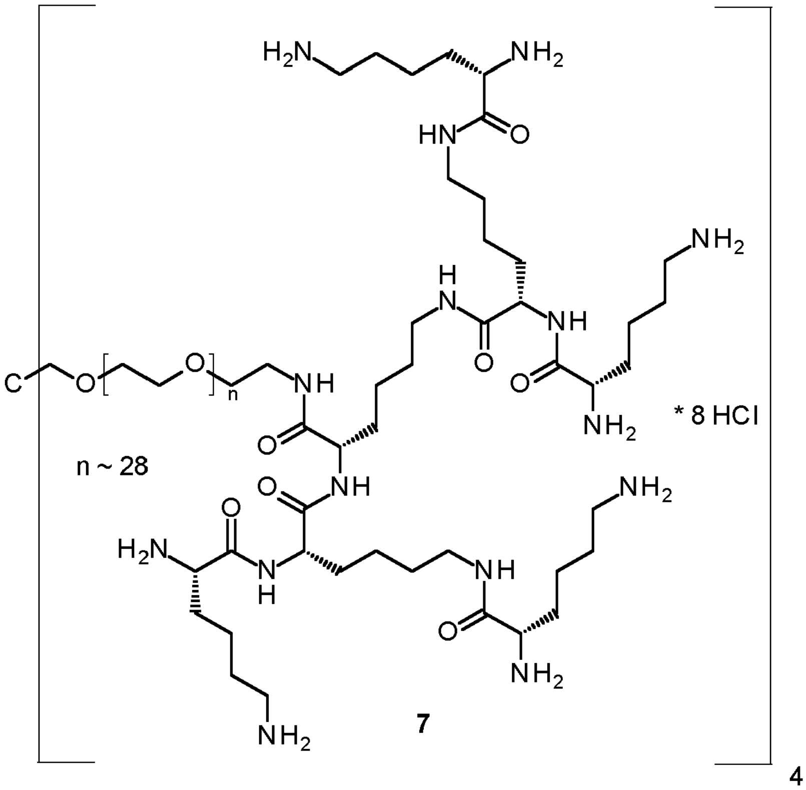

7

**Step 2: Polymerization**

7

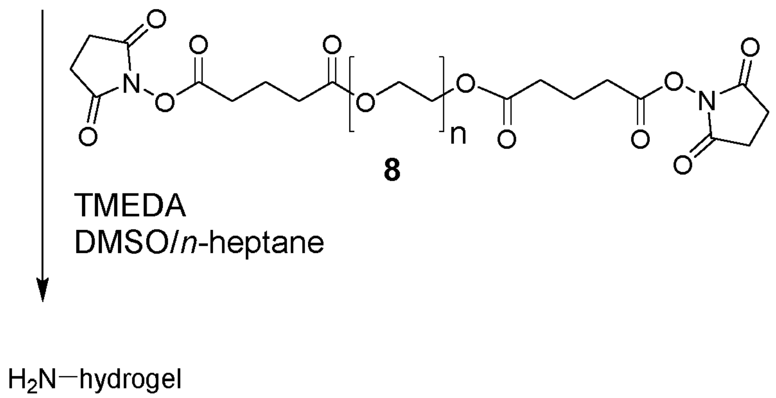


9

A cylindrical 250 mL reactor with bottom outlet, diameter 60 mm, equipped with baffles, was charged with an emulsion of CithrolTM DPHS (0.4 g) in heptane (80 mL). The reactor content was stirred with a pitch-blade stirrer, diameter 45 mm, at 460 rpm, at r.t. A solution of PEG-Disuccinimidylglutarate, 1 kDa **8** (Innochemie, 4290 mg) and backbone reagent **7** (2000 mg) in DMSO (38.6 g) was added to the reactor and stirred for 10 min to form an emulsion. TMEDA (8.9 mL) was added to effect polymerization and the mixture was stirred at r.t. for 16 h. Acetic acid (13.7 mL) was added while stirring. After 10 min, a sodium chloride solution (15 wt%, 100 mL) was added under stirring. After 10 min, the stirrer was stopped, and phases were allowed to separate. After 95 min, the aqueous phase containing the PEG-hydrogel beads was drained.

For bead size fractionation, the water-hydrogel suspension was diluted with ethanol (40 mL) and wet-sieved on

125, 100, 75, 63, and 50 μm (mesh opening) stainless steel sieves, diameter 200 mm using a sieving machine for 15 min. Sieving amplitude was 1.5 mm, liquid flow was 250 mL/min. Water (4000 mL) was used as the liquid for wet-sieving. Hydrogel beads were harvested from the sieves into 50 mL Falcon tubes using 20% ethanol in water. After centrifugation at 5000 rpm for 1 min, the yield of suspension was noted (see below). Fractions were worked up. Washing by centrifugation at 5000 rpm, 1 min, was performed 3x with 0.1% AcOH, then with EtOH until no more shrinkage of the volume was observed. The fractions were transferred into individual syringes with PE filter and dried for 3 d at < 1mbar.The amine content of the hydrogel was determined from dry material.

| | |
|---|---|
| Yields: | 63 μm sieve fraction: ≈ 15 mL of suspension, 1493 mg after drying |
| | 75 μm sieve fraction: ≈ 15 mL of suspension, 1433 mg after drying |
| Amine content: | 0.075 mmol/g |

**Example 3: Synthesis of PEG-hydrogel beads containing free amino groups (0.11-0.5 mmol/g)**

**7**

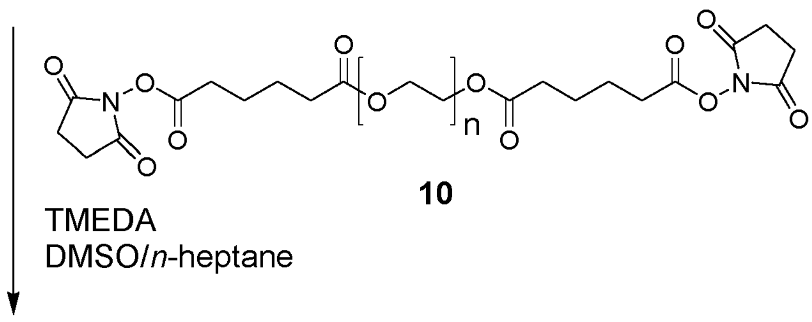


**10**

$H_2N$—hydrogel

**11a, 11b**

Hydrogels from reagent **7** and **10** (see WO 2011/012715 Al, example 2, compound **2d)** were prepared according to a procedure described in WO 2011/012715 Al, example 3.
Hydrogel **11a** was synthesized from 1398 mg of reagent 7 and 4473 mg of reagent **10** in 36.2 g of DMSO. The resulting amine load was 0.151 mmol/g.
Hydrogel **11b** was synthesized from 3.40 g of reagent 7 and 8.91 g of reagent **10** in 75.6 g of DMSO. The resulting amine load was 0.296 mmol/g.

**Example 4: Acetylation of hydrogels**

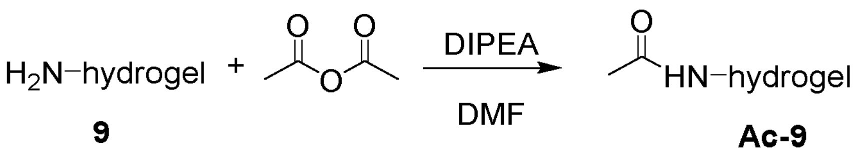


**9** **Ac-9**

Hydrogel **9** (3.184 g, 0.239 mmol) was filled into a 50 mL syringe equipped with a PE frit and washed 3x with a 1 % (v/v) solution of DIPEA in anhydrous DMF. A solution of acetic anhydride (0.45 mL; 4.77 mmol; 20.00 eq.) and DIPEA, (0.83 mL; 4.77 mmol; 20.00 eq.) in anhydrous DMF (38.18 mL) was drawn into the syringe, the syringe was closed with a sterile cap and shaken for 1 h at 1000 rpm at r.t. The solvent was expelled, and the syringe was washed 10x with anhydrous DMF, and 10x with ethanol. The volume of the swollen hydrogel after expelling the ethanol was 11 mL. The resulting hydrogel

was dried *in vacuo.* Under sterile conditions, hydrogel **Ac-9** (2.98 g; 1.00 eq.) was transferred into a 50 ml Falcon tube. Formulation buffer (30 mL) was added, and the Falcon tube was agitated for 30 min on a shaker until a homogenous suspension had formed.

In an analogous procedure, hydrogel **11a** was acetylated to yield **Ac-11a,** and hydrogel **11b** was acetylated to yield **Ac-11b.**

**Example 5: Loading of compound 6 on hydrogels**

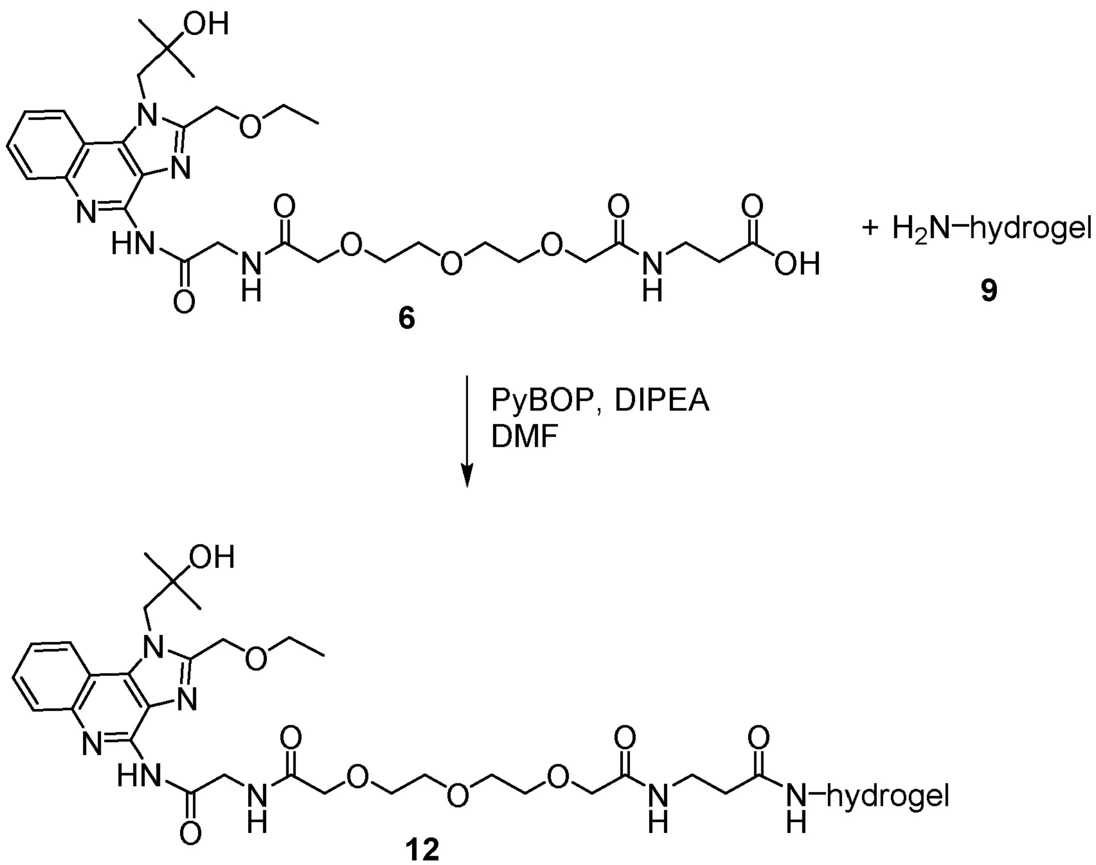


**6** **9** **12**

Under sterile conditions, hydrogel **9** (457.00 mg; 34.28 μmol; 1.00 eq.) was weighed into a 20 mL syringe equipped with a PE frit. The hydrogel was swollen by drawing anhydrous DMF (1% DIPEA, 10 mL) in the syringe, the syringe was shaken manually for 1 min and the solvent was expelled. This procedure was repeated three times. A solution of compound **6** in DMF (2.00 mL; 21.80 mg/mL; 67.42 μmol; 1.97 eq.) and DIPEA (35.82 μL; 205.66 μmol; 6.00 eq.) were mixed and drawn into the syringe containing the hydrogel, followed by a solution of PyBOP (35.67 mg; 68.55 μmol; 2.00 eq.) in anhydrous DMF (1.00 mL). Air was drawn into the syringe to drain canula and frit. The syringe was shaken for 3.5 h at r.t. The solution was expelled. The hydrogel was washed with DMF (10 x 10 mL), sterile, pyrogene-free water (10 x 10 mL) and formulation-buffer (10 x 10 mL). After the last washing step, ca. 10 mL of buffer were drawn into the syringe. The syringe was closed with a sterile stopper and incubated at 37°C for 1 h. The buffer was expelled, and the hydrogel was washed with formulation buffer (10 x 10 mL). The plunger was removed, and the suspension was transferred into a 50 mL Falcon tube. The buffer supernatant was removed, resulting in a suspension with a final volume of ca. 6 mL. The resiquimod content of the resulting hydrogel **12** was ca. 1.5 mg resiquimod eq./mL. In an analogous procedure, compound **6** was loaded on hydrogel **11a** to yield hydrogel **12a** with a resiquimod load of ca. 1.9 mg resiquimod eq./mL.

In an analogous procedure, compound **6** was loaded on hydrogel **11b** to yield hydrogel **12b** with a resiquimod load of ca. 4.9 mg resiquimod eq./mL.

In an analogous procedure, compound **6** was loaded on hydrogel **11a** to yield hydrogel **12c** with a resiquimod load of ca. 2.7 mg resiquimod eq./mL.

**Example 6: Dose adjustment**

Under sterile conditions, hydrogel suspension **Ac-9** (11.23 mL) was combined with hydrogel suspension **12** (1.52 mg Resiquimod eq./mL; 4.57 mL) in a sterile 50 mL Falcon tube. The combined hydrogel was homogenized by slowly vortexing the Falcon tube for 5min. The content of the resulting hydrogel suspension was 0.376 mg Resiquimod eq./mL.

In an analogous procedure, the following hydrogel suspensions were prepared from their acetylated and resiquimod-loaded components.

| Compound | Acetylated hydrogel | Resiquimod-hydrogel | Resiquimod content in hydrogel suspension | buffer |
|---|---|---|---|---|
| **13a** | **Ac-9** | **12** | 92 µg eq./mL | PBST |
| **13b** | **Ac-11a** | **12a** | 119 µg eq./mL | PBST |
| **13c** | **Ac-9** | **12** | 376 µg eq./mL | PBST |
| **13d** | **Ac-11b** | **12b** | 103 µg eq./mL | PTP |
| **13e** | **Ac-11b** | **12b** | 410 µg eq./mL | PTP |
| **13f** | **Ac-11b** | **12b** | 1649 µg eq./mL | PTP |
| **13g** | **Ac-11b** | **12b** | 4040 µg eq./mL | PTP |
| **13h** | **Ac-11b** | **12b** | 4321 µg eq./mL | PTP |
| **13i** | **Ac-11a** | **12c** | 226 µg eq./mL | PTP |

**Example 7: Loading of compound 6 on hydrogel with subsequent acetylation**

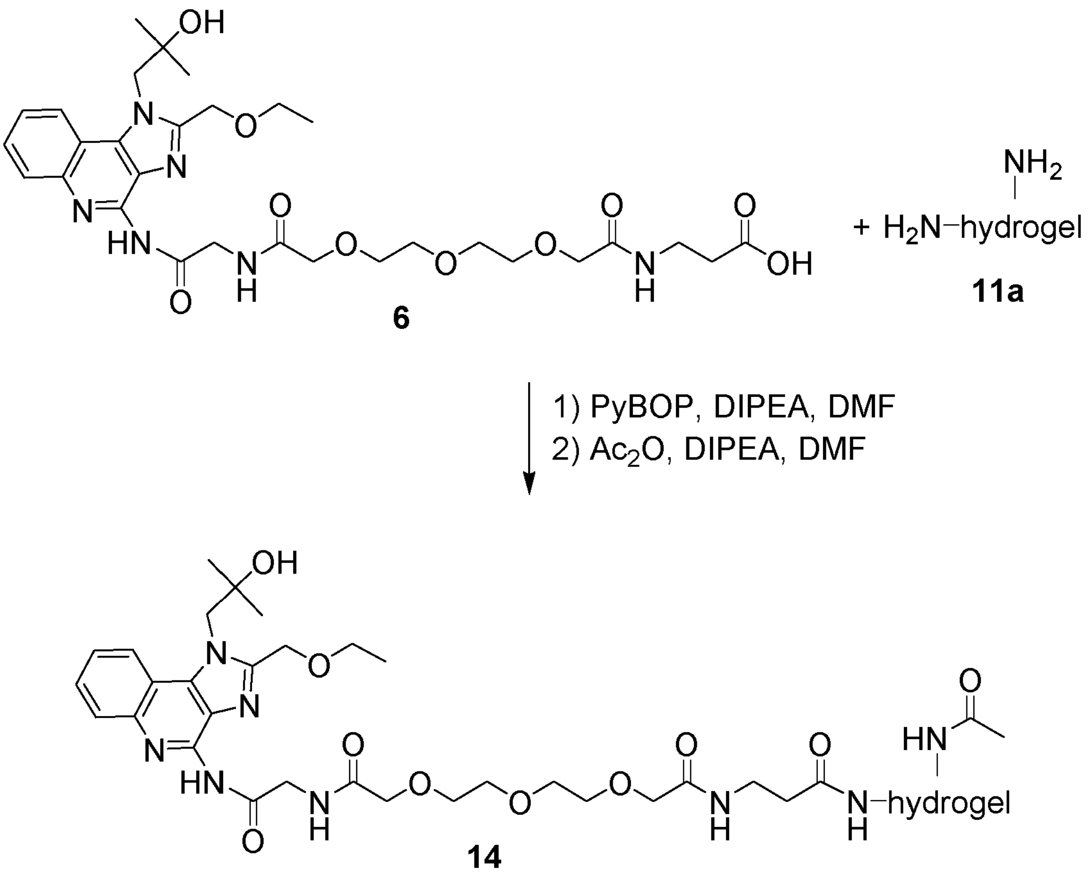


**6**

**11a**

**14**

Hydrogel **11a** (200 mg; 0.03 mmol) was weighed into a 10 mL syringe equipped with a PE frit. The hydrogel was

swollen by drawing anhydrous DMF (1% DIPEA, 3 mL) in the syringe, the syringe was shaken manually for 1 min and the solvent was expelled. This procedure was repeated three times.

A solution of compound **6** (7.76 mg, 12.0 μmol, 1.0 eq), PyBOP (7.5 mg, 14.4 μmol, 1.2 eq) and DIPEA (16.8 μL; 96 μmol; 8 eq) in DMF (3 mL) was added to the hydrogel, and the suspension was shaken at r.t. overnight. After completion of the reaction the hydrogel was washed with DMF (10 x 5 mL).

A solution of acetic anhydride (60 μL; 0.63 mmol) and DIPEA (110 μL; 0.63 mmol) in DMF (2.83 mL) was drawn into the syringe, and the suspension was shaken at r.t. for 2 hours. The supernatant was expelled and the hydrogel was washed with DMF (10 x 3 mL), water (10 x 3 mL), EtOH (10 x 3 mL) and dried *in vacuo.*

The resiquimod content of the resulting hydrogel **14** was 17.4 mg/g.

**Example 8: Release of Resiquimod from hydrogel 14**

A suspension of hydrogel **14** (0.23 % wt/wt) in pH 7.4 phosphate buffer was incubated at 37 °C. Over the course of 33 d, samples of the supernatant were withdrawn and the Resiquimod content was determined by UPLC against a calibration curve. Non-linear regression analysis of the obtained concentrations resulted in a release half-life of 15.3 d.

**Example 9: Preparation of biased IL-2 mutein polymer prodrug**

**Step 1: Preparation of cysteine protected IL-2 mutein 15**

IL-2 variant (mutein) was custom made and sourced from an external supplier where expression of the proteins was performed from E.coli followed by standard purification strategies known to the one skilled in the art. The following proteins were prepared

**15:** PTSSSTKKTQ LQLEHLLLDL QMILNGINNY KNPKLTC*MLT FKFYMPKKAT ELKHLQCLEE ELKPLEEVLN LAQSKNFHLR PRDLISNINV IVLELKGSET TFMCEYADET ATIVEFLNRW ITFSQSIIST LT (SEQ ID NO:1; cysteine marked with "*" is connected to a free cysteine via a disulfide bond)

**Step 2: Preparation of biased IL-2 mutein polymer prodrug 16**

23.2 mg of TCEP (Tris(2-carboxethyl)phosphine hydrochloride) were dissolved in 1.62 mL PBS (phosphate buffered saline) pH 7.4 to give a 50 mM solution. No adjustment of the pH was performed.

45.2 mL of **15** formulated at 1.8 mg/mL in PBS, 10% glycerin, pH approx. 9, were mixed with 13.6 mL 0.5 M sodium phosphate, pH 7.4, then 710 μL of the TCEP solution were added. The sample was incubated at ambient temperature for 30 min.

Subsequently, 5.5 mL of 5 mM 5 kDa PEG maleimide (Sunbright ME-050MA, CAS 883993-35-9, NOF Europe N.V., Grobbendonk, Belgium) in PBS, pH 7.4 (5 mol. eq.) were added to the reaction solution. After incubation at ambient temperature for 10 min, the formation of conjugates was confirmed by analytical size exclusion chromatography.

The buffer of the conjugation mixture was exchanged to 100 mM borate, pH 9.0 using an Aekta system equipped with a HiPrep Desalting 26/10 column. The sample was incubated at 25°C overnight, then concentrated to 5.3 mg/mL using Amicon Ultra-15, Ultracel 3 K centrifugation filters (Merck Millipore). 0.847g of 40 kDa mPEG-linker reagent (as described in patent WO 2016079114 example 2) were dissolved in 9.75 mL water to give a stock solution of $2.1*10^{-3}$ mol/L. The solution was stored on ice.

12.9 mL of the protein solution were diluted to 4 mg/mL by addition of 100 mM borate, pH 9.0, then 8.4 mL of the cooled 40 kDa mPEG-linker reagent stock solution were added (corresponding to 4 mol. eq. with respect to the protein). The conjugation mixture was placed in a water bath at 14°C for 2 h. The pH was shifted to pH 4 by addition of 8.4 mL of water and 33.5 mL of 200 mM sodium acetate, pH 3.6 followed by an incubation at 25°C overnight.

The conjugate with one single 40 kDa mPEG linker attached (mono-conjugate) was isolated from the reaction mixture using a HiScreen Capto MMC resin (column dimension: 0.77 x 10 cm) connected to an Aekta system. A flow rate of 1.2 mL/min and a linear gradient from 10 mM succinic acid, pH 5.5 to 80% of 10 mM succinic acid, 1 M NaCl, pH 5.5 in 12 column volumes was applied for all three runs. Fractions containing mainly mono-conjugate were identified by analytical size exclusion chromatography. The salt content of each fraction was adjusted to 150 mM by addition of 10 mM succinic acid, 1 M NaCl, pH 5.5, then the fractions were pooled and concentrated to 2.8 mg/mL in Amicon Ultra-15, Ultracel 10 K filters (Merck Millipore).

The concentrated solution (8.1 mL) was diluted with 0.4 mL of 10 mM succinic acid, 150 mM NaCl, 1% Tween20, pH 5.5 and 14.4 mL of 10 mM succinic acid, 150 mM NaCl, 0.05% Tween20, pH 5.5 to a final concentration of 1 mg/mL. The final sample was filtered through a 0.22 μm PVDF filter membrane.

**Example 10: In vivo PK study**

Resiquimod and resiquimod-releasing hydrogels were injected subcutaneously into rats and plasma levels of resiquimod were observed over the course of 28 d. Resiquimod **4** was dissolved in 10 mM succinate, 90.0 mg/mL trehalose dihydrate, pH 5.0 at a concentration of 104 μg/mL. Hydrogels were suspended (ca. 6% wt/v) PBST buffer at pH 7.4. Male WISTAR rats (n=3 per group) received a single subcutaneous injection of either resiquimod **4** solution or hydrogels **13a or 13b,** each corresponding to a dose of 25 μg eq. of resiquimod. Blood samples were withdrawn and used for plasma generation over the course of 28 d. The resiquimod concentration in the plasma samples was quantified by LC-MS/MS. Plasma concentration profiles were generated and analyzed with Phoenix WinNonlin software (Certara, Princeton, NJ, USA).

Results:

Maximum plasma concentrations, terminal elimination half-lives and calculated AUCs are summarized below:

| Compound | $C_{max}$ [pg/mL] | $t_{1/2}$ | $AUC_{Pred-\infty}$ [h*pg/mL] |
|---|---|---|---|
| **4** | 23100 | 1.5 h and 10 h (biphasic) | 65400 |
| **13a** | 281 | 13.6 d | 74700 |
| **13b** | 234 | 10.5 d | 65900 |

**Example 11: In vivo anti-tumor efficacy**

The study was conducted in female BALB/C mice with an age of 6-11 weeks at the day of tumor inoculation. Mice were subcutaneously implanted with 3 x $10^5$ CT26 tumor cells in the left and right flanks. When tumors to be injected were grown to a mean tumor volume of ~80 $mm^3$, mice were randomized into treatment cohorts (day 0). The day following randomization, animals received a single dose of either 20 μg of resiquimod **4** (dissolved in 10 mM succinate, 90.0 mg/mL trehalose dihydrate, pH 5.0) or hydrogel **13c** as a single intratumoral dose in an injection volume of 50 μL or a single intratumoral injection of 50 μL of a suspension of **Ac-9.** Hydrogels were administered as suspensions in PBST buffer. Following treatment initiation, anti-tumor efficacy was assessed by determination of tumor volumes at various time points from tumor size measurements with a caliper. Tumor volumes were calculated according to the formula:

$$\text{Tumor volume} = (L \times W^2) \times 0.5$$

where L is the length of the tumor and W the width (both in mm). Mice were removed from the study once tumors were greater than 1500 $mm^3$.

Results:

Absolute tumor volumes

| | | Days post-treatment | | | | | | |
|---|---|---|---|---|---|---|---|---|
| Group | | 0 | 2 | 4 | 7 | 9 | 11 | 14 |
| **Ac-9** | Mean ($mm^3$) | 89.01 | 125.62 | 151.98 | 449.94 | 792.78 | 1065.45 | 1402.37 |
| | SEM ($mm^3$) | 3.65 | 7.52 | 11.88 | 43.28 | 74.31 | 91.43 | 100.91 |
| | N | 10 | 10 | 10 | 10 | 10 | 10 | 6 |
| **4** | Mean ($mm^3$) | 88.82 | 108.44 | 128.42 | 316.07 | 549.72 | 957.90 | 1220.96 |
| | SEM ($mm^3$) | 3.48 | 5.34 | 8.69 | 49.35 | 56.21 | 125.30 | 137.76 |
| | N | 10 | 10 | 10 | 10 | 10 | 10 | 8 |

(continued)

| | | Days post-treatment | | | | | | |
|---|---|---|---|---|---|---|---|---|
| Group | | 0 | 2 | 4 | 7 | 9 | 11 | 14 |
| **13c** | Mean ($mm^3$) | 88.96 | 123.60 | 141.09 | 215.87† | 305.38†,‡ | 378.04†,‡ | 609.37†,‡ |
| | SEM ($mm^3$) | 2.15 | 6.78 | 9.55 | 33.39 | 58.73 | 75.61 | 129.13 |
| | N | 15 | 15 | 15 | 15 | 15 | 15 | 11 |
| SEM = standard error of the mean, N = sample size; †p<0.05 vs **Ac-9,** ‡p<0.05 vs **4.** Significance was determined by Two-way ANOVA followed by multiple comparisons using Tukey's Honest Significant Differences (HSD) post-hoc test. | | | | | | | | |

**Example 12: In vivo cytokine induction**

The study was conducted in female BALB/C mice with an age of 6-11 weeks at the day of tumor inoculation. Mice were subcutaneously implanted with 3 x $10^5$ CT26 tumor cells in the left and right flanks. When tumors to be injected were grown to a mean tumor volume of ~105 $mm^3$, mice were randomized into treatment cohorts (day 0). The day following randomization, animals received a single dose of either 20 μg of resiquimod **4** (dissolved in10 mM succinate, 90.0 mg/mL trehalose dihydrate, pH 5.0) or hydrogel **13c** as a single intratumoral dose in an injection volume of 50 μL or a single intratumoral injection of 50 μL of a suspension of **Ac-9.** Hydrogels were administered as suspensions in PBST buffer. $K_2$ EDTA-preserved blood samples were collected by retro-orbital bleed at various time points following drug administration and plasma was isolated following centrifugation at 2000 × g for 5 minutes at 4 °C and frozen. Plasma samples were stored at -80$^0$C. Plasma was thawed and undiluted samples were assessed for cytokine levels using the 36-Plex Mouse ProcartaPlex™ Cytokine Panel 1A (ThermoFisher Scientific) following manufacturer's recommendations. Cytokines were measured on the Bio-Plex 200 (BioRad) following kit instructions. For sample values below or at the lower limit of quantitation (LLOQ) of the assay, a value of 0.01 pg/mL was instead used in determining mean cytokine concentrations.

Results:

Plasma cytokine levels

| IFNγ | | | | | | |
|---|---|---|---|---|---|---|
| | | Hours post-treatment | | | | |
| Group | | 1 | 3 | 6 | 10 | 24 |
| **Ac-9** | Mean (pg/mL) | 0.01 | 0.01 | 0.01 | 0.01 | 0.01 |
| | SEM (pg/mL) | 0.00 | 0.00 | 0.00 | 0.00 | 0.00 |
| | N | 3 | 3 | 3 | 3 | 3 |
| **4** | Mean (pg/mL) | 4.04 | 15.49†,‡ | 40.72†,‡ | 28.19†,‡ | 2.38 |
| | SEM (pg/mL) | 0.45 | 6.64 | 3.49 | 4.50 | 1.21 |
| | N | 3 | 3 | 3 | 3 | 3 |
| **13c** | Mean (pg/mL) | 0.01 | 0.49 | 2.76 | 4.51 | 2.10 |
| | SEM (pg/mL) | 0.00 | 0.48 | 1.42 | 0.87 | 0.37 |
| | N | 3 | 3 | 3 | 3 | 3 |
| SEM = standard error of the mean, N = sample size; †p≤0.0002 vs **Ac-9,** ‡p≤0.0002 vs **13c.** Significance was determined by Two-way ANOVA followed by multiple comparisons using Tukey's Honest Significant Differences (HSD) post-hoc test. | | | | | | |

| IL-6 | | | | | | |
|---|---|---|---|---|---|---|
| | | Hours post-treatment | | | | |
| Group | | 1 | 3 | 6 | 10 | 24 |
| **Ac-9** | Mean (pg/mL) | 40.31 | 25.68 | 4.86 | 14.93 | 57.67 |
| | SEM (pg/mL) | 12.13 | 15.78 | 4.85 | 4.55 | 12.86 |
| | N | 3 | 3 | 3 | 3 | 3 |
| **4** | Mean (pg/mL) | 3618.87†,‡ | 1739.75†,‡ | 88.59 | 154.51 | 44.71 |
| | SEM (pg/mL) | 146.11 | 360.03 | 16.66 | 69.39 | 28.80 |
| | N | 3 | 3 | 3 | 3 | 3 |
| **13c** | Mean (pg/mL) | 52.18 | 141.69 | 80.47 | 71.58 | 270.01 |
| | SEM (pg/mL) | 18.14 | 55.52 | 11.99 | 22.72 | 96.47 |
| | N | 3 | 3 | 3 | 3 | 3 |
| SEM = standard error of the mean, N = sample size; †p<0.0001 vs **Ac-9,** ‡p<0.0001 vs **13c.** Significance was determined by Two-way ANOVA followed by multiple comparisons using Tukey's Honest Significant Differences (HSD) post-hoc test. | | | | | | |

| CCL2 / MCP-1 | | | | | | |
|---|---|---|---|---|---|---|
| | | Hours post-treatment | | | | |
| Group | | 1 | 3 | 6 | 10 | 24 |
| **Ac-9** | Mean (pg/mL) | 17.81 | 38.01 | 23.18 | 39.77 | 50.04 |
| | SEM (pg/mL) | 10.94 | 10.74 | 1.02 | 8.97 | 19.42 |
| | N | 3 | 3 | 3 | 3 | 3 |
| **4** | Mean (pg/mL) | 357.83 | 4230.35†,‡ | 1039.17†,‡ | 847.07† | 92.58 |
| | SEM (pg/mL) | 80.35 | 147.36 | 279.41 | 182.24 | 1.59 |
| | N | 3 | 3 | 3 | 3 | 3 |
| **13c** | Mean (pg/mL) | 62.28 | 282.21 | 309.21 | 508.54†† | 237.05 |
| | SEM (pg/mL) | 21.46 | 114.89 | 108.22 | 71.83 | 6.31 |
| | N | 3 | 3 | 3 | 3 | 3 |
| SEM = standard error of the mean, N = sample size †p≤0.0001 vs **Ac-9,** ‡p≤0.0001 vs **13c,** ††p < 0.02 vs **Ac-9.** Significance was determined by Two-way ANOVA followed by multiple comparisons using Tukey's Honest Significant Differences (HSD) post-hoc test. | | | | | | |

| TNFα | | | | | | |
|---|---|---|---|---|---|---|
| | | Hours post-treatment | | | | |
| Group | | 1 | 3 | 6 | 10 | 24 |
| **Ac-9** | Mean (pg/mL) | 2.29 | 1.93 | 4.73 | 1.36 | 2.11 |
| | SEM (pg/mL) | 2.28 | 1.92 | 0.25 | 1.35 | 2.10 |
| | N | 3 | 3 | 3 | 3 | 3 |
| **4** | Mean (pg/mL) | 830.84 | 136.25 | 35.49 | 43.32 | 9.74 |
| | SEM (pg/mL) | 99.38†,‡ | 17.86†,‡ | 1.05 | 14.06 | 2.14 |
| | N | 3 | 3 | 3 | 3 | 3 |

(continued)

| TNFα | | | | | | |
|---|---|---|---|---|---|---|
| | | Hours post-treatment | | | | |
| Group | | 1 | 3 | 6 | 10 | 24 |
| **13c** | Mean (pg/mL) | 7.34 | 27.00 | 26.46 | 13.78 | 19.70 |
| | SEM (pg/mL) | 0.80 | 4.31 | 8.56 | 3.21 | 0.99 |
| | N | 3 | 3 | 3 | 3 | 3 |
| SEM = standard error of the mean, N = sample size; †p<0.004 vs **Ac-9,** ‡p<0.02 vs **13c.** Significance was determined by Two-way ANOVA followed by multiple comparisons using Tukey's Honest Significant Differences (HSD) post-hoc test. | | | | | | |

**Example 13: In vivo dose escalation, tumor cytokine and chemokine profiling, and tumor efficacy study**

The study was conducted in female BALB/C mice with an age of 6-11 weeks at the day of tumor inoculation. Mice were implanted with 3 x $10^5$ CT26 tumor cells into the right flank. When tumors were grown to a mean tumor volume of ~115 $mm^3$, mice were randomized into treatment cohorts (day 0). The day following randomization, animals received either **13g, 13f, 13e,** or **13d** as a single intratumoral dose in an injection volume of 50 μL or a single intratumoral injection of 50 μL of a suspension of **Ac-11b.** Hydrogels were administered as suspensions in PTP buffer. Following treatment initiation, anti-tumor efficacy was assessed by determination of tumor volumes at various time points from tumor size measurements with a caliper. Tumor volumes were calculated according to the formula:

$$\text{Tumor volume} = (L \times W^2) \times 0.5$$

where L is the length of the tumor and W the width (both in mm). On the same day as tumor measurements, mice were weighed for absolute body weight. At defined time points (6 hours, 3 days, and 7 days post-treatment initiation), 2-3 mice per group were sacrificed and tumors were harvested and frozen while plasma was prepared after blood withdrawal. Plasma was also generated for all mice which were taken out of the study when termination criteria were reached. The concentration of resiquimod in the plasma samples was quantified by LC-MS/MS. Serum PK parameters for animals that received 13e or 13d were analysed using the noncompartmental (NCA) approach using Phoenix 64 (Version 8). Frozen tumors were cut in to pieces approximately 0.3-0.8mm in length, then mechanically homogenized via mortar and pestle while kept frozen. For tumor cytokine and chemokine protein assessment, an aliquot of homogenized tumor was lysed in 400 μL of ProcartaPlex cell lysis buffer (ThermoFisher Scientific) per every 50 mg of tissue. Samples were sonicated to facilitate tumor lysis. Lysates were centrifuged at 30,000 G for 20 minutes at $4^0$C, and supernatants were harvested. Protein concentrations were measured using the Bio-Rad DC Protein Assay kit (Bio-Rad) following manufacturer's recommendations. Samples were diluted with PBS to a protein concentration of 5.5 mg protein/mL. 25 μL of concentration adjusted samples were then assessed for chemokine and cytokine levels using the 36-Plex Mouse ProcartaPlex Cytokine Panel 1A (ThermoFisher Scientific) following manufacturer's recommendations. Cytokines were measured on the Bio-Plex 200 (Bio-Rad) following kit instructions. For sample values below or at the lower limit of quantitation (LLOQ) of the assay, a value of 0.01 pg/mL was instead used in determining mean cytokine concentrations. Fold changes were determined by dividing the mean cytokine concentrations of treated samples by the mean cytokine concentration of **Ac-11b** treated samples at each timepoint. For tumor cytokine and chemokine gene expression assessment, RNA was isolated from an aliquot of homogenized tumor using the mirVana miRNA Isolation kit (Ambion) following manufacturer's recommendations. Following the first column washing step, DNA was digested directly on the column using the RNase-free DNase Set (Qiagen) following manufacturer's recommendations. RNA was eluted with RNase-free water. RNA concentrations were measured using a NanoDrop (ThermoFisher) and then adjusted to 215-250 ng/mL with RNase-free water. RNA quality was assessed using a Bioanalyzer (Agilent). RNA integrity was confirmed to be of high quality (RIN between 6.5-10). 1 μg of RNA was reverse transcribed to cDNA using the M-MLV Reverse Transcriptase kit (ThermoFisher). Reverse transcription was performed using random primers, 10 mM dNTP mix, and RNase inhibitor (Promega). Reverse transcription was performed with the following thermal steps: $65^0$C for 5 minutes, $4^0$C for 5 minutes, $25^0$C for 10 minutes, $4^0$C for 5 minutes, $37^0$C for 50 minutes, $42^0$C for 10 minutes. 25 ng of cDNA was used for quantitative PCR using the KAPA SYBR FAST qPCR Master Mix (2X) kit (Kapa Biosystems) following manufacturer's recommendations. Primers used for qPCR reactions are as follows:

| Gene | Forward Sequence | Reverse Sequence |
|---|---|---|
| *Ubb* | GTCTGAGGGGTGGCTATTAA (SEQ ID NO:2) | GCTTACCATGCAACAAAACC (SEQ ID NO:3) |
| *Ccl2* | CAGCTCTCTCTTCCTCCACC (SEQ ID NO:4) | TGGGATCATCTTGCTGGTGA (SEQ ID NO:5) |
| *Ccl3* | CCAGCCAGGTGTCATTTTCC (SEQ ID NO:6) | AGGCATTCAGTTCCAGGTCA (SEQ ID NO:7) |
| *Ccl4* | TCTGTGCTAACCCCAGTGAG (SEQ ID NO:8) | CTCTCCTGAAGTGGCTCCTC (SEQ ID NO:9) |
| *Ccl5* | TGCCAACCCAGAGAAGAAGT (SEQ ID NO:10) | AGATGCCCATTTTCCCAGGA (SEQ ID NO:11) |
| *Csf2* | CTGCGTAATGAGCCAGGAAC (SEQ ID NO:12) | TCTCTCGTTTGTCTTCCGCT (SEQ ID NO:13) |
| *Cxel1* | TTGTATGGTCAACACGCACG (SEQ ID NO:14) | ACGAGACCAGGAGAAACAGG (SEQ ID NO:15) |
| *Cxcl2* | CTACATCCCACCCACACAGT (SEQ ID NO:16) | TGTTCTACTCTCCTCGGTGC (SEQ ID NO:17) |
| *Cxcl10* | GCCGTCATTTTCTGCCTCAT (SEQ ID NO:18) | GATAGGCTCGCAGGGATGAT (SEQ ID NO:19) |
| *Il1b* | ACTCATTGTGGCTGTGGAGA (SEQ ID NO:20) | TTGTTCATCTCGGAGCCTGT (SEQ ID NO:21) |
| *Il6* | TTCTTGGGACTGATGCTGGT (SEQ ID NO:22) | CAGGTCTGTTGGGAGTGGTA (SEQ ID NO:23) |
| *Il10* | ACCTGGTAGAAGTGATGCCC (SEQ ID NO:24) | AGGGTCTTCAGCTTCTCACC (SEQ ID NO:25) |
| *Il18* | GGACACTTTCTTGCTTGCCA (SEQ ID NO:26) | ACCCTCCCCACCTAACTTTG (SEQ ID NO:27) |
| *Tnf* | TGAGGTCAATCTGCCCAAGT (SEQ ID NO:28) | GGGGTCAGAGTAAAGGGGTC (SEQ ID NO:29) |

Cycle thresholds (CT) were collected using a StepOnePlus Real-Time PCR System (Applied Biosystems). *Ubb* was used as a housekeeping control gene. Data is reported as the average of the 2^ΔΔCT values for each treatment. 2^ΔΔCT values were calculated with the following formula:

$$2^{\Delta\Delta CT} = 2^{-(\Delta CT(treated) - \Delta CT(untreated))}$$

ΔCT(treated) = CT(treated)-CT(treated housekeeping) where CT(treated) = CT of the gene of interest of a sample replicate in the treatment group at a given timepoint and CT(treated housekeeping) = CT of the *UBB* housekeeping gene of the same sample replicate in the same treatment group at the same timepoint

ΔCT(untreated) = CT(**Ac-11b**)-CT(**Ac-11b** housekeeping) where CT**(Ac-11b)** = average of the CTs of the 3 **Ac-11b** samples at the same timepoint as the CT(treated) comparator and CT(**Ac-11b** housekeeping) = average of the *UBB* housekeeping gene CTs of the 3 **Ac-11b** samples of the same timepoint.

Results:

Absolute tumor volumes ($mm^3$)

| | | Days post-treatment | | | | |
|---|---|---|---|---|---|---|
| Group | | 0 | 3 | 6 | 8 | 10 |
| **Ac-11b** | Mean ($mm^3$) | 113.58 | 354.04 | 516.60 | 885.98 | 852.10 |
| | SEM ($mm^3$) | 6.66 | 34.52 | 61.18 | 101.23 | 81.08 |
| | N | 17 | 14 | 11 | 11 | 8 |
| **13d** | Mean ($mm^3$) | 114.19 | 359.94 | 497.52 | 639.44† | 795.98 |
| | SEM ($mm^3$) | 6.37 | 20.55 | 33.21 | 50.47 | 117.89 |
| | N | 17 | 14 | 11 | 11 | 8 |
| **13e** | Mean ($mm^3$) | 114.03 | 319.01 | 367.39 | 518.55† | 587.11† |
| | SEM ($mm^3$) | 6.17 | 29.95 | 48.16 | 68.48 | 79.87 |
| | N | 17 | 14 | 11 | 11 | 8 |

(continued)

| | | Days post-treatment | | | | |
|---|---|---|---|---|---|---|
| Group | | 0 | 3 | 6 | 8 | 10 |
| **13f** | Mean ($mm^3$) | 113.93 | 309.38 | 352.56 | 458.16† | 585.91† |
| | SEM ($mm^3$) | 6.10 | 24.91 | 47.46 | 60.90 | 72.34 |
| | N | 17 | 13 | 9 | 9 | 7 |
| **13g** | Mean ($mm^3$) | 114.17 | 240.09 | 287.44†,‡ | 369.03†,‡ | 411.80†,‡ |
| | SEM ($mm^3$) | 5.67 | 20.63 | 52.27 | 73.29 | 94.97 |
| | N | 17 | 14 | 10 | 9 | 7 |
| SEM = standard error of the mean, N = sample size; †p<0.01 vs **Ac-11b,** ‡p<0.02 vs **13d.** Significance was determined by Two-way ANOVA followed by multiple comparisons using Tukey's Honest Significant Differences (HSD) post-hoc test. | | | | | | |

Absolute body weight (g)

| | | Days post-treatment | | | | |
|---|---|---|---|---|---|---|
| Group | | 0 | 3 | 6 | 8 | 10 |
| **Ac-11b** | Mean (g) | 17.86 | 18.25 | 18.76 | 19.28 | 19.10 |
| | SEM (g) | 0.29 | 0.30 | 0.42 | 0.46 | 0.50 |
| | N | 17 | 14 | 11 | 11 | 8 |
| **13d** | Mean (g) | 17.82 | 18.08 | 18.73 | 19.29 | 19.41 |
| | SEM (g) | 0.27 | 0.26 | 0.30 | 0.30 | 0.42 |
| | N | 17 | 14 | 11 | 11 | 8 |
| **13e** | Mean (g) | 18.23 | 18.14 | 18.59 | 19.06 | 19.11 |
| | SEM (g) | 0.28 | 0.30 | 0.35 | 0.33 | 0.45 |
| | N | 17 | 14 | 11 | 11 | 8 |
| **13f** | Mean (g) | 17.97 | 17.85 | 18.23 | 18.39 | 18.57 |
| | SEM (g) | 0.30 | 0.29 | 0.40 | 0.41 | 0.47 |
| | N | 17 | 13 | 10 | 9 | 7 |
| **13g** | Mean (g) | 18.04 | 17.39 | 18.34 | 19.02 | 18.96 |
| | SEM (g) | 0.21 | 0.27 | 0.28 | 0.40 | 0.40 |
| | N | 17 | 14 | 11 | 9 | 7 |

Resiquimod concentration in plasma samples

| | | Time (days) | | | | | | |
|---|---|---|---|---|---|---|---|---|
| | | 0.25 | 3 | 7 | 9 | 12 | 14 | 16 |
| Group | | Resiquimod (pg/mL) | | | | | | |
| **13d** | Mean | 55.6 | 122 | 81.5 | 67.5 | 19.9 | 57 | ND |
| | SD | 8.1 | 59 | 23 | NC | NC | 8.5 | ND |
| | N | 3 | 3 | 3 | 1 | 1 | 3 | ND |
| | CV% | 14.6 | 48.2 | 27.9 | NC | NC | 14.9 | ND |
| **13e** | Mean | 216 | 383 | 319 | 160 | 155 | ND | 127 |
| | SD | 75 | 230 | 63 | NC | 39 | ND | NC |
| | N | 3 | 3 | 3 | 1 | 2 | ND | 1 |
| | CV% | 34.9 | 59.7 | 19.7 | NC | 25.5 | ND | NC |
| SD = standard deviation, CV% = coefficient of variation, N = sample size, NC = not calculable, ND = not determined | | | | | | | | |

Calculated PK parameters

| Dose | Mean Cmax (pg/ml) | Mean AUC (ng.h/ml) | MRT (hours) |
|---|---|---|---|
| **13d** | 122 (pg/mL) | 35.4 | 287 |
| **13e** | 383 (pg/ml) | 120.4 | 280 |
| MRT: Estimated Mean Residence Time; Estimated Area Under Plasma Concentration- Time Profile, Cmax: estimated maximum Plasma Concentration | | | |

Tumor lysate cytokine levels

| CXCL1 / GROα / KC | | | | |
|---|---|---|---|---|
| | | Hours post-treatment | | |
| Group | | 6 | 72 | 168 |
| **Ac-11b** | Mean (pg/mL) | 332.60 | 640.50 | 727.60 |
| | SEM (pg/mL) | 66.37 | 162.20 | 185.10 |
| | Fold change over **Ac-11b** | 1.00 | 1.00 | 1.00 |
| | N | 3 | 3 | 3 |
| **13e** | Mean (pg/mL) | 1270.00† | 3624.00† | 3339.00† |
| | SEM (pg/mL) | 360.70 | 752.10 | 492.00 |
| | Fold change over **Ac-11b** | 3.82 | 5.66 | 4.59 |
| | N | 3 | 3 | 3 |
| **13f** | Mean (pg/mL) | 875.40 | 3924.00† | 4538.00† |
| | SEM (pg/mL) | 145.20 | 968.60 | 751.00 |
| | Fold change over **Ac-11b** | 2.63 | 6.13 | 6.24 |
| | N | 3 | 3 | 2 |

(continued)

| CXCL1 / GROα / KC | | | | |
|---|---|---|---|---|
| | | Hours post-treatment | | |
| **13g** | Mean (pg/mL) | ND | 1419.00 | 2785.00 |
| | SEM (pg/mL) | ND | 335.40 | 989.40 |
| | Fold change over **Ac-11b** | ND | 2.22 | 3.83 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| IL-1β | | | | |
|---|---|---|---|---|
| | | Hours post-treatment | | |
| Group | | 6 | 72 | 168 |
| **Ac-11b** | Mean (pg/mL) | 30.83 | 23.30 | 17.86 |
| | SEM (pg/mL) | 1.31 | 0.15 | 1.47 |
| | Fold change over **Ac-11b** | 1.00 | 1.00 | 1.00 |
| | N | 3 | 3 | 3 |
| **13e** | Mean (pg/mL) | 132.10 | 54.65 | 53.85† |
| | SEM (pg/mL) | 35.22 | 10.35 | 3.67 |
| | Fold change over **Ac-11b** | 4.28 | 2.35 | 3.02 |
| | N | 3 | 3 | 3 |
| **13f** | Mean (pg/mL) | 119.80 | 64.03† | 69.12† |
| | SEM (pg/mL) | 26.84 | 7.54 | 8.70 |
| | Fold change over **Ac-11b** | 3.89 | 2.75 | 3.87 |
| | N | 3 | 3 | 2 |
| **13g** | Mean (pg/mL) | ND | 56.54 | 59.42† |
| | SEM (pg/mL) | ND | 10.20 | 16.10 |
| | Fold change over **Ac-11b** | ND | 2.43 | 3.33 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| IL-6 | | | | |
|---|---|---|---|---|
| | | Hours post-treatment | | |
| Group | | 6 | 72 | 168 |
| **Ac-11b** | Mean (pg/mL) | 59.01 | 86.57 | 100.10 |
| | SEM (pg/mL) | 1.71 | 19.11 | 18.59 |
| | Fold change over **Ac-11b** | 1.00 | 1.00 | 1.00 |
| | N | 3 | 3 | 3 |

(continued)

| IL-6 | | | | |
|---|---|---|---|---|
| | | Hours post-treatment | | |
| **13e** | Mean (pg/mL) | 755.40 | 270.60 | 286.10† |
| | SEM (pg/mL) | 301.20 | 51.04 | 41.06 |
| | Fold change over **Ac-11b** | 12.80 | 3.13 | 2.86 |
| | N | 3 | 3 | 3 |
| **13f** | Mean (pg/mL) | 744.10 | 523.60† | 82.92 |
| | SEM (pg/mL) | 136.80 | 159.70 | 17.37 |
| | Fold change over **Ac-11b** | 12.61 | 6.05 | 0.83 |
| | N | 3 | 3 | 2 |
| **13g** | Mean (pg/mL) | ND | 128.30 | 99.04 |
| | SEM (pg/mL) | ND | 31.05 | 13.27 |
| | Fold change over **Ac-11b** | ND | 1.48 | 0.99 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| CXCL10 / IP-10 | | | | |
|---|---|---|---|---|
| | | Hours post-treatment | | |
| Group | | 6 | 72 | 168 |
| **Ac-11b** | Mean (pg/mL) | 164.10 | 144.40 | 145.40 |
| | SEM (pg/mL) | 1.28 | 18.04 | 15.02 |
| | Fold change over **Ac-11b** | 1.00 | 1.00 | 1.00 |
| | N | 3 | 3 | 3 |
| **13e** | Mean (pg/mL) | 912.10† | 276.80 | 212.70 |
| | SEM (pg/mL) | 81.34 | 59.89 | 11.13 |
| | Fold change over **Ac-11b** | 5.56 | 1.92 | 1.46 |
| | N | 3 | 3 | 3 |
| **13f** | Mean (pg/mL) | 950.00† | 390.20† | 197.80 |
| | SEM (pg/mL) | 104.00 | 86.74 | 10.13 |
| | Fold change over **Ac-11b** | 5.79 | 2.70 | 1.36 |
| | N | 3 | 3 | 2 |
| **13g** | Mean (pg/mL) | ND | 426.40† | 225.70 |
| | SEM (pg/mL) | ND | 43.68 | 54.55 |
| | Fold change over **Ac-11b** | ND | 2.95 | 1.55 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| CCL2 / MCP-1 | | | | |
|---|---|---|---|---|
| | | Hours post-treatment | | |
| Group | | 6 | 72 | 168 |
| **Ac-11b** | Mean (pg/mL) | 1682.00 | 1619.00 | 1763.00 |
| | SEM (pg/mL) | 89.34 | 98.08 | 136.10 |
| | Fold change over **Ac-11b** | 1.00 | 1.00 | 1.00 |
| | N | 3 | 3 | 3 |
| **13e** | Mean (pg/mL) | 3470.00 | 2738.00† | 2690.00† |
| | SEM (pg/mL) | 533.60 | 185.80 | 260.40 |
| | Fold change over **Ac-11b** | 2.06 | 1.69 | 1.53 |
| | N | 3 | 3 | 3 |
| **13f** | Mean (pg/mL) | 3746.00 | 2891.00† | 2603.00 |
| | SEM (pg/mL) | 767.80 | 93.48 | 74.97 |
| | Fold change over **Ac-11b** | 2.23 | 1.79 | 1.48 |
| | N | 3 | 3 | 2 |
| **13g** | Mean (pg/mL) | ND | 2658.00† | 2457.00 |
| | SEM (pg/mL) | ND | 148.30 | 103.70 |
| | Fold change over **Ac-11b** | ND | 1.64 | 1.39 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| CCL3 / MIP-1α | | | | |
|---|---|---|---|---|
| | | Hours post-treatment | | |
| Group | | 6 | 72 | 168 |
| **Ac-11b** | Mean (pg/mL) | 475.30 | 525.20 | 518.50 |
| | SEM (pg/mL) | 44.13 | 33.93 | 31.84 |
| | Fold change over **Ac-11b** | 1.00 | 1.00 | 1.00 |
| | N | 3 | 3 | 3 |
| **13e** | Mean (pg/mL) | 1214.00 | 840.50 | 862.40 |
| | SEM (pg/mL) | 214.40 | 111.00 | 45.95 |
| | Fold change over **Ac-11b** | 2.55 | 1.60 | 1.66 |
| | N | 3 | 3 | 3 |
| **13f** | Mean (pg/mL) | 1260.00† | 1209.00† | 1131.00† |
| | SEM (pg/mL) | 244.50 | 214.30 | 118.40 |
| | Fold change over **Ac-11b** | 2.65 | 2.30 | 2.18 |
| | N | 3 | 3 | 2 |

(continued)

| CCL3 / MIP-1α | | | | |
|---|---|---|---|---|
| | | Hours post-treatment | | |
| Group | | 6 | 72 | 168 |
| **13g** | Mean (pg/mL) | ND | 1099.00† | 970.80† |
| | SEM (pg/mL) | ND | 62.99 | 212.50 |
| | Fold change over **Ac-11b** | ND | 2.09 | 1.87 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| CCL4 / MIP-1 β | | | | |
|---|---|---|---|---|
| | | Hours post-treatment | | |
| Group | | 6 | 72 | 168 |
| **Ac-11b** | Mean (pg/mL) | 160.50 | 142.20 | 99.26 |
| | SEM (pg/mL) | 10.87 | 24.60 | 13.86 |
| | Fold change over **Ac-11b** | 1.00 | 1.00 | 1.00 |
| | N | 3 | 3 | 3 |
| **13e** | Mean (pg/mL) | 1105.00 | 339.00 | 265.80 |
| | SEM (pg/mL) | 469.40 | 94.18 | 57.35 |
| | Fold change over **Ac-11b** | 6.88 | 2.38 | 2.68 |
| | N | 3 | 3 | 3 |
| **13f** | Mean (pg/mL) | 1031.00 | 432.00 | 458.50† |
| | SEM (pg/mL) | 388.30 | 58.90 | 67.76 |
| | Fold change over **Ac-11b** | 6.42 | 3.04 | 4.62 |
| | N | 3 | 3 | 2 |
| **13g** | Mean (pg/mL) | ND | 561.40† | 357.10 |
| | SEM (pg/mL) | ND | 106.60 | 138.10 |
| | Fold change over **Ac-11b** | ND | 3.95 | 3.60 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| CXCL2 / MIP-2α | | | | |
|---|---|---|---|---|
| | | Hours post-treatment | | |
| Group | | 6 | 72 | 168 |
| **Ac-11b** | Mean (pg/mL) | 188.00 | 548.30 | 1140.00 |
| | SEM (pg/mL) | 56.39 | 124.40 | 172.90 |
| | Fold change over **Ac-11b** | 1.00 | 1.00 | 1.00 |
| | N | 3 | 3 | 3 |

(continued)

| CXCL2 / MIP-2α | | | | |
|---|---|---|---|---|
| | | Hours post-treatment | | |
| Group | | 6 | 72 | 168 |
| **13e** | Mean (pg/mL) | 1122.00 | 1910.00 | 3144.00† |
| | SEM (pg/mL) | 365.00 | 439.60 | 84.67 |
| | Fold change over **Ac-11b** | 5.97 | 3.48 | 2.76 |
| | N | 3 | 3 | 3 |
| **13f** | Mean (pg/mL) | 921.00 | 2471.00† | 2884.00† |
| | SEM (pg/mL) | 299.70 | 564.80 | 285.20 |
| | Fold change over **Ac-11b** | 4.90 | 4.51 | 2.53 |
| | N | 3 | 3 | 2 |
| **13g** | Mean (pg/mL) | ND | 2570.00† | 2723.00† |
| | SEM (pg/mL) | ND | 221.20 | 317.60 |
| | Fold change over **Ac-11b** | ND | 4.69 | 2.39 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| TNFα | | | | |
|---|---|---|---|---|
| | | Hours post-treatment | | |
| Group | | 6 | 72 | 168 |
| **Ac-11b** | Mean (pg/mL) | 16.77 | 10.32 | 8.44 |
| | SEM (pg/mL) | 2.88 | 0.61 | 0.91 |
| | Fold change over **Ac-11b** | 1.00 | 1.00 | 1.00 |
| | N | 3 | 3 | 3 |
| **13e** | Mean (pg/mL) | 122.30 | 43.68 | 39.25 |
| | SEM (pg/mL) | 17.09 | 3.91 | 4.16 |
| | Fold change over **Ac-11b** | 7.29 | 4.23 | 4.65 |
| | N | 3 | 3 | 3 |
| **13f** | Mean (pg/mL) | 139.70† | 102.60 | 68.57† |
| | SEM (pg/mL) | 48.51 | 53.71 | 14.95 |
| | Fold change over **Ac-11b** | 8.33 | 9.94 | 8.12 |
| | N | 3 | 3 | 2 |
| **13g** | Mean (pg/mL) | ND | 57.06 | 33.27 |
| | SEM (pg/mL) | ND | 3.84 | 12.87 |
| | Fold change over **Ac-11b** | ND | 5.53 | 3.94 |
| | N | NA | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

Tumor gene expression

| *Ccl2* | | | | |
|---|---|---|---|---|
| | | Hours post-treatment | | |
| Group | | 6 | 72 | 168 |
| **Ac-11b** | Mean (2^ΔΔCT) | 1.02 | 1.00 | 1.01 |
| | SEM (2^ΔΔCT) | 0.13 | 0.04 | 0.10 |
| | Fold change over **Ac-11b** | 1.00 | 1.00 | 1.00 |
| | N | 3 | 3 | 3 |
| **13e** | Mean (2^ΔΔCT) | 4.76 | 1.76 | 1.33 |
| | SEM (2^ΔΔCT) | 2.33 | 0.40 | 0.12 |
| | Fold change over **Ac-11b** | 4.68 | 1.76 | 1.32 |
| | N | 3 | 3 | 3 |
| **13f** | Mean (2^ΔΔCT) | 5.12 | 1.43 | 1.44 |
| | SEM (2^ΔΔCT) | 1.61 | 0.25 | 0.57 |
| | Fold change over **Ac-11b** | 5.04 | 1.43 | 1.42 |
| | N | 3 | 3 | 2 |
| **13g** | Mean (2^ΔΔCT) | 7.14 | 2.00† | 2.31† |
| | SEM (2^ΔΔCT) | 1.67 | 0.11 | 0.27 |
| | Fold change over **Ac-11b** | 7.02 | 2.00 | 2.28 |
| | N | 3 | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Ccl3* | | | | |
|---|---|---|---|---|
| | | Hours post-treatment | | |
| Group | | 6 | 72 | 168 |
| **Ac-11b** | Mean (2^ΔΔCT) | 1.01 | 1.01 | 1.11 |
| | SEM (2^ΔΔCT) | 0.12 | 0.08 | 0.37 |
| | Fold change over **Ac-11b** | 1.00 | 1.00 | 1.00 |
| | N | 3 | 3 | 3 |
| **13e** | Mean (2^ΔΔCT) | 9.81 | 2.78 | 1.17 |
| | SEM (2^ΔΔCT) | 5.42 | 0.95 | 0.24 |
| | Fold change over **Ac-11b** | 9.68 | 2.76 | 1.05 |
| | N | 3 | 3 | 3 |
| **13f** | Mean (2^ΔΔCT) | 7.26 | 12.36 | 5.05 |
| | SEM (2^ΔΔCT) | 2.88 | 10.11 | 1.43 |
| | Fold change over **Ac-11b** | 7.17 | 12.27 | 4.54 |
| | N | 3 | 3 | 2 |

(continued)

| *Ccl3* | | | | |
|---|---|---|---|---|
| | | Hours post-treatment | | |
| Group | | 6 | 72 | 168 |
| **13g** | Mean (2^ΔΔCT) | 8.64 | 5.15 | 7.74† |
| | SEM (2^ΔΔCT) | 2.19 | 0.76 | 2.25 |
| | Fold change over **Ac-11b** | 8.53 | 5.11 | 6.95 |
| | N | 3 | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Ccl4* | | | | |
|---|---|---|---|---|
| | | Hours post-treatment | | |
| Group | | 6 | 72 | 168 |
| **Ac-11b** | Mean (2^ΔΔCT) | 1.01 | 1.04 | 1.09 |
| | SEM (2^ΔΔCT) | 0.09 | 0.23 | 0.33 |
| | Fold change over **Ac-11b** | 1.00 | 1.00 | 1.00 |
| | N | 3 | 3 | 3 |
| **13e** | Mean (2^ΔΔCT) | 6.93 | 1.54 | 0.68 |
| | SEM (2^ΔΔCT) | 3.49 | 0.42 | 0.10 |
| | Fold change over **Ac-11b** | 6.88 | 1.47 | 0.62 |
| | N | 3 | 3 | 3 |
| **13f** | Mean (2^ΔΔCT) | 5.42 | 5.49 | 2.37 |
| | SEM (2^ΔΔCT) | 2.36 | 4.41 | 0.68 |
| | Fold change over **Ac-11b** | 5.39 | 5.26 | 2.16 |
| | N | 3 | 3 | 2 |
| **13g** | Mean (2^ΔΔCT) | 8.91 | 1.95 | 2.68 |
| | SEM (2^ΔΔCT) | 2.37 | 0.24 | 0.75 |
| | Fold change over **Ac-11b** | 8.85 | 1.87 | 2.45 |
| | N | 3 | 3 | 2 |
| SEM = standard error of the mean, N = sample size, ND = not determined, NA = not applicable | | | | |

| *Ccl5* | | | | |
|---|---|---|---|---|
| | | Hours post-treatment | | |
| Group | | 6 | 72 | 168 |
| **Ac-11b** | Mean (2^ΔΔCT) | 1.01 | 1.09 | 1.08 |
| | SEM (2^ΔΔCT) | 0.10 | 0.32 | 0.27 |
| | Fold change over **Ac-11b** | 1.00 | 1.00 | 1.00 |
| | N | 3 | 3 | 3 |

(continued)

| *Ccl5* | | | | |
|---|---|---|---|---|
| | | Hours post-treatment | | |
| Group | | 6 | 72 | 168 |
| **13e** | Mean (2^ΔΔCT) | 12.58 | 2.13 | 1.08 |
| | SEM (2^ΔΔCT) | 7.87 | 0.89 | 0.20 |
| | Fold change over **Ac-11b** | 12.46 | 1.96 | 1.00 |
| | N | 3 | 3 | 3 |
| **13f** | Mean (2^ΔΔCT) | 15.64 | 7.50 | 3.92† |
| | SEM (2^ΔΔCT) | 7.95 | 5.77 | 1.16 |
| | Fold change over **Ac-11b** | 15.49 | 6.90 | 3.63 |
| | N | 3 | 3 | 2 |
| **13g** | Mean (2^ΔΔCT) | 22.13 | 3.98 | 3.05 |
| | SEM (2^ΔΔCT) | 5.66 | 0.68 | 1.10 |
| | Fold change over **Ac-11b** | 21.91 | 3.66 | 2.82 |
| | N | 3 | 3 | 2 |
| SEM = standard error of the mean, N = sample size, ND = not determined, NA = not applicable; †$p<0.05$ vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Csf2* | | | | |
|---|---|---|---|---|
| | | Hours post-treatment | | |
| Group | | 6 | 72 | 168 |
| **Ac-11b** | Mean (2^ΔΔCT) | 1.00 | 1.31 | 1.32 |
| | SEM (2^ΔΔCT) | 0.05 | 0.68 | 0.58 |
| | Fold change over **Ac-11b** | 1.00 | 1.00 | 1.00 |
| | N | 3 | 3 | 3 |
| **13e** | Mean (2^ΔΔCT) | 6.99 | 2.14 | 0.56 |
| | SEM (2^ΔΔCT) | 4.27 | 0.56 | 0.17 |
| | Fold change over **Ac-11b** | 6.97 | 1.64 | 0.43 |
| | N | 3 | 3 | 3 |
| **13f** | Mean (2^ΔΔCT) | 5.27 | 2.61 | 0.23 |
| | SEM (2^ΔΔCT) | 1.68 | 0.89 | 0.04 |
| | Fold change over **Ac-11b** | 5.25 | 2.00 | 0.17 |
| | N | 3 | 3 | 2 |
| **13g** | Mean (2^ΔΔCT) | 5.24 | 0.53 | 0.45 |
| | SEM (2^ΔΔCT) | 0.43 | 0.07 | 0.02 |
| | Fold change over **Ac-11b** | 5.22 | 0.41 | 0.34 |
| | N | 3 | 3 | 2 |
| SEM = standard error of the mean, N = sample size, ND = not determined, NA = not applicable | | | | |

| *Cxcl1* | | | | |
|---|---|---|---|---|
| | | Hours post-treatment | | |
| Group | | 6 | 72 | 168 |
| **Ac-11b** | Mean (2^ΔΔCT) | 1.00 | 1.33 | 1.47 |
| | SEM (2^ΔΔCT) | 0.03 | 0.71 | 0.64 |
| | Fold change over **Ac-11b** | 1.00 | 1.00 | 1.00 |
| | N | 3 | 3 | 3 |
| **13e** | Mean (2^ΔΔCT) | 2.00 | 5.28 | 1.03 |
| | SEM (2^ΔΔCT) | 1.00 | 1.86 | 0.50 |
| | Fold change over **Ac-11b** | 2.00 | 3.97 | 0.70 |
| | N | 3 | 3 | 3 |
| **13f** | Mean (2^ΔΔCT) | 1.23 | 2.08 | 0.42 |
| | SEM (2^ΔΔCT) | 0.30 | 0.96 | 0.02 |
| | Fold change over **Ac-11b** | 1.23 | 1.57 | 0.28 |
| | N | 3 | 3 | 2 |
| **13g** | Mean (2^ΔΔCT) | 3.74† | 0.70 | 0.77 |
| | SEM (2^ΔΔCT) | 0.41 | 0.14 | 0.25 |
| | Fold change over **Ac-11b** | 3.74 | 0.53 | 0.52 |
| | N | 3 | 3 | 2 |
| *Cxcl2* | | | | |
| | | Hours post-treatment | | |
| Group | | 6 | 72 | 168 |
| **Ac-11b** | Mean (2^ΔΔCT) | 1.08 | 1.10 | 1.31 |
| | SEM (2^ΔΔCT) | 0.28 | 0.33 | 0.64 |
| | Fold change over **Ac-11b** | 1.00 | 1.00 | 1.00 |
| | N | 3 | 3 | 3 |
| **13e** | Mean (2^ΔΔCT) | 4.94 | 4.00 | 1.03 |
| | SEM (2^ΔΔCT) | 2.06 | 1.50 | 0.24 |
| | Fold change over **Ac-11b** | 4.58 | 3.63 | 0.78 |
| | N | 3 | 3 | 3 |
| **13f** | Mean (2^ΔΔCT) | 2.66 | 13.18 | 3.91 |
| | SEM (2^ΔΔCT) | 0.61 | 10.73 | 1.26 |
| | Fold change over **Ac-11b** | 2.47 | 11.98 | 2.98 |
| | N | 3 | 3 | 2 |

(continued)

| *Cxcl2* | | | | |
|---|---|---|---|---|
| | | Hours post-treatment | | |
| Group | | 6 | 72 | 168 |
| **13g** | Mean (2^ΔΔCT) | 3.33 | 4.12 | 8.49† |
| | SEM (2^ΔΔCT) | 0.78 | 1.07 | 2.66 |
| | Fold change over **Ac-11b** | 3.09 | 3.75 | 6.48 |
| | N | 3 | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Cxcll0* | | | | |
|---|---|---|---|---|
| | | Hours post-treatment | | |
| Group | | 6 | 72 | 168 |
| **Ac-11b** | Mean (2^ΔΔCT) | 1.02 | 1.01 | 1.01 |
| | SEM (2^ΔΔCT) | 0.15 | 0.09 | 0.11 |
| | Fold change over **Ac-11b** | 1.00 | 1.00 | 1.00 |
| | N | 3 | 3 | 3 |
| **13e** | Mean (2^ΔΔCT) | 29.23 | 3.24 | 2.09 |
| | SEM (2^ΔΔCT) | 16.71 | 0.85 | 0.53 |
| | Fold change over **Ac-11b** | 28.57 | 3.21 | 2.07 |
| | N | 3 | 3 | 3 |
| **13f** | Mean (2^ΔΔCT) | 31.41 | 3.83† | 1.92 |
| | SEM (2^ΔΔCT) | 13.05 | 1.05 | 0.31 |
| | Fold change over **Ac-11b** | 30.70 | 3.81 | 1.90 |
| | N | 3 | 3 | 2 |
| **13g** | Mean (2^ΔΔCT) | 32.96 | 2.15 | 2.29 |
| | SEM (2^ΔΔCT) | 6.33 | 0.29 | 0.43 |
| | Fold change over **Ac-11b** | 32.22 | 2.14 | 2.27 |
| | N | 3 | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Il1b* | | | | |
|---|---|---|---|---|
| | | Hours post-treatment | | |
| Group | | 6 | 72 | 168 |
| **Ac-11b** | Mean (2^ΔΔCT) | 1.02 | 1.00 | 1.10 |
| | SEM (2^ΔΔCT) | 0.13 | 0.05 | 0.34 |
| | Fold change over **Ac-11b** | 1.00 | 1.00 | 1.00 |
| | N | 3 | 3 | 3 |
| **13e** | Mean (2^ΔΔCT) | 6.50 | 5.43 | 2.26 |
| | SEM (2^ΔΔCT) | 4.23 | 2.10 | 0.54 |
| | Fold change over **Ac-11b** | 6.39 | 5.41 | 2.06 |
| | N | 3 | 3 | 3 |
| **13f** | Mean (2^ΔΔCT) | 2.82 | 6.79 | 0.27 |
| | SEM (2^ΔΔCT) | 1.14 | 4.11 | 0.27 |
| | Fold change over **Ac-11b** | 2.77 | 6.77 | 0.25 |
| | N | 3 | 3 | 2 |
| **13g** | Mean (2^ΔΔCT) | 14.22† | 0.65 | 0.46 |
| | SEM (2^ΔΔCT) | 2.71 | 0.26 | 0.11 |
| | Fold change over **Ac-11b** | 13.98 | 0.65 | 0.41 |
| | N | 3 | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Il6* | | | | |
|---|---|---|---|---|
| | | Hours post-treatment | | |
| Group | | 6 | 72 | 168 |
| **Ac-11b** | Mean (2^ΔΔCT) | 1.02 | 1.24 | 1.45 |
| | SEM (2^ΔΔCT) | 0.16 | 0.58 | 0.70 |
| | Fold change over **Ac-11b** | 1.00 | 1.00 | 1.00 |
| | N | 3 | 3 | 3 |
| **13e** | Mean (2^ΔΔCT) | 6.25 | 2.77 | 1.31 |
| | SEM (2^ΔΔCT) | 4.00 | 0.88 | 0.49 |
| | Fold change over **Ac-11b** | 6.13 | 2.24 | 0.90 |
| | N | 3 | 3 | 3 |
| **13f** | Mean (2^ΔΔCT) | 6.68 | 4.54 | 4.31 |
| | SEM (2^ΔΔCT) | 1.98 | 1.41 | 3.78 |
| | Fold change over **Ac-11b** | 6.55 | 3.67 | 2.98 |
| | N | 3 | 3 | 2 |

(continued)

| *Il6* | | | | |
|---|---|---|---|---|
| | | Hours post-treatment | | |
| Group | | 6 | 72 | 168 |
| **13g** | Mean (2^ΔΔCT) | 5.05 | 0.98 | 0.60 |
| | SEM (2^ΔΔCT) | 0.93 | 0.25 | 0.15 |
| | Fold change over **Ac-11b** | 4.95 | 0.79 | 0.41 |
| | N | 3 | 3 | 2 |
| *Il10* | | | | |
| | | Hours post-treatment | | |
| Group | | 6 | 72 | 168 |
| **Ac-11b** | Mean (2^ΔΔCT) | 1.04 | 1.31 | 1.36 |
| | SEM (2^ΔΔCT) | 0.20 | 0.67 | 0.57 |
| | Fold change over **Ac-11b** | 1.00 | 1.00 | 1.00 |
| | N | 3 | 3 | 3 |
| **13e** | Mean (2^ΔΔCT) | 1.58 | 0.81 | 1.29 |
| | SEM (2^ΔΔCT) | 0.38 | 0.03 | 0.50 |
| | Fold change over **Ac-11b** | 1.52 | 0.62 | 0.94 |
| | N | 3 | 3 | 3 |
| **13f** | Mean (2^ΔΔCT) | 4.89† | 4.01 | 1.06 |
| | SEM (2^ΔΔCT) | 0.54 | 1.57 | 0.45 |
| | Fold change over **Ac-11b** | 4.70 | 3.06 | 0.78 |
| | N | 3 | 3 | 2 |
| **13g** | Mean (2^ΔΔCT) | 1.69 | 2.06 | 0.72 |
| | SEM (2^ΔΔCT) | 0.21 | 0.74 | 0.35 |
| | Fold change over **Ac-11b** | 1.63 | 1.57 | 0.53 |
| | N | 3 | 3 | 2 |
| SEM = standard error of the mean, N = sample size, ND = not determined, NA = not applicable<br>SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Il18* | | | | |
|---|---|---|---|---|
| | | Hours post-treatment | | |
| Group | | 6 | 72 | 168 |
| **Ac-11b** | Mean (2^ΔΔCT) | 1.01 | 1.15 | 1.21 |
| | SEM (2^ΔΔCT) | 0.09 | 0.45 | 0.43 |
| | Fold change over **Ac-11b** | 1.00 | 1.00 | 1.00 |
| | N | 3 | 3 | 3 |

(continued)

| *Il18* | | | | |
|---|---|---|---|---|
| | | Hours post-treatment | | |
| Group | | 6 | 72 | 168 |
| **13e** | Mean (2^ΔΔCT) | 2.14 | 0.94 | 0.97 |
| | SEM (2^ΔΔCT) | 0.56 | 0.14 | 0.31 |
| | Fold change over **Ac-11b** | 2.12 | 0.82 | 0.80 |
| | N | 3 | 3 | 3 |
| **13f** | Mean (2^ΔΔCT) | 4.45† | 3.27 | 0.53 |
| | SEM (2^ΔΔCT) | 0.83 | 1.71 | 0.38 |
| | Fold change over **Ac-11b** | 4.40 | 2.85 | 0.43 |
| | N | 3 | 3 | 2 |
| **13g** | Mean (2^ΔΔCT) | 2.58 | 1.78 | 0.72 |
| | SEM (2^ΔΔCT) | 0.47 | 0.92 | 0.19 |
| | Fold change over **Ac-11b** | 2.56 | 1.55 | 0.59 |
| | N | 3 | 3 | 2 |
| SEM = standard error of the mean, N = sample size, ND = not determined, NA = not applicable; †p<0.05 vs **Ac-11b** at the same timepoint. Significance was determined by One-way ANOVA followed by treatment group comparisons against **Ac-11b** treated controls for every time point using Dunnett's multiple comparisons post-hoc test. | | | | |

| *Tnf* | | | | |
|---|---|---|---|---|
| | | Hours post-treatment | | |
| Group | | 6 | 72 | 168 |
| **Ac-11b** | Mean (2^ΔΔCT) | 1.03 | 1.18 | 1.20 |
| | SEM (2^ΔΔCT) | 0.17 | 0.49 | 0.44 |
| | Fold change over **Ac-11b** | 1.00 | 1.00 | 1.00 |
| | N | 3 | 3 | 3 |
| **13e** | Mean (2^ΔΔCT) | 4.63 | 1.25 | 0.73 |
| | SEM (2^ΔΔCT) | 2.75 | 0.28 | 0.15 |
| | Fold change over **Ac-11b** | 4.51 | 1.06 | 0.61 |
| | N | 3 | 3 | 3 |
| **13f** | Mean (2^ΔΔCT) | 3.33 | 3.87 | 2.19 |
| | SEM (2^ΔΔCT) | 0.92 | 2.48 | 0.09 |
| | Fold change over **Ac-11b** | 3.25 | 3.28 | 1.83 |
| | N | 3 | 3 | 2 |
| **13g** | Mean (2^ΔΔCT) | 3.36 | 4.78 | 2.76 |
| | SEM (2^ΔΔCT) | 0.57 | 1.40 | 1.40 |
| | Fold change over **Ac-11b** | 3.27 | 4.05 | 2.30 |
| | N | 3 | 3 | 2 |
| SEM = standard error of the mean, N = sample size, ND = not determined, NA = not applicable | | | | |

**Example 14: In vivo WT IL-2 combination abscopal tumor efficacy and tumor rechallenge**

The study was conducted in female BALB/C mice with an age of 6-8 weeks at the day of tumor inoculation. Mice were implanted with 5x10$^5$ CT26 tumor cells into the left and right flanks. When right flank tumors were grown to a mean tumor volume of ~101 mm$^3$, mice were randomized into treatment cohorts (day 0). On the same day of randomization, animals received **13h** as a single intratumoral dose in an injection volume of 50 $\mu$L or a single intratumoral injection of 50 $\mu$L of a suspension of **Ac-11b,** in the right flank tumors. Hydrogels were administered as suspension in PTP buffer. Some cohorts were further treated with 20 $\mu$g human IL-2 (Peprotech, Rocky Hill, NJ), intraperitoneally (I.P.), twice a day for 5 days, followed by a 3-day dose holiday, then further treated with 20 ug human IL-2 I.P. once a day for 5 additional days. Following treatment initiation, anti-tumor efficacy was assessed by determination of tumor volumes at various time points from tumor size measurements with a caliper. Tumor volumes were calculated according to the formula:

$$\text{Tumor volume} = (L \times W^2) \times 0.5$$

where L is the length of the tumor and W the width (both in mm).

3 out of 7 mice that were treated with both **13h** and human IL-2 experienced complete regressions in both treated and untreated tumors and were reimplanted with 5x10$^5$ CT26 tumor cells in their right front flank ~60 days after initial treatment. Following reimplantation, mice were monitored for signs of tumor growth at the newly implanted site. Naive female BALB/C mice were also implanted with the same tumor on the same day as the reimplanted mice as naïve control mice for normal tumor growth comaprisons. Tumor growth was assessed by determination of tumor volumes at various time points following implantation from tumor size measurements with a caliper and calculated according to the formula:

$$\text{Tumor volume} = (L \times W^2) \times 0.5$$

where L is the length of the tumor and W the width (both in mm). No tumor growth was observed in mice that were treated with both **13h** and human IL-2 ~60 days earlier at the end of the study period.

Results:

Absolute tumor volumes (mm$^3$) of injected right flank tumors

| | | Days post-treatment | | | | | | | |
|---|---|---|---|---|---|---|---|---|---|
| Group | | 0 | 2 | 5 | 7 | 9 | 12 | 14 | 16 |
| **Ac-11b** | Mean (mm$^3$) | 101.57 | 222.96 | 390.09 | 676.66 | 975.86 | 1460.78 | 1836.12 | 2271.84 |
| | SEM (mm$^3$) | 2.86 | 14.07 | 36.74 | 84.40 | 88.75 | 106.17 | 122.25 | 101.39 |
| | N | 10 | 10 | 10 | 10 | 7 | 7 | 7 | 7 |
| **13h** | Mean (mm$^3$) | 101.70 | 147.86 | 222.54 | 359.25† | 503.92† | 691.12† | 864.10† | 1354.10† |
| | SEM (mm$^3$) | 3.09 | 5.89 | 18.62 | 38.62 | 63.80 | 101.80 | 135.92 | 149.33 |
| | N | 10 | 10 | 10 | 10 | 7 | 7 | 7 | 7 |
| **Ac11b** + human IL-2 | Mean (mm$^3$) | 101.79 | 144.70 | 210.24 | 311.11† | 413.93† | 539.45† | 659.38† | 856.24†,‡ |
| | SEM (mm$^3$) | 2.94 | 3.09 | 14.94 | 41.16 | 54.23 | 68.25 | 93.21 | 131.97 |
| | N | 10 | 10 | 10 | 10 | 7 | 7 | 7 | 7 |

(continued)

| | | Days post-treatment | | | | | | | |
|---|---|---|---|---|---|---|---|---|---|
| Group | | 0 | 2 | 5 | 7 | 9 | 12 | 14 | 16 |
| **13h** + human IL-2 | Mean ($mm^3$) | 101.79 | 135.21 | 161.22† | 183.69† | 228.72†-,‡ | 247.55†,‡-,†† | 255.14†,‡-,†† | 288.10†,‡-,†† |
| | **SEM** ($mm^3$) | 2.99 | 6.19 | 12.56 | 26.42 | 45.45 | 59.32 | 67.52 | 90.98 |
| SEM = standard error of the mean, N = sample size; †p<0.03 vs **Ac-11b,** ‡p<0.03 vs **13h,** ††p<0.02 vs **Ac-11b** + human IL-2. Significance was determined by Two-way ANOVA followed by multiple comparisons using Tukey's Honest Significant Differences (HSD) post-hoc test. | | | | | | | | | |

Absolute tumor volumes ($mm^3$) of uninjected left flank tumors

| | | Days post-treatment | | | | | | | |
|---|---|---|---|---|---|---|---|---|---|
| Group | | 0 | 2 | 5 | 7 | 9 | 12 | 14 | 16 |
| **Ac-11b** | Mean ($mm^3$) | 94.45 | 192.41 | 327.64 | 583.05 | 769.07 | 1192.25 | 1644.95 | 2223.11 |
| | SEM ($mm^3$) | 4.84 | 19.38 | 38.05 | 89.15 | 86.70 | 118.05 | 137.96 | 166.70 |
| | N | 10 | 10 | 10 | 10 | 7 | 7 | 7 | 7 |
| **13h** | Mean ($mm^3$) | 98.95 | 144.19 | 220.17 | 432.34 | 631.58 | 948.54 | 1239.77† | 1854.24† |
| | SEM ($mm^3$) | 4.08 | 5.30 | 21.21 | 43.94 | 63.59 | 97.86 | 136.23 | 187.27 |
| | N | 10 | 10 | 10 | 10 | 7 | 7 | 7 | 7 |
| **Ac-11b** + human IL-2 | Mean ($mm^3$) | 99.01 | 136.38 | 199.08 | 313.92 | 420.65† | 542.96†-,‡ | 718.21†-,‡ | 995.12†,‡ |
| | SEM ($mm^3$) | 8.07 | 8.19 | 17.95 | 46.42 | 58.92 | 86.36 | 135.03 | 219.23 |
| | N | 10 | 10 | 10 | 10 | 7 | 7 | 7 | 7 |
| **13h+** human IL-2 | Mean ($mm^3$) | 92.48 | 121.70 | 139.10 | 177.79† | 296.62†-,‡ | 347.49†-,‡ | 411.71†-,‡ | 484.22†,‡-,†† |
| | SEM ($mm^3$) | 4.47 | 9.59 | 13.12 | 34.35 | 82.23 | 101.27 | 139.26 | 165.58 |
| SEM = standard error of the mean, N = sample size; †p<0.002 vs **Ac-11b,** ‡p<0.04 vs **13h,** ††p<0.0003 vs **Ac-11b** + human IL-2. Significance was determined by Two-way ANOVA followed by multiple comparisons using Tukey's Honest Significant Differences (HSD) post-hoc test. | | | | | | | | | |

Absolute tumor volumes ($mm^3$) of reimplanted and newly implanted mice

| | | Days post-CT26 implantation | | | | | | | |
|---|---|---|---|---|---|---|---|---|---|
| Group | | 0 | 3 | 7 | 10 | 14 | 17 | 21 | 24 |
| Naive control mice | Mean ($mm^3$) | 0 | 0 | 17.94 | 87.77 | 444.76 | 672.99 | 1622.95 | 2024.37 |
| | SEM ($mm^3$) | 0 | 0 | 4.16 | 7.81 | 26.22 | 59.35 | 127.86 | 129.16 |
| | N | 10 | 10 | 10 | 10 | 10 | 10 | 10 | 10 |

(continued)

| | | Days post-CT26 implantation | | | | | | | |
|---|---|---|---|---|---|---|---|---|---|
| Group | | 0 | 3 | 7 | 10 | 14 | 17 | 21 | 24 |
| Reimplanted: **13h** + human IL-2 | Mean ($mm^3$) | 0 | 13.61 | 0 | 0 | 0 | 0 | 0 | 0 |
| | SEM ($mm^3$) | 0 | 13.61 | 0 | 0 | 0 | 0 | 0 | 0 |
| | N | 3 | 3 | 3 | 3 | 3 | 3 | 3 | 3 |
| SEM = standard error of the mean, N = sample size | | | | | | | | | |

**Example 15: Flow cytometric profiling of tumor draining immune cells**

The study was conducted in female BALB/C mice with an age of 6-8 weeks at the day of tumor inoculation. Mice were implanted with $5\times10^5$ CT26 tumor cells into the left and right flanks. When right flank tumors were grown to a mean tumor volume of ~101 $mm^3$, mice were randomized into treatment cohorts (day 0). On the same day of randomization, animals received a single dose of either 141 μg of resiquimod **4** (dissolved in10 mM succinate, 90.0 mg/mL trehalose dihydrate, pH 5.0), **13h** as a single intratumoral dose in an injection volume of 50 μL, or a single intratumoral injection of 50 μL of a suspension of **Ac-11b,** in the right flank tumors. Hydrogels were administered as suspension in PTP buffer. Some cohorts were further treated with 20 μg human IL-2 (Peprotech, Rocky Hill, NJ), intraperitoneally (I.P.), twice a day for 5 days. Mice were sacrificed 7 days after randomization (D0). Following sacrifice, tumor draining lymph nodes were isolated from both flanks and were dissociated mechanically to generate a single cell suspension at a cell concentration of $1 \times 10^6$ cells per sample. Cell suspensions were centrifuged at 300 g for 5 minutes. Supernatants were discarded and cells were resuspended in FACS buffer with 1 μg/ml Fc-Block and incubated at 4 °C for 10 minutes in the dark. Surface marker antibody mixtures (antibody concentration: 10 μg/mL) in FACS buffer were added to each sample and samples were incubated in the dark at 4°C for 30 minutes. Cells were centrifuged at 300 g for 5 minutes and supernatants were discarded. Cells were washed and then resuspended with FACS buffer before cytometer collection.

Summary of antibodies used for FACS profiling

| Markers | Fluorochrome | Clone | isotype |
|---|---|---|---|
| CD45 | BUV661 | 30-F11 | Rat IgG2b , κ |
| CD3 | BUV395 | 17A2 | Rat IgG2b , κ |
| CD4 | BV421 | GK1.5 | Rat IgG2b , κ |
| CD8 | PE-eFluor610 | 53-6.7 | Rat IgG2a , κ |
| CD335 | BV605 | 29A1.4 | Rat IgG2a, κ |
| I-A/I-E (MHCII) | BB515 | 2G9 | Rat IgG2a , κ |
| Ly-6C | APC | HK1.4 | Rat IgG2c, κ |
| L/D | eFluor780 | - | - |

After collection, FACS data was analyzed using FlowJo Version 10.6.1. Compensation was digitially adjusted using single antibody-stained beads. Samples with less than 90% viability, as determined by LiveDead cell staining, were excluded from the analysis. Cells were defined using the following gating strategy:

1) Ly-$6C^+$ antigen presenting cells: FSC-H/FSC-A Singlets/$LiveDead^-$/$CD45^+$/$CD3^-$ /$CD335^-$/Ly-$6C^+$
2) Ly-$6C^+$ $MHCII^+$ antigen presenting cells: FSC-H/FSC-A Singlets/$LiveDead^-$ /$CD45^+$/$CD3^-$/$CD335^-$/Ly-$6C^+$/IA/IE $(MHCII)^+$
3) $CD8^+$ T cells: FSC-H/FSC-A Singlets/$LiveDead^-$/$CD45^+$/$CD3^+$/CD8 single positive
4) Ly-$6C^+$ $CD8^+$ T cells: FSC-H/FSC-A Singlets/$LiveDead^-$/$CD45^+$/$CD3^+$/CD8 single positive/Ly-$6C^+$

Results:

Frequency of Ly-6C+ antigen presenting cells of non-T cells

| | | Tumor | |
|---|---|---|---|
| Group | | Injected, right flank | Uninjected, left flank |
| 4 | Mean (%) | 2.5 | 7.8 |
| | SEM (%) | 0.36 | 2.1 |
| | N | 3 | 2 |
| **Ac-11b** | Mean (%) | 2.27 | 4.21 |
| | SEM (%) | 0.40 | 0.68 |
| | N | 3 | 2 |
| **13h** | Mean (%) | 15.43†, ‡ | 25.23 |
| | SEM (%) | 5.22 | 7.91 |
| | N | 3 | 3 |
| **Ac-11b** + human IL-2 | Mean (%) | 3.49†† | 7.69 |
| | SEM (%) | 0.27 | 3.21 |
| | N | 3 | 2 |
| **13h+** human IL-2 | Mean (%) | 48.5†, ‡, ††, ‡‡ | 31 |
| | SEM (%) | 1 | 4.34 |
| | N | 2 | 3 |
| SEM = standard error of the mean, N = sample size; Injected tumors: †p<0.03 vs **Ac-11b**, ‡p<0.04 vs **4**, ††p<0.05 vs **13h**, ‡‡p<0.0001 vs **Ac-11b** + human IL-2. Significance was determined by One-way ANOVA followed by multiple comparisons using Tukey's multiple comparisons post-hoc test. | | | |

Frequency of IA-IE (MHCII)+ antigen presenting cells of Ly-6C+ antigen presenting cells

| | | Tumor | |
|---|---|---|---|
| Group | | Injected, right flank | Uninjected, left flank |
| **4** | Mean (%) | 69.63 | 35.05 |
| | SEM (%) | 4.06 | 6.15 |
| | N | 3 | 2 |
| **Ac-11b** | Mean (%) | 70.5 | 24.8 |
| | SEM (%) | 10.91 | 1.4 |
| | N | 3 | 2 |
| **13h** | Mean (%) | 94.77 | 95.6 |
| | SEM (%) | 4.48 | 3.01 |
| | N | 3 | 3 |
| **Ac-11b** + human IL-2 | Mean (%) | 66.3† | 45.25 |
| | SEM (%) | 1.47 | 29.45 |
| | N | 3 | 2 |

(continued)

| | | Tumor | |
|---|---|---|---|
| Group | | Injected, right flank | Uninjected, left flank |
| **13h+** human IL-2 | Mean (%) | 95.75 | 77.4 |
| | SEM (%) | 2.75 | 16.69 |
| | N | 2 | 3 |
| SEM = standard error of the mean, N = sample size; Injected tumors: †p = 0.049 vs **13h.** Significance was determined by One-way ANOVA followed by multiple comparisons using Tukey's multiple comparisons post-hoc test. | | | |

Frequency of $CD8^+$ T cells of $CD3^+$ T cells

| | | Tumor | |
|---|---|---|---|
| Group | | Injected, right flank | Uninjected, left flank |
| **4** | Mean (%) | 29.23 | 29 |
| | SEM (%) | 1.32 | 0.2 |
| | N | 3 | 2 |
| **Ac-11b** | Mean (%) | 29 | 28.55 |
| | SEM (%) | 1.10 | 2.05 |
| | N | 3 | 2 |
| **13h** | Mean (%) | 23.93 | 26.33 |
| | SEM (%) | 1.43 | 2.04 |
| | N | 3 | 3 |
| | Mean (%) | 38.83†, ‡, †† | 35 |
| | SEM (%) | 2.21 | 1.2 |
| **Ac-11b** + human IL-2 | N | 3 | 2 |
| **13h+** human IL-2 | Mean (%) | 41.45†, ‡, †† | 48.53†, ‡, †† |
| | SEM (%) | 2.75 | 3.93 |
| | N | 2 | 3 |
| SEM = standard error of the mean, N = sample size; Injected tumors: †p<0.02 vs **Ac-11b**, ‡p<0.02 vs **4**, ††p<0.001 vs **13h**; Uninjected tumors: †p = 0.0085 vs **Ac-11b**, ‡p = 0.0096 vs **4,** ††p = 0.0025 vs **13h.** Significance was determined by One-way ANOVA followed by multiple comparisons using Tukey's multiple comparisons post-hoc test. | | | |

Frequency of Ly-$6C^+$ T cells of $CD8^+$ T cells

| | | Tumor | |
|---|---|---|---|
| Group | | Injected, right flank | Uninjected, left flank |
| **4** | Mean (%) | 53.7 | 48 |
| | SEM (%) | 2.06 | 3.3 |
| | N | 3 | 2 |

(continued)

| | | Tumor | |
|---|---|---|---|
| Group | | Injected, right flank | Uninjected, left flank |
| **Ac-11b** | Mean (%) | 45.77 | 35.25 |
| | SEM (%) | 3.47 | 0.75 |
| | N | 3 | 2 |
| **13h** | Mean (%) | 49.1 | 50.4 |
| | SEM (%) | 6.03 | 5.56 |
| | N | 3 | 3 |
| **Ac-11b** + human IL-2 | Mean (%) | 56.17 | 54.8 |
| | SEM (%) | 2.03 | 2.8 |
| | N | 3 | 2 |
| **13h+** human IL-2 | Mean (%) | 68.35† | 68.17†, ‡ †† |
| | SEM (%) | 3.75 | 1.57 |
| | N | 2 | 3 |
| SEM = standard error of the mean, N = sample size; Injected tumors: †p = 0.024 vs **Ac-11b;** Uninjected tumors: †p = 0.0029 vs **Ac-11b,** ‡p = 0.039 vs **4,** ††p = 0.042 vs **13h.** Significance was determined by One-way ANOVA followed by multiple comparisons using Tukey's multiple comparisons post-hoc test. | | | |

Frequency of $CD4^+$ T cells of $CD3^+$ T cells

| | | Tumor | |
|---|---|---|---|
| Group | | Injected, right flank | Uninjected, left flank |
| **4** | Mean (%) | 69.1 | 69.25 |
| | SEM (%) | 1.31 | 0.05 |
| | N | 3 | 2 |
| **Ac-11b** | Mean (%) | 69.57 | 69.8 |
| | SEM (%) | 1.17 | 2.3 |
| | N | 3 | 2 |
| **13h** | Mean (%) | 73.23 | 70.27 |
| | SEM (%) | 1.09 | 1.45 |
| | N | 3 | 3 |
| **Ac-11b** + human IL-2 | Mean (%) | 58.9†, ‡, †† | 62.45 |
| | SEM (%) | 2.16 | 1.15 |
| | N | 3 | 2 |

(continued)

| | | Tumor | |
|---|---|---|---|
| Group | | Injected, right flank | Uninjected, left flank |
| **13h+** human IL-2 | Mean (%) | 54.5†, ‡, †† | 48.5†, ‡, ††, ‡‡ |
| | SEM (%) | 3 | 3.27 |
| | N | 2 | 3 |
| SEM = standard error of the mean, N = sample size; Injected tumors: †p<0.009 vs **Ac-11b,** ‡p<0.02 vs **4,** ††p<0.002 vs **13h;** Uninjected tumors: †p = 0.0021 vs **Ac-11b,** ‡p = 0.0025 vs **4,** ††p = 0.0009 vs **13h,** ‡‡p = 0.022 vs **Ac-11b** + human IL-2. Significance was determined by One-way ANOVA followed by multiple comparisons using Tukey's multiple comparisons post-hoc test. | | | |

**Example 16: Flow cytometric profiling of peripheral blood**

The study was conducted in female BALB/C mice with an age of 9-11 weeks at the day of tumor inoculation. Mice were implanted with 5 x $10^5$ CT26 tumor cells into the right rear flank. When tumors to be injected were grown to a mean tumor volume of ~80 $mm^3$, mice were randomized into treatment cohorts (day 0) and treated with either one intravenous dose on Day 0 and one intravenous dose on Day 6 of 200 μL of Buffer Control, one intravenous dose on Day 0 and one intravenous dose on Day 6 of 200 μL of 60μg of **16,** a single 50 μL intratumoral injection of **12c** on Day 0, or the combination of one intravenous dose on Day 0 and one intravenous dose on Day 6 of 200 μL of 60μg of **16** and a single 50 μL intratumoral injection of **12c** on Day 0. Hydrogels were administered as suspensions in PTP buffer buffer. Mice were bled 4 days after randomization for in vitro stimulation and flow cytometry (FACS). Blood was stimulated with Leukocyte Activation Cocktail, with BD GolgiPlug™ (BD Biosciences) for 5 hours in a 37°C humidified CO2 incubator then processed for FACS. Cells were washed with FACS buffer, supernatants were discarded and cells were resuspended in FACS buffer with 1 μg/ml Fc-Block and incubated at 4 °C for 10 minutes in the dark. Surface marker antibody mixtures in FACS buffer were added to each sample and samples were incubated in the dark at 4°C for 30 minutes. Red blood cell lysis buffer (Biogems) was added and cells were further incubated at 4°C for 10 minutes. Cells were washed twice with FACS buffer then fixed and permeabilized for 30 minutes at room temperature with Fix/Perm buffer (eBioscience). Cells were washed twice in Permeabilization Buffer and stained with intracellular antibodies in Permeabilization buffer for 60 minutes at room temperature. Cells were washed twice in FACS buffer and acquired in the presence of 123count Ebeads (eBioscience).

Summary of antibodies used for FACS profiling

| Markers | Fluorochrome | Clone | Isotype |
|---|---|---|---|
| CD45 | BV711 | 30-F11 | Rat IgG2b, κ |
| CD3 | BUV395 | 17A2 | Rat IgG2b, κ |
| CD4 | BUV737 | GK1.5 | Rat IgG2b, κ |
| CD8 | FITC | 53-6.7 | Rat IgG2a, k |
| CD25 | BV510 | PC61 | Rat IgG1, λ |
| CD335 | BV605 | 29A1.4 | Rat IgG2a, k |
| CD44 | BV421 | IM7 | Rat IgG2b, κ |
| Ly6C | BV785 | HK1.4 | Rat IgG2c, k |
| CTLA4 | PE | UC10-4B9 | Armenian Hamster IgG |
| FoxP3 | PerCP-Cy 5.5 | FJK-16S | Rat IgG2a, k |
| TNF-α | APC | MP6-XT22 | Rat IgG1, k |
| IFN-g | PE-Cy7 | XMG1.2 | Rat IgG1, k |
| GranzymeB | PE-ef610 | NGZB | Rat IgG2a, k |

(continued)

| Markers | Fluorochrome | Clone | Isotype |
|---|---|---|---|
| Live / Dead | efluo780 | NA | NA |

After collection, FACS data was analyzed using FlowJo Version 10.6.1. Compensation was digitally adjusted using single antibody-stained beads, single antibody-stained cells, and fluorescence minus one (FMO) controls. $CD8^+$ T cells were defined using the following gating strategy: FSC-A/SSC-A Cells/FSC-H/FSC-A Singlets/$LiveDead^-$ /$CD45^+$/$CD8^+$. This gating scheme was used to simultaneously gate CD4+ and CD8+ T cells; additional analyses confirmed that these cells co-expressed CD3 and are T cells.

Results:

Frequency of peripheral blood $CD8^+$ T cells within CD45+ cells:

| Group | Buffer Control | **16** | **12c** | **12c + 16** |
|---|---|---|---|---|
| N | 4 | 4 | 4 | 4 |
| Mean | 4.77 | 8.51 | 3.52 | 13.45 |
| SEM | 1.1 | 1.37 | 0.2691 | 1.664 |
| P-Value vs Control | NA | .051 | .484 | <.001 |
| P-Value vs **16** | .051 | NA | .014 | .014 |
| P-Value vs **12c** | .484 | .014 | NA | <.001 |

By this analysis, the combination of **12c** + **16** showed a significantly higher frequency of blood $CD8^+$ T cells within $CD45^+$ cells (mean: 13.45%) as compared to treatment with buffer control (mean: 4.77%) or treatment with either **16** alone (mean: 8.51%) or **12c** alone (mean: 3.52%). Treatment with **16** induced an approximately 1.78 fold increase in the percentage of $CD8^+$ T cells within total $CD45^+$ cells compared to treatment with Buffer Control. Treatment with **12c** + **16** induced an approximately 2.81 fold increase in the percentage of $CD8^+$ T cells within total $CD45^+$ cells compared to treatment with Buffer Control. Treatment with **12c + 16** induced an approximately 3.82 fold increase in the percentage of $CD8^+$ T cells within total $CD45^+$ cells compared to treatment with **12c** alone.

**Example 17: In vivo PK study of plasma and tumor resiquimod concentration and pharmacodynamic effects on peripheral blood mononuclear cell (PBMC) gene expression**

The study was conducted in female BALB/C mice with an age of 6-8 weeks at the day of tumor inoculation. Mice were implanted with $5 \times 10^5$ CT26 tumor cells into the right flank. When tumors were grown to a mean tumor volume of ~104 $mm^3$, mice were randomized into treatment cohorts (day 0). The day following randomization, animals received either a single intratumoral injection of 10 $\mu$g of Resiquimod **4** (dissolved in 50 $\mu$L of 10 mM succinate, 90.0 mg/mL trehalose dihydrate, pH 5.0) or hydrogel **13i** as a single intratumoral dose in an injection volume of 50 $\mu$L. Hydrogels were administered as suspensions in PTP buffer. At defined time points (0 hours, 6 hours, 22 hours and 72 hours post-treatment initiation), 5 mice per group were sacrificed and either plasma was prepared after blood withdrawal, or PBMCs were isolated. Untreated tumor bearing aniamls were sacrificed at the 0 hour timepoint to serve as untreated controls for PBMC gene expression assessment. Tumors were excised, weighed and snap frozen. Plasma samples underwent further processing by solid-phase extraction prior to Resiquimod concentration determination by LC-MS/MS.

The excised tumor samples (weights between 150 and 300 mg) were thawed and homogenized in the presence of 1 mL of saturated KOH in ethanol/water (9/1 v/v) with a FastPrep-24 5G homogenizer (MP Biomedicals, Eschwege) using a slight modification from the manufacturer's protocol (dry ice cooling, 2 times for 40 seconds with a speed of 6 m/s). The resulting cell lysate was further incubated at 37°C for 15 h. After incubation, the dissolved samples were vortexed and diluted 1:10,000 in plasma. These samples were processed as described above and submitted to LC-MS analysis to determine the Resiquimod concentration. The amount of Resiquimod in the tumor sample was back-calculated using the

dilution factor and the determined tumor weights.

For PBMC isolations, approximately 600 μl of whole blood was collected via cardiac puncture. The collected whole blood from each individual mouse was diluted with a 1:1 ratio of prewarmed PBS supplemented with 2% Fetal Bovine Serum (FBS). Then an equal volume of Histopaque-1083 was added to a new sterile 15 mL conical tube, where the diluted whole blood was layered over the Histopaque-1083. The mixture was then centrifuged at 400 g for 30 minutes. The top plasma layer was discarded, and the white translucent interlayer (mononuclear cells) was carefully transferred to a new sterile centrifuge tube. The mononuclear cells were then washed with PBS supplemented with 2% FBS and then were spun down at 250 g for 10 minutes. Afterwards, the cells were lysed with 2 ml of Ammonium-Chloride-Potassium (ACK) lysis buffer (Gibco) for 5 minutes at room temperature to get rid of the red blood cells following manufacturer's instruction. Subsequently, the cells were washed twice with PBS supplemented with 2% FBS and were centrifuged at 250 g for 10 minutes. Then, the supernatant was removed and the PBMC cell pellet was lysed in RLT buffer (Qiagen) and stored at -80°C before being proceeded to RNA extraction and isolation.

Lysates from untreated control samples and 6 hour treated samples were thawed and RNA was isolated using the RNeasy Mini Kit (QIAGEN) following manufacturer's recommendations. Following the first column washing step, DNA was digested directly on the column using the RNase-free DNase Set (TIANGEN) following manufacturer's recommendations. RNA was eluted with RNase-free water. RNA concentrations were measured using a NanoDrop (ThermoFisher) and then adjusted to 200 ng/mL with RNase-free water. RNA quality was assessed using a NanoDrop (ThermoFisher). The concentrations of all the RNA samples are >100ng/μl and the ratio of $A_{260}/A_{280}$ was confirmed to be close to or greater than 2, thus being suitable for downstream qPCR analysis. 2 μg of RNA was reverse transcribed to cDNA using the $RT^2$ First Strand Kit (QIAGEN). Reverse transcription was performed using random primers, 10 mM dNTP mix, and RNase inhibitor (TIANGEN). Reverse transcription was performed with the following thermal steps: $25^0$C for 10 minutes, $37^0$C for 120 minutes, $55^0$ C for 5 minutes. 200 ng of cDNA was used for quantitative PCR using the $RT^2$ SYBR Green ROX qPCR Master mix (2X) kit (QIAGEN) following manufacturer's recommendations. Probe sets used for qPCR reactions are as follows:

| Gene Symbol | Assay Catalog # |
|---|---|
| Il1a | PPM03010F |
| Ccl3 | PPM02949F |
| *Il1b* | PPM03109F |
| *Cxcl2* | PPM02969F |
| *Ccl2* | PPM03151G |
| *Ccl4* | PPM02948F |
| *Il10* | PPM03017C |
| *Ifna4* | PPM03549E |
| *Cxcl1* | PPM03058C |
| *Cxcll0* | PPM02978E |
| *Tnf* | PPM03113G |
| *B2m* | PPM03562A |
| *Ubc* | PPM03450A |
| *Gapdh* | PPM02946E |

Cycle thresholds (CT) were collected using a 384-well platform ABI-7900H real-time qPCR system (Applied Biosystems). *E2M*, *Ubb* and *GAPDH* were used as housekeeping control genes. Data is reported as the average of the 2^-ΔΔCT values for each treatment. 2^-ΔΔCT values were calculated with the following formula:

$$2\^{}\text{-}\Delta\Delta CT = 2\^{}\text{-}(\Delta CT(\text{treated})\text{-}\Delta CT(\text{untreated}))$$

ΔCT(treated) = CT(treated)-CT (average treated housekeeping) where CT(treated) = CT of the gene of interest of a sample triplicate in the treatment group and CT (treated housekeeping) = Total average CT of the *E2M, UBB* and *GAPDH* housekeeping genes of the same sample triplicate in the same treatment group.

ΔCT(untreated) = CT (untreated)-CT (untreated housekeeping) where CT (untreated) = average of the CTs of the

untreated triplicates at the same timepoint as the CT(treated) comparator and CT (untreated housekeeping) = Total average CT of the *B2M, UBB* and *GAPDH* housekeeping genes of the untreated triplicates.

For each gene, 3 technical replicates were analyzed per biological replicate. Undetermined technical replicate CT values were recorded as zero ΔCT values. 4-5 biological replicates were assessed in total.

Results:

Resiquimod concentration in plasma samples

| | | Time (hours) | | | |
|---|---|---|---|---|---|
| | | 0 | 6 | 22 | 72 |
| Group | | Resiquimod (pg/mL) | | | |
| **4** | Mean | 210,000 | 360 | 22 | 14† |
| | SD | 49,000 | 190 | 7 | NC |
| | N | 5 | 5 | 5 | 5 |
| | CV% | 23 | 52 | 33 | NC |
| **13i** | Mean | 280 | 230 | 200 | 180 |
| | SD | 130 | 49 | 46 | 31 |
| | N | 5 | 5 | 5 | 5 |
| | CV% | 47 | 22 | 23 | 17 |
| SD = standard deviation, CV% = coefficient of variation, N = sample size, NC = not calculable, ND = not determined. † 4/5 samples <LLOQ | | | | | |

Resiquimod content in tumors after full release from hydrogel:

| | Time (hours) | |
|---|---|---|
| Group | 0 h | 72 h |
| **13i** | 8.2 ± 2.3 μg (n = 5) | 8.2 ± 1.5 μg (n = 5) |

PBMC gene expression (6 hours post-treatment):

| Gene | | **4** | **13i** | Fold change **(4/13i)** |
|---|---|---|---|---|
| *Il1a* | Mean (2^ΔΔCT) | 0.41 | 0.23 | 1.78 |
| | SEM (2^ΔΔCT) | 0.26 | 0.11 | |
| | N | 4 | 5 | |
| *Ccl3* | Mean (2^ΔΔCT) | 1.43† | 0.87 | 1.64 |
| | SEM (2^ΔΔCT) | 0.09 | 0.18 | |
| | N | 4 | 5 | |

(continued)

| Gene | | 4 | 13i | Fold change (4/13i) |
|---|---|---|---|---|
| *Il1b* | Mean (2^ΔΔCT) | 3.71† | 0.47 | 7.92 |
| | SEM (2^ΔΔCT) | 1.12 | 0.27 | |
| | N | 4 | 5 | |
| *Cxcl2* | Mean (2^ΔΔCT) | 35.37 | 5.50 | 6.43 |
| | SEM (2^ΔΔCT) | 17.41 | 5.21 | |
| | N | 4 | 5 | |
| *Ccl2* | Mean (2^ΔΔCT) | 6.75 | 2.39 | 2.82 |
| | SEM (2^ΔΔCT) | 2.90 | 0.57 | |
| | N | 4 | 5 | |
| *Ccl4* | Mean (2^ΔΔCT) | 1.31 | 0.75 | 1.74 |
| | SEM (2^ΔΔCT) | 0.35 | 0.27 | |
| | N | 4 | 5 | |
| *Il10* | Mean (2^ΔΔCT) | 2.77† | 1.21 | 2.29 |
| | SEM (2^ΔΔCT) | 0.51 | 0.24 | |
| | N | 4 | 5 | |
| *Ifna4* | Mean (2^ΔΔCT) | 2.97 | 0.15 | 20.48 |
| | SEM (2^ΔΔCT) | 2.35 | 0.04 | |
| | N | 4 | 5 | |
| *Cxcl1* | Mean (2^ΔΔCT) | 7.41 | 0.64 | 11.54 |
| | SEM (2^ΔΔCT) | 6.41 | 0.39 | |
| | N | 4 | 5 | |
| *Cxcl10* | Mean (2^ΔΔCT) | 45.38 | 2.12 | 21.41 |
| | SEM (2^ΔΔCT) | 34.14 | 0.86 | |
| | N | 4 | 5 | |
| *Tnf* | Mean (2^ΔΔCT) | 4.64 | 1.04 | 4.45 |
| | SEM (2^ΔΔCT) | 1.91 | 0.47 | |
| | N | 4 | 5 | |
| SEM = standard error of the mean, N = sample size; †two-tailed $p<0.05$ vs **4.** Significance was determined via unpaired non-parametric t-test. | | | | |

**Abbreviations**

| | |
|---|---|
| AcOH | Acetic Acid |
| AUC | Area under curve |
| DCM | Dichloromethane |
| DIPEA | *N*,*N*-Diisopropylethylamine |
| DMAP | 4-(Dimethylamino)pyridine |
| EDC | *N*-(3-Dimethylaminopropyl)-*N'*-ethylcarbodiimide Hydrochloride |
| eq. | Equivalents |
| EtOH | Ethanol |

| | |
|---|---|
| Fmoc | Fluorenylmethyloxycarbonyl |
| HOBt | 1-Hydroxybenzotriazole |
| HOSu | *N*-hydroxysuccinimid |
| HPLC | High-Performance Liquid Chromatography |
| IV | intraveneous |
| LC-MS | Mass Spectrometry Coupled Liquid Chromatography |
| LPLC | Low Pressure Liquid Chromatography |
| MeCN | Acetonitrile |
| MeOH | Methanol |
| NHS | *N*-Hydroxysuccinimide |
| NMP | *N*-Methyl-2-pyrrolidone |
| PBST | Phosphate buffered saline with Tween 20 |
| PE | Polyethylene |
| PEG | Poly(ethylene glycol) |
| PK | Pharmacokinetic/s |
| PMM | poly(methyl methacrylate) |
| PTP | 5 mM phosphate, 90 g/L, trehalose dihydrate, 0.2 % Pluronic F-68, pH 7.4 |
| PyBOP | Benzotriazol-1-yl-oxytripyrrolidinophosphonium Hexafluorophosphate |
| RP-HPLC | Reversed Phase High-Performance Liquid Chromatography |
| RP-LPLC | Reversed Phase Low Pressure Liquid Chromatography |
| r.t. | Room Temperature |
| SC | Subcutaneous |
| TFA | Trifluoroacetic Acid |
| THF | Tetrahydrofurane |
| TMEDA | *N,N,N',N'*-Tetramethylethylenediamine |
| Tween 20 | Polyethylene Glycol Sorbitan Monolaurate |
| UHPLC | Ultra High Performance Liquid Chromatography |
| UPLC | Ultra Performance Liquid Chromatography |
| UPLC-MS | Mass Spectrometry Coupled Ultra Performance Liquid Chromatography |

**Claims**

1. A water-insoluble controlled-release pattern recognition receptor agonist ("PRRA") or its pharmaceutically acceptable salt or a pharmaceutical composition comprising such water-insoluble controlled-release PRRA or its pharmaceutically acceptable salt for use in the treatment of cancer, wherein the water-insoluble controlled-release PRRA is administered by intra-tumoral injection, wherein the water-insoluble controlled-release PRRA comprises a plurality of PRRA moieties covalently and reversibly conjugated to a carrier moiety, from which the PRRA moieties are released in aqueous buffer at pH 7.4 and 37°C with a half-life ranging from one day to three months, wherein the PRRA moieties are resiquimod moieties and wherein the carrier moiety is a hydrogel; and wherein the protein level of at least one cytokine selected from the group consisting of IL-6, CCL2 and IL-10 in plasma has a more than 10-fold lower maximum protein level within 24 hours compared to an equivalent molar dose of the corresponding free resiquimod upon intratumoral injection.

2. The water-insoluble controlled-release PRRA or its pharmaceutically acceptable salt or the pharmaceutical composition comprising such water-insoluble controlled-release PRRA or its pharmaceutically acceptable salt for use of claim 1, wherein the at least one cytokine is IL-6.

3. The water-insoluble controlled-release PRRA or its pharmaceutically acceptable salt or the pharmaceutical composition comprising such water-insoluble controlled-release PRRA or its pharmaceutically acceptable salt for use of claim 1, wherein the at least one cytokine is CCL2.

4. The water-insoluble controlled-release PRRA or its pharmaceutically acceptable salt or the pharmaceutical composition comprising such water-insoluble controlled-release PRRA or its pharmaceutically acceptable salt for use of claim 1, wherein the at least one cytokine is IL-10.

5. The water-insoluble controlled-release PRRA or its pharmaceutically acceptable salt or the pharmaceutical composition comprising such water-insoluble controlled-release PRRA or its pharmaceutically acceptable salt for use of any one of claims 1 to 4, wherein the maximum protein level of the at least one cytokine in plasma is more than 15-fold lower

following intra-tumoral administration of the water-insoluble controlled-release PRRA compared to intra-tumoral administration of an equivalent molar dose of the corresponding free resiquimod.

**6.** The water-insoluble controlled-release PRRA or its pharmaceutically acceptable salt or the pharmaceutical composition comprising such water-insoluble controlled-release PRRA or its pharmaceutically acceptable salt for use of any one of claims 1 to 5, wherein the cancer is selected from the group consisting of lip and oral cavity cancer, oral cancer, liver cancer/hepatocellular cancer, primary liver cancer, lung cancer, lymphoma, malignant mesothelioma, malignant thymoma, skin cancer, intraocular melanoma, metastatic squamous neck cancer with occult primary, childhood multiple endocrine neoplasia syndrome, mycosis fungoides, nasal cavity and paranasal sinus cancer, nasopharyngeal cancer, neuroblastoma, oropharyngeal cancer, ovarian cancer, pancreatic cancer, parathyroid cancer, pheochromocytoma, pituitary tumor, adrenocortical carcinoma, AIDS-related malignancies, anal cancer, bile duct cancer, bladder cancer, brain and nervous system cancer, breast cancer, bronchial adenoma/carcinoid, gastrointestinal carcinoid tumor, carcinoma, colorectal cancer, endometrial cancer, esophageal cancer, extracranial germ cell tumor, extragonadal germ cell tumor, extrahepatic bile duct cancer, gallbladder cancer, gastric (stomach) cancer, gestational trophoblastic tumor, head and neck cancer, hypopharyngeal cancer, islet cell carcinoma (endocrine pancreas), kidney cancer/renal cell cancer, laryngeal cancer, pleuropulmonary blastoma, prostate cancer, transitional cell cancer of the renal pelvis and ureter, retinoblastoma, salivary gland cancer, sarcoma, Sezary syndrome, small intestine cancer, genitourinary cancer, thyroid cancer, Wilms' tumor and cholangiocarcinoma.

**7.** The water-insoluble controlled-release PRRA or its pharmaceutically acceptable salt or the pharmaceutical composition comprising such water-insoluble controlled-release PRRA or its pharmaceutically acceptable salt for use of any one of claims 1 to 6, wherein the PRRA moieties are released in aqueous buffer at pH 7.4 and 37°C with a half-life ranging from two days to two months.

**8.** The water-insoluble controlled-release PRRA or its pharmaceutically acceptable salt or the pharmaceutical composition comprising such water-insoluble controlled-release PRRA or its pharmaceutically acceptable salt for use of any one of claims 1 to 7, wherein the PRRA moieties are released in aqueous buffer at pH 7.4 and 37°C with a half-life ranging from three days to one month.

**9.** The water-insoluble controlled-release PRRA or its pharmaceutically acceptable salt or the pharmaceutical composition comprising such water-insoluble controlled-release PRRA or its pharmaceutically acceptable salt for use of any one of claims 1 to 8, wherein the carrier is a PEG-based or hyaluronic acid-based hydrogel.

**10.** The water-insoluble controlled-release PRRA or its pharmaceutically acceptable salt or the pharmaceutical composition comprising such water-insoluble controlled-release PRRA or its pharmaceutically acceptable salt for use of any one of claims 1 to 9, wherein the carrier is a PEG-based hydrogel.

**11.** The water-insoluble controlled-release PRRA or its pharmaceutically acceptable salt or the pharmaceutical composition comprising such water-insoluble controlled-release PRRA or its pharmaceutically acceptable salt for use of claim 9 or 10, wherein the PEG-based hydrogel comprises a plurality of backbone moieties that are crosslinked via crosslinker moieties.

**12.** The water-insoluble controlled-release PRRA or its pharmaceutically acceptable salt or the pharmaceutical composition comprising such water-insoluble controlled-release PRRA or its pharmaceutically acceptable salt for use of claim 11, wherein the backbone moieties have a molecular weight ranging from 1 kDa to 20 kDa.

**13.** The water-insoluble controlled-release PRRA or its pharmaceutically acceptable salt or the pharmaceutical composition comprising such water-insoluble controlled-release PRRA or its pharmaceutically acceptable salt for use of claim 11, wherein the backbone moieties are of formula (pA)

$$B^*\text{-}(A\text{-}Hyp)x \quad (pA),$$

wherein

B* is a branching core,
A is a PEG-based polymer,
Hyp is a branched moiety,

x is an integer of from 3 to 16,
and wherein each backbone moiety is connected to one or more crosslinker moieties and to one or more spacer moieties $-L^2-$, which crosslinker moieties and moieties $-L^2-$ are connected to Hyp, either directly or through a spacer moiety.

**14.** The water-insoluble controlled-release PRRA or its pharmaceutically acceptable salt or the pharmaceutical composition comprising such water-insoluble controlled-release PRRA or its pharmaceutically acceptable salt for use of claim 13, wherein

B* is a pentaerythritol moiety,
-A- has a molecular weight ranging from 0.3 kDa to 40 kDa,
-Hyp has a molecular weight ranging from 0.3 kDa to 5 kDa.

**15.** The water-insoluble controlled-release PRRA or its pharmaceutically acceptable salt or the pharmaceutical composition comprising such water-insoluble controlled-release PRRA or its pharmaceutically acceptable salt for use of claim 11, wherein the backbone moieties are of formula (pC1)

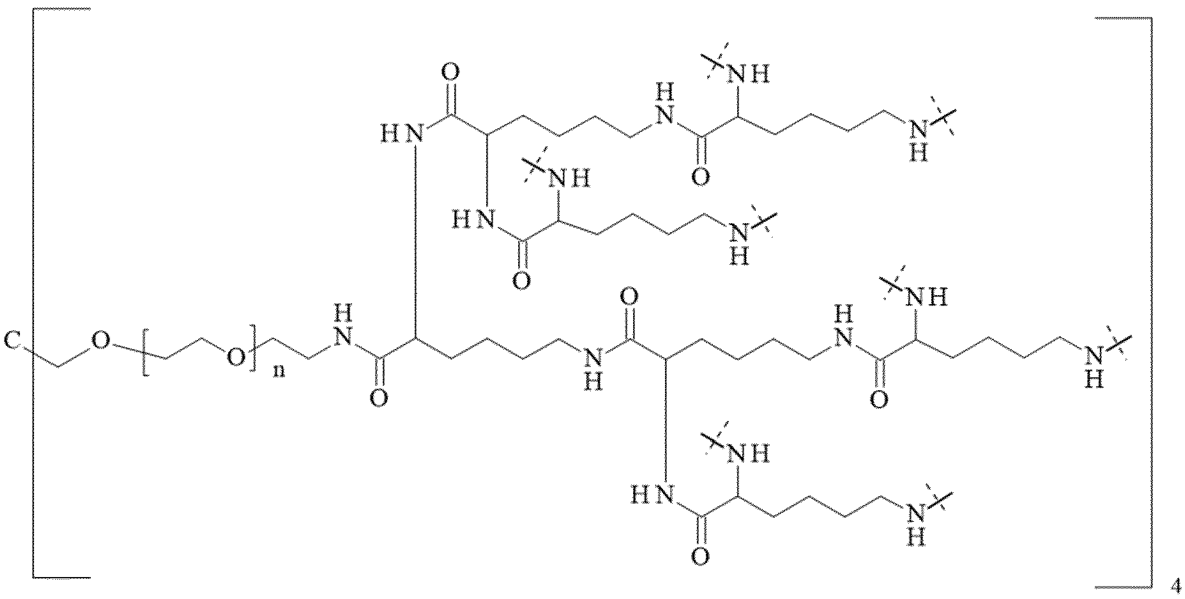

(pC1),

wherein

dashed lines indicate attachment to a spacer moiety or to a crosslinker moiety; and
n ranges from 10 to 40.

**16.** The water-insoluble controlled-release PRRA or its pharmaceutically acceptable salt or the pharmaceutical composition comprising such water-insoluble controlled-release PRRA or its pharmaceutically acceptable salt for use of any one of claims 1 to 15, wherein the treatment of cancer in addition to the administration of the water-insoluble controlled-release PRRA includes the administration of at least one cancer therapeutic.

**17.** The water-insoluble controlled-release PRRA or its pharmaceutically acceptable salt or the pharmaceutical composition comprising such water-insoluble controlled-release PRRA or its pharmaceutically acceptable salt for use of claim 16, wherein the at least one cancer therapeutic is selected from the group consisting of cytotoxic/chemotherapeutic agents, immune checkpoint inhibitors or antagonists, immune checkpoint agonists, multi-specific drugs, antibody-drug conjugates (ADC), radionuclides or targeted radionuclide therapeutics, DNA damage repair inhibitors, tumor metabolism inhibitors, pattern recognition receptor agonists, protein kinase inhibitors, chemokine and chemoattractant receptor agonists, chemokine or chemokine receptor antagonists, cytokine receptor agonists, death receptor agonists, CD47 or SIRP$\alpha$ antagonists, oncolytic drugs, signal converter proteins, epigenetic modifiers, tumor

peptides or tumor vaccines, heat shock protein (HSP) inhibitors, proteolytic enzymes, ubiquitin and proteasome inhibitors, adhesion molecule antagonists, and hormones including hormone peptides and synthetic hormones.

**18.** The water-insoluble controlled-release PRRA or its pharmaceutically acceptable salt or the pharmaceutical composition comprising such water-insoluble controlled-release PRRA or its pharmaceutically acceptable salt for use of claim 16 or 17, wherein the at least one cancer therapeutic is an immune checkpoint inhibitor.

**19.** The water-soluble controlled-release PRRA or its pharmaceutically acceptable salt or the pharmaceutical composition comprising such water-insoluble controlled-release PRRA or its pharmaceutically acceptable salt for use of claim 18, wherein the immune checkpoint inhibitor is an inhibitor of PD-1.

**20.** The water-soluble controlled-release PRRA or its pharmaceutically acceptable salt or the pharmaceutical composition comprising such water-insoluble controlled-release PRRA or its pharmaceutically acceptable salt for use of claim 18, wherein the immune checkpoint inhibitor is an inhibitor of PD-L1.

## Patentansprüche

**1.** Ein wasserunlöslicher Mustererkennungs-Rezeptoragonist ("Pattern Recognition Receptor Agonist", "PRRA") mit kontrollierter Freisetzung oder sein pharmazeutisch unbedenkliches Salz oder eine pharmazeutische Zusammensetzung enthaltend solch wasserunlöslichen PRRA mit kontrollierter Freisetzung oder ihr pharmazeutisch unbedenkliches Salz zur Verwendung in der Behandlung von Krebs, wobei der wasserunlösliche PRRA mit kontrollierter Freisetzung durch intra-tumorale Injektion verabreicht wird, wobei der wasserunlösliche PRRA mit kontrollierter Freisetzung eine Pluralität von kovalent und reversibel an einen Trägerrest konjugierte PRRA-Reste enthält, von dem die PRRA-Reste in wässrigem Puffer bei pH 7.4 und 37°C mit einer Halbwertszeit im Bereich von einem Tag bis zu drei Monaten freigesetzt werden, wobei die PRRA-Reste Resiquimod-Reste sind und wobei der Trägerrest ein Hydrogel ist; und wobei der Proteingehalt von mindestens einem Zytokin ausgewählt aus der Gruppe bestehend aus IL-6, CCL2 und IL-10 in Plasma ein mehr als 10-fach niedrigeren maximalen Proteingehalt innerhalb von 24 Stunden im Vergleich zu einer äquimolaren Dosis an entsprechendem freien Resiquimod nach intra-tumoraler Injektion hat.

**2.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 1, wobei das mindestens eine Zytokin IL-6 ist.

**3.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 1, wobei das mindestens eine Zytokin CCL2 ist.

**4.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 1, wobei das mindestens eine Zytokin IL-10 ist.

**5.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach einem der Ansprüche 1 bis 4, wobei der maximale Proteingehalt des mindestens einen Zytokins in Plasma mehr als 15-fach niedriger nach intra-tumoraler Injektion des wasserunlöslichen PRRA mit kontrollierter Freisetzung ist im Vergleich zu einer intra-tumoralen Injektion einer äquimolaren Dosis an entsprechendem freien Resiquimod.

**6.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach einem der Ansprüche 1 bis 5, wobei der Krebs ausgewählt ist aus der Gruppe bestehend aus Lippen- und Mundhöhlenkrebs, Mundkrebs, Leberkrebs/hepatozelluläres Karzinom, primärer Leberkrebs, Lungenkrebs, Lymphom, malignes Mesotheliom, malignes Thymom, Hautkrebs, intraokulares Melanom, metastasischer Plattenepithelhalskrebs mit unbekanntem Primärtumor, kindliches

multiples endokrines Neoplasie-Syndrom, Mycosis fungoides, Nasenhöhlen- und Nasennebenhöhlenkrebs, Nasopharynxkarzinom, Neuroblastom, Oropharynxkarzinom, Eierstockkrebs, Bauchspeicheldrüsenkrebs, Nebenschilddrüsenkrebs, Phäochromozytom, Hypophysentumor, Nebennierenrindenkarzinom, AIDS-assoziierte Malignome, Analkrebs, Gallengangskrebs, Blasenkrebs, Gehirn- und Nervensystemkrebs, Brustkrebs, bronchiales Adenom/Karzinom, gastrointestinaler Karzinoidtumor, Karzinom, Darmkrebs, Endometriumkarzinom, Speiseröhrenkrebs, extrakranieller Keimzelltumor, extragonadaler Keimzelltumor, extrahepatischer Gallengangskrebs, Gallenblasenkrebs, Magenkrebs, gestationaler trophoblastischer Tumor, Kopf- und Halskrebs, Hypopharynxkarzinom, Inselzellenkarzinom (endokrine Bauchspeicheldrüse), Nierenkrebs/Nierenzellkarzinom, Kehlkopfkrebs, pleuropulmonales Blastom, Prostatakrebs, Übergangszellkarzinom des Nierenbeckens und Harnleiters, Retinoblastom, Speicheldrüsenkrebs, Sarkom, Sézary-Syndrom, Dünndarmkrebs, urogenitaler Krebs, Schilddrüsenkrebs, Wilms-Tumor und Cholangiokarzinom.

**7.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach einem der Ansprüche 1 bis 6, wobei die PRRA-Reste in wässrigem Puffer bei pH 7.4 und 37°C mit einer Halbwertszeit im Bereich von zwei Tagen bis zwei Monaten freigesetzt werden.

**8.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach einem der Ansprüche 1 bis 7, wobei die PRRA-Reste in wässrigem Puffer bei pH 7.4 und 37°C mit einer Halbwertszeit im Bereich von drei Tagen bis einen Monat freigesetzt werden.

**9.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach einem der Ansprüche 1 bis 8, wobei der Träger ein PEG-basiertes oder Hyaluronsäure-basiertes Hydrogel ist.

**10.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach einem der Ansprüche 1 bis 9, wobei der Träger ein PEG-basiertes Hydrogel ist.

**11.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 9 oder 10, wobei das PEGbasierte Hydrogel eine Pluralität von Hauptkettenresten enthält, die mittels Quervernetzungsresten quervernetzt sind.

**12.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 11, wobei der Hauptkettenrest ein Molekulargewicht im Bereich on 1 kDa bis 20 kDa hat.

**13.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 11, wobei der Hauptkettenrest die Formel (pA) hat

$$B^*\text{-(A-Hyp)}x \quad \text{(pA)},$$

wobei

B* ein Verzweigungskern ist,
A ein PEG-basiertes Polymer ist,
Hyp ein verzweigter Rest ist,
x eine ganze Zahl von 3 bis 16 ist,
und wobei jeder Hauptkettenrest verbunden ist mit einem oder mehreren Quervernetzungsresten und mit einem

oder mehreren Abstandsresten $-L^2-$, welche Quervernetzungsreste und Reste $-L^2-$ verbunden sind mit Hyp, entweder direkt oder durch einen Abstandsrest.

**14.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 13, wobei

B* eine Pentaerythritol-Einheit ist,
-A- ein Molekulargewicht im Bereich von 0.3 kDa bis 40 kDa hat,
-Hyp ein Molekulargewicht im Bereich von 0.3 kDa bis 5 kDa hat.

**15.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 11, wobei der Hauptkettenrest die Formel (pC1) hat

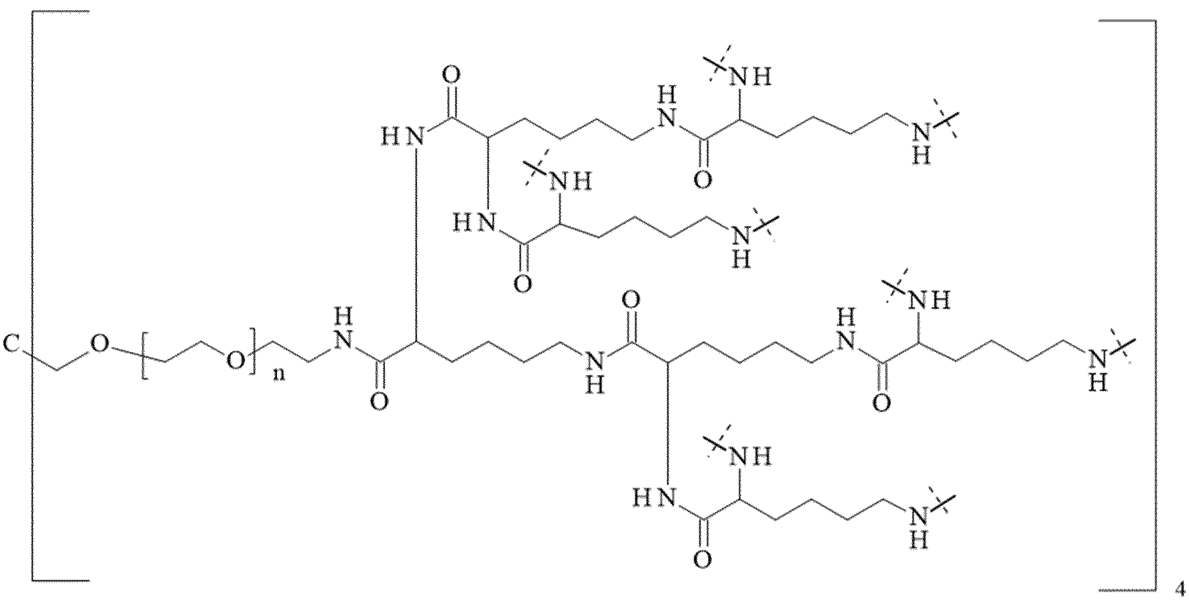

(pC1),

wobei

gestrichelten Linien Anknüpfung an einen Abstandsrest oder einen Quervernetzungsrest anzeigen; und
n sich im Bereich von 10 bis 40 erstreckt.

**16.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach einem der Ansprüche 1 bis 15, wobei die Behandlung des Krebses zusätzlich zu der Verabreichung des wasserunlöslichen PRRA mit kontrollierter Freisetzung die Verabreichung zumindest eines Krebstherapeutikums beinhaltet.

**17.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 16, wobei das zumindest eine Krebstherapeutikum ausgewählt ist aus der Gruppe bestehend aus zytotoxischen/chemotherapeutischen Wirkstoffen, Immun-Checkpoint-Inhibitoren oder -Antagonisten, Immun-Checkpoint-Agonisten, multi-spezifischen Medikamenten, Antikörper-Wirkstoff-Konjugaten (ADC), Radionukliden oder zielgerichtete Radionuklid-Therapeutika, DNA-Schadensreparatur-Inhibitoren, Tumormetabolismus-Inhibitoren, Mustererkennungsrezeptor-Agonisten, Proteinkinase-Inhibitoren, Chemokin- und Chemoattraktant-Rezeptor-Agonisten, Chemokin- oder Chemokinrezeptor-Antagonisten, Zytokinrezeptor-Agonisten, Todesrezeptor-Agonisten, CD47- oder SIRPα-Antagonisten, onkolytischen Medikamenten, Signalumwandlungsproteinen, epigenetische Modifikatoren, Tumorpeptiden oder Tumorimpfstoffen, Hitzeschockprotein (HSP)-Inhibitoren, proteolytischen Enzymen, Ubiquitin- und Proteasom-Inhibitoren, Adhäsionsmolekül-Antagonisten und Hormonen einschließlich Hormonpeptiden und synthetischen Hormonen.

**18.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 16 oder 17, wobei das zumindest eine Krebstherapeutikum ein Immun-Checkpoint-Inhibitor ist.

**19.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 18, wobei der Immun-Checkpoint-Inhibitor ein Inhibitor von PD-1 ist.

**20.** Der wasserunlösliche PRRA mit kontrollierter Freisetzung oder sein pharmazeutisch unbedenkliches Salz oder die pharmazeutische Zusammensetzung enthaltend solch wasserunlöslichen PRRA mit kontrollierter Freisetzung oder ihr pharmazeutisch unbedenkliches Salz zur Verwendung nach Anspruch 18, wobei der Immun-Checkpoint-Inhibitor ein Inhibitor von PD-L1 ist.

## Revendications

**1.** Agoniste des récepteurs de reconnaissance de motifs (« PRRA ») à libération contrôlée insoluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé dans le traitement du cancer, le PRRA à libération contrôlée insoluble dans l’eau étant administré par injection intratumorale, le PRRA à libération contrôlée insoluble dans l’eau comprenant une pluralité de fragments PRRA conjugués de façon covalente et réversible à un fragment de support, à partir duquel les fragments PRRA sont libérés dans un tampon aqueux à pH 7,4 et 37 °C avec une demi-vie allant d’un jour à trois mois, les fragments PRRA étant des fragments résiquimod et le fragment de support étant un hydrogel ; et le taux de protéine d’au moins une cytokine choisie dans le groupe constitué par IL-6, CCL2 et IL-10 dans le plasma présente un taux de protéines maximal plus de 10 fois inférieur dans les 24 heures par rapport à une dose molaire équivalente du résiquimod libre correspondant après injection intratumorale.

**2.** PRRA à libération contrôlée insoluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon la revendication 1, la ou les cytokines étant IL-6.

**3.** PRRA à libération contrôlée insoluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon la revendication 1, la ou les cytokines étant CCL2.

**4.** PRRA à libération contrôlée insoluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon la revendication 1, la ou les cytokines étant IL-10.

**5.** PRRA à libération contrôlée insoluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon l’une quelconque des revendications 1 à 4, le taux de protéine maximal de la ou des cytokines dans le plasma étant plus de 15 fois inférieur après administration intratumorale du PRRA à libération contrôlée insoluble dans l’eau par rapport à l’administration intratumorale d’une dose molaire équivalente du résiquimod libre correspondant.

**6.** PRRA à libération contrôlée insoluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon l’une quelconque des revendications 1 à 5, dans laquelle le cancer est choisi dans le groupe constitué par un cancer des lèvres et de la cavité buccale, un cancer buccal, un cancer du foie/cancer hépatocellulaire, un cancer primitif du foie, un cancer du poumon, un lymphome, un mésothéliome malin, un thymome

malin, un cancer de la peau, un mélanome intraoculaire, cancer épidermoïde métastatique du cou avec syndrome de néoplasie endocrinienne multiple primitif occulte de l'enfant, le mycosis fongoïde, un cancer des fosses nasales et des sinus paranasaux, un cancer du nasopharynx, un neuroblastome, un cancer de l'oropharynx, un cancer des ovaires, un cancer du pancréas, un cancer de la parathyroïde, un phéochromocytome, une tumeur hypophysaire, un carcinome corticosurrénalien, des tumeurs malignes liées au SIDA, un cancer anal, un cancer des voies biliaires, un cancer de la vessie, un cancer du cerveau et du système nerveux, un cancer du sein, un adénome/tumeur carcinoïde bronchique, une tumeur carcinoïde gastro-intestinale, un carcinome, un cancer colorectal, un cancer de l'endomètre, un cancer de l'œsophage, une tumeur germinale extracrânienne, une tumeur germinale extragonadique, un cancer des voies biliaires extrahépatiques, un cancer de la vésicule biliaire, un cancer gastrique (de l'estomac), une tumeur trophoblastique gestationnelle, un cancer de la tête et du cou, un cancer de l'hypopharynx, carcinome des cellules des îlots (du pancréas endocrinien), un cancer du rein/cancer des cellules rénales, un cancer du larynx, un blastome pleuropulmonaire, un cancer de la prostate, un cancer à cellules transitionnelles du bassinet du rein et de l'uretère, un rétinoblastome, un cancer des glandes salivaires, un sarcome, le syndrome de Sézary, un cancer de l'intestin grêle, un cancer génito-urinaire, un cancer de la thyroïde, une tumeur de Wilms et un cholangiocarcinome.

**7.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon l'une quelconque des revendications 1 à 6, les fragments PRRA étant libérés dans un tampon aqueux à pH 7,4 et 37 °C avec une demi-vie allant de deux jours à deux mois.

**8.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon l'une quelconque des revendications 1 à 7, les fragments PRRA étant libérés dans un tampon aqueux à pH 7,4 et 37 °C avec une demi-vie allant de trois jours à un mois.

**9.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon l'une quelconque des revendications 1 à 8, le support étant un hydrogel à base de PEG ou à base d'acide hyaluronique.

**10.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon l'une quelconque des revendications 1 à 9, le support étant un hydrogel à base de PEG.

**11.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon la revendication 9 ou 10, l'hydrogel à base de PEG comprenant une pluralité de fragments de squelette qui sont réticulés par l'intermédiaire de fragments de réticulation.

**12.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon la revendication 11, les fragments de squelette ayant un poids moléculaire allant de 1 kDa à 20 kDa.

**13.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon la revendication 11, les fragments de squelette étant de formule (pA)

B*-(A-Hyp)x (pA), dans laquelle
B* est un noyau à ramification,
A est un polymère à base de PEG,
Hyp est un fragment ramifié,
x est un entier de 3 à 16,
et chaque fragment de squelette étant relié à un ou plusieurs fragments de réticulation et à un ou plusieurs fragments d'espacement $-L^2-$, lesquels fragments de réticulation et fragments $-L^2-$ étant reliés à Hyp, directement ou par l'intermédiaire d'une fragment d'espacement.

**14.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon la revendication 13, dans lequel

B* est un fragment pentaérythritol,
-A- a un poids moléculaire allant de 0,3 kDa à 40 kDa,
-Hyp a un poids moléculaire allant de 0,3 kDa à 5 kDa.

**15.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon la revendication 11, les fragments de squelette étant de formule (pC1)

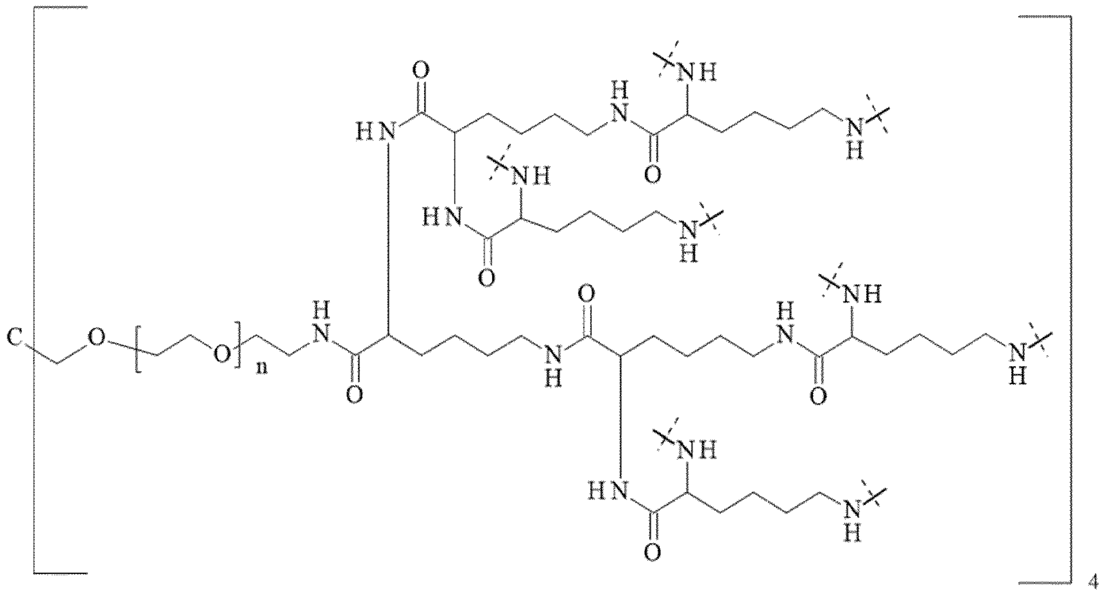

(pC1),

dans laquelle

les lignes pointillées indiquent la liaison à un fragment d'espacement ou à un fragment de réticulation ; et
n est dans la plage de 10 à 40.

**16.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon l'une quelconque des revendications 1 à 15, le traitement du cancer en plus de l'administration du PRRA à libération contrôlée insoluble dans l'eau comprenant l'administration d'au moins un agent thérapeutique contre le cancer.

**17.** PRRA à libération contrôlée insoluble dans l'eau ou son sel pharmaceutiquement acceptable ou composition pharmaceutique comprenant un tel PRRA à libération contrôlée insoluble dans l'eau ou son sel pharmaceutiquement acceptable destiné à être utilisé selon la revendication 16, le ou les agents thérapeutiques contre le cancer étant choisis dans le groupe constitué par des agents cytotoxiques/chimiothérapiques, des inhibiteurs ou antagonistes de point de contrôle immunitaire, des agonistes de point de contrôle immunitaire, des médicaments multispécifiques, des conjugués anticorps-médicament (ADC), des radionucléides ou des agents thérapeutiques à base de radionucléides ciblés, des inhibiteurs de réparation des dommages à l'ADN, des inhibiteurs du métabolisme tumoral, des agonistes des récepteurs de reconnaissance de motifs, des inhibiteurs de protéine kinase, des agonistes des récepteurs de chimiokines et de facteurs chimiotactiques, des antagonistes des chimiokines ou des récepteurs de chimiokines, des agonistes des récepteurs de cytokines, des agonistes des récepteurs de mort, des antagonistes de CD47 ou de SIRPα, des médicaments oncolytiques, des protéines de conversion de signal, des modificateurs épigénétiques, des peptides tumoraux ou des vaccins tumoraux, des inhibiteurs de protéine de choc thermique (HSP), des enzymes protéolytiques, des inhibiteurs de l'ubiquitine et du protéasome, des antagonistes des molécules

d’adhésion et des hormones comprenant des peptides hormonaux et des hormones synthétiques.

**18.** PRRA à libération contrôlée insoluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon la revendication 16 ou 17, le ou les agents thérapeutiques contre le cancer étant des inhibiteurs de point de contrôle immunitaire.

**19.** PRRA à libération contrôlée soluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon la revendication 18, l’inhibiteur de point de contrôle immunitaire étant un inhibiteur de PD-1.

**20.** PRRA à libération contrôlée soluble dans l’eau ou son sel pharmaceutiquement acceptable ou composition pharmaceutique comprenant un tel PRRA à libération contrôlée insoluble dans l’eau ou son sel pharmaceutiquement acceptable destiné à être utilisé selon la revendication 18, l’inhibiteur de point de contrôle immunitaire étant un inhibiteur de PD-L1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160082123 A1
- WO 2011012715 A1
- WO 2016079332 A1
- WO 2018045058 A1
- WO 2010138193 A2
- US 20150010634 A1
- WO 2019185705 A1
- WO 2005099768 A2
- WO 2006136586 A2
- WO 2011089216 A1
- WO 2013024053 A1
- WO 2011012722 A1
- WO 2011089214 A1
- WO 2011089215 A1
- WO 2013024052 A1
- WO 2013160340 A1
- WO 2009095479 A2
- WO 2016020373 A1
- EP 1536334 B1
- WO 2009009712 A1
- WO 2008034122 A1
- WO 2009143412 A2
- WO 2011082368 A2
- US 8618124 B2
- US 8946405 B2
- US 8754190 B2
- WO 2013036857 A1
- US 7585837 B2
- WO 2002089789 A1
- WO 2006003014 A
- WO 2011012715 A
- WO 2014056926 A
- WO 2018175788 A
- WO 2013053856 A
- WO 2016079114 A


### Non-patent literature cited in the description


- **IWASAKI** ; **MEDZHITOV**. *Nat Immunol.*, October 2004, vol. 5 (10), 987-995
- **ENGEL et al.** *Expert Rev Clin Pharmacol.*, March 2011, vol. 4 (2), 275-289
- **PARK et al.** *Sci. Transl. Med.*, 2018, vol. 10, eaar1926
- **GUDE, M.** ; **J. RYF et al.** *Letters in Peptide Science*, 2002, vol. 9 (4), 203-206
- *CHEMICAL ABSTRACTS*, 883993-35-9